# EUROPEAN PATENT APPLICATION

(11) **EP 4 321 503 A1**
(43) Date of publication of application: **14.02.2024**
(21) Application number: 22784091.5
(22) Date of filing: 07.04.2022
(51) Int. Cl.: C07C 219/06, C07C 217/08, C07C 229/16, C07C 229/26, C07C 235/10, C07C 323/25, A61K 9/127, A61K 47/18, A61K 47/20, A61K 45/00, A61K 31/7088, A61K 31/7105, A61K 31/713, A61P 43/00

(54) **PEGYLATED LIPID, LIPOSOME MODIFIED THEREBY, PHARMACEUTICAL COMPOSITION CONTAINING LIPOSOME, PREPARATION THEREOF, AND USE THEREOF**

(30) Priority: 08.04.2021 CN 202110379685; 09.07.2021 CN 202110780188; 23.07.2021 CN 202110839008
(71) Applicant: Xiamen Sinopeg Biotech Co., Ltd., Xiamen, Fujian 361100 (CN)
(72) Inventor: WENG, Wengui, Xiamen, Fujian 361100 (CN); LIU, Chao, Xiamen, Fujian 361100 (CN); WANG, Ailan, Xiamen, Fujian 361100 (CN); LIN, Congming, Xiamen, Fujian 361100 (CN); WANG, Linlin, Xiamen, Fujian 361100 (CN)
(74) Representative: Algemeen Octrooi- en Merkenbureau B.V.
(86) International application number: PCT/CN2022/085526
(87) International publication number: WO 2022/214025

(57) **Abstract**

The present invention provides a novel PEGylated lipid and preparation methods thereof, a cationic liposome containing the PEGylated lipid, a pharmaceutical composition containing the liposome, a formulation and application thereof, belonging to the field of drug delivery. The PEGylated lipid of the present invention can be used for modifying a liposome, and the PEGylated lipid can also be further modified and coupled with a targeting group and then used for modifying a liposome to obtain a liposome having the targeting group. Due to the presence of a long-chain PEG and the targeting group on the PEGylated lipid, the modified liposome can not only effectively avoid being removed by the reticuloendothelial system in a human body, but also realize a targeting function. Therefore, when the PEGylated lipid-modified liposome delivers an active drug to cells or a patient, especially when delivering a nucleic acid drug and anti-tumor drug, the liposome can not only realize long circulation in vivo and improve the transport efficiency of drug, but also has a targeting function, so that the therapeutic effect of a drug is improved under a synergistic effect.

## Description

### TECHNICAL FIELD

The present invention relates to the field of drug delivery, specifically to a PEGylated lipid as pharmaceutical carrier, in particular to a PEGylated lipid with a nitrogen-atom branching center and the preparation method thereof, cationic liposomes containing the lipid, pharmaceutical compositions containing the liposome, formulations of the compositions and application thereof.

### BACKGROUND OF THE INVENTION

Liposome is a microvesicle formed by encapsulating drugs in the lipoid bilayer. Liposomal nanoparticles contain liposomes and drugs, especially nucleic acid drugs, and their structures are similar to biofilms, which makes liposomal nanoparticle a biocompatible and non-toxic nanomaterial. They can encapsulate water-soluble and lipo-soluble drugs, with advantages such as reduced drug dose, sustained release, targeted drug release, and protection of encapsulated nucleic acids from degradation and clearance in serum. In addition, nanoliposome is also an excellent antigen carrier, not only encapsulating a series of antigens with different physicochemical properties and immune adjuvants, protecting protein polypeptide antigens from degradation, but also promoting the phagocytosis and presentation of antigen-presenting cells to antigen and enhancing the specific immune response of the body as well. Therefore, liposomal nanoparticles are widely used in the field of drug delivery.

Cationic lipids bearing positive charges on their surface form cationic liposome-nucleic acid drug complexes by electrostatic interaction with the negatively charged nucleic acids, while the whole cationic liposome-nucleic acid drug complexes bearing positive charges on their surface are prone to generate non-specific adsorption with serum proteins in plasma to form large-size aggregates, and the large-size aggregates are easily cleared by the reticuloendothelial system (RES) to cause short blood circulation time, poor stability and low transportation efficiency. For these reasons, the surfaces of cationic liposomes need to be modified to prepare long circulating cationic liposomes. Currently, the commonly used long circulating cationic liposomes are modified by PEGylated lipids, such as PEG-DMG. PEG interacts with the water molecules in solvent via hydrogen bonding to form a hydrated layer on the surface of the modified cationic liposome, and the hydrated layers conceal the positive charges on the surfaces of cationic liposomes to inhibit the protein adsorption and reduce the recognition by the phagocytic system. Therefore, PEGylated lipids are important for preparing long circulating cationic liposomes and further improving the nucleic acid transport efficiency.

Although PEGylated lipids, such as PEG-DMG, have made great progress in drug delivery, there is still a need for novel PEGylated lipids that are suitable for regular therapeutic uses in this field.

### SUMMARY OF THE INVENTION

The present invention provides novel PEGylated lipids and preparation methods thereof, cationic liposomes containing the PEGylated lipids, pharmaceutical compositions containing the liposome and formulations thereof, especially the nucleic acid pharmaceutical compositions containing the liposome and formulations thereof. The formulations of cationic liposome-nucleic acid pharmaceutical compositions can deliver nucleic acid drugs into cells, and improve the transportation efficiency of nucleic acid drugs, thereby improving the treatment effect of nucleic acid drugs.

The above-mentioned aim can be achieved as follows.

In one embodiment, provided herein is a PEGylated lipid:
The PEGylated lipid having the structure represented by the following general formula (2): 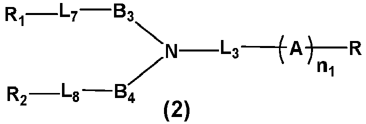
or pharmaceutically acceptable salts, tautomers, or stereoisomers thereof,
wherein, L₇ and L₈ are each independently a linking bond, -OC(=O)- or -C(=O)O-;
L₃ is a linking bond, -L₄-, -L₄-Z-, -Z-L₄-, -Z-L₄-Z-, -L₄-Z-L₅-, -Z-L₄-Z-L₅-, -L₄-Z-L₅-Z-, -Z-L₄-Z-L₅-Z- or -L₄-Z-L₄-Z-L₅-Z-; said L₄ and L₅ are carbon chain linking groups, each independently represented by -(CRₐR_{b})ₜ-(CRₐR_{b})ₒ-(CRₐR_{b})ₚ-, wherein, t, o and p are each independently an integer from 0 to 12, and t, o and p are not 0 simultaneously; Rₐ and R_{b} are, at each occurrence, each independently a hydrogen atom or a C₁₋₁₂ alkyl group; said Z is, at each occurrence, each independently selected from the group consisting of -C(=O)-, - OC(=O)-, -C(=O)O-, -OC(=O)O-, -O-, -S-, -C(=O)S-, -SC(=O)-, -NR_{c}C(=O)-, -C(=O)NR_{c}-, - NR_{c}C(=O)NR_{c}-, -OC(=O)NR_{c}-, -NR_{c}C(=O)O-, -SC(=O)NR_{c}-, -NR_{c}C(=O)S-, and 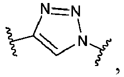 wherein, R_{c} is, at each occurrence, independently a hydrogen atom or a C₁₋₁₂ alkyl group;
B₃ and B₄ are each independently a linking bond or a C₁₋₃₀ alkylene group;
R₁ and R₂ are each independently a C₁₋₃₀ aliphatic hydrocarbon group;
R is a hydrogen atom, -R_{d}, -OR_{d}, -NR_{d}R_{d}, -SR_{d}, -C(=O)R_{d}, -C(=O)OR_{d}, -OC(=O)R_{d}, - OC(=O)OR_{d} or 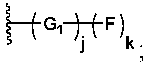 wherein, R_{d} is, at each occurrence, independently a C₁₋₁₂ alkyl group, G₁ is a (k+1)-valent terminal branching group, j is 0 or 1, and F contains the functional group R₀₁; when j is 0, G₁ is absent; when j is 1, G₁ protrudes F with the number of k, and k is an integer from 2 to 8; when L₃ is -C(=O)CH₂-, R is not a hydrogen atom, an alkyl group, an alkoxy group or a hydroxyl group;
A is -(CRₐR_{b})ₛO- or -O(CRₐR_{b})ₛ-, wherein s is 2, 3 or 4;
n₁ is an integer from 20 to 250;
said alkyl group, alkylene group, alkoxy group, and aliphatic hydrocarbon group are each independently substituted or unsubstituted.

Four preparation methods of the aforementioned PEGylated lipids are disclosed herein, specifically as follows:
Method-1: The foregoing PEGylated lipids can be prepared by the following steps:
Step 1: Activating the carboxyl terminus of the acid A-1 or A-1' containing an unprotected carboxyl group with carboxyl activating agents to obtain the ester A-2 or A-2' containing an activated carboxyl terminus, wherein B₃' and B₄' are each independently a linking bond or an alkylene group having one less methylene group than B₃ and B₄; R₁' and R₂' are each independently R₁, R₂ or an aliphatic hydrocarbon group having one less methylene group than R₁ and R₂; R₇ is a carboxyl-activating group; and, when either B₃' or L₇ is not a linking bond, R₁' is R₁; when both B₃' and L₇ are linking bonds; R₁' is an aliphatic hydrocarbon group having one less methylene group than R₁; when either B₄' or L₈ is not a linking bond, R₂' is R₂; when both B₄' and L₈ are linking bonds, R₂' is an aliphatic hydrocarbon group having one less methylene group than R₂;
Step 2: Carrying out the condensation reaction between the ester A-2 or A-2' containing an activated carboxyl terminus and the primary amine derivative A-3 or A-3' containing a nitrogen-source terminal group to obtain the amide intermediate A-4 or A-4';
Step 3: Using reducing agents to reduce the amide intermediate A-4 or A-4' to the secondary amine intermediate A-5 or A-5';
Step 4: Carrying out the coupling reaction between the secondary amine intermediate and the dual end-functionalized PEG derivative which is, the biLPEG molecule A-6, to obtain the PEGylated lipid derivative A-7 or A-7'; wherein, biLPEG can be monodisperse or polydisperse; wherein, the two terminal functional groups of biLPEG can be the same or different; wherein, the R' end of biLPEG contains the functional group R₀₁ or a micro variant form of R₀₁; said micro variant form refers to the group which can be transformed into R₀₁ through any chemical process selected from deprotection, salt complexation and decomplexation, ionization, protonation, deprotonation, and leaving group transformation; wherein, the F₁ contains active functional groups, which can react with the amino group of the secondary amine intermediate A-5 or A-5' to obtain the nitrogen atom as a branching center and the divalent linking group L₃;
   when R' is R, the structure of A-7 or A-7' is represented by the general formula (2);
   when R' is not R, A-8 or A-8' represented by the general formula (2) is obtained via terminal micro-modification of A-7 or A-7'; said terminal micro-modification is selected from chemical reactions including deprotection, salt complexation and decomplexation, ionization, protonation, deprotonation, and leaving group transformation.

### Step 1:

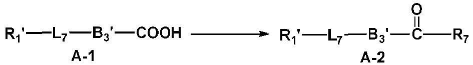

or

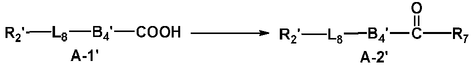

### Step 2:

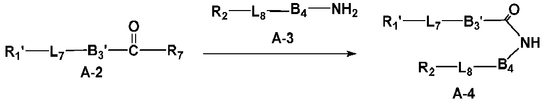

or

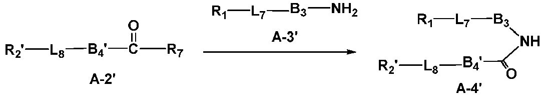

### Step 3:

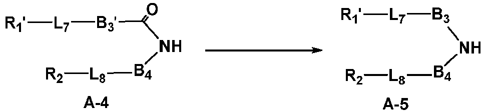

or or

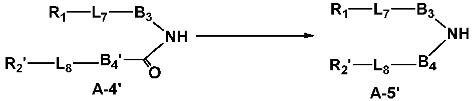

### Step 4:

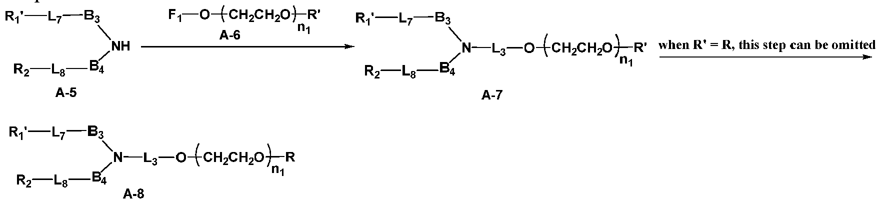

or

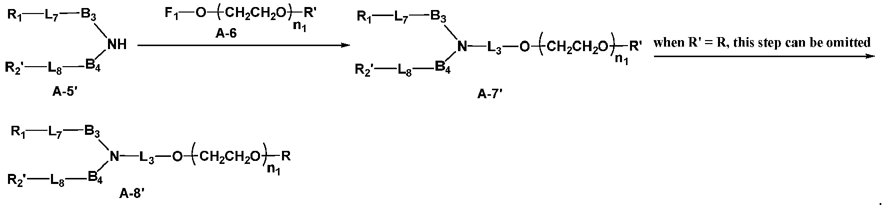

Method-2: The foregoing PEGylated lipids can be prepared by the following steps:
Step 1: Carrying out the coupling reaction between the PEG derivative biLPEG molecule B-1, and the primary amine derivative B-2 or B-2' containing a nitrogen-source terminal group to obtain the secondary amine derivative B-3 or B-3'; wherein, biLPEG can be monodisperse or polydisperse, the two terminal functional groups of biLPEG can be the same or different, and the R' end of biLPEG contains the functional group R₀₁ or a micro variant form of R₀₁; said micro variant form refers to the group which can be transformed into R₀₁ through any chemical process selected from deprotection, salt complexation and decomplexation, ionization, protonation, deprotonation, and leaving group transformation; wherein, the F₁ contains active functional groups, which can react with the amino group of the primary amine B-2 or B-2' to obtain the secondary amine derivative B-3 or B-3' containing the divalent linking group L₃;
Step 2: Carrying out the alkylation reaction between the secondary amine derivative B-3 or B-3' and the compound B-4 or B-4' with the functional group F_{N} to obtain the PEGylated lipid derivative B-5 or B-5'; said F_{N} is a functional group which can react with amino group or secondary amino group, preferably -OMs, -OTs, -CHO, -F, -Cl, or -Br;
   when R' is R, the structure of B-5 or B-5' is represented by the general formula (2);
   when R' is not R, B-6 or B-6' represented by the general formula (2) is obtained via terminal micro-modification of B-5 or B-5'; said terminal micro-modification is selected from chemical reactions including deprotection, salt complexation and decomplexation, ionization, protonation, deprotonation and leaving group transformation.

### Step 1:

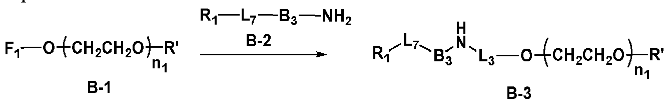

or

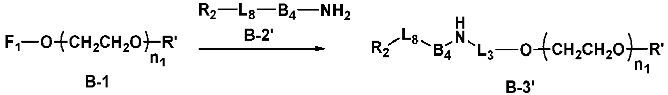

### Step 2:

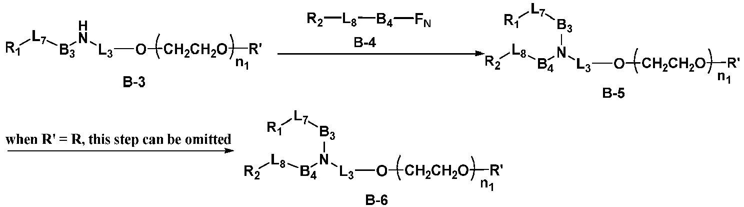

or

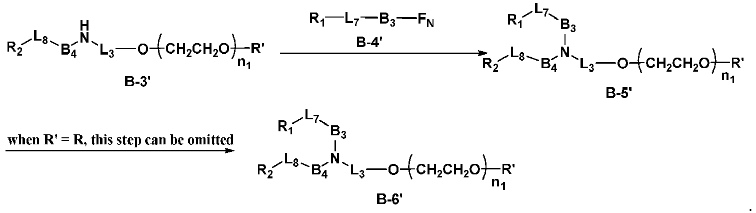

Method-3: The foregoing PEGylated lipids can be prepared by the following steps:
Step 1: Carrying out the reaction between the small molecule C-1 and the small molecule C-2 to obtain the small molecule intermediate C-3 which contains the divalent linking group L₇, the functional group F_{N} at one end, and an aliphatic hydrocarbon group R₁ at the other end; wherein, F₃ and F₄ are each independently a functional group, which can be transformed into the divalent linking group L₇ via reaction; wherein, C-2 contains a heterofunctional group pair F₃ and F_{N}, said F_{N} is the functional group which can react with amino group or secondary amino group, preferably -OMs, -OTs, -CHO, -F, -Cl or -Br;
Step 2: Carrying out the alkylation reaction between two molecules of the small molecule intermediate C-3 and the PEG primary amine derivative C-4 containing a nitrogen-source terminal group to obtain the PEGylated lipid C-5, wherein the R' end contains the functional group R₀₁ or a micro variant form of R₀₁; said micro variant form refers to the group which can be transformed into R₀₁ through any chemical process selected from deprotection, salt complexation and decomplexation, ionization, protonation, deprotonation, and leaving group transformation;
   when R' is R, the structure of C-5 is represented by the general formula (2);
   when R' is not R, C-6 represented by the general formula (2) is obtained via terminal micro-modification of C-5; said terminal micro-modification is selected from chemical reactions including deprotection, salt complexation and decomplexation, ionization, protonation, deprotonation and leaving group transformation; wherein, R₁ is the same as R₂, B₃ is the same as B₄, and L₇ is the same as L₈.

### Step 1:

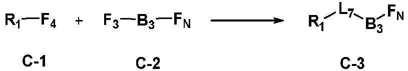

### Step 2:

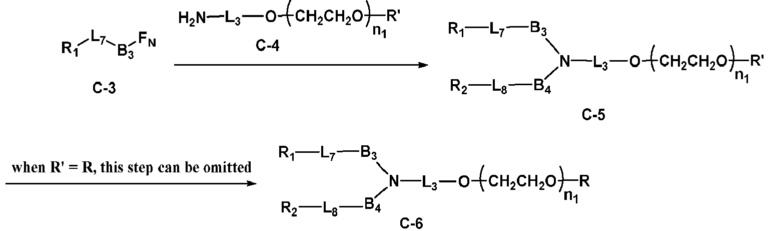

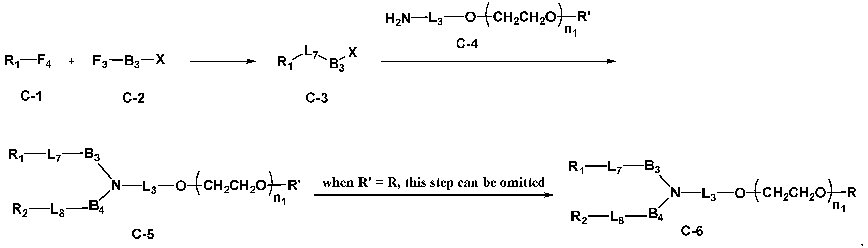

Method-4: The foregoing PEGylated lipids can be prepared by the following steps:
Step 1: Carrying out the reaction between the small molecule D-1 and the small molecule D-2 to obtain the small molecule intermediate D-3 which contains the divalent linking group L₇, a hydroxyl group at one end, and an aliphatic hydrocarbon group R₁ at the other end; wherein, F₃ and F₄ are each independently a functional group, which can be transformed into the divalent linking group L₇ via reaction; wherein, D-2 contains a heterofunctional group pair consisting of F₃ and a hydroxyl group;
Step 2: Carrying out the reaction in which the hydroxyl group of the small molecule intermediate D-3 is oxidized to an aldehyde group to obtain the small molecule intermediate D-4 containing an aldehyde group, wherein B₃' is an alkylene group having one less methylene group than B₃;
Step 3: Carrying out the addition reaction between two molecules of the small molecule intermediate D-4 containing an aldehyde group and the PEG primary amine derivative D-5 containing a nitrogen-source terminal group to obtain the PEGylated lipid D-6, wherein the R' end contains the functional group R₀₁ or a micro variant form of R₀₁; said micro variant form refers to the group which can be transformed into R₀₁ through any chemical process selected from deprotection, salt complexation and decomplexation, ionization, protonation, deprotonation, and leaving group transformation;
   when R' is R, the structure of D-6 is represented by the general formula (2);
   when R' is not R, D-6 represented by the general formula (2) is obtained via terminal micro-modification of D-7; said terminal micro-modification is selected from chemical reactions including deprotection, salt complexation and decomplexation, ionization, protonation, deprotonation and leaving group transformation; wherein, R₁ is the same as R₂, B₃ is the same as B₄, and L₇ is the same as L₈.

### Step 1:

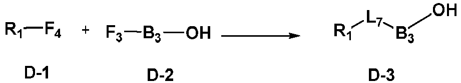

### Step 2:

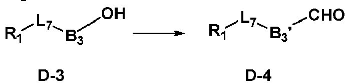

### Step 3:

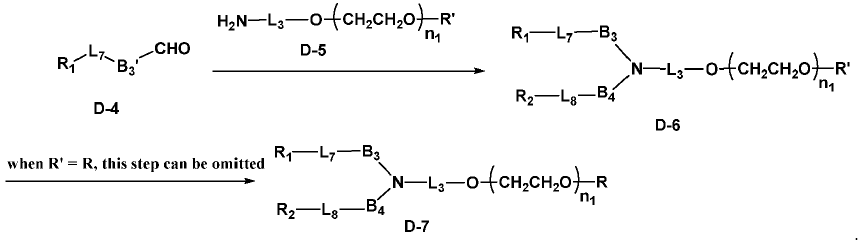

Embodiment as disclosed herein provides a cationic liposome:
A cationic liposome contains the PEGylated lipids represented by the formula (2).

Embodiment as disclosed herein provides a cationic liposome pharmaceutical composition:
A liposome pharmaceutical composition contains cationic liposomes and drugs, and the said cationic liposomes contain the PEGylated lipids represented by the formula (2).

Embodiment as disclosed herein provides a formulation of liposome pharmaceutical composition:
A formulation of liposome pharmaceutical composition contains the foregoing liposome pharmaceutical compositions and the pharmaceutically acceptable diluents or excipients.

### Compared with the prior art, the present invention brings the following beneficial effects:

The nitrogen atom as the branching center of the PEGylated lipid in the present invention is easily protonated under physiological pH to produce partial positive charges, and thus the lipid is capable to bind negatively charged nucleic acids, thereby improving the loading efficiency of nucleic acid drugs.

The surfaces of the cationic liposomes can be modified by the novel PEGylated lipids of the present invention to obtain PEGylated cationic liposomes. The presence of the long-chain PEG overcomes the shortcoming of conventional cationic liposomes which are cleared by the phagocytes because of the serum proteins in plasma, improves the stability of cationic liposomes in serum, prolongs the circulation time in vivo, and further improves the transportation efficiency and treatment effect of drugs.

In the present invention, formulations of cationic liposome pharmaceutical compositions containing PEGylated lipids have stronger gene complexation ability and higher biocompatibility, and contribute to improving the therapeutic effect of drugs.

In the present invention, the terminals of the polyethylene glycol chains of the PEGylated lipids can also contain fluorescent groups or targeting groups, which furtherly improve the targeted therapeutic or diagnostic effect of the modified cationic liposome pharmaceutical compositions.

In the present invention, the polyethylene glycol chains of the PEGylated lipids can be degradable. In certain therapeutic areas, such as tumor therapy, the stable PEG coated on the surface of the liposome can keep the liposome components from leaving the endosome so that it prevents the delivery of the liposome contents into the cytoplasm, while the degradable PEG can be degraded to leave the liposome surface in the unique acidic environment of endosomal vacuoles or tumor tissues so that the therapeutic agent loaded in liposomes can be delivered to the target site efficiently.

### DETAILED DESCRIPTION OF THE INVENTION

### Description of terms

In the present invention, unless otherwise defined, all terms are defined as follows.

In the present invention, when a structure has isomers, it may refer to any form of the isomers unless otherwise specified. For example, when *cis-* and *trans-* isomers are present, it can refer to either a cis-structure or a *trans*-structure; when *E* and *Z* isomers are present, it can refer to either an (*E*)-structure or a (*Z*)-structure; and, when the structure has optical activity, it can be either a laevoisomer or a dextroisomer.

In the present invention, the definition of the numerical interval includes both the numerical interval indicated by a dash (e.g., 0-12) and the numerical interval indicated by a wavy line (e.g., 0~12). In the present invention, an integer interval denoted as an interval can represent the group consisting of all integers within the range of the interval unless otherwise specified, and said range includes two endpoints as well. For example, the integer interval 0-12 represents the group consisting of 0, 1, 2, 3, 4, 5, 6, 7, 8, 9, 10, 11 and 12. The numerical interval in the present invention includes but is not limited to the numerical intervals represented by integers, non-integers, percentages and fraction, and all of the foregoing numerical interval include two endpoints unless otherwise specified.

In the present invention, a numerical value described with "about" or "approximately" generally indicates a numerical range of ±10% which, in some cases, can be amplified to ±15%, not exceeding ±20%, based on the preset numerical value. For example, provided that the molar percentage of steroid lipids in the total lipids in a solvent-containing solution is about 40%, it is generally considered that the molar percentage of steroid lipids is 30%-50%.

In the present invention, unless otherwise specified, the terms "include", "involve", "contain", and similar expressions in the description and claims shall be interpreted as "include but are not limited to", openly and inclusively.

In the present invention, when two or more objects are "preferably each independently selected from" something and there are multiple levels of optimum conditions, the objects are not necessarily selected from the optimum conditions of the same level but allowed to be any of the following cases: one is selected from a wider range of options while the another is selected from a narrower range of options, one is selected from the maximum range of options while another is selected from any allowable options, or all of the objects are selected from the optimum conditions of the same level. For example, "R₁ and R₂ are preferably each independently a linear alkyl group, more preferably a C₁₋₂₅ linear alkyl group, more preferably a C₁₋₁₇ linear alkyl group" means R₁ is a C₁₋₂₅ linear alkyl group and R₂ is a C₁₋₁₇ linear alkyl group, or R₁ is a C₁₋₁₇ linear alkyl group and R₂ is a C₁₋₂₅ linear alkyl group, or R₁ and R₂ are both C₁₋₂₅ linear alkyl groups, or R₁ and R₂ are both C₁₋₁₇ linear alkyl groups.

In the present invention, a divalent linking group, e.g., a hydrocarbylene group, an alkylene group, an arylene group, an amide bond, and the like, can use either one of its two connecting ends to be connected to another group, unless otherwise specified. For example, when an amide bond is used as a divalent linking group between C-CH₂CH₂- and -CH₂-D, both C-CH₂CH₂-C(=O)NH-CH₂-D and C-CH₂CH₂-NHC(=O)-CH₂-D are allowable.

In the present invention, when a terminal group of a linking group in a structural formula is easily confused with a substituent group of said linking group, " 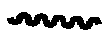 " is used to mark the location where the linking group is connected to other groups. For example, in the structural formulas 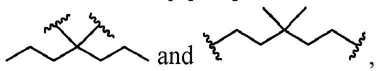 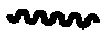 are used to mark the two locations where the divalent linking group is connected to other groups; the two aforementioned structural formulas represent -CH(CH₂CH₂CH₃)₂- and -CH₂CH₂CH(CH₃)₂-CH₂CH₂-, respectively.

In the present invention, the range of the number of carbon atoms in a group is denoted as a subscript of C, representing the number of carbon atoms of the group. For example, C₁₋₁₂ represents a group "having 1 to 12 carbon atoms", and C₁₋₃₀ indicates a group "having 1 to 30 carbon atoms". A "substituted C₁₋₁₂ alkyl group" refers to a compound obtained from one or more hydrogen atoms of a C₁₋₁₂ alkyl group being replaced by substituents. A "C₁₋₁₂ substituted alkyl group" refers to a compound having 1 to 12 carbon atoms which is obtained from one or more hydrogen atoms of an alkyl group being replaced by substituents. As another example, when a group can be selected from C₁₋₁₂ alkylene groups, it can be any alkylene group with the number of carbon atoms in the range indicated by the subscript, that is, the group can be selected from the group consisting of C₁, C₂, C₃, C₄, C₅, C₆, C₇, C₈, C₉, C₁₀, C₁₁ and C₁₂ alkylene groups. In the present invention, subscript marked in interval form represents any integer within the range could be available unless otherwise specified, and the numerical range includes two endpoints.

In the present invention, heteroatoms are not particularly limited, including but not limited to O, S, N, P, Si, F, Cl, Br, I, B, etc.

In the present invention, the heteroatom used for substitution is referred to as a "substituent atom", and the group used for substitution is referred to as a "substituent group".

In the present invention, the term "substituted" indicates that at least one hydrogen atom of any aforementioned group (e.g., aliphatic hydrocarbon groups, hydrocarbon groups, alkyl groups or alkylene groups) is replaced by a bond connected to non-hydrogen atoms, said non-hydrogen atoms including but not limited to a halogen atom (F, Cl, Br, or I), an oxo group (=O), a hydroxyl group (-OH), a hydrocabyloxy group (-OR_{d}, wherein R_{d} is a C₁₋₁₂ alkyl group), a carboxyl group (-COOH), an amine group (-NR_{c}R_{c}, wherein both R_{c} are each independently a hydrogen atom or a C₁₋₁₂ alkyl group), a C₁₋₁₂ alkyl group, and a cycloalkyl group. In some embodiments, said substituent group is a C₁₋₁₂ alkyl group. In another embodiment, said substituent group is a cycloalkyl group. In another embodiment, said substituting group is a halogenated group, e.g., fluorination. In another embodiment, said substituent group is an oxo group. In another embodiment, said substituent group is a hydroxyl group. In another embodiment, said substituent group is an alkoxy group. In another embodiment, said substituent group is a carboxyl group. In another embodiment, said substituent group is an amine group.

In the present invention, the term "atom spacing" refers to the number of main chain atoms spaced along the main chain, taking into account no pendant groups or side chains. It is usually the shortest atomic distance, which can be used to represent the length of the linking group. For example, the atom spacing between A and B in A-CO-NH-B is 2, that in A-*p*-Ph-CH₂-B is 5 (wherein *p*-Ph is *p*-phenylene), and that in A-CH(CH₂CH₂CH₂CH₃)-B is 1. The "main-chain atoms" accounted for the atom spacing can only be non-hydrogen atoms. Wherein, concerning the divalent linking group with a ring structure, its atom spacing refers to the minimum number of atoms calculated along the ring. For example, the atom spacing of *p*-phenylene, namely 1,4-phenylene, is 4, the atom spacing of *m*-phenylene is 3, and the atom spacing of *o*-phenylene is 2. For another example, the atom spacing of -CH₂-, -CH(CH₃)-, - C(CH₃)₂-, -CH(CH₂Ph)₂- and -C(CH₂OX)- are all 1.

In the present invention, a "carbon chain linking group" refers to the linking group whose main chain atoms are all carbon atoms, while the pendant chains are allowed to contain heteroatoms or heteroatom-containing groups that replace the hydrogen atoms connected to the main chain carbon atoms. When a "main chain atom" is a heteroatom, it can also be termed a "main chain heteroatom". For example, A-S-CH₂-B, A-O-CH₂-B and 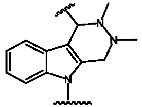 (wherein the atom spacing is 4) are considered to contain main chain heteroatoms. Carbon chain linking groups can be divided into hydrocarbylene groups and carbon chain linking groups containing heteroatoms in their pendant groups; said carbon chain linking group whose pendant groups contain heteroatoms included but are not limited to an oxo group (=O), a thioxo group (=S), an imino group (connected to the main chain carbon through a carbon-nitrogen double bond), an oxa-hydrocarbon group in the form of an ether bond, a thia-hydrocarbon group in the form of a thioether bond, an aza-hydrocarbon group in the form of a tertiary amino group, etc. The main chain of the "carbon chain linking group" is entirely composed of carbon atoms, and the pendant groups of the carbon chain are allowed to contain heteroatoms, that is, the main chain is constructed by connecting methylene groups or substituted methylene groups. Said substituted methylene group can be replaced by one monovalent substituent, two monovalent substituents, or one divalent substituent (e.g., a divalent oxygen atom, or forming a three-membered ring 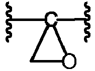 with a divalent methylene group). Said substituted methylene group can be a methylene group with one hydrogen atom being substituted (e.g., -CH(CH₃)-), two hydrogen atoms being substituted respectively (e.g., -(CH₃)C(OCH₃)-), or two hydrogen atoms being substituted at the same time (e.g., a carbonyl group, a thiocarbonyl group, -C(=NH)- and -C(=N⁺H₂)-), or a cyclic pendant group (e.g., 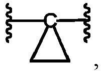 wherein the atom spacing is 1).

In the present invention, secondary amino bonds and hydrazine bonds refer to the linking bond "-NH-" capped with hydrocarbylene groups at both ends, e.g., -CH₂-NH-CH₂-; while -C(=O)-NH- is denoted as amide bond not a secondary amino bond.

In the present invention, a compound, a group, or an atom can be substituted and heterosubstituted at the same time, for example, a hydrogen atom is substituted by a nitrophenyl group, and -CH₂-CH₂-CH₂- is substituted by -CH₂-S-CH(CH₃)-.

In the present invention, a "linking bond" containing no atoms is only for connection, that is, when a group is defined as a linking bond, it means the group can be absent.

In the present invention, the expression "at each occurrence, each independently" not only mean that different groups can be selected from the defined groups independently, but also the different positions in the same group can be selected from the defined groups independently. For example, Z is, at each occurrence, each independently selected from the group consisting of -C(=O)-, -OC(=O)-, -C(=O)O-, -OC(=O)O-, -O-, -S-, -C(=O)S-, - SC(=O)-, -NR_{c}C(=O)-, -C(=O)NR_{c}-, -NR_{c}C(=O)NR_{c}-, -OC(=O)NR_{c}-, -NR_{c}C(=O)O-, - SC(=O)NR_{c}-, -NR_{c}C(=O)S-, and 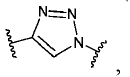 wherein R_{c} is, at each occurrence, each independently a hydrogen atom or a C₁₋₁₂ alkyl group; in the group "-NR_{c}C(=O)NR_{c}-", two R_{c} groups can be the same or different, which are each independently a hydrogen atom or a C₁₋₁₂ alkyl group.

In the present invention, the term "group" contains at least one atom, referring to the radical formed by a compound losing one or more atoms. Relative to a compound, the group formed by a compound losing part of its groups is also termed as a residue. The valence of a group is not particularly limited, and examples include a monovalent group, a divalent group, a trivalent group, a tetravalent group, ..., a hectovalent group, etc. Wherein, groups whose valence is equal to or greater than two are collectively defined as linking groups. A linking group can also contain only one atom, such as an oxygen group and a sulfur group.

In the present invention, the term "hydrocarbon" refers to a class of compounds that contains only carbon atoms and hydrogen atoms.

In the present invention, hydrocarbons are classified into aliphatic hydrocarbons and aromatic hydrocarbons by category. Hydrocarbons containing neither phenyl rings nor hydrocarbyl-substituted phenyl rings are defined as aliphatic hydrocarbons. Hydrocarbons containing at least one phenyl ring or hydrocarbyl-substituted phenyl ring are defined as aromatic hydrocarbons. An aromatic hydrocarbon can contain aliphatic hydrocarbon groups, such as toluene, diphenylmethane, 2,3-dihydroindene, etc.

In the present invention, hydrocarbons are classified into saturated hydrocarbons and unsaturated hydrocarbons by saturation. All aromatic hydrocarbons are unsaturated hydrocarbons. Saturated aliphatic hydrocarbons are also termed as alkanes. The degree of unsaturation of unsaturated aliphatic hydrocarbons is not particularly limited. For example, it includes but is not limited to alkenes (containing carbon-carbon double-bonds), alkynes (containing carbon-carbon triple-bonds), dienes (containing two conjugated carbon-carbon double-bonds), and the like. When the aliphatic moieties are saturated in an aromatic hydrocarbon, said aromatic hydrocarbon is also termed as arylalkane, such as toluene.

In the present invention, the structures of hydrocarbons are not particularly limited, including linear structures without pendant groups, branched structures bearing pendant groups, cyclic structures containing at least one ring, dendritic structures, comb-like structures, hyperbranched structures, etc. Unless otherwise specified, preferable structures include linear structures without pendant groups, branched structures bearing pendant groups and cyclic structures, which correspond to linear hydrocarbons, branched hydrocarbons and cyclic hydrocarbons, respectively. Wherein, hydrocarbons without cyclic structures are termed as open-chain hydrocarbons, including but not limited to linear structures without pendant groups, and branched structures bearing pendant groups. Open-chain hydrocarbons fall into the scope of aliphatic hydrocarbons, so linear hydrocarbons are also described as linear aliphatic hydrocarbons and branched hydrocarbons are also described as branched aliphatic hydrocarbons.

In the present invention, hydrocarbons with any carbon atom being replaced by heteroatom are generally referred to as heterosubstituted hydrocarbons.

In the present invention, aliphatic-derived heterosubstituted hydrocarbon refers to the heterosubstituted hydrocarbons that deriving from aliphatic hydrocarbons, including aliphatic-derived heterocyclic hydrocarbons and aliphatic-derived open-chain heterosubstituted hydrocarbons. Saturated aliphatic-derived heterohydrocarbons are also termed as heteroalkanes.

In the present invention, "hydrocarbon group" refers to the residue of a hydrocarbon molecule with at least one hydrogen atom being removed. According to the number of the lost hydrogen atoms, hydrocarbon groups can be classified into monovalent hydrocarbon groups (with one hydrogen atom lost), divalent hydrocarbon groups (with two hydrogen atoms lost, which also referred to as hydrocarbylene groups), trivalent hydrocarbon groups (with three hydrogen atoms lost), and the like. Accordingly, when n hydrogen atoms are lost, the valence of the resulting hydrocarbon group is n. In the present invention, hydrocarbon groups refer specifically to monovalent hydrocarbon groups, unless otherwise specified.

In the present invention, the sources of hydrocarbon groups are not particularly limited, for example, from aliphatic hydrocarbons or aromatic hydrocarbons, from saturated hydrocarbons or unsaturated hydrocarbons, from linear hydrocarbons, branched hydrocarbons or cyclic hydrocarbons, from hydrocarbons or heterohydrocarbons, etc. According to the degree of saturation, e.g., the hydrocarbon groups can be derived from alkanes, alkenes, alkynes, dienes, etc.; with respect to cyclic hydrocarbons, e.g., from alicyclic hydrocarbons or aromatic hydrocarbons, from monocyclic hydrocarbons or polycyclic hydrocarbons; and with respect to heterocyclic hydrocarbons, e.g., from aliphatic heterocyclic hydrocarbons or aromatic heterocyclic hydrocarbons.

In the present invention, the "aliphatic hydrocarbon group" refers to the residue of an aliphatic hydrocarbon molecule with at least one hydrogen atom being removed. Aliphatic hydrocarbon groups in the present invention refer specifically to monovalent aliphatic hydrocarbon groups, unless otherwise specified. Aliphatic hydrocarbon groups include saturated aliphatic hydrocarbon groups and unsaturated aliphatic hydrocarbon groups.

In the present invention, the "alkyl group" refers to a hydrocarbon group obtained from an alkane losing hydrogen atom at any location, unless otherwise specified. Said alkyl group can be linear or branched, substituted or unsubstituted. Specific examples include that a propyl group refers to either a 1-propyl group or an isopropyl group, and a propylene group refers to a 1,3-propylene group, a 1,2-propylene group or an isopropylidene group.

In the present invention, the "unsaturated hydrocarbon group" refers to a hydrocarbon group obtained from an unsaturated hydrocarbon losing hydrogen atoms. The hydrocarbon group obtained from an unsaturated hydrocarbon losing hydrogen atoms bonded to unsaturated carbon atoms, can be an alkenyl group, an alkynyl group, or a dienyl group, etc., e.g., a propenyl group or a propynyl group. According to the difference of unsaturated bonds, the hydrocarbon group obtained from an unsaturated hydrocarbon losing hydrogen atoms bonded to saturated carbon atoms, can be termed, e.g., an alkenyl hydrocarbon group, analkyne, a dienyl hydrocarbon group, or the like, and specifically, e.g., an allyl group or a propargyl group.

In the present invention, the "alkenyl" or "alkenyl group" refers to a substituted or unsubstituted, linear or branched alkenyl group which contains two or more carbon atoms (e.g., two, three, four, five, six, seven, eight, nine, ten, eleven, twelve, thirteen, fourteen, fifteen, sixteen, seventeen eighteen, nineteen, twenty or more carbon atoms) and at least one carbon-carbon double bond. The term "C₂₋₁₅ alkenyl group" refers to a substituted or unsubstituted, linear or branched alkenyl group which contains 2-15 carbon atoms and at least one carbon-carbon double bond, that is, an alkenyl group can contain one, two, three, four, or more carbon-carbon double bonds. Alkenyl groups in the present invention include substituted and unsubstituted alkenyl groups, unless otherwise specified.

In the present invention, the "alkynyl" or "alkynyl group" refers to a linear or branched, optionally substituted hydrocarbon which contains two or more carbon atoms (e.g., two, three, four, five, six, seven, eight, nine, ten, eleven, twelve, thirteen, fourteen, fifteen, sixteen, seventeen eighteen, nineteen, twenty or more carbon atoms) and at least one carbon-carbon triple bond. The term "C₂₋₁₅ alkynyl group" refers to a substituted or unsubstituted, linear or branched alkynyl group which contains 2-15 carbon atoms and at least one carbon-carbon triple bond, that is, an alkynyl group can contain one, two, three, four, or more carbon-carbon triple bonds. Alkynyl groups in the present invention include substituted and unsubstituted alkynyl groups, unless otherwise specified.

In the present invention, the term "molecular weight" represents the mass of a compound, and the term "average molecular weight" represents the mass of a compound component of a general formula in macroscopic matter. Unless otherwise specified, the "average molecular weight" refers to the "number-average molecular weight" (Mₙ). The number-average molecular weight refers to the molecular weight of polydisperse blocks or substances, or that of monodisperse blocks or substances. Unless otherwise specified, the unit of measurement of "molecular weight" and "average molecular weight" are dalton, Da. The molecular weight of a polyethylene glycol chain can also be measured by "degree of polymerization" which specifically refers to the number of repeating units (oxyethylene units, EO-units) in a compound molecule. Accordingly, "average degree of polymerization", "number-average degree of polymerization" or "number of EO units" represents the average value or the number average value of the number of repeating units.

In the present invention, for polydispersity, when a term such as "equal", "same", "equivalent", or "approximately equal" (including other forms of equivalent expression) is used to describe the molecular weight or the degree of polymerization of a single compound and the number-average molecular weight or the number-average degree of polymerization of a compound component in macroscopic matter, unless otherwise specified, the term does not impose a strict numerical equality but indicates an approximation or an approximate equality in value; said approximation or approximate equality preferably refers to a deviation within ±10%, more preferably a deviation within ±5%, generally based on the preset value. The terms "about" and "approximately" generally indicate a numerical range of ±10% which, in some cases, can be amplified to ±15%, not exceeding ±20%. For example, the deviations of 10 kDa from 11 kDa and 12 kDa are 10% and 20%, respectively. As another example, when the molecular weight of a certain PEG component in the specified general formula is 5 kDa, it is allowed that the corresponding molecular weight or number-average molecular weight is 5 kDa±10%, that is, variable within the range of 4500-5500 Da. However, for monodisperse, the sameness or equality with respect to the number of oxyethylene units in a single compound molecule or general formula thereof indicates the strict numerical equality; for example, if the number of EO units of a certain PEG component is set to 11, then a value being 12 is beyond the scope; whereas, in order to obtain the compound component with the desired number of EO units, the products in macroscopic matter obtained by certain preparation methods may also contain impurities with other numbers of EO units besides the component with the target number of EO units because of the limitation of preparation methods and purification methods; in this case, if the deviation of the average number of EO units from the preset number of EO units is within ±5% (preset value ≥10) or within ±0.5 (preset value <10), it can be considered to have obtained the monodisperse product in macroscopic matter with the target component; moreover, when the content of the component with the number of EO units as desired or within the range of average number reaches certain percentages (preferably ≥90%, more preferably >95%, more preferably >96%, more preferably >98%, more preferably 99%-100%), the obtained product in macroscopic matter falls within the protected scope of the present invention; even if the above mentioned content percentages are not met, the resulting products of insufficient contents and the components in the form of co-products or by-products, as long as prepared by the preparation methods of the present invention or by similar methods using basically the same preparation ideas, whether separation and purification are performed or not, all fall within the protected scope of the present invention.

In the present invention, when Da, kDa, repeating unit, and number of EO units are used to describe the molecular weight of a compound of a general formula containing polydisperse components, for a single molecule, the value falls within a certain range covering the specified value (including endpoints, preferably within the range of ±10%); when the number of oxyethylene units is used to describe the preset molecular weight of a compound of a general formula containing monodisperse components, the value is not variable in any range but a discrete point; however, the average number of the EO units might be variable within a certain range (not exceeding ±10% or ±1, preferably not exceeding ±5% or ±0.5) because of heterogeneity in molecular weights of the prepared product. For example, the molecular weight of mPEG is 5 kDa, it means the molecular weight of a single molecule of the general formula is between 4500-5500 Da, and the average molecular weight of the corresponding component of the prepared product is 5 kDa, that is, the product with the average molecular weight between 4500-5500 Da is regarded as the target product, and only the components whose molecular weights fall within the scope contribute to the content of the target component; for another example, if the designed mPEG contains 22 oxyethylene units, then the numbers of EO units of all compound molecules of the general formula should be strictly 22, while the prepared product could be a mixture of compounds with the numbers of EO units being 20, 21, 22, 23, and 24, and in this case, when the average number of EO units is within the range of 22±2.2 (preferably in the range of 22±1.1), it is considered to have obtained the target component, meanwhile the components whose molecular weights fall within the numerical range can be regarded as the target component and used to calculate the purity. In the present invention, the product with PDI<1.005 can be considered to be monodisperse with PDI=1.

In the present invention, concerning the polydispersity coefficient PDI in the present invention, when the other parameters are the same or considered to be the same, it can be determined that there is no significant difference between the materials from different batches, and these materials can be regarded as the same material, as long as the PDI values do not exceed the preset value.

In the present invention, concerning the percentage, "about" and "approximately" generally refer to a tolerance of ±0.5%.

In the present invention, the terms "stable" and "degradable" regarding groups are a pair of opposing concepts.

In the present invention, the term "degradable" ("be degradable" or "can be degraded") means that a chemical bond can be cleaved into at least two independent residues. If a linking group remains whole, but with its structure altered after chemical changes, then it still belongs to the scope of "being stable". The conditions to be "degradable" are not particularly limited, which can be physiological conditions in vivo, simulated physiological environments in vitro, or other conditions, preferably physiological conditions in vivo and simulated physiological environments in vitro. Said physiological condition is not particularly limited, including but not limited to physiological environments of serum, heart, liver, spleen, lung, kidney, bone, muscle, fat, brain, lymph node, small intestine, gonad, etc., which are intracellular or in the extracellular matrix, in normal tissues or in pathologic tissues (e.g., tumor, inflammation, etc.). Said simulated physiological environment in vitro is not particularly limited, including but not limited to physiological saline, buffer, culture medium, and the like. The degradation is not particularly limited with respect to the rate, e.g., rapid degradation via enzymolysis, slow hydrolysis under physiological conditions, etc. Said physiological conditions in vivo include physiological conditions during treatment such as ultraviolet radiation, thermal therapy, etc. The conditions for degradation include but are not limited to light, heat, low temperature, enzymatic condition, oxidation-reduction condition, acidic condition, basic condition, physiological condition, simulated physiological environment in vitro, etc., preferably light, heat, enzymatic condition, oxidation-reduction condition, acidic condition, basic condition, etc. Said "degradable" means that the degradation can occur under any stimulation from the above conditions. Said light condition includes but is not limited to visible light, ultraviolet light, infrared light, near-infrared light, mid-infrared light, etc. Said heat condition refers to the temperature higher than the normal physiological temperature, normally the temperature higher than 37°C and below 45°C, and preferably below 42°C. Said low temperature refers to the temperature below the human physiological temperature, preferably below 25°C, more preferably ≤10°C, with specific examples such as refrigeration temperature, freezer temperature, temperature for liquid nitrogen treatment, 2~10°C, 4~8°C, 4°C, 0°C, -20±5°C, etc. Said enzymatic condition is not particularly limited, and all enzymes that can be physiologically generated are included, e.g., peptidases, proteases, lyases, etc. Said oxidation-reduction condition is not particularly limited, e.g., redox transformation or hydrogenation-reduction transformation between a mercapto group and a disulfide bond. Said acidic condition and basic condition mainly refer to the pH conditions of internal body parts such as normal tissues, pathologic tissues, and organs or tissues in treatment; for example, the stomach is under acidic condition, and a tumor site is usually under acidic condition as well. Said "degradable" means that the degradation can be realized through metabolism in vivo (e.g., physiological effect, enzymatic reaction, oxidation-reduction, etc.), microenvironment stimulation in specific areas inside the body (e.g., acidic condition and basic condition), clinical therapeutic stimulation (e.g., light, heat, and low temperature), etc. What should be noted is that some conditions in organic chemistry that are extreme for organisms, e.g., bond cleavage under conditions such as strong acid, strong base, high temperature (e.g., above 100°C), etc., are not included in the scope of the conditions for degradation of degradable bonds in the present invention. For example, although an ether bond can be cleaved under strong acid conditions (e.g., hydrobromic acid), it is always classified as a stable linking group in the present invention.

In the present invention, the term "stable" means that a linking group can keep as whole (i.e., stably and covalently connected to the adjacent groups) and therefore it is defined as "stable", wherein chemical changes without breaking the wholeness of the linking group are allowed. Said chemical changes are not particularly limited, including but not limited to isomerization, oxidation, reduction, ionization, protonation, deprotonation, substitution reaction, etc. The conditions to be "stable" are not particularly limited, including but not limited to light, heat, low temperature, enzymatic condition, oxidation-reduction, neutral condition, acidic condition, basic condition, physiological condition, simulated physiological environment in vitro, etc., preferably light, heat, enzymatic condition, oxidation-reduction, acidic condition, basic condition, etc. Being stable indicates that, without particular stimulation (e.g., pH conditions in specific areas, and light, heat, low temperature in treatment, etc.), the stable linking can be maintained in the metabolic cycle in vivo and the molecular weight will not be reduced due to the chain cleavage (as long as the integrity remains).

In the present invention, for a specific linking group, "stable" is not an absolute concept. For example, an amide bond is much more stable than an ester bond under acidic or basic condition, and in the present invention, "stable" linking groups include amide bonds. However, the peptide bond, for example, is a special kind of amide bond formed via the dehydration condensation of two amino acids that respectively provide an α-carboxyl group and an α-amino group for the reaction, could also be cleaved when encountering specific enzymatic interactions, and therefore it is also within the scope of "degradable" linking groups. Similarly, a urethane group, a thiourethane group, or the like could be either a "stable" linking group or a "degradable" linking group. More commonly, a urethane group, a thiourethane group, and the like tend to degrade slowly, while an amide bond in the form of a non-peptide bond can remain stable in the circulative metabolism in vivo. For another example, common ester bonds will degrade under acidic or basic condition, while an ester bond contained in a special structure could also degrade under UV exposure. As another example, even though some chemical bonds will degrade under specific enzymatic conditions, they can still be regard as stable if their circulation in clinical use (e.g., point-of-site administration) does not or basically not go through said enzymatic conditions.

In the present invention, in order to define more clearly the degradability of a compound structure, provided herein is a criterion for reference which is, a threshold being a specific percentage (e.g., 90%) of the examined chemical bond maintained within a limited time interval. Take 90% as an example, it usually takes the pharmacokinetic curve of functionalized PEGylated products as a reference, and is based on the dose percentage that meets the clinical evaluation criteria. For example, for intravenously administered PEGylated drugs, when the plasma concentration (calculated in terms of effective drug ingredients including PEGylated drugs and non-PEGylated components after degradation) is lower than 15% of the initial concentration (or another percentage more consistent with the clinical evaluation of the drug), the remaining 85% is taken as the base value; if the proportion of a linking group with its chemical bonds maintained exceeds 90%, then it is regarded as a stable group in the present invention; on the contrary, if said 90% is not met, then the linking group belongs to degradable groups. The hydrolytic stabilization and enzymatic degradation reported in the published literature are also included in the present invention. Take hydrolytic stabilization as an example, the hydrolysis rate in the process of hydrolytic stabilization reported in the published literature is also included herein, preferably referring to the hydrolysis rate less than 1-2% per day (generally 2%) under physiological conditions, in mass or molar weight. The hydrolysis rate of typical chemical bonds can be found in most standard handbooks of chemistry.

In the present invention, the term "hydroxyl protecting group" includes all the groups which can be used as common hydroxyl protecting groups. A hydroxyl protecting group is preferably selected from the group consisting of an alkanoyl group (e.g., an acetyl group and a butyryl group), an aromatic alkanoyl group (e.g., a benzoyl group), a benzyl group, a triphenylmethyl group, a trimethylsilyl group, a *t*-butyldimethylsilyl group, an allyl group, an acetal group, and a ketal group. The removal of an acetyl group is generally carried out under basic condition, most commonly by the ammonolysis with NH₃/MeOH or by the methanolysis catalyzed by methanol anions. The benzyl group is easily removed via palladium-catalyzed hydrogenolysis in a neutral solution at room temperature, or via the reduction reaction with metallic sodium in ethanol or liquid ammonia. The triphenylmethyl group is usually removed by catalytic hydrogenolysis. The trimethylsilyl group is usually removed with reagents containing fluoride ions (e.g., tetrabutylammonium fluoride/anhydrous THF, etc.). The *t*-butyldimethylsilyl ether is relatively stable, which can withstand the ester hydrolysis with alcoholic potassium hydroxide and mild reduction conditions (e.g., Zn/CH₃OH and the like), and can be removed by fluoride ions (e.g., Bu₄N⁺F⁻) in THF solution or by aqueous acetic acid at room temperature.

In the present invention, the "carboxyl protecting group" refers to the protecting group which can be transformed into a carboxyl group through hydrolysis or the deprotection reaction of the carboxyl protecting group itself. A carboxyl protecting group is preferably selected from the group consisting of an alkyl group (e.g., a methyl group, an ethyl group and a butyl group) and an aralkyl group (e.g., a benzyl group), and more preferably selected from the group consisting of a butyl group (tBu), a methyl group (Me), and an ethyl group (Et). In the present invention, the "protected carboxyl group" refers to the group obtained from a carboxyl group being protected by an appropriate carboxyl protecting group, preferably selected from the group consisting of a methoxycarbonyl group, an ethoxycarbonyl group, a *t*-butyloxycarbonyl group, and a benzyloxycarbonyl group. Said carboxyl protecting group can be removed through hydrolysis catalyzed by acids or alkalis, or through pyrolysis reaction occasionally; for example, *t*-butyl groups can be removed under mild acidic condition, and benzyl groups can be removed by hydrogenolysis. The reagent used for removing carboxyl protecting groups is selected from the group consisting of TFA, H₂O, LiOH, NaOH, KOH, MeOH, EtOH and combinations thereof, preferably selected from the combination of TFA and H₂O, the combination of LiOH and MeOH, and the combination of LiOH and EtOH. The deprotection of a protected carboxyl group can produce the corresponding free acid, which can be carried out in the presence of an alkali that forms pharmaceutically acceptable salts with said free acid generated during said deprotection.

In the present invention, the term "amino protecting group" includes all the groups which can be used as common amino protecting groups, such as an aryl C₁₋₆ alkyl group, a C₁₋₆ alkoxy C₁₋₆ alkyl group, a C₁₋₆ alkoxycarbonyl group, an aryloxycarbonyl group, C₁₋₆ alkylsulfonyl group, an arylsulfonyl group, a silyl group, etc. An amino protecting group is preferably selected from the group consisting of a *t*-butoxycarbonyl group (Boc), a *p-*methoxybenzyloxycarbonyl group (Moz) and a 9-fluorenylmethoxycarbonyl group (Fmoc). The reagent used for removing amino protecting groups is selected from the group consisting of TFA, H₂O, LiOH, MeOH, EtOH and combinations thereof, preferably selected from the combination of TFA and H₂O, the combination of LiOH and MeOH, and the combination of LiOH and EtOH. The reagent used for removing the Boc protecting group is TFA or HCl/EA, preferably TFA. The reagent used for removing the Fmoc protecting group is the *N*,*N-*dimethylformamide (DMF) solution containing 20% piperidine.

In the present invention, the "activation of a carboxyl group" refers to the activation of a carboxyl group with carboxyl activating agents. The activated carboxyl group can promote the condensation reactions, for example, by inhibiting the generation of racemic impurities, by accelerating the reaction through catalysis, etc. The "carboxyl activating group" refers to the residue of a carboxyl activating agent. Said carboxyl activating agent is selected from the group consisting of *N*-hydroxysuccinimide (NHS), 1-ethyl-3-(3-dimethylaminopropyl)carbodiimide hydrochloride (EDCI), N hydroxy-5-norbornene-2,3-dicarboximide (HONb), and *N*,*N*'-dicyclohexylcarbodiimide (DCC), and combinations thereof, preferably selected from the combination of NHS/EDCI, NHS/DCC, and HONb/DCC, and more preferably the combination of NHS/EDCI.

In the present invention, the term "cation" refers to the corresponding structure bearing a positive charge, either permanently, or non-permanently but in response to certain conditions (such as pH). Therefore, the cations include permanent cations and those cationisable compounds, groups, or atoms. Permanent cations refer to the corresponding compounds, groups, or atoms that bear positive charges under conditions of any pH value or hydrogen ion activity of their environment. Typically, a positive charge is generated by the presence of quaternary nitrogen atom. When a compound carries multiple such positive charges, it can be called a permanent cation. A cationisable substance refers to a compound, group or atom that is positively charged at a lower pH and uncharged at a higher pH of its environment. On the other hand, in non-aqueous environments where the pH cannot be determined, a cationisable compound, group, or atom is positively charged at a high hydrogen ion concentration and uncharged at a low concentration or activity of hydrogen ions. It depends on the individual properties of the cationisable or polycationisable compound, especially the pKa of the respective cationisable group or atom, at which pH or hydrogen ion concentration said compound is charged or uncharged. In the diluted aqueous environment, the so-called Henderson-Hasselbalch equation can be used to estimate the fraction of positively charged cationisable compounds, groups or atoms, which is well-known to those skilled in the art. For example, in some embodiments, if a compound or moiety is cationisable, it is preferred that it is positively charged at a pH value of about 1 to 9, preferably 4 to 9, 5 to 8 or even 6 to 8, more preferably at a pH value of or below 9, of or below 8, of or below 7, and most preferably at physiological pH values (e.g., about 7.3 to 7.4), i.e., under physiological conditions, particularly under physiological salt conditions of the cell in vivo. In other embodiments, it is preferred that the cationisable compound or moiety is predominantly neutral at physiological pH values (e.g., about 7.0-7.4), but becomes positively charged at lower pH values. In some embodiments, the preferred range of pKa for the cationisable compound or moiety is about 5 to about 7.

In the present invention, the "cationic component/compound" typically refers to a charged molecule, which is positively charged (cation) at a pH value of typically about 1 to 9. In some embodiments, the cationic component/compound is preferably charged at a pH value of or below 9 (e.g., 5 to 9), of or below 8 (e.g., 5 to 8), of or below 7 (e.g., 5 to 7), and most preferably at physiological pH values (e.g., about 7.3 to 7.4). Therefore, a cationic peptide, protein, polysaccharide, lipid or polymer according to one embodiment of the present invention is positively charged under physiological conditions, particularly under physiological salt conditions of the cell in vivo.

In the present invention, a lipid nanoparticle, cationic peptide, protein, polysaccharide, lipid, or polymer is uncharged, having a neutral charge or being respectively electrically neutral under physiological conditions, particularly under the physiological salt conditions of the cell in vivo. A cationic peptide or protein preferably contains a larger amount of cationic amino acids, e.g., a greater number of Arg, His, Lys or Orn than other amino acid residues (especially more cationic amino acids than anionic amino acid residues like Asp or Glu), or contains blocks predominantly formed by cationic amino acid residues. The expression "cation" may also refer to "polycationic" components/compounds. The cationic component/compound may also refer to a cationic lipid capable of being positively charged. For example, cationic lipids include one or more amine groups bearing positive charges, the preferred cationic lipids are ionizable so that they can exist in a positively charged or neutral form depending on pH. The ionization of the cationic lipid affects the surface charge of a lipid nanoparticle (LNP) under different pH conditions. This charge state can influence plasma protein absorption, blood clearance and tissue distribution as well as the ability to form non-bilayer structures critical to the intracellular delivery of nucleic acids.

In the present invention, the "PEGylated lipid" refers to a molecule containing lipid and polyethylene glycol moieties. In addition to those represented by the general formula (2) in the present invention, PEGylated lipids also include but are not limited to 1,2-dimyristoyl-sn-glycerol methoxypolyethylene glycol (PEG-DMG), 1,2-distearoyl-sn-glycero-3-phosphoethanolamine-N-[amino(polyethylene glycol)] (PEG-DSPE), PEG-cholesterol, PEG-diacylglycamide (PEG-DAG), PEG-dialkyloxypropyl (PEG-DAA), and specifically PEG500-dipalmitoylphosphatidylcholine, PEG2000-dipalmitoylphosphatidylcholine, PEG500-stearyl phosphatidylethanolamine PEG2000-distearylphosphatidylethanolamine, PEG500-1,2-dioleoylphosphatidylethanolamine, PEG2000-1,2-dioleoylphosphatidylethanolamine, PEG2000-2,3-distearoylglycerol (PEG-DMG), and the like.

In the present invention, the "neutral lipid" refers to any lipid substance which is uncharged or exists in the form of neutral zwitterion at the chosen pH, preferably phospholipid. This kind of lipid includes but is not limited to 1,2-dilinoleoyl-sn-glycero-3-phosphocholine (DLPC), 1,2-dimyristoyl-sn-glycero-phosphocholine (DMPC), 1,2-dioleoyl-sn-glycero-3-phosphocholine (DOPC), 1,2-dipalmitoyl-sn-glycero-3-phosphocholine (DPPC), 1,2-distearoyl-sn-glycero-3-phosphocholine (DSPC), 1,2-diundecanoyl-sn-glycero-phosphocholine (DUPC), 1-palmitoyl-2-oleoyl-sn-glycero-3-phosphocholine (POPC), 1,2-di-O-octadecenyl-sn-glycero-3-phosphocholine (18:0 Diether PC), 1-oleoyl-2-cholesterylhemisuccinoyl-sn-glycero-3-phosphocholine (OChemsPC), 1-hexadecyl-sn-glycero-3-phosphocholine (C16 Lyso PC), 1,2-dilinolenoyl-sn-glycero-3-phosphocholine, 1,2-diarachidonoyl-sn-glycero-3-phosphocholine, 1,2-didocosahexaenoyl-sn-glycero-3-phosphocholine, 1,2-dioleoyl-sn-glycero-3-phosphoethanola mine (DOPE), 1,2-diphytanoyl-sn-glycero-3-phosphoethanolamine (ME 16.0 PE), 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didocosahexaenoyl-sn-glycero-3-phosphoethanolamine, 1,2-dioleoyl-sn-glycero-3-phospho-rac-(1-glycerol) sodium salt (DOPG), dioleoylphosphatidylserine (DOPS), dipalmitoylphosphatidylglycerol (DPPG), palmitoyloleoylphosphatidylethanolamine (POPE), distearoylphosphatidylethanolamine (DSPE), dipalmitoylphosphatidylethanolamine (DPPE), dimyristoylphosphoethanolamine (DMPE), 1-stearoyl-2-oleoyl-phosphatidyethanolamine (SOPE), 1-stearoyl-2-oleoyl-phosphatidylcholine (SOPC), sphingomyelin, phosphatidylcholine, phosphatidylethanolamine, phosphatidylserine, phosphatidylinositol, phosphatidic acid, palmitoyloleoylphosphatidylcholine, lysophosphatidylcholine, lysophosphatidylethanolamine (LPE), and combinations thereof. The neutral lipid can be synthetic or natural.

In the present invention, a "steroid lipid" is selected from the group consisting of cholesterol, coprostanol, sitosterol, ergosterol, campesterol, stigmasterol, brassicasterol tomatidine, ursolic acid, α-tocopherol, and mixtures thereof.

In the present invention, an "amino acid residue" is an amino acid from which, formally, a hydrogen atom has been removed from an amino group and/or from which, formally, a hydroxy group has been removed from a carboxy group and/or from which, formally, a hydrogen atom has been removed from a sulfhydryl group and/or with a protected amino group and/or with a protected carboxyl group and/or with a protected sulfhydryl group. Imprecisely, an amino acid residue can be described as an amino acid. The source of the amino acid in the present invention is not particularly limited unless otherwise specified, which can be either natural or unnatural, or a mixture of both. The configuration of the amino acid in the present invention is not particularly limited unless otherwise specified, which can be either L-type or D-type, or a mixture of both. In one embodiment of the invention, the amino acid is a hydrophobic amino acid, selected from the group consisting of tryptophan (Trp), phenylalanine (Phe), valine (Val), isoleucine (Ile), leucine (Leu), and tyrosine (Tyr). In another embodiment of the invention, the amino acid is a hydrophilic amino acid, selected from the group consisting of glutamic acid (Glu), aspartic acid (Asp), histidine (His), glutamine (Gln), asparagine (Asn), serine (Ser), threonine (Thr), proline (Pro), glycine (Gly), lysine (Lys), and arginine (Arg), preferably selected from glycine and lysine, and more preferably lysine.

In the present invention, the term "functional group source" refers to those having reaction activity or potential reaction activity, photosensitivity or potential photosensitivity, targeting properties or potential targeting properties. Said "potential" means that the functional group source can be converted into a reactive group through chemical processes including but not limited to functional modification (e.g., grafting, substitution, etc.), deprotection, salt complexation and decomplexation, ionization, protonation, deprotonation, leaving group transformation, etc., and can exhibit luminescence or targeting properties through external stimuli such as light, heat, enzymes, specific binding molecules, microenvironment in vivo, etc. Said luminescence is not particularly limited, including but not limited to visible light, fluorescence, phosphorescence, etc.

In the present invention, a variant form refers to a structural form that can be transformed into the target reactive group after any process of chemical change selected from the group consisting of oxidation, reduction, hydration, dehydration, electronic rearrangement, structural rearrangement, salt complexation and decomplexation, ionization, protonation, deprotonation, substitution, deprotection, leaving group transformation, etc.

In the present invention, a "variant form of a reactive group" refers to a form which still have reactivity after a reactive group undergoing at least one process of chemical change selected from oxidation, reduction, hydration, dehydration, electronic rearrangement, structural rearrangement, salt complexation and decomplexation, ionization, protonation, deprotonation, substitution, deprotection, leaving group transformation, etc., or a non-reactive form that has been protected.

In the present invention, the "micro-modification" refers to a chemical modification process that can be completed through simple chemical reaction processes. Said simple chemical reaction processes mainly refer to deprotection, salt complexation and decomplexation, ionization, protonation, deprotonation, leaving group transformation, etc.

The "micro variant form" corresponds to the "micro-modification", referring to the structural form that can be transformed into the target reactive group after simple chemical reaction processes such as deprotection, salt complexation and decomplexation, ionization, protonation, deprotonation, leaving group transformation, etc. Said leaving group transformation includes the transformation from an ester form to an acyl chloride form.

The description "any appropriate" in expressions such as "any appropriate linking group", "any appropriate reactive group", etc., indicates that the structure accords with the fundamental principle of chemical structure and can enable the preparation method in the present invention to be implemented successfully. A chemical structure described in this way can be considered to have a clear and determined scope.

When at least two structural types are listed, the "any combination" of the listed structural types refers to a combination of any two or more afore-listed types of relevant structures; the number of a structural unit is not limited, which means, the number of any structural unit can be 0, 1, or greater than 1; and, when the number of structural units of the same type is greater than 1, the structural units could be from the same or different chemical structures, wherein the total number of said structural units is at least 2. For example, any combination of alkylene groups, divalent cycloalkyl groups, divalent cycloalkenyl groups, divalent cycloalkynyl groups, divalent cyclodienyl groups, arylene groups, carbon-carbon double bonds, carbon-carbon triple bonds, conjugated carbon-carbon double bonds, divalent aliphatic heterocyclic linking groups, divalent aromatic heterocyclic linking groups, and carbon chain linking groups with heteroatom-containing pendant groups, can be -Ph-CH₂-Ph-(arylene-alkylene-arylene), -CH₂-Ph-CH₂CH₂-(alkylene-arylene-alkylene; wherein, the alkylene group has a quantity of 2, with different structures), or a structure obtained from the phenyl ring of any aforementioned example being replaced with the ring of a hexane, diazepine, or 1-(2-pyridinyl) hexahydro-1H-1,4-diazepine. As another example, a cycloalkenyl hydrocarbon group = a cycloalkenyl group + a hydrocarbylene group = a hydrocarbon group substituted with a cycloalkenyl group, and a cyclodienyl hydrocarbon group = a hydrocarbon group substituted with a cyclodienyl group. In the present invention, the alkylene group (i.e., divalent alkyl group) includes the open-chain alkylene group and the divalent cycloalkyl group; the open-chain alkylene group refers to the divalent alkyl group containing no ring structures, and the divalent cycloalkyl group refers to the ring-containing divalent alkyl group.

In the present invention, the "adjuvant" or "adjuvant component" is typically a (e.g., pharmacological or immunological) agent or composition that may modify (e.g., enhance) the efficacy of other agents (e.g., drugs or vaccines). Conventionally, the term refers in the context of the invention to a compound or composition that serves as a carrier or auxiliary substance for immunogens and/or other pharmaceutically active compounds. It is to be interpreted in a broad sense and refers to a broad spectrum of substances that are able to increase the immunogenicity of antigens incorporated into or co-administered with an adjuvant in question. In the present invention, an adjuvant will preferably enhance the specific immunogenic effect of the active agents of the present invention. Typically, "adjuvant" or "adjuvant component" has the same meaning and can be used mutually. Adjuvants may be divided, e.g., into immuno potentiators, antigenic delivery systems or even combinations thereof.

In the present invention, the "N/P ratio" refers to molar ratio of nitrogen atoms in cationic lipids to phosphoric acid in nucleic acids.

In the present invention, the "nucleic acid" refers to DNA, RNA or modified form thereof, including purines and pyrimidine bases in DNA (adenine "A", cytosine "C", guanine "G", thymine "T"), or purines and pyrimidine bases in RNA (adenine "A", cytosine "C", guanine "G", uracil "U").

In the present invention, the term "RNA" refers to a ribonucleic acid that may be naturally or non-naturally occurring. For example, an RNA may include modified and/or non-naturally occurring components such as one or more nucleobases, nucleosides, nucleotides, or linkers. An RNA may include a cap structure, a chain terminating nucleoside, a stem loop, a polyA sequence, and/or a polyadenylation signal. An RNA may have a nucleotide sequence encoding a polypeptide of interest. For example, an RNA may be a messenger RNA (mRNA). The translation of an mRNA encoding a particular polypeptide, for example, the in vivo translation of an mRNA inside a mammalian cell, may produce the encoded polypeptide. An RNA may be selected from the non-limiting group consisting of small interfering RNA (siRNA), asymmetrical interfering RNA (aiRNA), microRNA (miRNA), Dicer-substrate RNA (dsRNA), small hairpin RNA (shRNA), mRNA, single guide RNA (sgRNA), cas9 mRNA and mixtures thereof.

In the present invention, an antisense oligonucleotide or small interfering RNA (siRNA) can inhibit the expression of the target gene and the target protein in vitro or in vivo.

In the present invention, the Flue mRNA can express the luciferase protein which emits bioluminescence in the presence of fluorescein substrates, so Flue is commonly used in mammalian cell culture to measure gene expression and cell activity.

In the present invention, the term "inhibiting the expression of a target gene" refers to the ability of nucleic acids to silence, reduce or inhibit the expression of a target gene. To examine the extent of gene silencing, a test sample (e.g., a sample of cells in culture expressing the target gene) is contacted with nucleic acids that inhibit the expression of the target gene. The expression of the target gene in the test sample or test animal is compared to the expression of the target gene in a control sample (e.g., a sample of cells in culture expressing the target gene) which is not contacted with or administered the nucleic acids. The expression of the target gene in the control sample may be assigned a value of 100%. In particular embodiments, inhibition of expression of a target gene is achieved when the level of target gene expression in the test sample or the test mammal relative to the level of target gene expression in the control sample or the control mammal is about 95%, 90%, 85%, 80%, 75%, 70%, 65%, 60%, 55%, 50%, 45%, 40%, 35%, 30%, 25%, 20%, 15%, 10%, 5%, or 0%.

In the present invention, assays for determining the level of target gene expression include but are not limited to dot blots, northern blots, in situ hybridization, ELISA, immunoprecipitation, enzyme function, and phenotypic assays.

In the present invention, the term "transfection" refers to the introduction of a species (e.g., RNA) into a cell. Transfection may occur, for example, in vitro, ex vivo, or in vivo.

In the present invention, the term "antigen" typically refers to a substance that can be recognized by the immune system, preferably recognized by the adaptive immune system, and triggers an antigen-specific immune response, for example, forming antibodies and/or antigen-specific T cells as a part of the adaptive immune response. Typically, the antigen may be or may contain a peptide or a protein that can be presented to T cells by MHC. In the present invention, the antigen may be a translation product of the provided nucleic acid molecule (preferably mRNA as defined herein). In this context, fragments, variants, and derivatives of peptides and proteins containing at least one epitope are also recognized as antigens.

In the present invention, the term "delivery" refers to providing an entity to the target, for example, delivering drugs and/or therapeutic agents and/or prophylactic agents to subjects, said subjects being tissues and/or cells of human and/or other animals.

In the present invention, the "pharmaceutically acceptable carrier" refers to a diluent, adjuvant, excipient, or vehicle administered together with the therapeutic agent, which is, in a reasonable medical judgment range, suitable for the contact of the human and/or other animal without excessive toxicity, stimulation, hypersensitive response, other problems or complications corresponding to a reasonable benefit/risk ratio. Pharmaceutically acceptable carriers that can be used in the pharmaceutical composition in the present invention include but are not limited to sterile liquids, e.g., water and oil, including the oil from petroleum, animal, plant, or synthetic origin, e.g., peanut oil, soybean oil, mineral oil, sesame oil, etc. When said pharmaceutical composition is administered intravenously, water is an exemplary carrier. Physiological saline, glucose and aqueous glycerol solution can also be used as liquid carriers, and are especially used for injection. Suitable pharmaceutical excipients include starch, glucose, lactose, sucrose, gelatin, maltose, chalk, silica gel, sodium stearate, glyceryl monostearate, talc, sodium chloride, defatted milk powder, glycerol, propylene glycol, water, ethanol, etc. Said composition can also contain a small amount of humectant, emulsifier or pH buffer as needed. Oral preparations can contain standard carriers, e.g., pharmaceutical grade mannitol, lactose, starch, magnesium stearate, sodium saccharin, cellulose, magnesium carbonate, etc. Specifically, excipients include but are not limited to anti-adherents, antioxidants, binders, coatings, compression aids, disintegrants, dyes (pigments), emollients, emulsifiers, fillers (diluents), film formers or coatings, flavors, fragrances, glidants (flow enhancers), lubricants, preservatives, printing inks, sorbents, suspending or dispersing agents, sweeteners, and waters for hydration. More specifically, excipients include but are not limited to butylated hydroxytoluene (BHT), calcium carbonate, calcium phosphate (dibasic), calcium stearate, croscarmellose, crosslinked polyvinyl pyrrolidone, citric acid, crospovidone, cysteine, ethylcellulose, gelatin, hydroxypropyl cellulose, hydroxypropyl methylcellulose, lactose, magnesium stearate, maltitol, mannitol, methionine, methylcellulose, methyl paraben, microcrystalline cellulose, polyethylene glycol, polyvinyl pyrrolidone, povidone, pregelatinized starch, phenyl paraben, retinyl palmitate, shellac, silicon dioxide, sodium carboxymethyl cellulose, sodium citrate, sodium starch glycolate, sorbitol, starch (corn), stearic acid, sucrose, talc, titanium dioxide, vitamin A, vitamin E (α-tocopherol), vitamin C, and xylitol.

In the present invention, pharmaceutical compositions can act systemically and/or locally. For this purpose, they can be administered by appropriate routes such as injection (e.g., intravenous, intraarterial, subcutaneous, intraperitoneal, or intramuscular injection, including instillation) or transdermal administration, or administered by oral, buccal, transnasal, transmucosal or topical routes or in the form of ophthalmic preparation or by inhalation. Regarding these administration routes, the pharmaceutical compositions of the present invention can be administered in a suitable dosage form. The dosage forms include but are not limited to tablets, capsules, lozenges, hard sugar agents, powders, sprays, creams, ointments, suppositories, gels, pastes, emulsions, ointments, aqueous suspensions, injectable solutions, elixirs, and syrups.

In the present invention, vaccines are preventive or therapeutic materials that provide at least one antigen or antigenic function. An antigen or antigenic function can stimulate the body's adaptive immune system to provide an adaptive immune response.

In the present invention, treatment refers to the management and care of patients to resist diseases, obstacles or symptoms, which is intended to delay the development of diseases, obstacles or symptoms, reduce or alleviate symptoms and complications, and/or cure or eliminate diseases, obstacles or symptoms. The patients to be treated are preferably mammals, especially humans.

**1.1.** The PEGylated lipid having the structure represented by the following general formula (2): 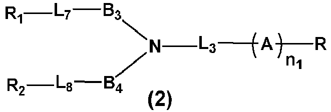
or pharmaceutically acceptable salts, tautomers, or stereoisomers thereof,
wherein, L₇ and L₈ are each independently a linking bond, -OC(=O)- or -C(=O)O-;
L₃ is a linking bond, -L₄-, -L₄-Z-, -Z-L₄-, -Z-L₄-Z-, -L₄-Z-L₅-, -Z-L₄-Z-L₅-, -L₄-Z-L₅-Z-, -Z-L₄-Z-L₅-Z- or -L₄-Z-L₄-Z-L₅-Z-; said L₄ and L₅ are carbon chain linking groups, each independently represented by -(CRₐR_{b})ₜ-(CRₐR_{b})ₒ-(CRₐR_{b})ₚ-, wherein, t, o and p are each independently an integer from 0 to 12, and t, o and p are not 0 simultaneously; Rₐ and R_{b} are, at each occurrence, each independently a hydrogen atom or a C₁₋₁₂ alkyl group; said Z is, at each occurrence, each independently selected from the group consisting of -C(=O)-, - OC(=O)-, -C(=O)O-, -OC(=O)O-, -O-, -S-, -C(=O)S-, -SC(=O)-, -NR_{c}C(=O)-, -C(=O)NR_{c}-, - NR_{c}C(=O)NR_{c}-, -OC(=O)NR_{c}-, -NR_{c}C(=O)O-, -SC(=O)NR_{c}-, -NR_{c}C(=O)S-, and 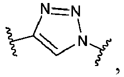 wherein, R_{c} is, at each occurrence, independently a hydrogen atom or a C₁₋₁₂ alkyl group;
B₃ and B₄ are each independently a linking bond or a C₁₋₃₀ alkylene group;
R₁ and R₂ are each independently a C₁₋₃₀ aliphatic hydrocarbon group;
R is a hydrogen atom, -R_{d}, -OR_{d}, -NR_{d}R_{d}, -SR_{d}, -C(=O)R_{d}, -C(=O)OR_{d}, -OC(=O)R_{d}, - OC(=O)OR_{d} or 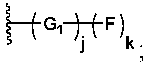 wherein, R_{d} is, at each occurrence, independently a C₁₋₁₂ alkyl group, G₁ is a (k+1)-valent terminal branching group, j is 0 or 1, and F contains the functional group R₀₁; when j is 0, G₁ is absent; when j is 1, G₁ protrudes F with the number of k, and k is an integer from 2 to 8; when L₃ is -C(=O)CH₂-, R is not a hydrogen atom, an alkyl group, an alkoxy group or a hydroxyl group;
A is -(CRₐR_{b})ₛO- or -O(CRₐR_{b})ₛ-, wherein s is 2, 3 or 4;
n₁ is an integer from 20 to 250;
said alkyl group, alkylene group, alkoxy group, and aliphatic hydrocarbon group are each independently substituted or unsubstituted.

### 1.1.1. Divalent linking groups L₃, L₄, L₅, L₇, L₈, Z, Z₁, and Z₂

In the present invention, the structures of L₃, L₄, L₅, L₇, L₈, Z, Z₁, and Z₂ are not particularly limited, each independently including but not limited to linear structures, branched structures or ring-containing structures.

In the present invention, the number of non-hydrogen atoms of L₃, L₄, L₅, L₇, L₈, Z, Z₁, and Z₂ are not particularly limited, each independently preferably from 1 to 50, more preferably from 1 to 20, and more preferably from 1 to 10. Said non-hydrogen atom is a carbon atom or a heteroatom. Said heteroatom includes but is not limited to O, S, N, P, Si, B, etc. When the number of non-hydrogen atoms is 1, the non-hydrogen atom can be a carbon atom or a heteroatom. When the number of non-hydrogen atoms is greater than 1, the species of non-hydrogen atoms are not particularly limited, which can be one, two or more than two species; when the number of non-hydrogen atoms is greater than 1, they can be any combination of carbon atoms and carbon atoms, carbon atoms and heteroatoms, or heteroatoms and heteroatoms.

In the present invention, two identical or different reactive groups may form a divalent linking group after reaction. The reaction conditions are related to the types of the resulting divalent linking groups, and the prior art can be introduced herein. For example, amino groups can react with active esters, active formates, sulfonate esters, aldehydes, α,β-unsaturated bonds, carboxylic groups, epoxides, isocyanates, and isothiocyanates, respectively, to obtain divalent linking groups such as amide groups, urethane groups, amino groups, imide groups (which can be further reduced to secondary amino groups), amino groups, amide groups, amino alcohols, urea bonds, thiourea bonds, etc.; mercapto groups can react with active esters, active formates, sulfonate groups, mercapto groups, maleimide groups, aldehyde groups, α,β-unsaturated bonds, carboxyl groups, iodoacetamide groups, and anhydride groups, respectively, to obtain divalent linking groups such as thioester groups, thiocarbonate groups, thioether groups, disulfide groups, thioether groups, thiohemiacetal linking groups, thioether groups, thioester groups, thioether groups, imide groups, etc.; unsaturated bonds can react with mercapto groups to obtain thioether groups; carboxyl groups or acyl halides can respectively react with mercapto groups and amino groups to obtain groups such as thioester bonds, amide bonds, etc.; hydroxyl groups can react with carboxyl groups, isocyanates, epoxides, and chlorocarbonyloxy groups to obtain divalent linking groups such as ester groups, carbamate groups, ether bonds, carbonate groups, etc.; carbonyl groups or aldehyde groups can react with amino groups, hydrazines and hydrazides to obtain divalent linking groups such as imine groups, hydrazone groups, acylhydrazone groups, etc.; reactive groups such as azido groups, alkynyl groups, alkenyl groups, mercapto groups, azido groups, dienyl groups, maleimide groups, 1,2,4-triazoline-3,5-dione groups, dithioester groups, hydroxylamine groups, hydrazide groups, acrylate groups, allyloxy groups, isocyanate groups, tetrazole groups and the like can undergo click reactions to form various divalent linking groups including but not limited to the structures of triazoles, isoxazoles, thioether bonds, and the like.

The stability of divalent linking groups L₃, L₄, L₅, L₇, L₈, Z, Z₁, and Z₂ are not particularly limited, wherein any divalent linking group or a divalent linking group consisting of any aforementioned divalent linking group and adjacent heterosubstituted groups thereof is independently a stable linking group STAG or a degradable linking group DEGG.

### 1.1.1.1. divalent linking groups L₇, L₈

In the present invention, L₇ and L₈ are each independently a linking bond, -OC(=O)- or - C(=O)O-.

In one specific embodiment of the present invention, said L₇ and L₈ are preferably selected from the following situations:
(1) L₇ and L₈ are both linking bonds;
(2) one of L₇ and L₈ is a linking bond, and the other is -OC(=O)- or -C(=O)O-;
(3) L₇ and L₈ are each independently -OC(=O)- or -C(=O)O-; more preferably, L₇ and L₈ are both -OC(=O)- or -C(=O)O-.

### 1.1.1.2. Divalent linking group L₃

In the present invention, L₃ is a linking bond, -L₄-, -L₄-Z-, -Z-L₄-, -Z-L₄-Z-, -L₄-Z-L₅-, - Z-L₄-Z-L₅-, -L₄-Z-L₅-Z-, -Z-L₄-Z-L₅-Z-, or -L₄-Z-L₄-Z-L₅-Z-, said L₄ and L₅ are carbon chain linking groups, each independently represented by -(CRₐR_{b})ₜ-(CRₐR_{b})ₒ-(CRₐR_{b})ₚ-, wherein, t, o and p are each independently an integer from 0 to 12, and t, o and p are not 0 simultaneously; Rₐ and R_{b} are, at each occurrence, each independently a hydrogen atom or a C₁₋₁₂ alkyl group; said Z is, at each occurrence, each independently selected from the group consisting of -C(=O)-, -OC(=O)-, -C(=O)O-, -OC(=O)O-, -O-, -S-, -C(=O)S-, -SC(=O)-, - NR_{c}C(=O)-, -C(=O)NR_{c}-, -NR_{c}C(=O)NR_{c}-, -OC(=O)NR_{c}-, -NR_{c}C(=O)O-, -SC(=O)NR_{c}-, - NR_{c}C(=O)S-, and 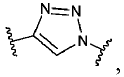 wherein, R_{c} is, at each occurrence, independently a hydrogen atom or a C₁₋₁₂ alkyl group;

In one specific embodiment of the present invention, R_{c} is preferably a hydrogen atom.

In one specific embodiment of the present invention, said L₃ contains degradable groups, said degradable group refers to the group which can degrade under any condition selected from the group consisting of light, heat, low temperature, enzymatic condition, oxidation-reduction condition, acidic condition, basic condition, physiological condition, and simulated physiological environment in vitro; L₃ is more preferably selected from the group consisting of -(CH₂)ₜ-, -(CH₂)ₜZ-, -Z(CH₂)ₜ-, -(CH₂)ₜZ(CH₂)ₜ-, -Z(CH₂)ₜZ-, -(CH₂)ₜZ(CH₂)ₜZ-, - Z(CH₂)ₜZ(CH₂)ₜ-, and -Z(CH₂)ₜZ(CH₂)ₜZ-, wherein, t is an integer from 0 to 12, and Z is, at each occurrence, independently selected from the group consisting of -C(=O)-, -OC(=O)-, - C(=O)O-, -OC(=O)O-, -O-, -S-, -C(=O)S-, -SC(=O)-, -NR_{c}C(=O)-, -C(=O)NR_{c}-, - NR_{c}C(=O)NR_{c}-, -OC(=O)NR_{c}-, -NR_{c}C(=O)O-, -SC(=O)NR_{c}-, and -NR_{c}C(=O)S-; L₃ is more preferably selected from the group consisting of -(CH₂)ₜ-, -(CH₂)ₜO-, -(CH₂)ₜC(=O)-, - (CH₂)ₜC(=O)O-, -(CH₂)ₜOC(=O)-, -(CH₂)ₜC(=O)NH-, -(CH₂)ₜNHC(=O)-, -(CH₂)ₜOC(=O)O-, - (CH₂)ₜNHC(=O)O-, -(CH₂)ₜOC(=O)NH-, -(CH₂)ₜNHC(=O)NH-, -O(CH₂)ₜ-, -C(=O)(CH₂)ₜ-, - C(=O)O(CH₂)ₜ-, -OC(=O)(CH₂)ₜ-, -C(=O)NH(CH₂)ₜ-, -NHC(=O)(CH₂)ₜ-, -OC(=O)O(CH₂)ₜ-, - NHC(=O)O(CH₂)ₜ-, -OC(=O)NH(CH₂)ₜ-, -NHC(=O)NH(CH₂)ₜ-, -(CH₂)ₜO(CH₂)ₜ-, - (CH₂)ₜC(=O)(CH₂)ₜ-, -(CH₂)ₜC(=O)O(CH₂)ₜ-, -(CH₂)ₜOC(=O)(CH₂)ₜ-, - (CH₂)ₜC(=O)NH(CH₂)ₜ-, -(CH₂)ₜNHC(=O)(CH₂)ₜ-, -(CH₂)ₜOC(=O)O(CH₂)ₜ-, - (CH₂)ₜNHC(=O)O(CH₂)ₜ-, -(CH₂)ₜOC(=O)NH(CH₂)ₜ-, -(CH₂)ₜNHC(=O)NH(CH₂)ₜ-, - O(CH₂)ₜO-, -C(=O)(CH₂)ₜC(=O)-, -C(=O)O(CH₂)ₜC(=O)O-, -OC(=O)(CH₂)ₜOC(=O)-, - C(=O)O(CH₂)ₜOC(=O)-, -OC(=O)(CH₂)ₜC(=O)O-, -OC(=O)O(CH₂)ₜOC(=O)O-, - C(=O)NH(CH₂)ₜC(=O)NH-, -NHC(=O)(CH₂)ₜNHC(=O)-, -NHC(=O)(CH₂)ₜC(=O)NH-, - C(=O)NH(CH₂)ₜNHC(=O)-, -NHC(=O)O(CH₂)ₜNHC(=O)O-, - OC(=O)NH(CH₂)ₜOC(=O)NH-, -NHC(=O)O(CH₂)ₜOC(=O)NH-, - OC(=O)NH(CH₂)ₜNHC(=O)O-, -NHC(=O)NH(CH₂)ₜNHC(=O)NH-, -C(=O)(CH₂)ₜO-, - C(=O)(CH₂)ₜC(=O)O-, -C(=O)(CH₂)ₜOC(=O)-, -C(=O)(CH₂)ₜOC(=O)O-, - C(=O)(CH₂)ₜNHC(=O)O-, -C(=O)(CH₂)ₜOC(=O)NH-, -C(=O)(CH₂)ₜNHC(=O)NH-, - C(=O)(CH₂)ₜC(=O)O(CH₂)ₜ-, -C(=O)(CH₂)ₜOC(=O)(CH₂)ₜ-, -C(=O)(CH₂)ₜOC(=O)O(CH₂)ₜ-, - C(=O)(CH₂)ₜNHC(=O)O(CH₂)ₜ-, -C(=O)(CH₂)ₜOC(=O)NH(CH₂)ₜ-, - C(=O)(CH₂)ₜNHC(=O)NH(CH₂)ₜ-, and -C(=O)(CH₂)ₜC(=O)(CH₂)ₜNHC(=O)O-, wherein, t is an integer from 2 to 12, and most preferably selected from the group consisting of -C(=O)O-, -(CH₂)ₜO-, -(CH₂)ₜC(=O)O-, -(CH₂)ₜOC(=O)-, -(CH₂)ₜO-, -C(=O)(CH₂)ₜO-, - C(=O)(CH₂)ₜOC(=O)-, -C(=O)(CH₂)ₜC(=O)O-, -C(=O)(CH₂)ₜC(=O)NH-, - C(=O)(CH₂)ₜOC(=O)NH-, -C(=O)(CH₂)ₜNHC(=O)O-, -C(=O)(CH₂)ₜOC(=O)NH(CH₂)ₜ-, and -C(=O)(CH₂)ₜC(=O)(CH₂)ₜNHC(=O)O-.

### 1.1.2. Description of stable and degradable groups

In the present invention, a stable linking group STAG or a degradable linking group DEGG can exist within any of the divalent linking groups L₃, L₄, L₅, L₇, L₈, Z, Z₁, and Z₂ mentioned above, or within a divalent linking group consisting of any afore-mentioned divalent linking group and adjacent heterosubstituted groups thereof.

### 1.1.2.1. Stable divalent linking groups STAG in the present invention

The condition of being stable for a stable divalent linking group STAG is not particularly limited, including but not limited to any condition selected from light, heat, low temperature, enzymatic condition, oxidation-reduction, acidic condition, basic condition, physiological condition, simulated physiological environment in vitro, etc., preferably light, heat, enzymatic condition, oxidation-reduction, acidic condition, basic condition, etc.

The type of stable divalent linking group STAG is not particularly limited, including but not limited to an alkylene group, a divalent heteroalkyl group, a carbon-carbon double bond, a carbon-carbon triple bond, a divalent dienyl group, a divalent cycloalkyl group, a divalent cycloalkenyl group, a divalent cycloalkenyl hydrocarbon group, a divalent cycloalkynyl hydrocarbon group, an aromatic ring, an aliphatic heterocyclic group, a heterophenylene group, an aromatic-fused heterocyclic group, a hetero-fused heterocyclic group, a substituted alkylene group, a substituted heteroalkyl group, a substituted divalent heteroalkyl group, a substituted double bond, a substituted triple bond, a substituted dienyl group, a substituted divalent cycloalkyl group, a substituted divalent cycloalkenyl group, a substituted divalent cycloalkenyl hydrocarbon group, a substituted divalent cycloalkynyl hydrocarbon group, a substituted aromatic ring, a substituted aliphatic heterocyclic group, a substituted heterophenylene group, a substituted aromatic-fused heterocyclic group, a substituted hetero-fused heterocyclic group, an ether bond, a thioether bond, a urea bond, a thiourea bond, a carbamate bond, a thiocarbamate bond, -P(=O)-, a divalent silyl group without active hydrogen atoms, a divalent boron-containing linking group, a secondary amino group, a tertiary amino group, a carbonyl group, a thiocarbonyl group, an amide group, a thioamide group, a sulfonamide group, an enamino group, a triazole group, a 4,5-dihydroisoxazole group, a skeleton of an amino acid or its derivative, and a stable divalent linking group composed of any two or more groups thereof.

Specifically, a stable divalent linking group STAG includes but is not limited to the structures described or listed in the references CN104530413A, CN104530415A and CN104530417A. Taking CN104530417A as an example, the corresponding paragraphs are from [0627] to [0704]. The way in which a STAG is composed of two or more stable divalent linking groups is not particularly limited, which includes but is not limited to those in the paragraph [0704] of CN104530417A.

### 1.1.2.2. Degradable divalent linking group DEGG in the present invention

The condition of degradable divalent linking groups DEGG to be degraded is not particularly limited, including but not limited to light illumination, heat, low temperature, enzymatic condition, oxidation-reduction, acidic condition, basic condition, physiological condition, simulated physiological environment in vitro, etc., preferably light, heat, enzymatic condition, oxidation-reduction, acidic condition, basic condition, etc.

The divalent linking group formed by the combination of any degradable divalent linking group DEGG and any stable divalent linking group STAG is still a degradable linking group. As for an aromatic ring-containing degradable divalent linking group, it can also be formed by the combination of aromatic rings and degradable divalent linking groups.

The type of degradable divalent linking group DEGG is not particularly limited, including but not limited to the degradable divalent linking group containing any divalent linking group or combination of any two or more thereof selected from the group consisting of a disulfide bond, a vinylether bond, an ester group, a thioate group, a thioester group, a dithioester group, a carbonate group, a thiocarbonate group, a dithiocarbonate group, a trithiocarbonate group, a carbamate group, a thiocarbamate group, a dithiocarbamate group, an acetal group, a cycloacetal group, a thioacetal group, an azaacetal group, an azacycloacetal group, an azathiaacetal group, a dithioacetal group, a hemiacetal group, a thiohemiacetal group, an azahemiacetal group, a ketal group, a thioketal group, an azaketal group, an azacycloketal group, an azathiaketal group, an imine bond, a hydrazone bond, an acylhydrazone bond, an oxime bond, a thiooxime ether bond, a semicarbazone bond, a thiosemicarbazone bond, a hydrazino group, a hydrazide group, a thiocarbohydrazide group, an azocarbohydrazide group, an azothiocarbohydrazide group, a hydrazino formate group, a hydrazino thioformate group, a carbohydrazide group, a thiocarbohydrazide group, an azo group, an isourea group, an isothiourea group, an allophanate group, a thioallophanate group, a guanidino group, an amidino group, an aminoguanidinyl group, a carbamimidamido group, an imino acid group, a thioimidate group, a sulfonate group, a sulfinate group, a sulfonyl hydrazide group, a sulfonyl ureido group, a maleimide group, an orthoester group, a phosphate group, a phosphirate group, a phosphinate group, a phosphonate group, a phosphosilicate group, a silicate group, an amide group, a thioamide group, a sulfonamide bond, a polyamide group, a phosphamide group, a phosphoramidite group, a pyrophosphamide group, a cyclophosphamide group, an ifosfamide group, a thiophosphamide group, an aconityl group, a peptide fragment, a skeleton of a nucleotide or its derivative, and a skeleton of a deoxynucleotide or its derivative.

Herein, said carbamate group, thiocarbamate group, amide group, phosphamide group, and the like, can be regarded as either a stable linking group or a degradable linking group, depending on the environmental characteristics of its use.

Specifically, a degradable divalent linking group DEGG includes but is not limited to the structures described or listed in the references CN104530413A, CN104530415A and CN104530417A. Taking CN104530417A as an example, the corresponding paragraphs are from [0705] to [0725].

### 1.1.3. Alkylene groups B₃ and B₄

In the present invention, B₃ and B₄ are each independently a linking bond or a C₁₋₃₀ alkylene group, more preferably a linking bond or a C₁₋₂₀ alkylene group.

In one specific embodiment of the present invention, B₃ and B₄ are both linking bonds.

In one specific embodiment of the present invention, one of B₃ and B₄ is a linking bond, and the other is a C₁₋₂₀ alkylene group.

In one specific embodiment of the present invention, B₃ and B₄ are each independently a C₁₋₂₀ alkylene group; specifically, each independently selected from the group consisting of a methylene group, an ethylene group, a propylene group, a butylene group, a pentylene group, a hexylene group, a heptylene group, an octylene group, a nonylene group, a decylene group, an undecylene group, a dodecylene group, a tridecylene group, a tetradecylene group, a pentadecylene group, a hexadecylene group, a heptadecylene group, an octadecylene group, a nonadecylene group, and an eicosylene group.

### 1.1.4. Aliphatic hydrocarbon groups R₁ and R₂

In the present invention, R₁ and R₂ are each independently a C₁₋₃₀ aliphatic hydrocarbon group.

In one specific embodiment of the present invention, R₁ and R₂ are preferably each independently a C₅₋₃₀ aliphatic hydrocarbon group, more preferably a C₅₋₂₀ aliphatic hydrocarbon group.

In one specific embodiment of the present invention, R₁ and R₂ are preferably each independently a linear alkyl group, a branched alkyl group, a linear alkenyl group, a branched alkenyl group, a linear alkynyl group, or a branched alkynyl group, preferably a linear aliphatic hydrocarbon group, more preferably a C₅₋₂₅ linear aliphatic hydrocarbon group, more preferably a C₅₋₂₀ linear aliphatic hydrocarbon group, more preferably selected from the group consisting of a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an eicosyl group, a (*Z*)-tridec-8-enyl group, a (*Z*)-tetradec-9-enyl group, a (*Z*)-pentadec-8-enyl group, a (*Z*)-hexadec-9-enyl group, a (*Z*)-heptadec-5-enyl group, a (*Z*)-heptadec-8-enyl group, a (*E*)-heptadec-8-enyl group, a (*Z*)-heptadec-10-enyl group, a (8*Z*, 11*Z*)-heptadec-8,11-dienyl group, a (*Z*)-octodec-6-enyl group, a (*Z*)-octodec-9-enyl group, a (*E*)-octodec-9-enyl group, a (*Z*)-octodec-11-enyl group, a (9*Z*, 12*Z*)-octodec-9,12-dienyl group, a (9*Z*, 12*Z*, 15*Z*)-octodec-9,12,15-trienyl group, a (8*Z*, 11*Z*, 14*Z*)-octodec-8,11,14-trienyl group, a (*Z*)-eicos-11-enyl group, a (11*Z*, 14*Z*)-eicos-11,14-dienyl group, a (*Z*)-nonadec-10-enyl group, a (10*Z*, 13*Z*)-nonadec-10,13-dienyl group, a 2,6,10-trimethylundec-1,5,9-trienyl group, a 3,7,11-trimethyldodec-2,6,10-trienyl group, and a 3,7,11,15-tetramethylhexadec-2-enyltridecyl group, and most preferably selected from the group consisting of a dodecyl group, a tridecyl group, a tetradecyl group, a pentadecyl group, a hexadecyl group, a heptadecyl group, and an octadecyl group.

In one specific embodiment of the aforementioned PEGylated lipid of the present invention, R₁ and R₂ are each independently a branched alkyl group, a branched alkenyl group, or a branched alkynyl group, and each independently represented as 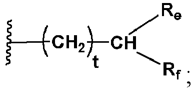 wherein, Rₑ and R_{f} are each independently selected from the group consisting of a C₁₋₁₅ alkyl group, a C₂₋₁₅ alkenyl group, and a C₂₋₁₅ alkynyl group, more preferably the group consisting of a methyl group, an ethyl group, a propyl group, a butyl group, a pentyl group, a hexyl group, a heptyl group, an octyl group, a nonyl group, a decyl group, an undecyl group, a dodecyl group, a tridecyl group, a tetradecyl group, a vinyl group, a propenyl group, an allyl group, a butenyl group, an enyl butyl group, a pentenyl group, an enyl pentyl group, a hexenyl group, an enyl hexyl group, a heptenyl group, an enyl heptyl group, an octenyl group, an enyl octyl group, a nonenyl group, an enyl nonyl group, a decenyl group, an enyl decyl group, an ethynyl group, a propynyl group, a propargyl group, a butynyl group, an ynyl butyl group, a pentynyl group, an ynyl pentyl group, a hexynyl group, an ynyl hexyl group, a heptynyl group, an ynyl heptyl group, an octynyl group, an ynyl octyl group, a nonynyl group, an ynyl nonyl group, a decynyl group, and an ynyl decyl group, and more preferably the group consisting of a methyl group, an ethyl group, a propyl group, a butyl group, a pentyl group, a hexyl group, a heptyl group, an octyl group, a nonyl group, and a decyl group. R₁ and R₂ are further preferably each independently selected from the group consisting of the following structures: 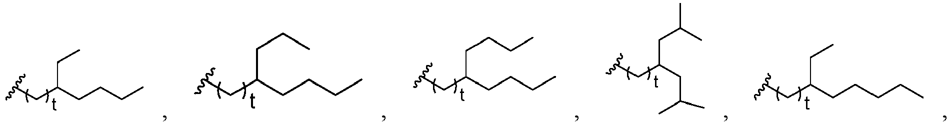 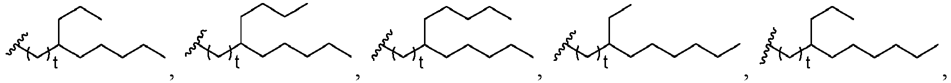 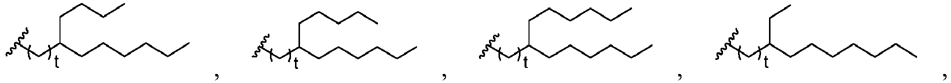 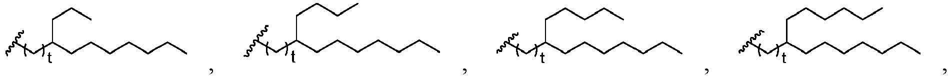 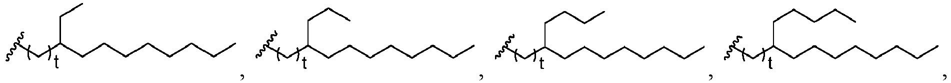 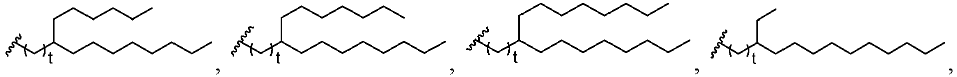 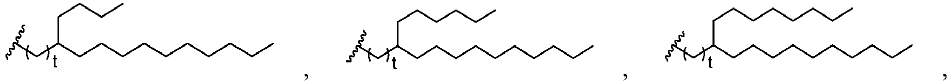 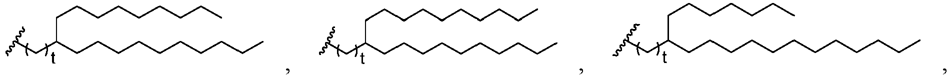 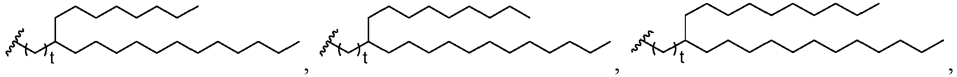 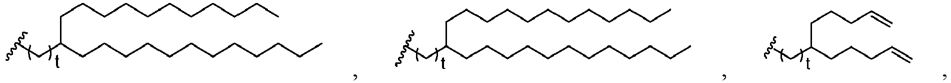 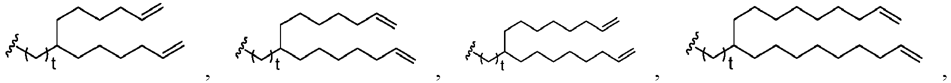 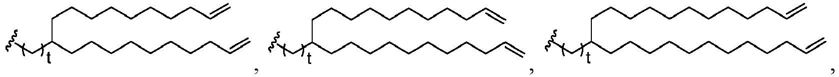 wherein t is an integer from 0 to 12.

### 1.1.5. R

In the present invention, R is a hydrogen atom, -R_{d}, -OR_{d}, -NR_{d}R_{d}, -SR_{d}, -C(=O)R_{d}, - C(=O)OR_{d}, -OC(=O)R_{d}, -OC(=O)R is a hydrogen atom, -R_{d}, -OR_{d}, -NR_{d}R_{d}, -SR_{d}, -C(=O)R_{d}, -C(=O)OR_{d}, -OC(=O)R_{d}, -OC(=O)OR_{d}, or 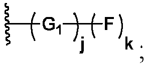 wherein, R_{d} is independently, at each occurrence, a C₁₋₁₂ alkyl group, G₁ is a (k+1)-valent end-branching group, j is 0 or 1, F contains functional group R₀₁, when j is 0, G₁ is absent, when j is 1, G₁ protrudes F with the number of k, k is an integer from 2 to 8; and when L₃ is -C(=O)CH₂-, R is not a hydrogen atom, an alkyl group, an alkoxy group, or a hydroxyl group.

### 1.1.5.1. R_{d}

In the present invention, R_{d} is, at each occurrence, independently a C₁₋₁₂ alkyl group.

In one specific embodiment of the present invention, R_{d} is preferably a C₁₋₈ alkyl group; specifically, R_{d} is preferably a methyl group, an ethyl group, a propyl group, a butyl group, a pentyl group, a hexyl group, a heptyl group, or an octyl group.

### 1.1.5.2. G₁

In the present invention, j is 0 or 1; when j is 0, G₁ is absent; when j is 1, G₁ exists as a (k+1)-valent terminal branching group and leads to k F containing functional groups, and has k functional groups F, wherein k is an integer from 2 to 8, and preferably 2, 3 or 4.

In one specific embodiment of the present invention, when G₁ is preferably a branching group with a valence of three or more, G₁ is preferably a trivalent or tetravalent branching group; G₁ is preferably a trivalent branching group, more preferably a trivalent branching group of glycerol or amino acid residues.

In the present invention, when a terminal bifunctionalization is performed, G₁ is preferably derived from the compounds consisting of alcohols, thiols, primary amines, secondary amines, sulfonates, and halide compounds wherein each contains two free or protected hydroxyl groups (e.g., triethanolamine *p*-toluenesulfonate, glycerol monomercaptoacetate, 3,4-dihydroxy-2'-chloro-acetophenone, and hydroxyl-protected forms thereof), or the compounds consisting of alcohols, thiols, primary amines, secondary amines, sulfonates, and halide compounds wherein each contains two free or protected mercapto groups (e.g., dimercaprol and mercapto-protected forms thereof), or the compounds consisting of alcohols, thiols, primary amines, secondary amines, sulfonates, and halide compounds where each contains two primary amino groups, two secondary amino groups, two protected primary amino groups, or two protected secondary amino groups, etc. Wherein, the compounds include alcohols containing two primary amino groups (e.g., 1,3-diamino-2-propanol), aldehydes containing an epoxy group, alcohols containing an epoxy group (e.g., 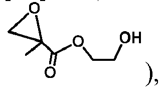 sulfonates containing an epoxy group, halides containing an epoxy group, and compounds containing one epoxy group and another reactive group. The terminal bifunctionalization can be obtained by Michael addition reactions of primary amines and two molecules of acrylic acid esters. The compound containing two terminal mercapto groups can be obtained through reduction and ring-opening reactions of the disulfide bond of compound containing lipoic acid as terminal protecting group.

In the present invention, when a terminal trifunctionalization is performed, G₁ is preferably derived from a tetrafunctionalized small molecule (htetraSM) which contains three hydroxyl groups and one different kind of reactive group, including but not limited to *N-*tris(hydroxymethyl)methyl-2-aminoethanesulfonic acid, 3-[tris(hydroxymethyl)methylamino]-1-propanesulfonic acid, methyl 6-O-tosyl-α-D-glucopyranoside, 2-(bromomethyl)-2-(hydroxymethyl)-1,3-propanediol, tris(hydroxymethyl)aminomethane, 2-amino-1,3,4-octadecanetriol, 3-aminopropylsilanetriol, 4-(2-amino-1-hydroxylethyl)-1,2-benzenediol, 4-[1-hydroxy-2-(isopropylamino)ethyl]-1,2-benzenediol, 3,4-dihydroxy-α-(methylaminomethyl)benzyl alcohol, 2,5-anhydro-1-azido-1-deoxy-D-glucitol, 2,3,4-trihydroxybutanal (L-erythrose, D-erythrose, L-(+)-threose and D-(+)-threose), 2,3,4-trihydroxybenzaldehyde, 3,4,5-trihydroxybenzaldehyde, *N-*[tris(hydroxymethyl)methyl]glycine, 2,3,4-trihydroxybutyric acid (including but not limited to erythorbic acid and threonic acid), 2,4,6-trihydroxybenzoic acid, shikimic acid, 3,4,5-trihydroxybenzoic acid, 2,3,4-trihydroxybenzoic acid, arjunolic acid, 1,4,7-tris(tert-butoxycarbonyl)-1,4,7,10-tetraazacyclododecane, tri-(t-butoxycarbonyl)spermine, 1,4,7-tris(tert-butoxycarbonyl)-1,4,7,10-tetraazacyclododecane, etc., and any hydroxyl-protected form thereof. Also included herein is a group consisting of citric acid, laricic acid, N-(2-hydroxyethyl)ethylenediamine-triacetic acid, pentaerythritol triacrylate, aminomethanetrispropionic acid, tri(tert-butyl) aminomethanetrispropionate, etc. Also included herein is the tetravalent silicon-atom branching center obtained via the terminal branching reaction based on alkenyl, trichlorosilane and allylmagnesium chloride, referring to the literature "Macromolecules, Vol. 33, No. 12, 2000". Also included herein are trifunctionalized small molecules such as 1,4,7-tris(t-butoxycarbonylmethyl)-1,4,7,10-azacyclotetradecane (NOTA), which require an excess amount in the reaction.

### 1.1.5.3. F containing functional groups

In one specific embodiment of the present invention, F is preferably -(Z₂)_{q}-(Z₁)_{q1}-R₀₁, wherein, q and q1 are each independently 0 or 1; Z₁ and Z₂ are each independently a divalent linking group; R₀₁ is a functional group capable of interreacting with bio-related substances.

In one specific embodiment of the present invention, Z₁ and Z₂ are more preferably each independently selected from the group consisting of -L₄-, -L₄-Z-, -Z-L₄-, -Z-L₄-Z-, -L₄-Z-L₅-, -Z-L₄-Z-L₅-, and -L₄-Z-L₅-Z-, wherein, t is an integer from 1 to 12.

In a more specific embodiment of the present invention, R₀₁ is preferably selected from the group consisting of a functional group, a variant form of a functional group, a therapeutic targeting functional group, and a fluorescent functional group; wherein, said variant form includes precursors of functional groups, active forms with functional groups as precursors, substituted active forms, protected forms, and deprotected forms; wherein, said precursors of functional groups refer to structures which can be transformed into the functional group through at least one process selected from the group consisting of oxidation, reduction, hydration, dehydration, electronic rearrangement, structural rearrangement, salt complexation and decomplexation, ionization, protonation, and deprotonation; wherein, the variant forms of functional groups refer to the reactive forms of functional groups after at least one chemical change process selected from the group consisting of oxidation, reduction, hydration, dehydration, electronic rearrangement, structural rearrangement, salt complexation and decomplexation, ionization, protonation, deprotonation, substitution, and deprotection, or refer to the non-reactive forms after protection; said R₀₁ is more preferably a functional group selected from the group consisting of the functional groups in the following Classes A to H and variant forms thereof, or from the group consisting of the functional groups in the following Classes I to J:
Class A: active ester groups and analogs thereof; wherein, an active ester group includes a succinimidyl active ester group, a *p*-nitrophenyl active ester group, an *o*-nitrophenyl active ester group, a benzotriazole active ester group, a 1,3,5-trichlorophenyl active ester group, a 1,3,5-trifluorophenyl active ester group, a pentafluorophenyl active ester group, and an iminazole active ester group; wherein, analogs of active ester groups include a 2-thione-3-thiazolidine-formate group, a 2-thioxo-thiazolidine-3-carboxylate group, a 2-thione-pyrrolidine-*N*-carboxylate group, a 2-thione-pyrrolidine-*N*-formate group, a 2-thione-benzothiazole-*N*-formate group, and a 1-oxo-3-thioxoisoindoline-*N*-formate group;
Class B: a sulfonate group, a sulfinate group, a sulfonyl group, a sulfoxide group, a 1,3-disulfonyl-2-propylcarbonylphenyl group, and a (2-sulfonylmethyl)acryl group;
Class C: a hydroxylamine group, a mercapto group, a primary amino group, a secondary amino group, a halogen atom, a haloacetamide group, a tetramethylpiperidinyloxy group, a dioxapiperidinyloxy group, an ammonium salt group, a hydrazino group, a disulfide group, an ester group, thioate group, a thioester group, a carbonate group, a thiocarbonate group, a dithiocarbonate group, a trithiocarbonate group, a xanthate group, a perthiocarbonate group, a tetrathiodiester group, an *O*-carbonyl hydroxylamine group, an amide group, an imide group, a hydrazide group, a sulfonyl hydrazide group, a hydrazone group, an imine group, an enamino group, an alkynyl amine group, a urethane group, a thiourethane group, a dithiourethane group, and a protected amino group;
Class D: a carboxyl group, a sulfonic acid group, a sulfenic acid group, a hydroxamic acid group, a thiohydroxamic acid group, a xanthogenic acid group, an acyl halide group, a sulfonyl chloride group, an aldehyde group, a glyoxal group, an acetal group, a hemiacetal group, a hydrated aldehyde group, a ketone group, a ketal group, a hemiketal group, a hemiketal group, a ketal group, a hydrated ketone group, an orthoacid group, an orthoester group, a cyanate group, a thiocyanate group, an isocyanato group, an isothiocyanate group, an ester group, an oxycarbonyl halide group, an oxazolinyl group, an isoxazolinyl group, a thioaldehyde group, a thione group, a thioacetal group, a thione hydrate group, a thioketal group, a thioate group, a thioester group, a dithioester group, a thiohemiacetal group, a monothiohydrate group, a dithiohydrate group, a thiolhydrate group, a monothiocarboxylic acid group containing a thiocarbonyl group, a monothiocarboxylic acid group containing a thiol group, a dithiocarboxylic acid group, a ureido group, a thioureido group, a guanidino group and its protonated form, an amidino group and its protonated form, an anhydride group, a squaric acid group, a squarate group, a semi-squaric acid group, a semi-squarate group, a *N-*carbamoyl-3-imidazole group, a *N*-carbamoyl-3-methylimidazolium iodide group, an imino acid group, an imidate group, a nitrone group, an oxime group, and a pseudourea group;
Class E: a maleimide group, an acrylate group, a *N-*acrylamide group, a methacrylate group, a *N*-methacrylamide group, a protected maleimide group, a maleamic acid group, a 1,2,4-triazoline-3,5-dione group, a linear azo compound group, a cyclic azo compound group, a cycloalkenyl group; wherein, the cycloalkenyl group includes a cyclooctenyl group, a norbornenyl group, a 7-oxa-bicyclo[2.2.1]hept-5-en-2-yl, a bicycloheptadienyl group, and a 7-oxa-bicycloheptadienyl group;
Class F: an epoxy group, an ethenyl group, a propenyl group, an alkenyl hydrocarbon group, an alkynyl group, and an alkynyl hydrocarbon group.

### Class G,

Class Ga: a cycloalkynyl group, a cycloalkynyl heterohydrocarbon group, a linear conjugated dienyl group, a cyclic conjugated dienyl group, a heterosubstituted cyclic conjugated dienyl group, and a 1,2,4,5-tetrazinyl group;
Class Gb: an azide group, a nitrile oxide group, a cyano group, an isocyano group, an aldoxime group, a diazo group, a diazonium group, an azo oxide group, a nitrilimine group, a *N*-aldimine oxide group, a tetrazole group, a 4-acetyl-2-methoxy-5-nitrophenoxy group and its diazo form, and other functional groups which can undergo 1,3-dipolar cycloaddition reactions;
Class H: a hydroxyl group, a protected hydroxyl group, a siloxy group, a protected dihydroxyl group, a trihydroxysilyl group, a protected trihydroxysilyl group; wherein, the hydroxyl group includes an alcoholic hydroxyl group, a phenolic hydroxyl group, an enolic hydroxyl group, and a hemiacetal hydroxyl group;
Class I: targeting groups and pharmaceutically acceptable salts thereof;
Class J: fluorescent groups, including a fluorescein group, a rhodamine group, an anthracenyl group, a pyrenyl group, a coumarin group, a fluorescent yellow 3G group, a carbazole group, an imidazole group, an indole group, an alizarin violet group, and residues of functional derivatives thereof.

Further, R₀₁ is preferably selected from the group consisting of the functional groups in the following Classes A to H and variant forms thereof, and the functional derivatives in Classes I to J; said variant form is a precursor of functional group, an active form with functional group as precursor, a substituted active form, a protected form, or an unprotected form:
Class A: 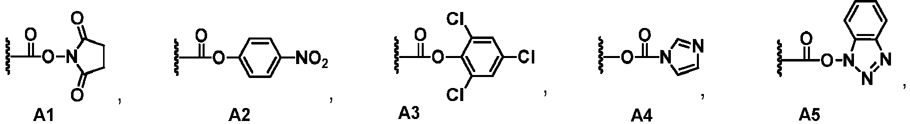 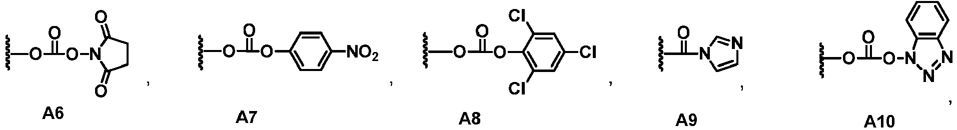 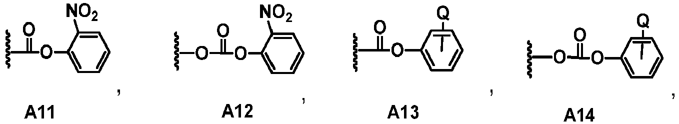 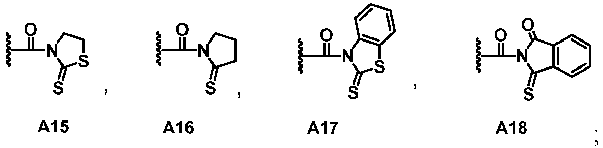
or Class B: 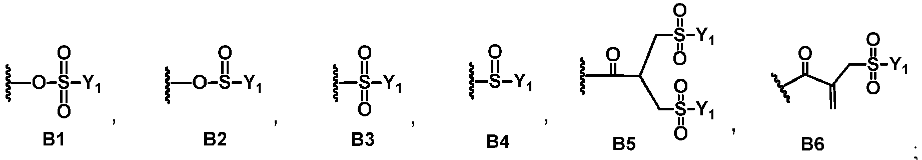
or Class C: 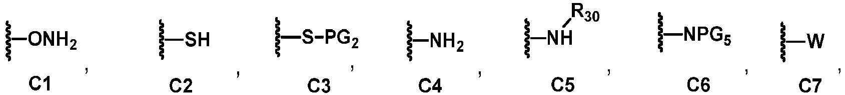 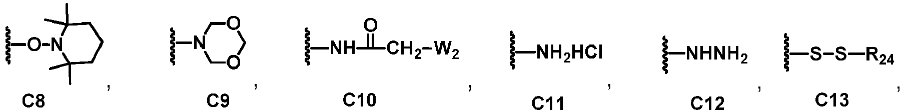 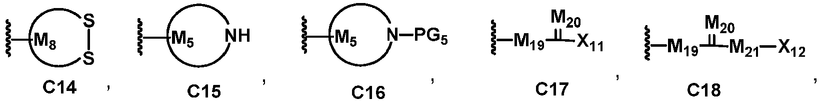 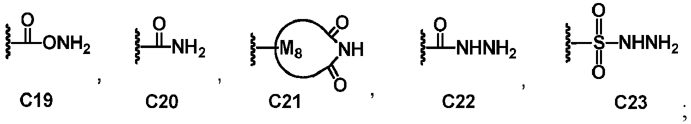
or Class D: 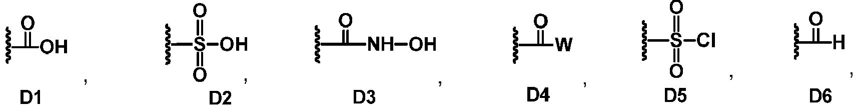 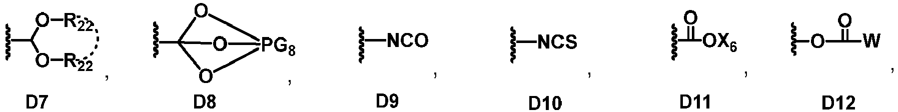 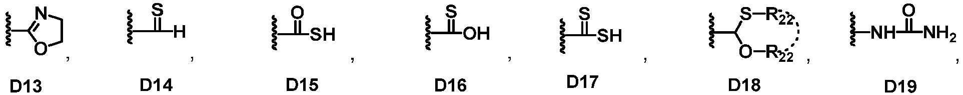 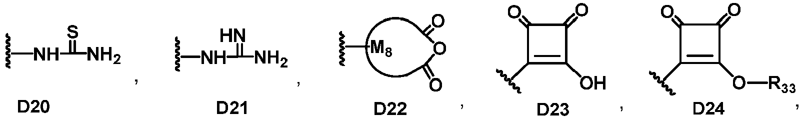 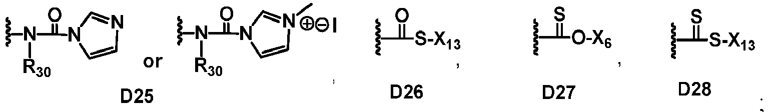
or Class E: 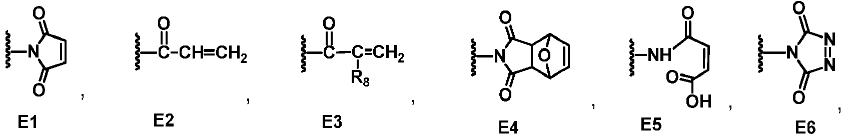 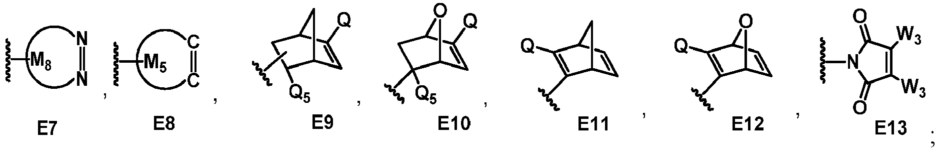
or Class F: 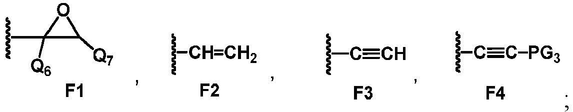
or Class G:
   Class Ga: 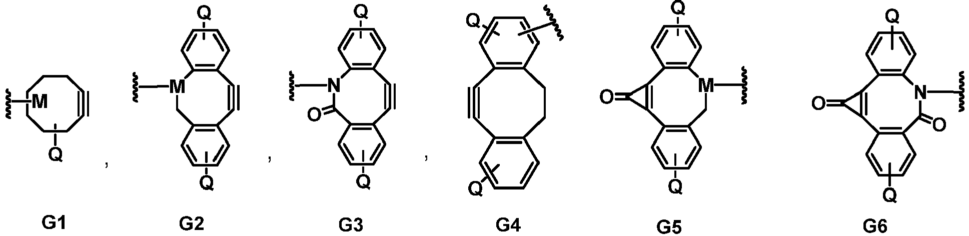 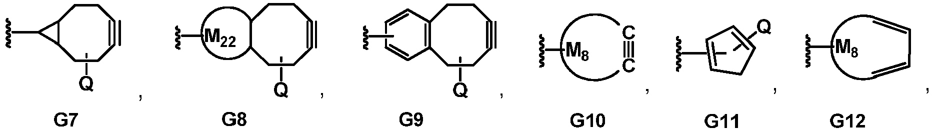 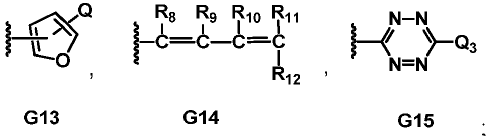
   or Class Gb: 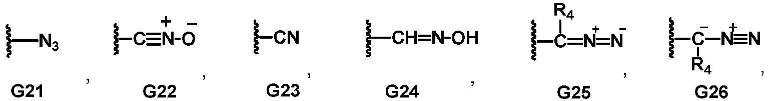 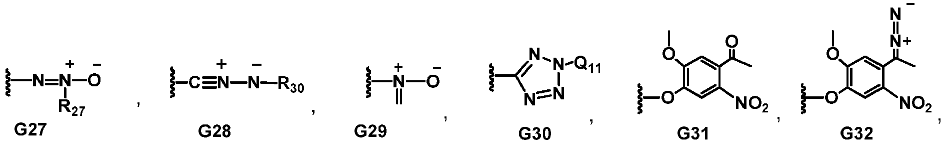
   or Class H: 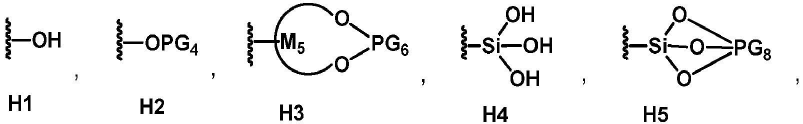 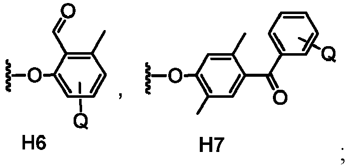
   or Class I: 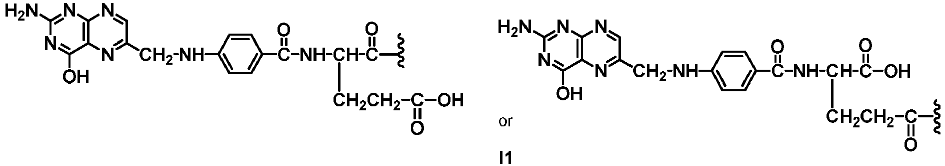 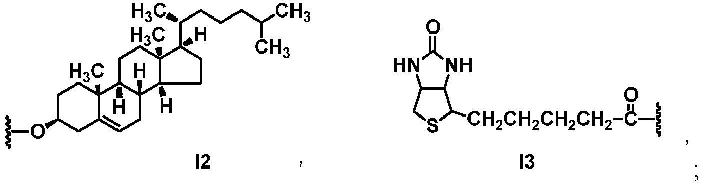
   or Group J: 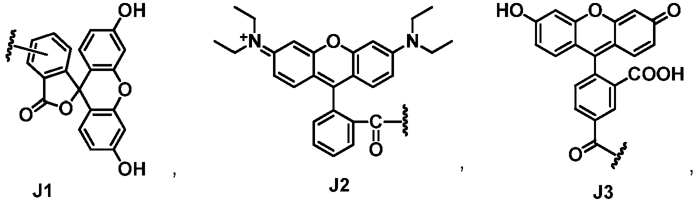 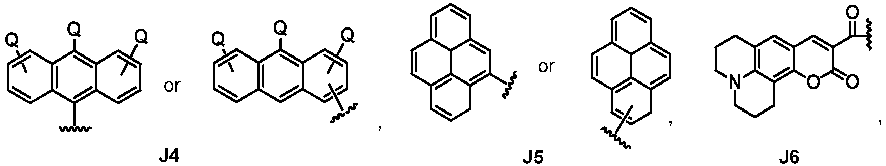 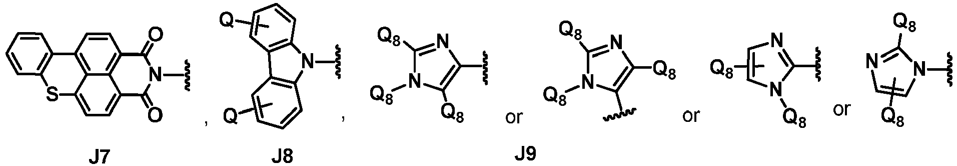 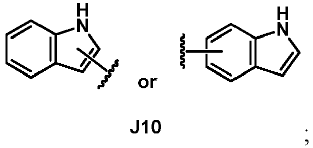
      wherein, Ms is a ring-constituting atom, selected from the group consisting of a carbon atom, a nitrogen atom, a phosphorus atom, and a silicon atom; the cyclic structure containing Ms is a 3- to 50-membered ring, preferably a 3- to 32-membered ring, more preferably a 3- to 18-membered ring, and more preferably a 5- to 18-membered ring; said cyclic structure is preferably selected from the group consisting of cyclohexane, furanose ring, pyranose ring, benzene, tetrahydrofuran, pyrrolidine, thiazolidine, cyclohexene, tetrahydropyran, piperidine, 1,4-dioxane, pyridine, pyridazine, pyrimidine, pyrazine, 1,3,5-triazine, 1,4,7-triazacyclononane, cyclotripeptide, indene, indane, indole, isoindole, purine, naphthalene, dihydroanthracene, xanthene, thioxanthene, dihydrophenanthrene, 10,11-dihydro-5H-dibenzo[a,d]cycloheptane, dibenzocycloheptene, 5-dibenzosuberenone, quinoline, isoquinoline, fluorene, carbazole, iminodibenzyl, acenaphthene, dibenzocyclooctyne, azadibenzocyclooctyne, substituted forms thereof, and heterosubstituted forms thereof;
      wherein, Yi is a leaving group connected to a sulfonyl group, a sulfinyl group, an oxysulfonyl group, or an oxysulfinyl group, which is selected from the group consisting of a methyl group, an ethyl group, an *n*-propyl group, an isopropyl group, a butyl group, a pentyl group, a hexyl group, a heptyl group, an octyl group, a nonyl group, a decyl group, a vinyl group, a phenyl group, a benzyl group, a *p*-methylphenyl group, a 4-(trifluoromethoxy)phenyl group, a trifluoromethyl group, and a 2,2,2-trifluoroethyl group;
      wherein, W is F, Cl, Br or I;
      wherein, W₂ is F, Cl, Br or I;
      wherein, W₃ is a leaving group, selected from the group consisting of F, Cl, Br, I and PhS;
      wherein, 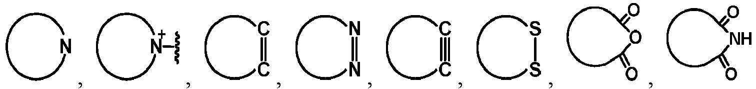 and 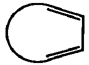 are cyclic structures, of which the skeletons contain a nitrogen atom, a nitrogenium ion, a double bond, an azo bond, a triple bond, a disulfide bond, an anhydride group, an imide group, and a dienylene, respectively; said cyclic structures are selected from the group consisting of a carbocycle, a heterocycle, a benzoheterocycle, a substituted carbocycle, a substituted heterocycle, and a substituted benzoheterocycle;
      wherein, M is a carbon atom, a nitrogen atom, a phosphorus atom, or a silicon atom of the ring skeleton;
      wherein, Ms is a carbon atom, a nitrogen atom, a phosphorus atom, or a silicon atom of the ring skeleton; the number of ring-constituting atoms of the cyclic structure containing Ms is from 4 to 50, preferably from 4 to 32, and more preferably from 5 to 32;
      wherein, M₂₂ is a carbon atom, a nitrogen atom, a phosphorus atom, or a silicon atom of an alicycle or heteroalicycle; the number of ring-constituting atoms of the cyclic structure containing M₂₂ is 4, 5, 6, 7, or 8;
      wherein, R₂₂ is a terminal group or a divalent linking group connected to an oxygen atom or a sulfur atom, which is any atom or group selected from the group consisting of hydrogen atom, R₂₁, and R₃₃;
      wherein, R₂₁ is a divalent linking group and participates in forming a ring; R₂₁ is selected from the group consisting of a C₁₋₂₀ hydrocarbylene group, a divalent C₁₋₂₀ heterohydrocarbon group, a substituted C₁₋₂₀ hydrocarbylene group, a substituted divalent C₁₋₂₀ heterohydrocarbon group, and combinations of any two or three thereof; R₂₁ is preferably selected from the group consisting of a methylene group, an ethylene group, a propylene group, a butylene group, a pentylene group, a hexylene group, a heptylene group, an octylene group, a nonylene group, a decylene group, a 1,2-phenylene group, a benzylene group, a C₁₋₂₀ oxa-alkylene group, a C₁₋₂₀ thia-alkylene group, a C₁₋₂₀ aza-alkylene group, an aromatic azahydrocarbyl group, substituted forms thereof, and combinations of any two or more identical, different, or substituted forms thereof;
      wherein, R₃₃ is a terminal group connected to an oxygen group or a sulfur group, which is selected from the group consisting of a C₁₋₂₀ hydrocarbon group, a C₁₋₂₀ heterohydrocarbon group, a C₁₋₂₀ substituted hydrocarbon group, and a C₁₋₂₀ substituted heterohydrocarbon group, and preferably the group consisting of a methyl group, an ethyl group, an *n*-propyl group, an isopropyl group, a butyl group, a pentyl group, a hexyl group, a heptyl group, an octyl group, a nonyl group, a decyl group, a benzyl group, an allyl group, and substituted forms thereof;
      wherein, R₄ is a hydrogen atom, substituent atom, or substituent group connected to the carbon atom of a structure with the formula of -(R₄)C=N⁺=N⁻ or -(R₄)C⁻-N⁺≡N, and preferably selected from the group consisting of a hydrogen atom, a methyl group, an ethyl group, an *n*-propyl group, an isopropyl group, a butyl group, a pentyl group, a hexyl group, a heptyl group, an octyl group, a nonyl group, a decyl group, an allyl group, a propenyl group, an ethenyl group, a phenyl group, a methylphenyl group, a butylphenyl group, and a benzyl group;
      wherein, R₈, R₉, R₁₀, R₁₁ and R₁₂ are each independently a hydrogen atom, a substituent atom, or a substituent group connected to a double bond (-C=C-); R₈, R₉, R₁₀, R₁₁, and R₁₂ can be the same or different in one molecule; R₈, R₉, R₁₀, R₁₁ and R₁₂ are each independently selected from the group consisting of a hydrogen atom, a fluorine atom, and a methyl group; in the class E3, R₈ is preferably a methyl group;
      wherein, R₂₄ is a terminal group connected to a disulfide bond, which is selected from the group consisting of a C₁₋₂₀ alkyl group, an aryl group, and a heterosubstituted phenyl group;
      wherein, R₂₇ is a substituent connected to an azo group, which is selected from the group consisting of a phenyl group, a substituted phenyl group and a heterosubstituted phenyl group;
      wherein, R₃₀ is a hydrocarbon group, selected from the group consisting of a C₁₋₂₀ alkyl group, a benzyl group, and a benzyl group with hydrogen atoms at the benzene ring replaced by C₁₋₂₀ hydrocarbon groups;
      wherein, M₁₉, M₂₀, and M₂₁ are each independently an oxygen atom or a sulfur atom, and in one molecule, M₁₉, M₂₀, and M₂₁ can be the same or different;
      wherein, X₆ is a terminal group connected to an oxygen atom of an ester group, which is a hydroxyl protecting group or the group LG₄; LG₄ is selected from the group consisting of a C₁₋₂₀ alkyl group, an aryl group, an aralkyl group, a C₁₋₂₀ heteroalkyl group, a heteroaryl group, a heteroaralkyl group, a C₁₋₂₀ alkylcarbonyl group, an arylcarbonyl group, an arylalkylcarbonyl group, a C₁₋₂₀ heteroalkylcarbonyl group, a heteroarylcarbonyl group, a heteroarylalkylcarbonyl group, a C₁₋₂₀ alkoxycarbonyl group, an aryloxycarbonyl group, an arylalkoxycarbonyl group, a C₁₋₂₀ (alkylthio)carbonyl group, an (arylthio)carbonyl group, an (arylalkylthio)carbonyl group, a C₁₋₂₀ alkylaminocarbonyl group, an arylaminocarbonyl group, an arylalkylaminocarbonyl group, a C₁₋₂₀ heteroalkoxycarbonyl group, a heteroaryloxycarbonyl group, a heteroarylalkoxycarbonyl group, a C₁₋₂₀ hetero(alkylthio)carbonyl group, a hetero(arylthio)carbonyl group, a hetero(arylalkylthio)carbonyl group, a C₁₋₂₀ heteroalkylaminocarbonyl group, a heteroarylaminocarbonyl group, a heteroarylalkylaminocarbonyl group, a C₁₋₂₀ (alkyl)thiocarbonyl group, an (aryl)thiocarbonyl group, an (arylalkyl)thiocarbonyl group, a C₁₋₂₀ hetero(alkyl)thiocarbonyl group, a hetero(aryl)thiocarbonyl group, a hetero(arylalkyl)thiocarbonyl group, a C₁₋₂₀ alkoxy-thiocarbonyl group, an aryloxy-thiocarbonyl group, an arylalkoxy-thiocarbonyl group, a C₁₋₂₀ (alkylthio)thiocarbonyl group, an (arylthio)thiocarbonyl group, an (arylalkylthio)thiocarbonyl group, a C₁₋₂₀ alkylaminothiocarbonyl group, an arylaminothiocarbonyl group, an arylalkylaminothiocarbonyl group, a C₁₋₂₀ heteroalkyloxy-thiocarbonyl group, a heteroaryloxy-thiocarbonyl group, a heteroarylalkoxy-thiocarbonyl group, a C₁₋₂₀ hetero(alkylthio)thiocarbonyl group, a hetero(arylthio)thiocarbonyl group, a hetero(arylalkylthio)thiocarbonyl group, a C₁₋₂₀ heteroalkylaminothiocarbonyl group, a heteroarylaminothiocarbonyl group, a heteroarylalkylaminothiocarbonyl group, and substituted forms thereof; wherein, the substituent atom or substituent group is a fluorine atom, an alkoxy group, or a nitro group;
      wherein, X₁₁ is a terminal group connected to a carbonyl group or a thiocarbonyl group, selected from C₁₋₂₀ alkyl groups;
      wherein, X₁₂ is a terminal group connected to a carbonate group or a thiocarbonate group, selected from C₁₋₂₀ hydrocarbon groups;
      wherein, X₁₃ is a terminal group connected to a sulfur group, selected from the group consisting of a mercapto protecting group and the group LG₂;
      wherein, LG₂ is selected from the group consisting of a methyl group, an ethyl group, an *n*-propyl group, an isopropyl group, a *t*-butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an eicosyl group, an allyl group, a trityl group, a phenyl group, a benzyl group, a methylbenzyl group, a nitrobenzyl group, a *t*-butylthio group, a benzylthio group, a 2-pyridylthio group, an acetyl group, a benzoyl group, a methoxycarbonyl group, an ethoxycarbonyl group, a *t*-butoxycarbonyl group, a phenoxycarbonyl group, a benzyloxycarbonyl group, a (methylthio)carbonyl group, an (ethylthio)carbonyl group, a (*t*-butylthio)carbonyl group, a (phenylthio)carbonyl group, a (benzylthio)carbonyl group, a 2-pyridylcarbonyl group, a methylaminocarbonyl group, an ethylaminocarbonyl group, a *t*-butylaminocarbonyl group, a benzylaminocarbonyl group, an ethyl-thiocarbonyl group, a phenyl-methylthiocarbonyl group, a methoxy-thiocarbonyl group, an ethoxy-thiocarbonyl group, a *t*-butyloxy-thiocarbonyl group, a phenoxy-thiocarbonyl group, a benzyloxy-thiocarbonyl group, a (methylthio)thiocarbonyl group, an (ethylthio)thiocarbonyl group, a (*t*-butylthio)thiocarbonyl group, a (phenylthio)thiocarbonyl group, a (benzylthio)thiocarbonyl group, a methylaminothiocarbonyl group, an ethylaminothiocarbonyl group, a *t*-butylaminothiocarbonyl group, a benzylaminothiocarbonyl group, a C₁₋₁₀ halohydrocarbon group, a trifluoroacetyl group, a nitrophenyl group, and substituted forms thereof; wherein, the substituent atom or substituent group is a fluorine atom, an alkoxy group or a nitro group;
      wherein, Q is an atom or a substituent that can promote the inductive or conjugate effect of electrons of unsaturated bonds; when Q is connected to the ring, the number of Q can be one or greater than one; when the number of Q is greater than one, they can be of the same structure or a combination of two or more different structures; when Q is a substituent group, it can have a linear structure, a branched structure bearing pendant groups, or a ring-containing structure;
      wherein, Q₃ is a hydrogen atom or a group that can promote the inductive or conjugate effect of electrons of unsaturated bonds, which can be any atom or group selected from the group consisting of a hydrogen atom, a fluorine atom, a chlorine atom, a bromine atom, an iodine atom, a methyl group, an ethyl group, an *n*-propyl group, an isopropyl group, a butyl group, a pentyl group, a hexyl group, a heptyl group, an octyl group, a nonyl group, a decyl group, an ethenyl group, a propenyl group, an allyl group, a propynyl group, a propargyl group, a cyclopropyl group, a cyclopropenyl group, a phenyl group, a benzyl group, a butylphenyl group, a *p*-methylphenyl group, a *p*-nitrophenyl group, an *o*-nitrophenyl group, a *p*-methoxyphenyl group, an azaphenyl group, a methoxy group, an ethoxy group, a phenoxy group, a benzyloxy group, a methylthio group, an ethylthio group, a phenylthio group, a benzylthio group, a trifluoromethyl group, a 2,2,2-trifluoroethyl group, and substituted forms thereof;
      wherein, Q₅ is a hydrogen atom, a methyl group, an ethyl group, or a propyl group; when Q₅ is connected to the ring, the number of Q can be one or greater than one; when the number of Q is greater than one, they can have the same structure, or be a combination of two or more different structures;
      wherein, Q₆ is a hydrogen atom or a methyl group; Q₇ is a hydrogen atom, a methyl group, a phenyl group, or a substituted phenyl group; in one molecule, Q₆ and Q₇ can be identical or different from each other;
      wherein, Q₈ is a substituent atom or substituent group of an imidazole group, selected from the group consisting of a hydrogen atom, a methyl group, an ethyl group, a propyl group, a butyl group, and a phenyl group; the number of Q₈ can be one or greater than one; when the number of Q₈ is greater than one, they can have the same structure, or be a combination of two or more different structures;
      wherein, Q₁₁ is a substituent group connected to a nitrogen atom of a tetrazole group, selected from the group consisting of a phenyl group, a substituted phenyl group, and an azaphenyl group;
      wherein, PG₂ is a mercapto protecting group, and the protected mercapto group is represented as SPG₂ and preferably selected from the group consisting of a thioether, a disulfide, a silyl thioether, and a thiocarboxylate;
      wherein, PG₃ is an alkynyl protecting group, preferably a silyl group;
      wherein, PG₄ is a hydroxyl protecting group, and the protected hydroxyl group is represented as OPG₄ and preferably selected from the group consisting of an ether, a silyl ether, an ester, a carbonate, and a sulfonate;
      wherein, PG₅ is an amino protecting group, and the protected amino group is represented as NPG₅ and preferably selected from the group consisting of a carbamate, an amide, an imide, an *N*-alkyl amine, an *N-*aryl amine, an imine, an enamine, an imidazole, a pyrrole, and an indole;
      wherein, PG₆ is a dihydroxyl protecting group, forming a five- or six-membered cyclic acetal structure with two oxygen atoms; PG₆ is a methylene group or a substituted methylene group; wherein, the substituent of PG₆ is a hydrocarbyl substituent or a heteroatom-containing substituent, which is selected from the group consisting of a methylene group, a 1-methylmethylene group, a 1,1-dimethylmethylene group, a 1,1-cyclopentylene group, a 1,1-cyclohexylene group, a 1-phenylmethylene group, and a 3,4-dimethylphenylmethylene group;
      wherein, PG₈ is a protecting group of orthocarbonic acid or orthosilicic acid.

### 1.1.5.4. Specific examples of R

In one specific embodiment of the present invention, R preferably contains any atom or group selected from the group consisting of a hydrogen atom, an alkyl group, an alkoxy group, an alcoholic hydroxyl group, a protected alcoholic hydroxyl group, a thiol group, a protected thiol group, a carboxyl group, a protected carboxyl group, an amino group, a protected amino group, an aldehyde group, a protected aldehyde group, an ester group, a carbonate group, a urethane group, a succinimidyl group, a maleimide group, a protected maleimide group, a dimethylamino group, an alkenyl group, an alkenoate group, an azido group, an alkynyl group, a folate group, a rhodamine group, a biotinyl group, a monosaccharide group, and a polysaccharide group, and more preferably selected from the group consisting of H, -CH₃, -CH₂CH₃, -(CH₂)ₜOH, -(CH₂)ₜSH, -OCH₃, -OCH₂CH₃, - (CH₂)ₜNH₂, -(CH₂)ₜC(=O)OH, -C(=O)(CH₂)ₜC(=O)OH, -C(=O)CH₃, -(CH₂)ₜN₃, - C(=O)CH₂CH₃, -C(=O)OCH₃, -OC(=O)OCH₃, -C(=O)OCH₂CH₃, -OC(=O)OCH₂CH₃, - (CH₂)ₜN(CH₃)₂, -(CH₂)ₜN(CH₂CH₃)₂, -(CH₂)ₜCHO, 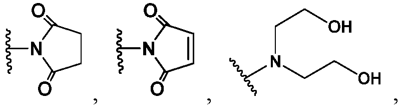 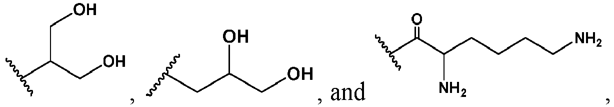 wherein, t is an integer from 0 to 12.

### 1.1.6. A

In the present invention, A is -(CRₐR_{b})ₛO- or -O(CRₐR_{b})ₛ-; wherein, s is 2, 3, or 4, Rₐ and R_{b} are each independently a hydrogen atom or a C₁₋₁₂ alkyl group.

In one specific embodiment of the present invention, Rₐ and R_{b} are both hydrogen atoms, and s is 2, specifically corresponding to A being -CHzCHzO- or -OCH₂CH₂-.

In the present invention, the number-average molecular weight of a polyethylene glycol chain of a PEGylated lipid is selected from the group consisting of 900, 1000, 1500, 2000, 2500, 3000, 3350, 3500, 4000, 5000, 5500, 6000, 6500, 7000, 7500, 8000, 8500, 9000, 9500, 10000, and 11,000.

In one specific embodiment of the present invention, a polyethylene glycol chain of a PEGylated lipid is monodisperse or polydisperse.

In one specific embodiment of the present invention, a polyethylene glycol chain of a PEGylated lipid is preferably polydisperse, and the number-average degree of polymerization (n₁) is preferably an integer from approximately 20 to 100, more preferably an integer from approximately 20 to 60, and more preferably an integer from approximately 40 to 60.

In one specific embodiment of the present invention, a polyethylene glycol chain of a PEGylated lipid is preferably monodisperse, and the number of EO units is preferably from 20 to 70, more preferably from 20 to 60, more preferably selected from the group consisting of 20, 22, 24, 26, 27, 28, 29, 30, 32, 34, 36, 38, 40, 42, 44, 45, 46, 48, 50, 52, 54, 56, 58 and 60, and more preferably selected from the group consisting of approximately 44, 45, 46, 48, 50, 52, 54, 56, 58, and 60.

### 1.1.7. Examples of specific general formulas

In one specific embodiment of the present invention, the structure of PEGylated lipid of the present invention is preferably selected from the group consisting of the following structural formulas:

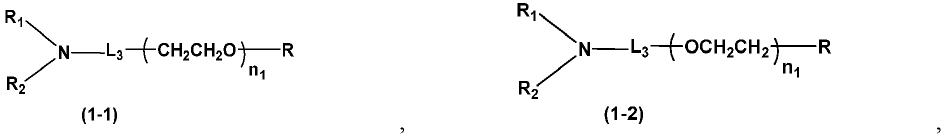

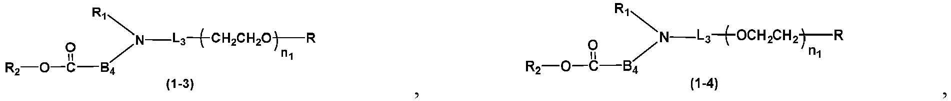

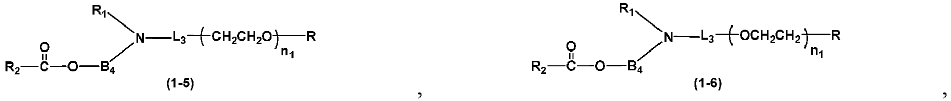

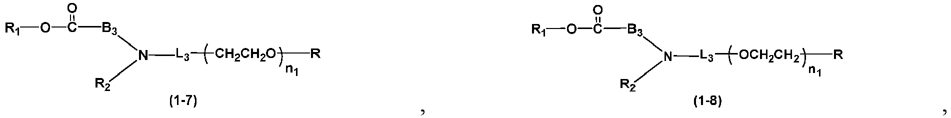

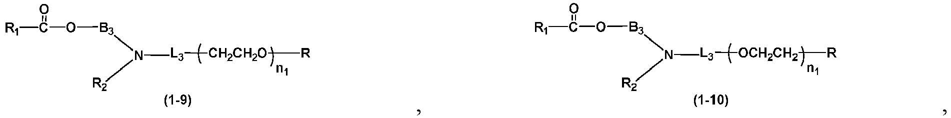

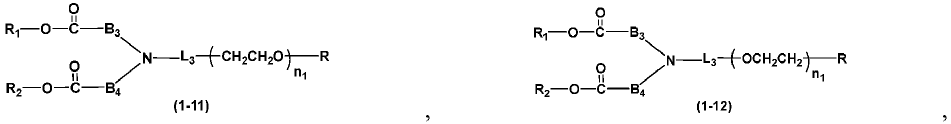

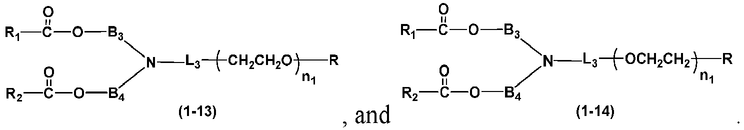

### 1.1.8. Examples of specific structures of PEGylated lipid

In some specific embodiments of the present invention, PEGylated lipids with the following structures are finally obtained, which include but are not limited to the group consisting of the following structures: 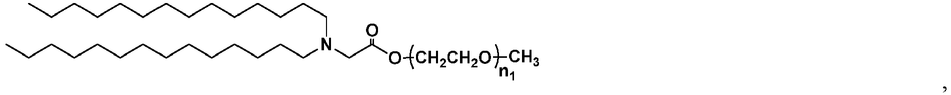 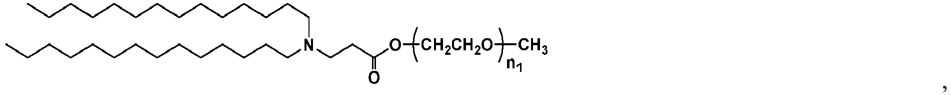 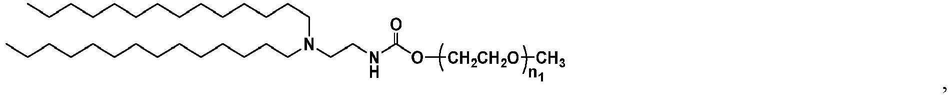 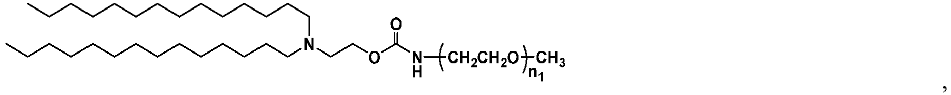 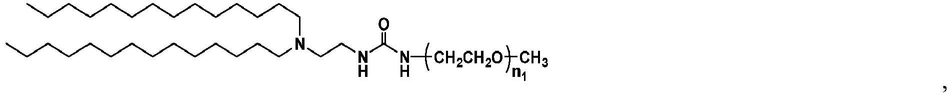 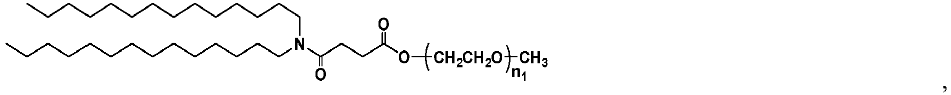 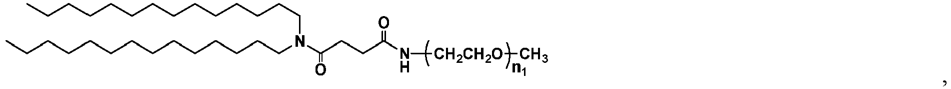 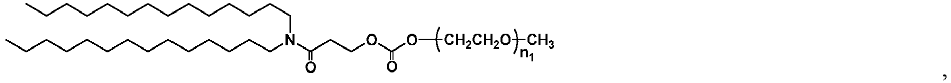 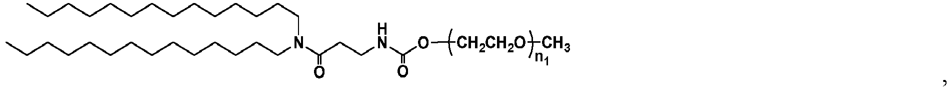 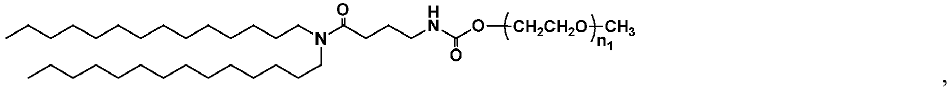 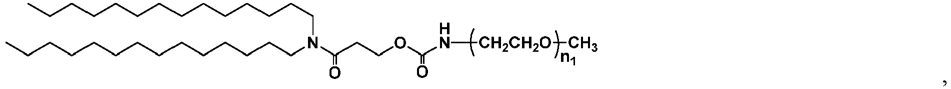 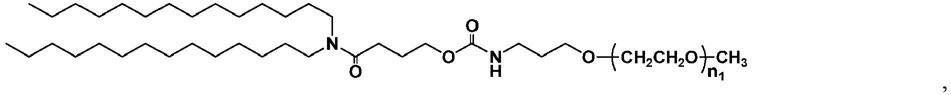 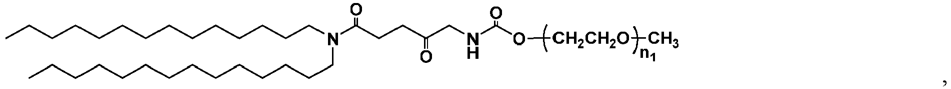 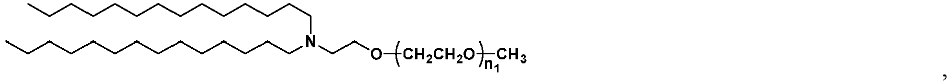 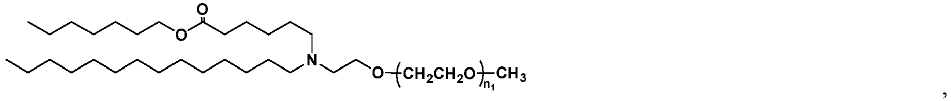 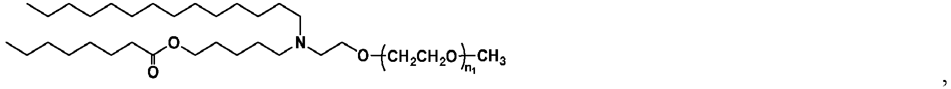 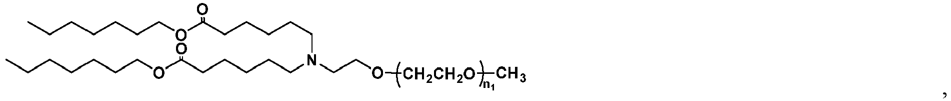 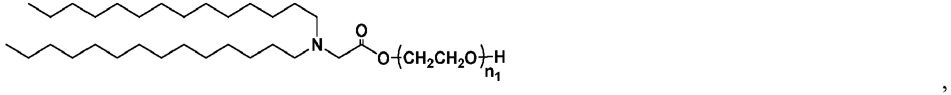 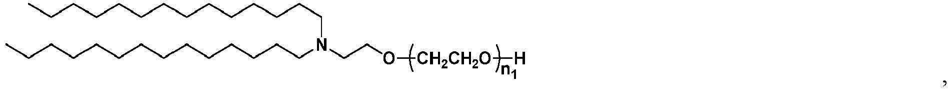 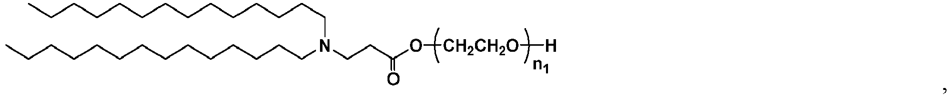 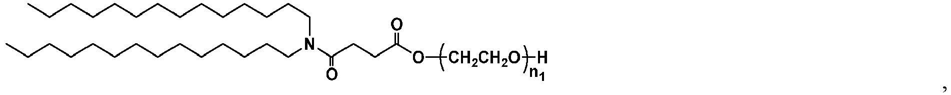 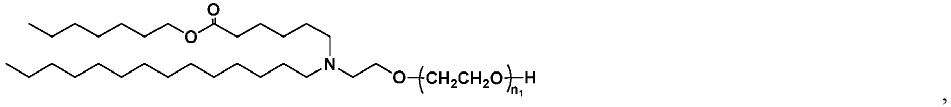 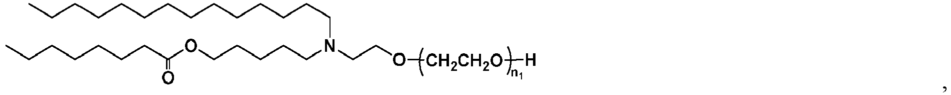 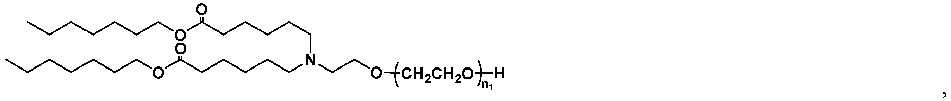 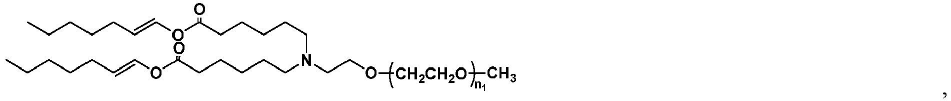 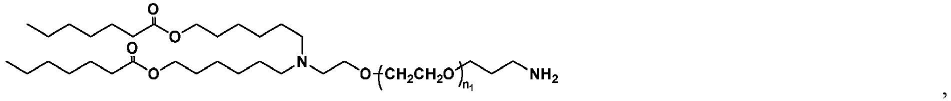 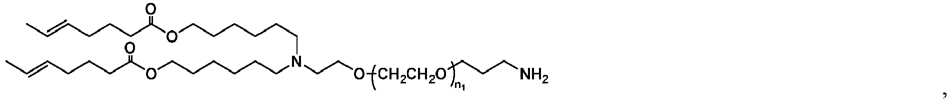 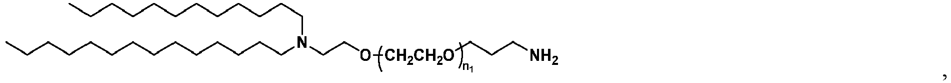 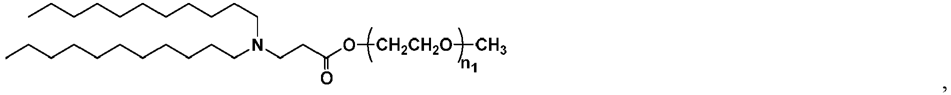 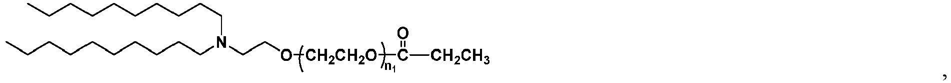 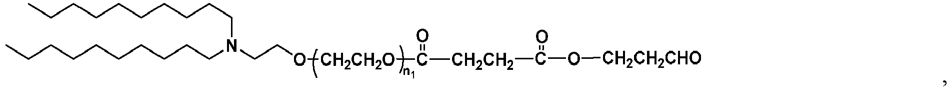 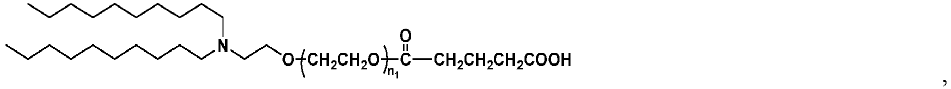 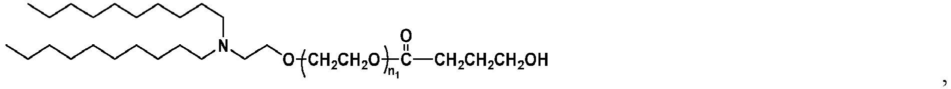 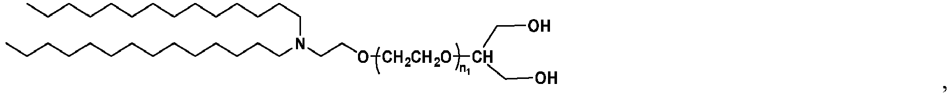 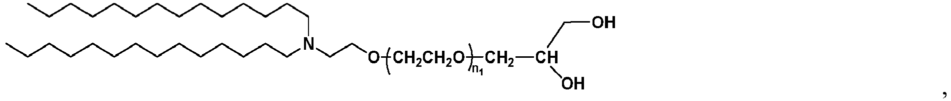 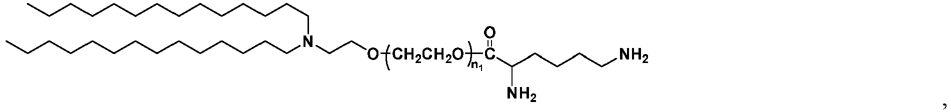 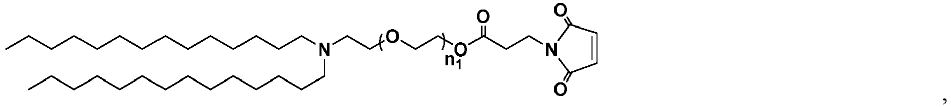 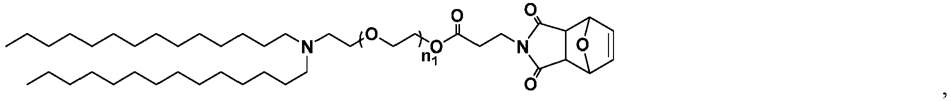 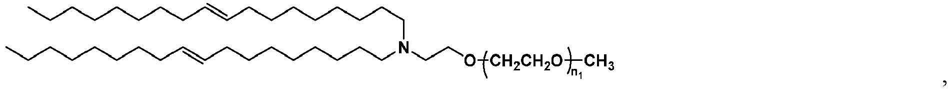 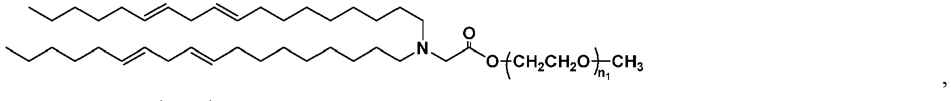 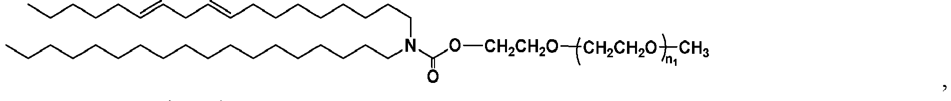 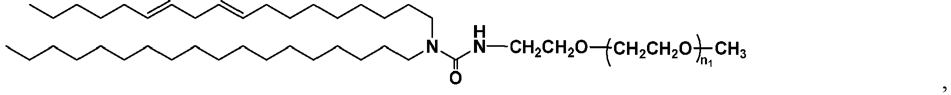 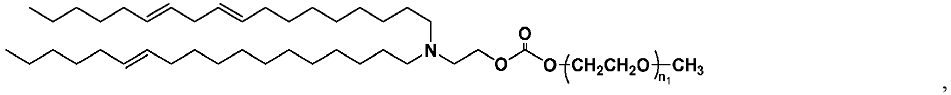 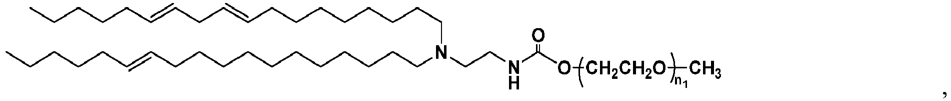 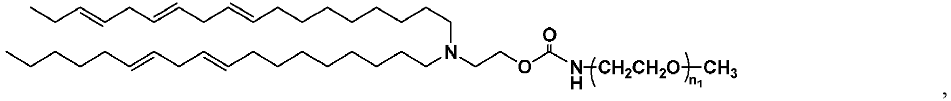 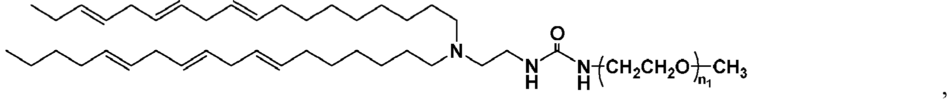 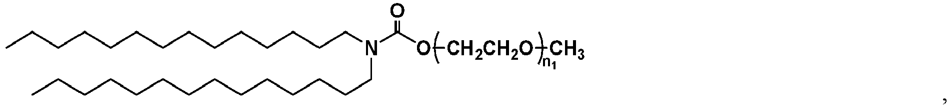 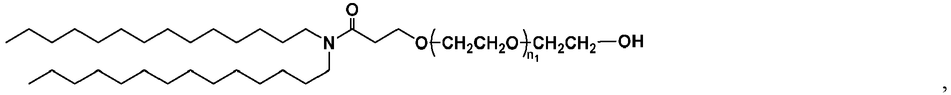 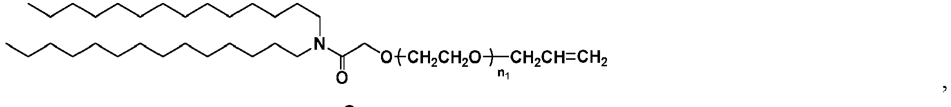 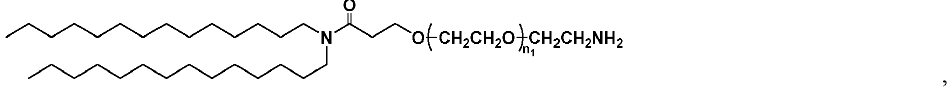 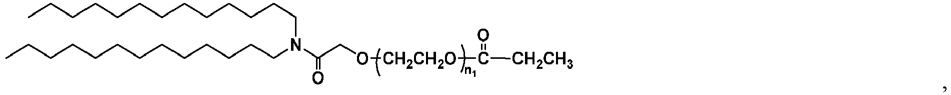 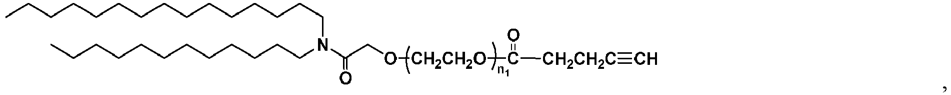 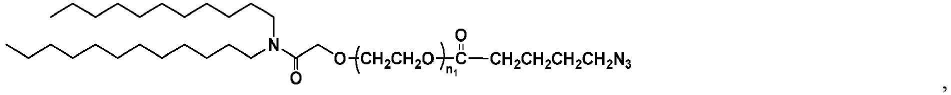 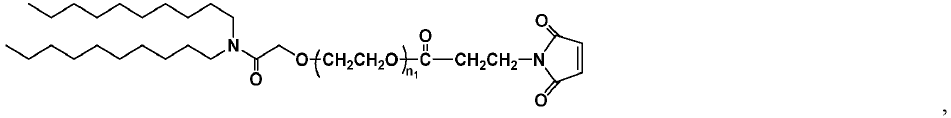 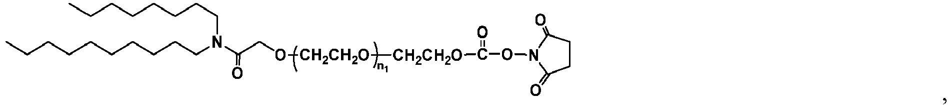 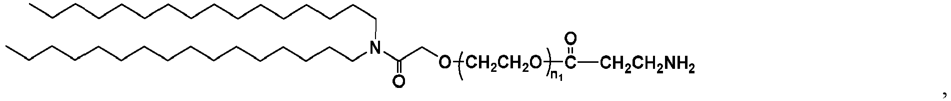 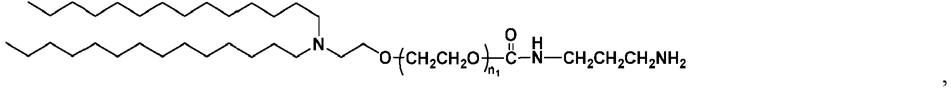 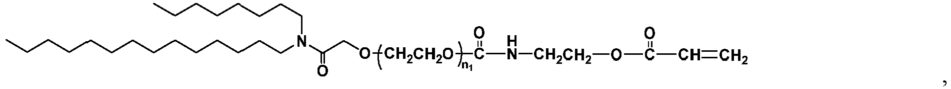 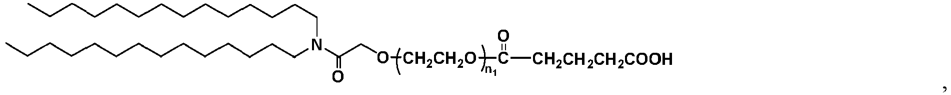 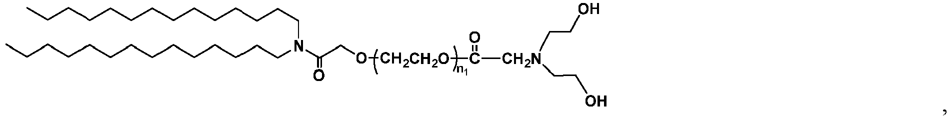 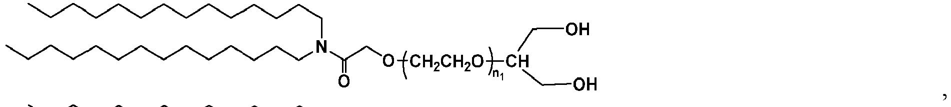 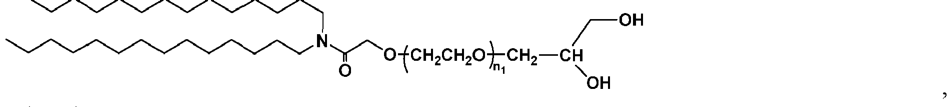 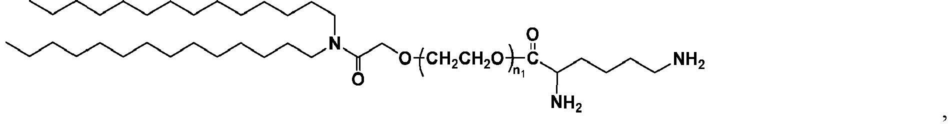 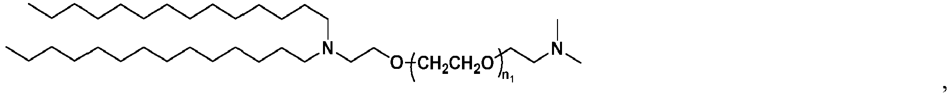 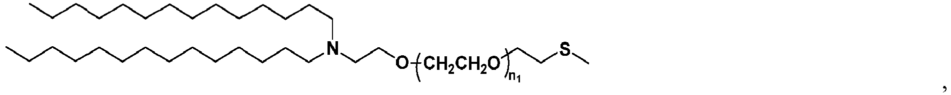 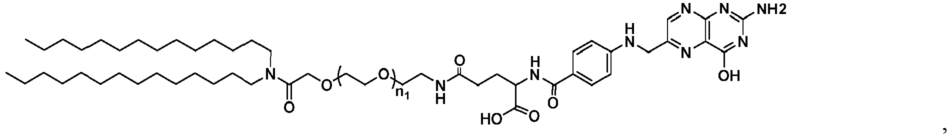 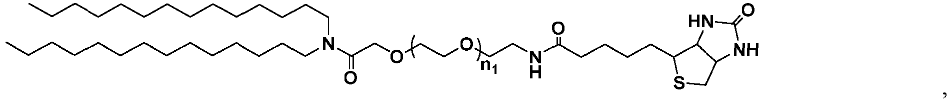 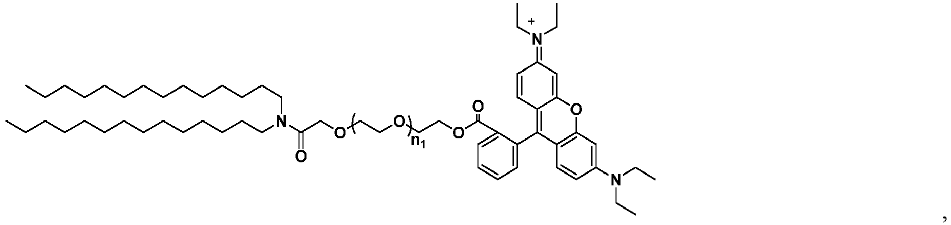 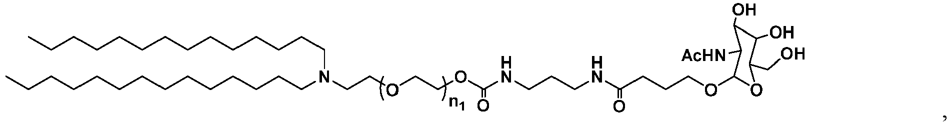 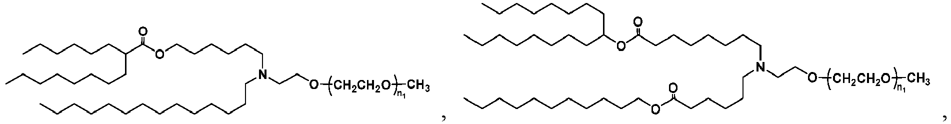 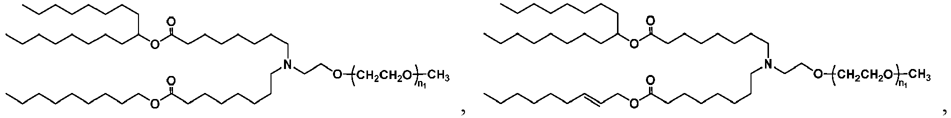 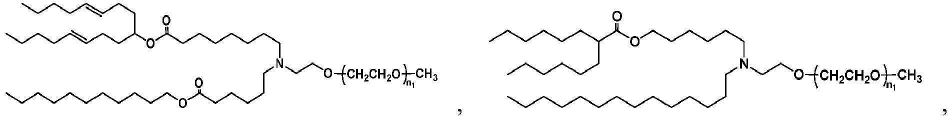 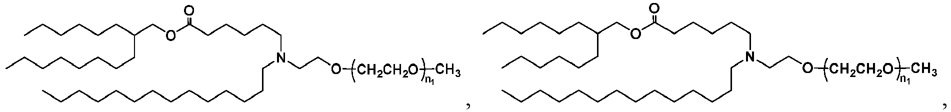 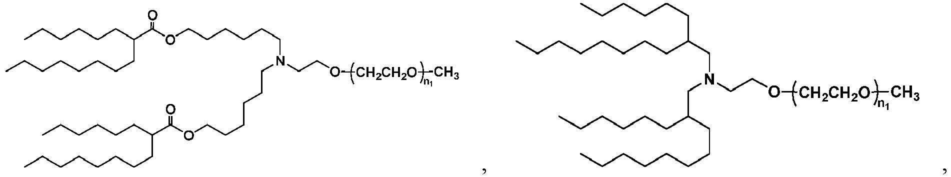 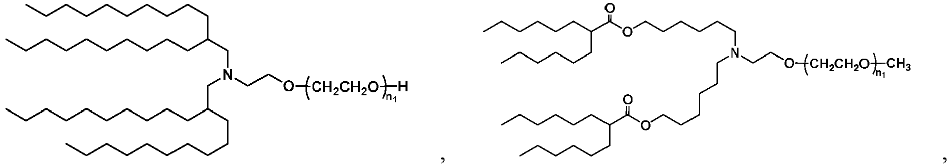 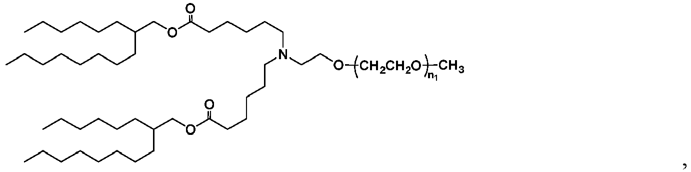 and 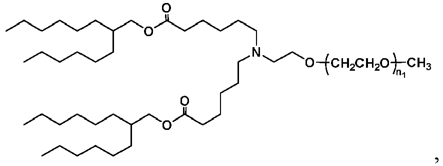 wherein n₁ is an integer from 20 to 250.

### 2. Preparation of PEGylated lipids

In the present invention, any of the above-mentioned PEGylated lipids can be prepared by methods including but not limited to the following:

### 2.1. Method-1:

Step 1: Activating the carboxyl terminus of the acid A-1 or A-1' containing an unprotected carboxyl group with carboxyl activating agents to obtain the ester A-2 or A-2' containing an activated carboxyl terminus, wherein B₃' and B₄' are each independently a linking bond or an alkylene group having one less methylene group than B₃ and B₄; R₁' and R₂' are each independently R₁, R₂ or an aliphatic hydrocarbon group having one less methylene group than R₁ and R₂; R₇ is a carboxyl-activating group; and, when either B₃' or L₇ is not a linking bond, R₁' is R₁; when both B₃' and L₇ are linking bonds; R₁' is an aliphatic hydrocarbon group having one less methylene group than R₁; when any of B₄' and L₈ is not a linking bond, R₂' is R₂; when both B₄' and L₈ are linking bonds, R₂' is an aliphatic hydrocarbon group having one less methylene group than R₂;
Step 2: Carrying out the condensation reaction between the ester A-2 or A-2' containing an activated carboxyl terminus and the primary amine derivative A-3 or A-3' containing a nitrogen-source terminal group to obtain the amide intermediate A-4 or A-4';
Step 3: Using reducing agents to reduce the amide intermediate A-4 or A-4' to the secondary amine intermediate A-5 or A-5';
Step 4: Carrying out the coupling reaction between the secondary amine intermediate and the dual end-functionalized PEG derivative which is, the biLPEG molecule A-6, to obtain the PEGylated lipid derivative A-7 or A-7'; wherein, the biLPEG can be monodisperse or polydisperse; wherein, the two terminal functional groups of biLPEG can be the same or different; wherein, the R' end of biLPEG contains the functional group R₀₁ or a micro variant form of R₀₁; said micro variant form refers to the group which can be transformed into R₀₁ through any chemical process selected from deprotection, salt complexation and decomplexation, ionization, protonation, deprotonation, and leaving group transformation; wherein, the F₁ contains active functional groups, which can react with the amino group of the secondary amine intermediate A-5 or A-5' to obtain the nitrogen atom as a branching center and the divalent linking group L₃;
   when R' is R, the structure of A-7 or A-7' is represented by the general formula (2);
   when R' is not R, A-8 or A-8' represented by the general formula (2) is obtained via terminal micro-modification of A-7 or A-7'; said terminal micro-modification is selected from chemical reactions including deprotection, salt complexation and decomplexation, ionization, protonation, deprotonation, and leaving group transformation.

### Step 1:

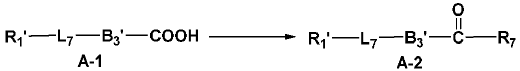

or

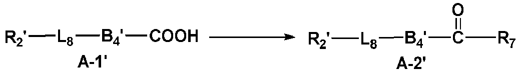

### Step 2:

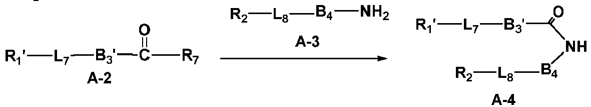

or

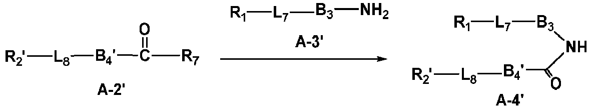

### Step 3:

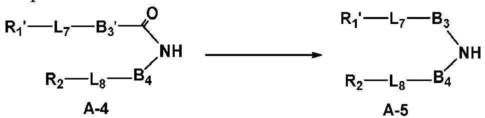

or

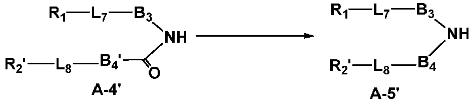

### Step 4:

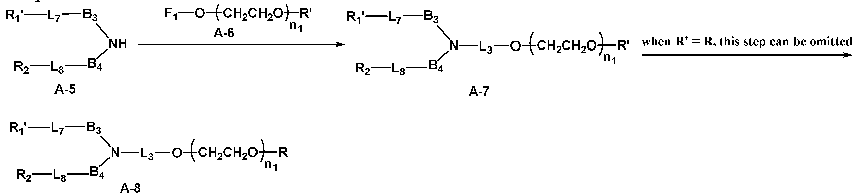

or

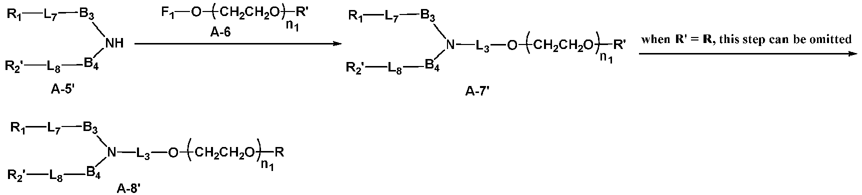

Wherein, the descriptions of L₃, L₇, L₈, B₃, B₄, R, R₁, R₂, and n₁ are in line with those of the general formula (2), which are not repeated here.

In one specific embodiment of the present invention, in the foregoing Method-1, said A-1 is preferably R₁'-COOH or R₂'-COOH, said A-3 is preferably R₁-NH₂ or R₂-NH₂, said carboxyl activating agent is preferably selected from the group consisting of *N-*hydroxysuccinimide (NHS), 1-ethyl-3-(3-dimethylaminopropyl)carbodiimide hydrochloride (EDCI), *N*-hydroxy-5-norbornene-2,3-dicarboximide (HONb), and *N*,*N'-*dicyclohexylcarbodiimide (DCC), and said PEGylated lipid is preferably prepared by the following routes:

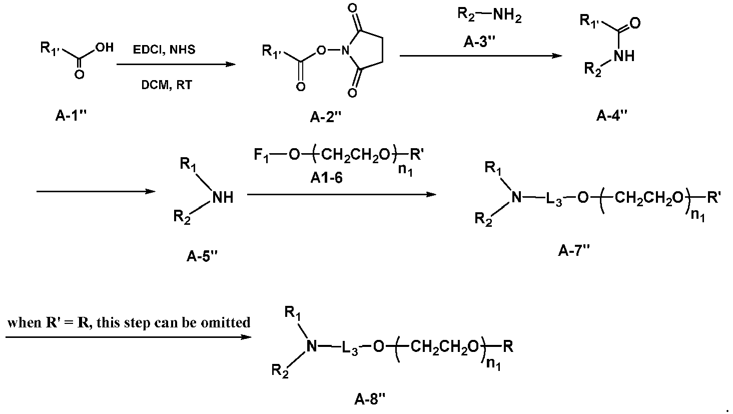

### 2.2 Method-2:

Step 1: Carrying out the coupling reaction between the PEG derivative biLPEG molecule B-1, and the primary amine derivative B-2 or B-2' containing a nitrogen-source terminal group to obtain the secondary amine derivative B-3 or B-3'; wherein, biLPEG can be monodisperse or polydisperse, the two terminal functional groups of biLPEG can be the same or different, and the R' end of biLPEG contains the functional group R₀₁ or a micro variant form of R₀₁; said micro variant form refers to the group which can be transformed into R₀₁ through any chemical process selected from deprotection, salt complexation and decomplexation, ionization, protonation, deprotonation, and leaving group transformation; wherein, the F₁ contains active functional groups, which can react with the amino group of the primary amine B-2 or B-2' to obtain the secondary amine derivative B-3 or B-3' containing the divalent linking group L₃;
Step 2: Carrying out the alkylation reaction between the secondary amine derivative B-3 or B-3' and the compound B-4 or B-4' with the functional group F_{N} to obtain the PEGylated lipid derivative B-5 or B-5'; said F_{N} is a functional group which can react with amino group or secondary amino group, preferably -OMs, -OTs, -CHO, -F, -Cl, or -Br;
   when R' is R, the structure of B-5 or B-5' is represented by the general formula (2);
   when R' is not R, B-6 or B-6' represented by the general formula (2) is obtained via terminal micro-modification of B-5 or B-5'; said terminal micro-modification is selected from chemical reactions including deprotection, salt complexation and decomplexation, ionization, protonation, deprotonation, and leaving group transformation.

### Step 1:

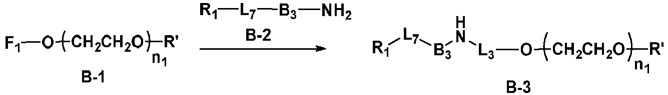

or

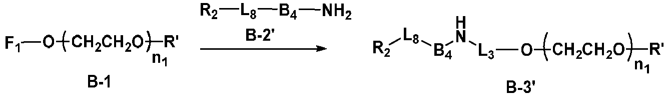

### Step 2:

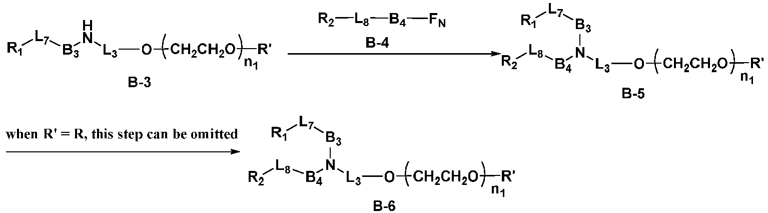

or

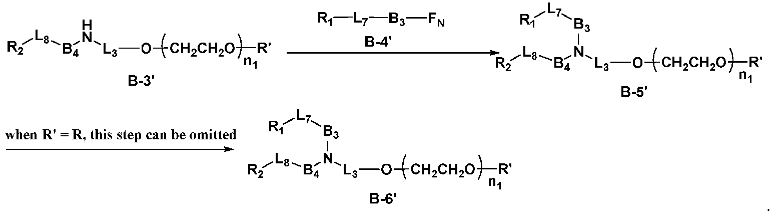

Wherein, the descriptions of L₃, L₇, L₈, B₃, B₄, R, R₁, R₂, and n₁ are in line with those of the general formula (2), and no more repeated here.

In one specific embodiment of the present invention, in the foregoing Method-2, said B-3 or B-3' is R₁-NH₂ or R₂-NH₂, said B-4 or B-4' is R₂-L₈-B₄-Br or R₁-L₇-B₃-Br, and said F₁ contains the -OMs group.

### 2.3. Method-3:

Step 1: Carrying out the reaction between the small molecule C-1 and the small molecule C-2 to obtain the small molecule intermediate C-3 which contains the divalent linking group L₇, the functional group F_{N} at one end, and an aliphatic hydrocarbon group R₁ at the other end; wherein, F₃ and F₄ are each independently a functional group, which can be transformed into the divalent linking group L₇ via reaction; wherein, C-2 contains a pair of heterofunctional groups of F₃ and F_{N}, said F_{N} is the functional group which can react with amino group or secondary amino group, preferably -OMs, -OTs, -CHO, -F, -Cl or -Br;
Step 2: Carrying out the alkylation reaction between two molecules of the small molecule intermediate C-3 and the PEGylated primary amine derivative C-4 containing a nitrogen-source terminal group to obtain the PEGylated lipid C-5, wherein, the R' end contains the functional group R₀₁ or a micro variant form of R₀₁; said micro variant form refers to the group which can be transformed into R₀₁ through any chemical process selected from deprotection, salt complexation and decomplexation, ionization, protonation, deprotonation, and leaving group transformation;
   when R' is R, the structure of C-5 is represented by the general formula (2);
   when R' is not R, C-6 represented by the general formula (2) is obtained via terminal micro-modification of C-5; said terminal micro-modification is selected from chemical reactions including deprotection, salt complexation and decomplexation, ionization, protonation, deprotonation and leaving group transformation; wherein, R₁ is the same as R₂, B₃ is the same as B₄, and L₇ is the same as L₈.

Wherein, the descriptions of L₃, L₇, L₈, B₃, B₄, R, R₁, R₂, and n₁ are in line with those of the general formula (2), and no more repeated here.

### Step 1:

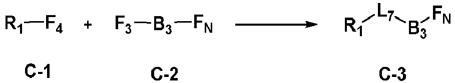

### Step 2:

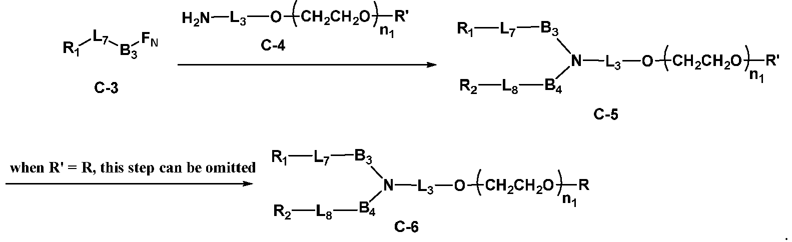

### 2.4. Method-4:

Step 1: Carrying out the reaction between the small molecule D-1 and the small molecule D-2 to obtain the small molecule intermediate D-3 which contains the divalent linking group L₇, a hydroxyl group at one end, and an aliphatic hydrocarbon group R₁ at the other end; wherein, F₃ and F₄ are each independently a functional group, which can be transformed into the divalent linking group L₇ via reaction; wherein, D-2 contains a pair of heterofunctional groups of F₃ and a hydroxyl group;
Step 2: Carrying out the reaction in which the hydroxyl group of the small molecule intermediate D-3 is oxidized to an aldehyde group to obtain the small molecule intermediate D-4 containing an aldehyde group, wherein B₃' is an alkylene group having one less methylene group than B₃;
Step 3: Carrying out the addition reaction between two molecules of the small molecule intermediate D-4 containing an aldehyde group and the PEGylated primary amine derivative D-5 containing a nitrogen-source terminal group to obtain the PEGylated lipid D-6, wherein, the R' end contains the functional group R₀₁ or a micro variant form of R₀₁; said micro variant form refers to the group which can be transformed into R₀₁ through any chemical process selected from deprotection, salt complexation and decomplexation, ionization, protonation, deprotonation, and leaving group transformation;
   when R' is R, the structure of D-6 is represented by the general formula (2);
   when R' is not R, D-6 represented by the general formula (2) is obtained via terminal micro-modification of D-7; said terminal micro-modification is selected from chemical reactions including deprotection, salt complexation and decomplexation, ionization, protonation, deprotonation and leaving group transformation; wherein, R₁ is the same as R₂, B₃ is the same as B₄, and L₇ is the same as L₈.

### Step 1:

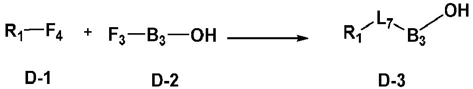

### Step 2:

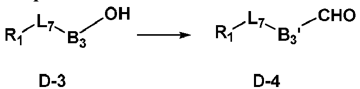

### Step 3:

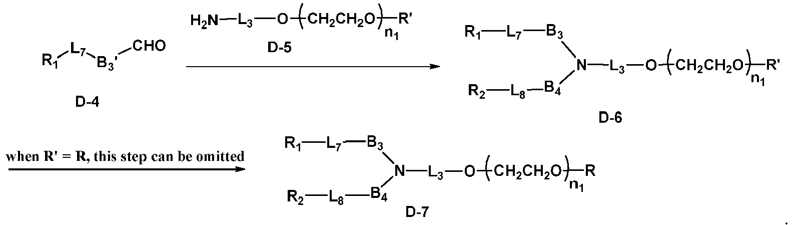

Wherein, the descriptions of L₃, L₇, L₈, B₃, B₄, R, R₁, R₂, and n₁ are in line with those of the general formula (2), and no more repeated here.

### 2.5. Description of relevant starting materials and/or steps in the preparation process

### 2.5.1. Carboxyl activating agent, condensing agent, oxidizing agent, and reducing agent

In the present invention, the "activation of a carboxyl group" refers to the activation of a carboxyl group with carboxyl activating agents. The activated carboxyl group can promote the condensation reactions, for example, by inhibiting the generation of racemic impurities, by accelerating the reaction through catalysis, etc. The "carboxyl activating group" refers to the residue of a carboxyl activating agent. Said carboxyl activating agent is selected from the group consisting of *N*-hydroxysuccinimide (NHS), 1-ethyl-3-(3-dimethylaminopropyl)carbodiimide hydrochloride (EDCI), *N*-hydroxy-5-norbornene-2,3-dicarboximide (HONb), and *N*,*N*'-dicyclohexylcarbodiimide (DCC), and combinations thereof, preferably the combination of NHS/EDCI, NHS/DCC, or HONb/DCC, and more preferably the combination of NHS/EDCI.

In the present invention, the condensing agent used in reactions is not particularly limited, but preferably selected from the group consisting of *N*,*N*'-dicyclohexylcarbodiimide (DCC), 1-ethyl-3-(3-dimethylaminopropyl)carbodiimide hydrochloride (EDC·HCl), 2-(7-azobenzotriazole-*N*,*N*,*N*',*N*'-tetramethyluronium hexafluorophosphate (HATU) and (benzotriazol-1-yl)-*N*,*N*,*N'*,*N'*-tetramethyluronium hexafluorophosphate (HBTU), and most preferably DDC. Generally, the amount of the condensing agent used is 1 to 20 folds of the molar equivalent of the carboxylic acid, preferably 5-10 folds, and a suitable catalyst (such as 4-dimethylaminopyridine) can be added to the reaction.

In the present invention, the oxidizing agents used in reactions are not particularly limited, as long as they can increase the valence of the substrate; said oxidizing agents are preferably selected from the group consisting of phenyliodine(III) bis(trifluoroacetate), 1,4-benzoquinone, benzyl trimethyl ammonium tribromide, pyridinium dichromate, potassium dichromate, ozone, oxygen, hydrofluoric acid, sodium hypochlorite, cobaltic acetate, cobalt acetate, manganous acetate, palladium(II) acetate, cupric acetate, monoperoxyphthalic acid, iodine, *N*-iodosuccinimide, iodylbenzene, 2-iodylbenzoic acid, dimethyldioxirane, dimethyl sulfoxide-oxalyl chloride, DDQ, dichlorotris(triphenylphosphine)ruthenium, manganese dioxide, (diacetoxyiodo)benzene, periodic acid, sodium periodate, sodium periodate-osmium tetraoxide, potassium permanganate, sodium perborate, perbenzoic acid, dibenzoyl peroxide, nickel peroxide, hydrogen peroxide, cumyl hydroperoxide, t-butyl hydroperoxide, peracetic acid, *m*-chloroperbenzoic acid, *N*-chlorosuccinimide, pyridinium chlorochromate, palladium chloride-cupric chloride, urea hydrogen peroxide adduct, triphenylcarbenium tetrafluoroborate, tributyltin oxide, cobalt trifluoride, vanadium oxytrifluoride, chromium trioxide, manganese triacetate, TEMPO, diammonium cerium nitrate, bromine, pyridine *N-*oxide, silver oxide, *O*-ethylperoxycarbonic acid, manganese acetylacetonate, vanadyl acetylacetonate, aluminium isopropoxide, potassium peroxymonosulfate, dichloroiodobenzene, etc., and combinations thereof, and more preferably selected from the group consisting of oxygen, sodium hypochlorite, hydrogen peroxide, dichloroiodobenzene, potassium peroxymonosulfate, etc., and combinations thereof. The molar equivalent of the oxidizing agent used is 1 to 50 folds of that of the hydroxyl groups of intermediate compounds, preferably 1 to 20 folds, and more preferably 5 to 10 folds.

In the present invention, the reducing agents are not particularly limited, as long as the Schiff bases formed via the reaction between amines and aldehydes or ketones can be reduced to amine groups; said reducing agents are preferably selected from the group consisting of sodium borohydride, sodium cyanoborohydride, lithium aluminum hydride, borane, diborane, diisobutylaluminum hydride, diisopinocampheylborane, lithium borohydride, zinc borohydride, borane-pyridine complex, borane-methyl sulfide complex, borane-tetrahydrofuran complex, etc., and combinations thereof, and more preferably sodium cyanoborohydride. The molar equivalent of the reducing agent used is 1 to 50 folds of that of the amino groups to be modified, preferably 1 to 20 folds, and more preferably 5 to 10 folds.

In the present invention, the reaction temperature is 0 to 200°C, preferably 0 to 100°C, and more preferably 0 to 25°C. The reaction time is preferably 10 min to 48 h, and more preferably 30 min to 24 h. The obtained product can be purified by a purification means such as extraction, recrystallization, adsorption treatment, precipitation, reverse precipitation, membrane dialysis, supercritical extraction, etc.

In the present invention, the reaction solvent can be absent or an aprotic solvent; said aprotic solvent includes toluene, benzene, xylene, acetonitrile, ethyl acetate, diethyl ether, tert-butyl methyl ether, tetrahydrofuran, chloroform, dichloromethane, dimethylsulfoxide, dimethylformamide, and dimethylacetamide, and is preferably tetrahydrofuran, dichloromethane, dimethylsulfoxide, or dimethylformamide.

In the present invention, the bases used in reactions can be organic bases (e.g., triethylamine, pyridine, 4-dimethylaminopyridine, imidazole, or *N*,*N*-diisopropylethylamine), preferably triethylamine or pyridine. The molar equivalent of the base used is 1 to 50 folds of that of carboxylic acids, preferably 1 to 10 folds, and more preferably 2 to 3 folds.

### 2.5.2. "Protection" and "deprotection" of relevant groups involved in the reaction process

In the present invention, the reaction process also involves the "protection" and "deprotection" processes of relevant groups. In order to prevent a functional group from affecting the reaction, the functional group is usually protected. In addition, when there are two or more functional groups, only the target functional group reacts selectively, so the other functional groups should be protected. The protecting group not only protects the functional group stably, but also need to be removed easily as needed. Therefore, in organic synthesis, it is important to remove only the protecting group bonded to the specified functional group under appropriate conditions.

In the present invention, a "carboxyl protecting group" refers to the protecting group which can be transformed into a carboxyl group via hydrolysis or deprotection. A carboxyl protecting group is preferably an alkyl group (e.g., a methyl group, an ethyl group, a tert-butyl group) or an aralkyl group (e.g., a benzyl group), and more preferably a tert-butyl group (tBu), a methyl group (Me), or an ethyl group (Et). In the present invention, a "protected carboxyl group" refers to the group formed via the protection of a carboxyl group with an appropriate carboxyl protecting group, and is preferably a methoxycarbonyl group, an ethoxycarbonyl group, a *t*-butoxycarbonyl group, or a benzyloxycarbonyl group. Said carboxyl protecting group can be removed through hydrolysis catalyzed by acids or alkalis, or through pyrolysis reactions occasionally; for example, *t*-butyl groups can be removed under mild acidic conditions, and benzyl groups can be removed by hydrogenolysis. The reagents used for the removal of carboxyl protecting groups are selected from the group consisting of TFA, H₂O, LiOH, NaOH, KOH, MeOH, EtOH, and combinations thereof, preferably the combination of TFA and H₂O, the combination of LiOH and MeOH, or the combination of LiOH and EtOH. A protected carboxyl group can undergo deprotection and then produce the corresponding free acid; said deprotection can be conducted in the presence of an alkali, and said alkali forms pharmaceutically acceptable salts with said free acid obtained from said deprotection.

In the present invention, the "amino protecting group" includes all the groups which can be used as common amino protecting groups, such as an aryl C₁₋₆ alkyl group, a C₁₋₆ alkoxy C₁₋₆ alkyl group, a C₁₋₆ alkoxycarbonyl group, an aryloxycarbonyl group, C₁₋₆ alkylsulfonyl group, an arylsulfonyl group, a silyl group, etc. An amino protecting group is preferably selected from the group consisting of a *t*-butoxycarbonyl group (Boc), a *p-*methoxybenzyloxycarbonyl group (Moz) and a 9-fluorenylmethoxycarbonyl group (Fmoc). The reagent used for removing amino protecting groups is selected from the group consisting of TFA, H₂O, LiOH, MeOH, EtOH and combinations thereof, preferably selected from the combination of TFA and H₂O, the combination of LiOH and MeOH, and the combination of LiOH and EtOH. The reagent used for removing the Boc protecting group is TFA or HCl/EA, preferably TFA. The reagent used for removing the Fmoc protecting group is the *N*,*N-*dimethylformamide (DMF) solution containing 20% piperidine.

In the present invention, said hydroxyl group protected by a hydroxyl protecting group is not particularly limited, e.g., an alcoholic hydroxyl group, a phenolic hydroxyl group, and the like. Wherein, said amino group protected by an amino protecting group is not particularly limited, selected from the group consisting of a primary amine, a secondary amine, a hydrazine, an amide, and the like. In the present invention, amino groups are not particularly limited, including but not limited to primary amino groups, secondary amino groups, tertiary amino groups, and quaternary ammonium ions.

In the present invention, the deprotection of protected hydroxyl groups is related to the type of hydroxyl protecting group. Said type of hydroxyl protecting group is not particularly limited; for example, benzyl groups, silyl ethers, acetals, or tert-butyl groups can be used to protect terminal hydroxyl groups, and the corresponding deprotection methods include the follows:

### A: Removal of benzyl groups

The removal of the benzyl groups can be achieved via hydrogenation using a hydrogenative reduction catalyst and a hydrogen donor. As used herein, the water content should be less than 1% in order to facilitate the reaction. When the water content is more than 1%, the decomposition reaction of the polyethylene glycol chain occurs and generate the low-molecular-weight polyethylene glycol with a hydroxyl group, which can participate in the subsequent polymerization reaction or functionalization reaction, will introduce impurities into the target product. Such impurities may even react with the bio-related substance and change the property of the preparation.

The catalyst for hydrogenation reduction is not particularly limited, preferably palladium or nickel. The carrier is not particularly limited, preferably alumina or carbon, more preferably carbon. The amount of palladium is 1 to 100 wt% of that of compounds containing protected hydroxyl groups, preferably 1 to 20 wt%. When the amount of palladium is less than 1wt%, the rate and the conversion of removal decrease. For the deprotected compounds are not allowed to participate in the subsequent polymerization or functionalization, the ratio of functionalization of the final product will be low. However, when the amount of palladium exceeds 100 wt%, the polyethylene glycol chain tends to undergo a decomposition reaction.

The reaction solvent is not particularly limited, as long as both the starting materials and the products can be dissolved, but is preferably methanol, ethanol, ethyl acetate, tetrahydrofuran, or acetic acid, more preferably methanol. The hydrogen donor is not particularly limited, but is preferably hydrogen, cyclohexene, 2-propanol, or ammonium formate, etc. The reaction temperature is preferably in the range of 25 to 40°C. When the temperature is higher than 40 °C, the decomposition reaction of the polyethylene glycol chain may occur. The reaction time is not particularly limited, which is negatively correlated with the amount of catalyst, preferably 1 to 5 hours, when the reaction time is shorter than 1 hour, the conversion is relatively low, when the reaction time is longer than 5 hours, the polyethylene glycol chain may undergo a decomposition reaction.

### B: Removal of acetals and ketals

The compounds used for protecting hydroxyl groups in the forms of acetals or ketals are preferably ethyl vinyl ether, tetrahydropyran, acetone, 2,2-dimethoxypropane, or benzaldehyde, etc. The removal of such acetals or ketals can be realized under an acidic condition wherein the pH of the solution is preferably 0 to 4. When the pH is higher than 4, the acidity is too weak for the protecting group to be completely removed. When the pH is lower than 0, the acidity is too strong so that the polyethylene glycol chain tends to undergo a decomposition reaction. The acid is not particularly limited, preferably acetic acid, phosphoric acid, sulfuric acid, hydrochloric acid, or nitric acid, more preferably hydrochloric acid. The reaction solvent is not particularly limited as long as it allows the starting materials and the products can be dissolved. The solvent is preferably water. The reaction temperature is preferably 0 to 30 °C. When the temperature is lower than 0 °C, the reaction rate is relatively slow, and the protecting group cannot be completely removed; when the temperature is higher than 30 °C, the decomposition reaction of the polyethylene glycol chain tends to occur under an acidic condition.

### C: Removal of silyl ethers

The protected hydroxyl groups in the forms of silyl ethers include trimethylsilyl ether, triethylsilyl ether, tert-butyldimethylsilyl ether, tert-butyldiphenylsilyl ether, etc. The removal of such silyl ethers uses compounds containing fluoride ions; wherein, said compounds are preferably selected from the group consisting of tetrabutylammonium fluoride, tetraethylammonium fluoride, hydrofluoric acid, and potassium fluoride, and more preferably selected from tetrabutylammonium fluoride and potassium fluoride. The molar equivalent of the fluorine-containing compound used is 5 to 20 folds of that of the protected hydroxyl group, preferably 8 to 15 folds of that of the initiator. When the molar equivalent of the fluorine-containing compound used is less than 5 folds of that of the protected hydroxyl group, the deprotonation might be incomplete. When the molar equivalent of the removal reagent used exceeds 20 folds of that of the protected hydroxyl group, the excess reagents or compounds will bring difficulty in the purification and possibly cause side reactions in subsequent steps. The reaction solvent is not particularly limited, as long as the reactants and the products can be dissolved, but is preferably an aprotic solvent and more preferably tetrahydrofuran or dichloromethane. The reaction temperature is preferably 0 to 30°C; when the temperature is lower than 0°C, the reaction rate is relatively slow, and the protecting group cannot be completely removed.

### D: Removal of tert-butyl groups

The removal of tert-butyl groups is carried out under an acidic condition wherein the pH of the solution is preferably 0 to 4. When the pH is higher than 4, the acidity is too weak for the protecting group to be completely removed; when the pH is lower than 0, the acidity is too strong, and there is a tendency for the polyethylene glycol chain to undergo a decomposition reaction. The acid is not particularly limited, preferably acetic acid, phosphoric acid, sulfuric acid, hydrochloric acid, or nitric acid, more preferably hydrochloric acid. The reaction solvent is not particularly limited as long as the starting materials and the products can be dissolved. The solvent is preferably water. The reaction temperature is preferably 0 °C to 30 °C. When the temperature is lower than 0 °C, the reaction rate is relatively slow, and the protective group cannot be completely removed; when the temperature is higher than 30 °C, the decomposition reaction of the polyethylene glycol chain tends to occur.

In the following preparation methods for linear functionalization of terminal hydroxyl groups of polyethylene glycol chains, it is preferred that q=0, q₁=1, and Z₁ is a 1,2-methylene group. When q is not 0, for example, when a linking group such as an amino acid group or a succinyl group is present between PEG and R₀₁, the prior art capable of generating Z₂ or Z₁ (including but not limited to alkylation, condensation, click reactions, etc.) can be used, and the preparation process can be carried out referring to the following linear functionalization methods.

### 2.5.3. Alkylation reaction

In the present invention, the alkylation reaction is preferably based on hydroxyl groups, mercapto groups, or amino groups, corresponding to the formation of ether bonds, thioether bonds, secondary amino groups, and tertiary amino groups, respectively. Specific examples are as follows:

### 2.5.3.1. Alkylation reaction between the substrate alcohols and sulfonates or halide derivatives

An amine intermediate can be obtained via the nucleophilic substitution with a sulfonate or halide on the substrate alcohol under basic conditions. Wherein, the molar equivalent of the sulfonate or halide is 1 to 50 folds of that of the substrate alcohol, preferably 1 to 5 folds. When the molar equivalent of the sulfonate or halide is less than 1 fold of that of the substrate alcohol, the substitution might be incomplete and cause difficulty in the purification process. When the molar equivalent of the sulfonate or halide exceeds 50 folds of that of the substrate alcohol, the excess sulfonate or halide tends to bring difficulty in the purification process, and might be brought into the subsequent step and therefore cause side reactions which further increases difficulty in the purification.

The resulting product is a mixture of ether intermediate, excess sulfonate, and excess halide, and can be purified by means such as anion exchange resin, osmosis, ultrafiltration, etc. Wherein, the anion exchange resin is not particularly limited, as long as the target product can undergo ion-exchange and adsorption in the resin, and is preferably the ion exchange resin of tertiary amines or quaternary ammonia salts, with the matrix being dextran, agarose, polyacrylate, polystyrene, or poly(diphenylethylene), etc. The solvents used for osmosis or ultrafiltration are not limited, generally water or an organic solvent. Said organic solvent is not particularly limited, as long as the product can be dissolved within, but is preferably dichloromethane, chloroform, or the like.

The reaction solvent is not limited, preferably an aprotic solvent such as toluene, benzene, xylene, acetonitrile, ethyl acetate, tetrahydrofuran, chloroform, dichloromethane, dimethylsulfoxide, dimethylformamide, and dimethylacetamide, and more preferably dimethylformamide, dichloromethane, dimethylsulfoxide, or tetrahydrofuran.

The base used can be an organic base (e.g., triethylamine, pyridine, 4-dimethylaminopyridine, imidazole, diisopropylethylamine) or an inorganic base (e.g., sodium carbonate, sodium hydroxide, sodium bicarbonate, sodium acetate, potassium carbonate, potassium hydroxide), preferably an organic base, and more preferably triethylamine or pyridine. The molar equivalent of the base used is 1 to 50, preferably 1 to 10, and more preferably 3 to 5 folds of that of the sulfonate or halide.

### 2.5.3.2. Alkylation reaction between the substrate amines and sulfonate or halide derivatives

### Method (A): Alkylation reaction between the substrate amines and sulfonate or halide derivatives

An amine intermediate can be obtained via the nucleophilic substitution between the substrate amine and sulfonate or halide derivatives under a basic condition. Wherein, the amount of the sulfonate or halide derivatives is 1 to 50 folds of that of the substrate amine, preferably 1 to 5 folds. When the amount of the sulfonate or halide derivative is less than 1 fold of that of the substrate alcohol, the substitution may not sufficiently proceed and the purification tends to be difficult. When the amount of the sulfonate or halide derivative exceeds 50 folds, the excess reagent tends to cause difficulty in the purification process and result in side reactions in the subsequent steps.

The resulting product is a mixture of amine intermediate, excess sulfonate, and excess halide, the mixture can be purified by purification means such as cation exchange resin, osmosis treatment, ultrafiltration treatment or the like. Wherein, the anion exchange resin is not particularly limited as long as the target product can undergo ion-exchange and adsorption in the resin, and is preferably the ion exchange resin of tertiary amines or quaternary ammonia salts, with the matrix being dextran, agarose, polyacrylate, polystyrene, or poly(diphenylethylene), etc. The solvents used for osmosis or ultrafiltration are not limited, generally water or an organic solvent. Said organic solvent is not particularly limited, as long as the product can be dissolved within, but is preferably dichloromethane, chloroform, or the like.

The reaction solvent is not limited, preferably an aprotic solvent such as toluene, benzene, xylene, acetonitrile, ethyl acetate, tetrahydrofuran, chloroform, dichloromethane, dimethylsulfoxide, dimethylformamide, and dimethylacetamide, and more preferably dimethylformamide, dichloromethane, dimethylsulfoxide, or tetrahydrofuran.

The base used can be an organic base (e.g., triethylamine, pyridine, 4-dimethylaminopyridine, imidazole, diisopropylethylamine) or an inorganic base (e.g., sodium carbonate, sodium hydroxide, sodium bicarbonate, sodium acetate, potassium carbonate, potassium hydroxide), preferably an organic base, and more preferably triethylamine or pyridine. The molar equivalent of the base used is 1 to 50, preferably 1 to 10, and more preferably 3 to 5 folds of that of the sulfonate or halide.

### 2.5.3.3. Alkylation reaction between the substrate amines and aldehyde derivatives

The substrate amine reacts with an aldehyde derivative to obtain an imine intermediate, which is followed by obtaining an intermediate using reducing agents. Wherein, the molar equivalent of the aldehyde derivative is 1 to 20, preferably 1 to 2, and more preferably 1 to 1.5 folds of that of the substrate amine. When the molar equivalent of the aldehyde derivative exceeds 20 folds of that of the substrate amine, the excess reagent tends to cause difficulty in the purification process, and might be brought into the subsequent step and therefore increase difficulty in the purification. When the molar equivalent of the aldehyde derivative is less than 1 fold of that of the substrate amine, the reaction might be incomplete, causing further difficulty in the purification. Wherein, the resulting product can be obtained after purification by means such as cation exchange resin, osmosis, ultrafiltration, etc. Said cation exchange resin is not particularly limited, as long as it can undergo ion-exchange with quaternary ammonium cations and realize the isolation. The solvents used for osmosis or ultrafiltration treatment are not limited, generally water or an organic solvent. Said organic solvent is not particularly limited, as long as the product can be dissolved within, but is preferably dichloromethane, chloroform, or the like.

The reaction solvent is not limited, preferably an organic solvent such as methanol, ethanol, water, toluene, benzene, xylene, acetonitrile, ethyl acetate, tetrahydrofuran, chloroform, dichloromethane, dimethylsulfoxide, dimethylformamide, dimethylacetamide, etc., and more preferably water or methanol.

The reducing agent is not particularly limited, as long as the imine can be reduced to an amine, but is preferably sodium borohydride, lithium aluminum hydride, sodium cyanoborohydride, or Zn/AcOH, etc., and more preferably sodium cyanoborohydride. The amount of the reducing agent used is generally 0.5 to 50 folds and preferably 1 to 10 folds of that of the aldehyde derivative.

### 2.5.4. The linear di-end-functionalized PEG derivative biLPEG

Said biLPEG contains a functional group which can react with amino groups (primary amino groups or secondary amino groups) at one end, and the functional group forms the divalent linking group L₃ via coupling reaction.

The other end of said biLPEG with the structure being the same or different with the target structure R, contains a functional group which can be the same or different with the target functional group. The functional group on this end of the biLPEG includes but is not limited to the functional groups in the aforementioned classes A~J, including the precursors of any aforementioned functional groups, variant forms with the functional groups as precursors, substituted forms, protected forms, deprotected forms, and the like.

**The functional groups at both ends of said biLPEG may be the same or different,** preferably a linear heterofunctionalized PEG derivative (biheteroLPEG) with two different functional groups at both ends. Wherein, in the present invention, a pair of heterofunctional groups which can exist simultaneously includes but is not limited to the group consisting of a hydroxyl group paired with a protected hydroxyl group, a hydroxyl group or a protected hydroxyl group with a non-hydroxyl reactive group belonging to classes A~H (e.g., an amino group, a protected amino group, an ammonium salt group, an aldehyde group, an active ester group, a maleimide group, a carboxyl group, a protected carboxyl group, an alkynyl group, a protected alkynyl group, an azido group, an alkenyl group, an acrylic acid group, an acrylate group, a methacrylate group, an epoxy group, an isocyanato group, etc.), a hydroxyl group or a protected hydroxyl group paired with a functional group or derivative thereof belonging to the classes I-J (such as a targeting group, a photosensitive group, etc.), an active ester group paired with a maleimide group, an active ester group paired with an aldehyde group, an active ester group paired with an azido group, an active ester group paired with an alkynyl group or a protected alkynyl group, an active ester group paired with an acrylate group, an active ester group paired with a methacrylate group, an active ester group paired with an acrylic acid group, a maleimide group paired with an azido group, a maleimide group paired with an alkynyl group or a protected alkynyl group, a maleimide group paired with an acrylate group, a maleimide group paired with a methacrylate group, a maleimide group paired with an acrylic acid group, a maleimide group paired with a carboxyl group, a maleimide group paired with an amino group or a protected amino group or an ammonium salt group, a maleimide group paired with an isocyanato group, a maleimide group paired with a protected mercapto group, an aldehyde group paired with an azido group, an aldehyde group paired with an acrylate group, an aldehyde group paired with a methacrylate group, an aldehyde group paired with an acrylic acid group, an aldehyde group paired with an epoxy group, an aldehyde group paired with a carboxyl group, an aldehyde group paired with an alkynyl group or a protected alkynyl group, an azido group paired with a mercapto group or a protected mercapto group, an azido group paired with an amino group or a protected amino group or an ammonium salt group, an azido group paired with an acrylate group, an azido group paired with a methacrylate group, an azido group paired with an acrylic acid group, an azido group paired with a carboxyl group, an acrylate group paired with an amino group or a protected amino group or an ammonium salt group, an acrylate group paired with an isocyanato group, an acrylate group paired with an epoxy group, an acrylate group paired with a methacrylate group, an acrylate group paired with a carboxyl group, a methacrylate group paired with a carboxyl group, a methacrylate group paired with an amino group or a protected amino group or an ammonium salt group, a methacrylate group paired with an isocyanato group, a methacrylate group paired with an epoxy group, an alkynyl group or a protected alkynyl group paired with an amino or a protected amino group or an ammonium salt group, an alkynyl group or a protected alkynyl group paired with an isocyanato group, an alkynyl group or a protected alkynyl group paired with an acrylate group, an alkynyl group or a protected alkynyl group paired with a methacrylate group, an alkynyl group or a protected alkynyl group paired with acrylic acid group, an alkynyl group or a protected alkynyl group paired with an epoxy group, an alkynyl group or a protected alkynyl group paired with a carboxyl group, a protected alkynyl group paired with an azido group, an acrylic acid group paired with an isocyanato group, an acrylic acid group paired with an acrylate group, an acrylic acid group paired with an epoxy group, an acrylic acid group paired with a carboxyl group, a carboxyl group paired with a mercapto group or a protected mercapto group, a carboxyl group paired with an amino group or a protected amino group or an ammonium salt group, a carboxyl group paired with an isocyanato group, a carboxyl group paired with an epoxy group, an amino group or a protected amino group or an ammonium salt group paired with a mercapto or a protected mercapto group, a targeting group paired with a non-hydroxyl reactive group, a photosensitive group paired with a non-hydroxyl reactive group, and the like. Wherein, said active ester groups include but are not limited to any succinimidyl active ester groups (e.g., a succinimidyl carbonate group), p-nitrophenyl active ester groups, *o-*nitrophenyl active ester groups, benzotriazole active ester groups, 1,3,5-trichlorophenyl active ester groups, 1,3,5-trifluorophenyl active ester groups, pentafluorophenyl active ester groups, imidazole active ester groups, 2-thioxo-thiazolidine-3-carboxylate groups, and 2-thione-pyrrolidine-1-carboxylate groups in the present invention; said amino groups include primary amino groups and secondary amino groups. Said ammonium salt is preferably a hydrochloride form of an amino group, such as NH₂HCl.

### Said biLPEG can be polydisperse or monodisperse.

When a biLPEG is polydisperse, the polydispersity index is not particularly limited, while the polydispersity index of the starting material is preferably below 1.15, more preferably below 1.10, more preferably below 1.08, and more preferably below 1.05. The lower the PDI is, the more uniform the molecular weight and the narrower the molecular weight distribution, which leads to a higher quality of modified products when applied in the modification of molecules such as drugs and then meets the practical needs to a greater extent.

When a biLPEG is monodisperse, PDI=1 and a PEGylated lipid with a defined molecular structure and molecular weight can be obtained.

The products prepared with monodisperse starting materials have a more uniform molecular weight distribution; however, the molecular weight is mostly limited by preparation methods with lengthy steps. The advantage of using polydisperse starting materials is to provide a simple route and a larger range for molecule-weight adjustment.

The methods for preparing monodisperse polyethylene glycol chains can employ techniques in the prior art, including but not limited to the following literatures "J. Org. Chem. 2006, 71, 9884-9886" and cited references therein, "Angew. Chem. 2009, 121, 1274-1278" and cited references therein, "Expert Rev. Mol. Diagn. 2013, 13(4), 315-319" and cited references therein, "Angew. Chem. Int. Ed. 2014, 53, 6411-6413" and cited references therein, "Bioorganic & Medicinal Chemistry Letters, 2015, 25:38-42" and cited references therein, "Angew. Chem. Int. Ed., 2015, 54:3763-3767" and cited references therein, etc.

### 2.5.5. Linear functionalization of the terminal of a polyethylene glycol chain

The method for linear functionalization of the terminal of a polyethylene glycol chain is not particularly limited, but is relevant to the type of the final functional group or its protected form. It can be the linear functionalization of the terminal hydroxyl group of a polyethylene glycol chain, or the conversion of a reactive group into the target functional group or its protected form, or the combination of both.

The method for linear functionalization of the terminal hydroxyl group of a polyethylene glycol chain, starting from the terminal hydroxyl group of the polyethylene glycol chain, obtains a functional group of classes A~J or its protected form after functionalization. The specific preparation method is as documented in the paragraphs from [0960] to [1205] of the document CN104530417A. The general formula of the reaction is as follows: 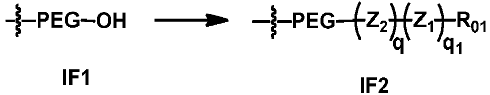 wherein, the structure of -PEG-OH is -(CH₂CH₂O)ₙCH₂CH₂OH, and n is n₁-1; the definitions of q, Z₂, q₁, Z₁, and R₀₁ are the same as those described above.

**The conversion of reactive groups into the target functional groups or protected forms thereof,** can be achieved by any of the following approaches:
Approach 1: Direct modification. The target functional group or its protected form can be obtained via direct modification of a reactive group. Examples include, the conversion of a carboxyl group to an acyl halide group, a hydrazide group, an ester group, a thioester group, or a dithioester group, the conversion of a hydroxyl group, a mercapto group, an alkynyl group, an amino group, a carboxyl group, or the like to the corresponding protected form, and so on. Another example is, the modification of a hydroxyl group, an amino group, or the like with an anhydride.
Approach 2: Coupling reaction between two reactive groups. Said coupling reaction uses a heterofunctional reagent as the starting material containing 1 type of reactive group and the target functional group or its protected form, to introduce the target functional group or its protected form via the reaction between any said reactive group and the terminal reactive group of the polyethylene glycol chain. The reaction between two reactive groups is not particularly limited with respect to the manner and method of the reaction; wherein, the reaction conditions are related to the type of the divalent linking group formed in the reaction, and the prior art can be employed, for example, alkylation, addition reaction of alkenes, addition reaction of alkynes, Schiff base reaction-reduction reaction, condensation reaction, etc. Wherein, the alkylation reaction is preferably selected from those based on a mercapto group or an amino group, corresponding to the formation of a thioether bond, a secondary amino group or a tertiary amino group, respectively. Wherein, the condensation reaction includes but is not limited to reactions forming an ester group, a thioester group, an amide group, an imine bond, a hydrazone bond, a carbamate group, and the like. Examples also include, introducing the target functional group or its protected form via click reactions by using heterofunctional reagents as the starting materials containing not only an azido group, an alkynyl group, an alkenyl group, a trithioester group, a mercapto group, a dienyl group, a furyl group, a 1,2,4,5-tetrazinyl group, a cyanate group, or the like, but also the target functional group or its protected form. The reaction between two reactive groups brings the formation of new bonds. The representative newly formed divalent linking groups include an amide bond, a urethane bond, an ester bond, a secondary amino bond, a thioether bond, a triazole group, and the like.
Approach 3: Combination of direct modifications and coupling reactions. The target functional group or its protected form can thus be obtained.

In the present invention, starting materials used in every preparation method can be obtained by purchase or synthetic means.

The intermediates and end-products prepared in the present invention can all be purified by purification methods including but not limited to extraction, recrystallization, adsorption treatment, precipitation, reverse precipitation, membrane dialysis, supercritical extraction and the like. The characterization of the structure and molecular weight of the end-products can use characterization methods including but not limited to NMR, electrophoresis, UV-visible spectrophotometer, FTIR, AFM, GPC, HPLC, MALDI-TOF, circular dichroism spectroscopy, and the like.

### 3. Cationic liposome

### 3.1. Cationic liposome

In the present invention, provided herein is a cationic liposome containing any foregoing PEGylated lipid whose structure is represented by the general formula (2).

In one specific embodiment of the present invention, the cationic liposome preferably contains not only the PEGylated lipid whose structure is represented by the general formula (2), but also one or more types of lipids selected from the group consisting of neutral lipid, steroid lipid, and cationic lipid; more preferably simultaneously contains three types of lipids as neutral lipid, steroid lipid, and cationic lipid.

In one specific embodiment of the present invention, neutral lipids in the cationic liposomes preferably include but are not limited to 1,2-dilinoleoyl-sn-glycero-3-phosphocholines (DLPC), 1,2-dimyristoleoyl-sn-glycero-3-phosphocholines (DMPC), 1,2-dioleoyl-sn-glycero-3-phosphocholines (DOPC), 1,2-dipalmitoyl-sn-glycero-3-phosphocholines (DPPC), 1,2-distearoyl-sn-glycero-3-phosphatidylcholines (DSPC), 1,2-diundecanoyl-sn-glycero-3-phosphatidylcholines (DUPC), 1-plamitoyl-2-oleoyl-sn-glycero-3-phosphocholines (POPC), 1,2-di-O-octadecenyl-sn-glycero-3-phosphatidylcholines (18:0 Diether PC), 1 -oleoyl-2-cholesterylhemisuccinyl-sn-glycero-3 -phosphocholines (OChemsPC), 1-O-hexadecyl-sn-glycero-3-phosphatidylcholines (C16 Lyso PC), 1,2-dilinolenoyl-sn-glycero-3-phosphatidylcholines, 1,2-diarachidonoyl-sn-glycero-3-phosphatidylcholines, 1,2-didecosahexaenoyl-sn-glycero-3-phosphocholines, 1,2-dioleoyl-sn-glycero-3-phosphoethanolamines (DOPE), 1,2-diphytanyl-sn-glycero-3-phosphoethanolamines (ME 16.0 PE), 1,2-distearoyl-sn-glycero-3-phosphoethanolamines, 1,2-dilinoleoyl-sn-glycero-3-phosphoethanolamines, 1,2-dilinolenoyl-sn-glycero-3-phosphoethanolamines, 1,2-diarachidonoyl-sn-glycero-3-phosphoethanolamines, 1,2-didecosahexaenoyl-sn-glycero-3-phosphoethanolamines, 1,2-dioleoyl-sn-glycero-3-phospho-rac-(1-glycerol) sodium salts (DOPG), dioleoyl phosphatidylserines (DOPS), dipalmitoylphosphatidylglycerols (DPPG), palmitoyloleoyl phosphatidylethanolamines (POPE), distearoyl phosphatidylethanolamines (DSPE), dipalmitoyl phosphatidylethanolamines (DPPE), dimyristoleoyl phosphoethanolamines (DMPE), 1-stearoyl-2-oleoyl-stearoylethanolamines (SOPE), 1-stearoyl-2-oleoyl-phosphatidylcholines (SOPC), sphingomyelins, phosphatidylcholines, phosphatidylethnolamines, phosphatidylserines, phosphatidylinositols, phosphatidic acids, palmitoyloleoyl phosphatidylcholines, lysophosphatidylcholines, lysophosphatidylethanolamines (LPE), and combinations thereof.

In one specific embodiment of the present invention, PEGylated lipids in the cationic liposomes are, apart from the PEGylated lipids represented by the general formula (2), preferably selected from the group consisting of PEG500-dipalmitoylphosphatidylcholine, PEG2000-dipalmitoylphosphatidylcholine, PEG500-distearylphosphatidylethanolamine, PEG2000-distearylphosphatidylethanolamine, PEG500-1,2-dioleoylphosphatidylethanolamine, PEG2000-1,2-dioleoylphosphatidylethanolamine and PEG2000-2,3-distearoylglycerol, preferably selected from PEG2000-dipalmitoylphosphatidylcholine, PEG2000-distearylphosphatidylethanolamine, and PEG2000-1,2-dioleoylphosphatidylethanolamine, more preferably PEG2000-distearylphosphatidylethanolamine.

In one specific embodiment of the present invention, steroid lipids in the cationic liposomes are preferably selected from the group consisting of cholesterol, coprostanol, sitosterol, ergosterol, campesterol, stigmasterol, brassicasterol, tomatidine, ursolic acid, α-tocopherol, and mixtures thereof.

In one specific embodiment of the present invention, the structure of a cationic lipid in a cationic liposome is preferably represented by the following general formula (1): 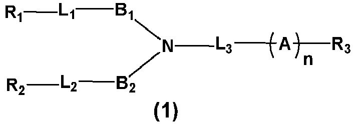
wherein, N is the nitrogen branching center;
L₁ and L₂ are divalent linking groups, each independently selected from the group consisting of -OC(=O)-, -C(=O)O-, -OC(=O)O-, -C(=O)-, -O-, -S-, -C(=O)S-, -SC(=O)-, - NR_{c}C(=O)-, -C(=O)NR_{c}-, -NR_{c}C(=O)NR_{c}-, -OC(=O)NR_{c}-, -NR_{c}C(=O)O-, -SC(=O)NR_{c}-, and -NR_{c}C(=O)S-; wherein, R_{c} is a hydrogen atom or a C₁₋₁₂ alkyl group;
L₃ is a linking bond or -L₄-Z-L₅-; said L₄ and L₅ are carbon chain linking groups, each independently -(CRₐR_{b})ₜ- or -(CRₐR_{b})ₜ-(CRₐR_{b})ₚ-(CRₐR_{b})_{q}-; wherein, t, p and q are each independently an integer from 0 to 12, Rₐ and R_{b} are each independently a hydrogen atom or a C₁₋₁₂ alkyl group; said Z is a linking bond or a divalent linking group selected from the group consisting of -OC(=O)-, -C(=O)O-, -OC(=O)O-, -C(=O)-, -O-, -S-, -C(=O)S-, - SC(=O)-, -NR_{c}C(=O)-, -C(=O)NR_{c}-, -NR_{c}C(=O)NR_{c}-, -OC(=O)NR_{c}-, -NR_{c}C(=O)O-, - SC(=O)NR_{c}-, and -NR_{c}C(=O)S-;
B₁ and B₂ are each independently a C₁₋₁₂ alkylene group;
R₁ and R₂ are each independently a C₂₋₃₀ aliphatic hydrocarbon group;
R₃ is a hydrogen atom, an alkyl group, an alkoxy group, -C(=O)R_{d}, -C(=O)OR_{d}, - OC(=O)R_{d}, -OC(=O)OR_{d}, or 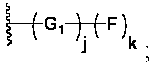 wherein, R_{d} is a C₁₋₁₂ alkyl group, G₁ is a terminal branching group with the valence of k+1, j is 0 or 1, and F contains functional groups; when j is 0, G₁ is absent; when j is 1, G₁ protrudes F with the number of k, and k is an integer from 2 to 8;
A is selected from the group consisting of -(CRₐR_{b})ₛO-, -O(CRₐR_{b})ₛ-, -(CRₐR_{b})ₛS-, - S(CRₐR_{b})ₛ-, -(CRₐR_{b})ₛO(CRₐR_{b})ₛS-, -(CRₐR_{b})ₛS(CRₐR_{b})ₛO-, -(CRₐR_{b})ₛNR_{c}(CRₐR_{b})ₛS-, - (CRₐR_{b})ₛS(CRₐR_{b})ₛNR_{c}-, -(CRₐR_{b})ₛNR_{c}(CRₐR_{b})ₛO-, and -(CRₐR_{b})ₛO(CRₐR_{b})ₛNR_{c}-; wherein, s is 2, 3 or 4, Rₐ and R_{b} are each independently a hydrogen atom or a C₁₋₁₂ alkyl group;
when A is -(CRₐR_{b})ₛO- or -O(CRₐR_{b})ₛ-, n is an integer from 2 to 6; when A is not - (CRₐR_{b})ₛO- or -O(CRₐR_{b})ₛ-, n is an integer from 1 to 6;
said alkyl group, alkylene group, alkoxy group, and aliphatic hydrocarbon group are each independently substituted or unsubstituted.

In one specific embodiment of the present invention, the structure of a cationic lipid in a cationic liposome is represented by the general formula (1), and preferably selected from the group consisting of the following structures:

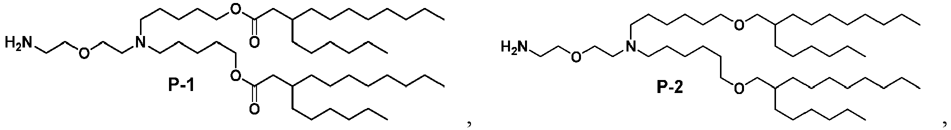

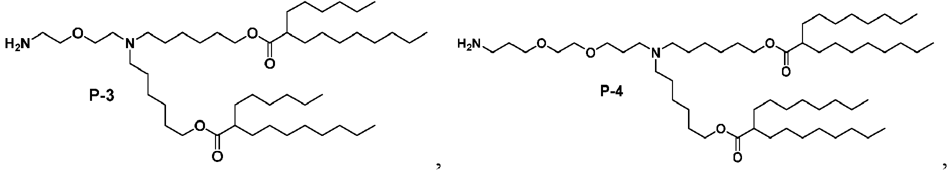

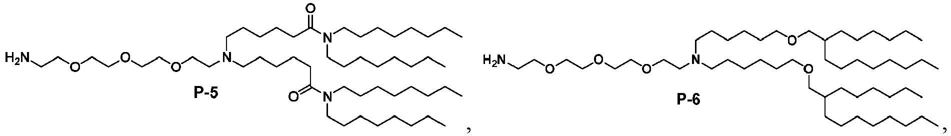

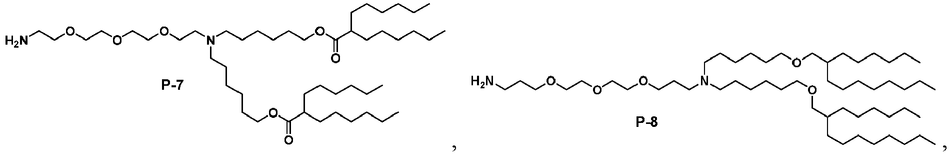

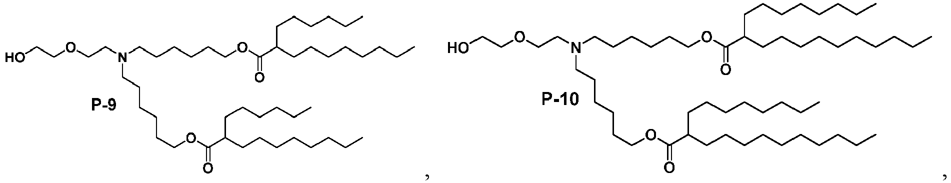

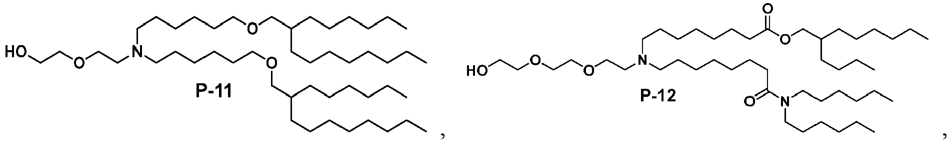

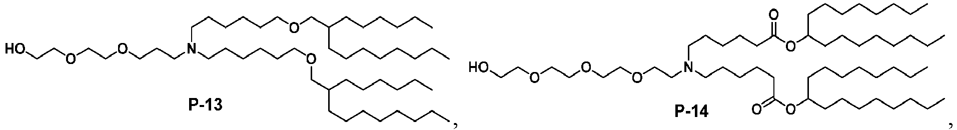

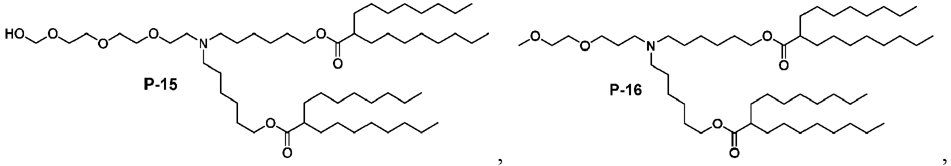

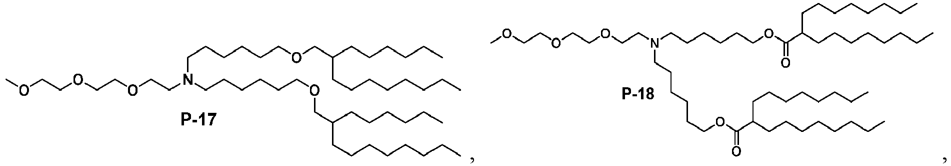

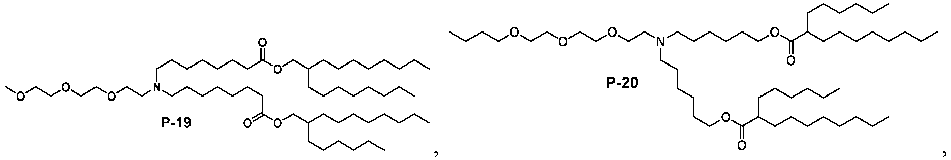

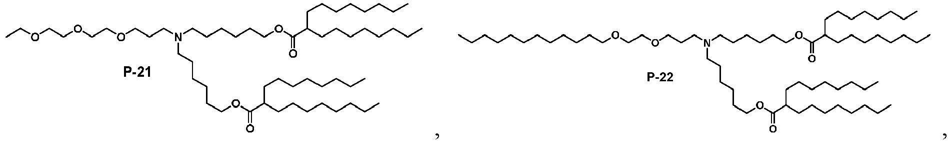

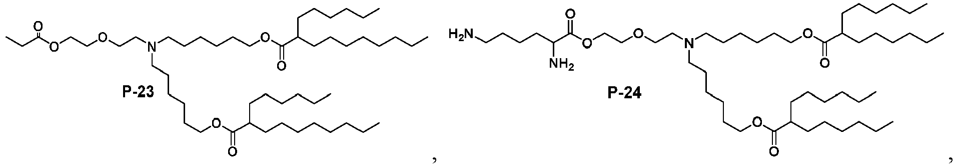

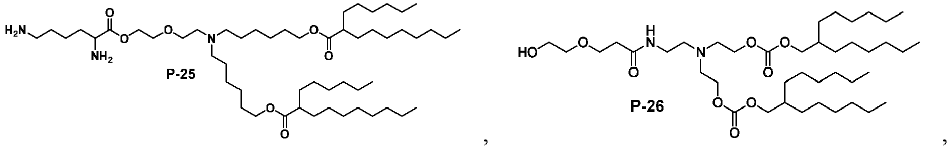

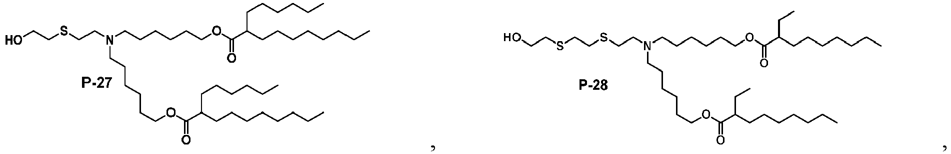

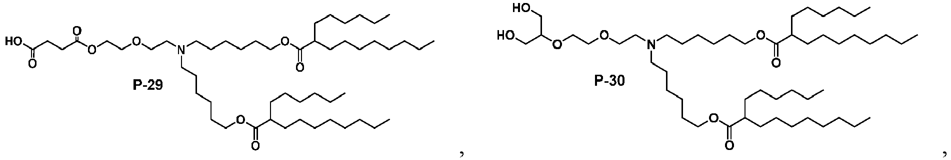

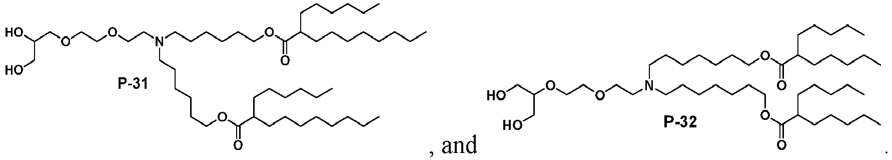

In one specific embodiment of the present invention, the cationic lipids in the cationic liposomes can be selected from not only the cationic lipids represented by the general formula (1), but also the group consisting of *N,N*-dioleyl-*N,N*-dimethylammonium chloride (DODAC), *N,N-*distearyl-*N,N-*dimethylammonium bromide (DDAB), 1,2-dioleoyl-3-trimethylammonium-propane (DOTAP), *N*-[1-(2,3-dioleoyloxy)propyl]-*N,N,N-*trimethylammonium chloride (DOTMA) 1,2-dioleyloxy-*N,N*-dimethylaminopropane (DODMA), 3-(didodecylamino)-*N1,N1*,4-tridodecyl-1-piperazineethanamine (KL10), *N1*-[2-(didodecylamino)ethyl]-*N1,N4,N4*-tridodecyl-1,4-piperazinediethanamine (KL22), 14,25-ditridecyl-15,18,21,24-tetraaza-octatriacontane (KL25), 1,2-dilinoleyloxy-*N,N-*dimethylaminopropane (DLin-DMA), 2,2-dilinoleyl-4-dimethylaminomethyl-[1,3]-dioxolane (DLin-K-DMA), heptatriaconta-6,9,28,31-tetraen-19-yl 4-(dimethylamino)butanoate (DLin-MC3-DMA), 2,2-dilinoleyl-4-(2-dimethylaminoethyl)-[1,3]-dioxolane (DLin-KC2-DMA), and mixtures thereof.

In one specific embodiment of the present invention, any foregoing cationic liposome preferably contains 20% to 80% cationic lipids represented by the general formula (1), 5% to 15% neutral lipids, 25% to 55% steroid lipids, and 0.5% to 10% PEGylated lipids, said percentage is the molar percentage of each lipid relative to the total lipids in a solution containing the solvent.

In one specific embodiment of the present invention, it is preferable that in any foregoing cationic liposome, the molar percentage of cationic lipids relative to the total lipids in a solution containing the solvent is 30% to 65%; more preferably about 35%, 40%, 45%, 46%, 47%, 48%, 49%, 50%, or 55%.

In one specific embodiment of the present invention, it is preferable that in any foregoing cationic liposomes, the molar percentage of neutral lipids relative to the total lipids in a solution containing the solvent is 7.5% to 13%; more preferably about 8%, 9%, 10%, 11%, or 12%.

In one specific embodiment of the present invention, it is preferable that in any foregoing cationic liposomes, the molar percentage of steroid lipids relative to the total lipids in a solution containing the solvent is 35% to 50%; more preferably about 40%, 41%, 42%, 43%, 44%, 45%, 46%, 47%, 48%, 49%, or 50%.

In one specific embodiment of the present invention, it is preferable that in any foregoing cationic liposomes, the molar percentage of PEGylated lipids relative to the total lipids in a solution containing the solvent is 0.5% to 5%; preferably 1% to 3%; more preferably about 1.5%, 1.6%, 1.7%, 1.8%, or 1.9%.

### 3.2. Preparation of cationic liposomes

In the present invention, cationic liposomes can be prepared by the following methods, including but not limited to thin-film dispersion method, ultrasonic dispersion method, reverse phase evaporation method, freeze-drying method, freeze-thaw method, double emulsion method and/or injection method, preferably film dispersion method, ultrasonic dispersion method and/or reverse phase evaporation method.

In the preparation methods of the cationic liposomes of the present invention, said thin-film dispersion method could include the following steps:
Step (1): weigh cationic lipids, steroid lipids, neutral lipids, and PEGylated lipids, dissolve them fully in organic solvents, shake well, remove the organic solvents by rotary evaporation under reduced pressure to form an oil film, and dry with a vacuum pump to remove the organic solvents;
Step (2): add a phosphate buffer solution containing dissolved cryoprotectants, and use water-bath ultrasonication to obtain a translucent emulsion;
Step (3): add the emulsion to a high-pressure homogenizer for overpressure, and then add the overpressurized emulsion to the liposome extruder to pass through films to obtain cationic liposomes;
Step (4): optionally, dry said cationic liposomes in a freeze dryer to obtain cationic liposome powders;
   wherein, said organic solvent is preferably selected from dichloromethane, chloroform, and/or methanol, more preferably chloroform and methanol; the rotational speed of said rotary evaporation under reduced pressure is preferably 30-300 rpm, more preferably 50-200 rpm, and most preferably 100-170 rpm; the temperature of said rotary evaporation under reduced pressure is preferably 10~200°C, more preferably 20~200°C, and most preferably 40~80°C;
   the time of said drying with a vacuum pump is preferably 1-72 h, more preferably 5-48 h, and most preferably 15-36 h;
   the mass concentration of said cryoprotectants dissolved in a phosphate buffer solution is preferably 0.1-80%, preferably 1-50 %, and more preferably 5-20%;
   the frequency of said water-bath ultrasonication is preferably 10-300 kHz, more preferably 30-200 kHz, and most preferably 60-150 kHz;
   the time of said water-bath ultrasonication is preferably 0.1-5 h, more preferably 0.2-2 h, and most preferably 0.25~1 h;
   the pressure of said high-pressure homogenizer is preferably 50-240 MPa, more preferably 80-200 MPa, and most preferably 100-150 MPa;
   the times of said overpressure in the high-pressure homogenizer is preferably any integer from 1 to 50, more preferably any integer from 3 to 20, and most preferably any integer from 5 to 10;
   the pressure of said liposome extruder is preferably 50~300 MPa, more preferably 80-250 MPa, and most preferably 120-200 MPa;
   the times of said passing through films in the liposome extruder is preferably any integer from 1 to 50, more preferably any integer from 3 to 30, and most preferably any integer from 5 to 20;
   the time of said drying in a freeze dryer is preferably 1~120 h, more preferably 5~72 h, and most preferably 10-36 h.

In the preparation methods of the cationic liposomes of the present invention, the ratio of the cationic liposomes to the phosphate buffer solution containing dissolved cryoprotectants could be 1 mg : (0.1~100) mL, preferably 1 mg : (0.3~50) mL, and more preferably 1 mg : (0.5~5) mL.

### 4. Cationic liposome pharmaceutical composition

In one embodiment of the present invention, provided herein is a cationic liposome pharmaceutical composition containing any foregoing cationic liposome and a drug, wherein the cationic liposome contains any foregoing PEGylated lipid whose structure is represented by the general formula (2).

In one specific embodiment of the present invention, in a cationic liposome pharmaceutical composition, the drug is preferably a nucleic acid drug or an anti-tumor agent; said nucleic acid drug is selected from the group consisting of DNA, antisense nucleic acid, plasmid, mRNA (messenger RNA), interfering nucleic acid, aptamer, miRNA inhibitor (antagomir), microRNA (miRNA), ribozyme, and small interfering RNA (siRNA), and preferably the group consisting of RNA, miRNA and siRNA.

In one specific embodiment of the present invention, a cationic liposome pharmaceutical composition is preferably used as a drug, selected from the group consisting of drugs for treating cancer, drugs for treating malignant tumor, anti-infectious agents, antiviral agents, antifungal agents, and vaccines.

In the present invention, said drugs are further preferably selected from those including but not limited to doxorubicin, mitoxantrone, camptothecin, cisplatin, bleomycin, cyclophosphamide, streptozotocin, actinomycin D, vincristine, vinblastine, cytosine arabinoside, anthracycline, nitrogen mustard, tioteppa, chlorambucil, rachelmycin, melphalan, carmustine, romustine, busulfan, dibromannitol, mitomycin C, cis-diammineplatinum(II) dichloride, methotrexate, 6-mercaptopurine, 6-thioguanine, cytosine arabinoside, 5-fluorouracil dacarbazine, dibucaine, chlorpromazine, propranolol, demorol, labetalol, clonidine, hydralazine, imipramine, amitriptyline, doxepin, phenytoin, diphenhydramine, chlorpheniramine, promethazine, gentamicin, ciprofloxacin, cefoxitin, miconazole, terconazole, econazole, isoconazole, butoconazole, clotrimazole, itraconazole, nystatin, naftifine, amphotericin B, antiparasitic agents, hormones, hormone antagonists, immunomodulators, neurotransmitter antagonists, glaucoma drugs, vitamins, tranquilizers, imaging agents, taxol, cytochalasin B, gramicidin D, ethidium bromide, emetine, mitomycin, etoposide, teniposide, colchicine, daunorubicin, quinizarin, mithramycin, 1-dihydrotestosterone, glucocorticoid, procaine, tetracaine, lidocaine, puromycin, and maytansinoid.

In one specific embodiment of the present invention, the N/P ratio of said cationic liposomes to said nucleic acids is preferably (0.1~100):1, more preferably (0.2~30):1, and most preferably (0.5-20): 1.

In one specific embodiment of the present invention, the working solution of said formulation of nucleic acid pharmaceutical composition is preferably deionized water, ultrapure water, phosphate buffer, or physiological saline, more preferably phosphate buffer or physiological saline, and most preferably physiological saline; the ratio of the cationic liposomes to the working solution is preferably (0.05~20) g:100 mL, more preferably (0.1~10) g:100 mL, and most preferably (0.2~5) g:100 mL.

### 5. Formulation of cationic liposome pharmaceutical composition

In the present invention, provided herein is a formulation of cationic liposome pharmaceutical composition containing any foregoing cationic liposome pharmaceutical composition and pharmaceutically acceptable diluents or excipients; said diluents or excipients are preferably selected from the group consisting of deionized water, ultrapure water, phosphate buffer, and physiological saline, more preferably selected from phosphate buffer and physiological saline, and most preferably physiological saline.

In the present invention, the preparation method of the formulation of cationic liposome-nucleic acid pharmaceutical composition includes the following steps:
Step (1): equilibrate said cationic liposome in said diluents or excipients;
Step (2): complex a nucleic acid drug with the equilibrated mixture of the cationic liposome and the diluents or excipients;
   wherein, the time of said equilibration is 0.1~12 h, preferably 0.2~6 h, and more preferably 0.5-3 h; the time of said complexation is 0.1-12 h, preferably 0.2-5 h, and more preferably 0.5-2 h.

The following is a further description with specific examples of the preparation methods of cationic lipids, cationic liposomes, and cationic liposome pharmaceutical compositions, and the biological activity assays for cationic liposome pharmaceutical compositions. The specific examples are for further illustration of the present invention, not limiting the protected scope of present invention. Wherein, in the embodiments of preparing PEGylated lipids, the structures of end-products were characterized by NMR, and the molecular weight was determined by MALDI-TOF.

### Example-1: synthesis of methoxy-PEGylated lipid E1-1

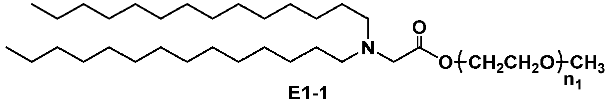

**E1-1** corresponds to the general formula (2); wherein, R₁ and R₂ are both tetradecyl groups, B₃ and B₄ are both linking bonds, L₇ and L₈ are both linking bonds, L₃ is -CH₂C(=O)- or -CH₂C(=O)O-, A is -OCH₂CH₂- or -CH₂CH₂O-, Rₐ and R_{b} are both hydrogen atoms, s is 2, n₁≈45, R is a methyl group or a methoxy group, and the total molecular weight is approximately 2460 Da.

The preparation process is represented as follows:
Step a: Compound **S1-1** (5.02 g, 22.0 mmol), 1-ethyl-3-(3-dimethylaminopropyl)carbodiimide hydrochloride (**EDCI**, 5.07 g, 26.4 mmol), and *N-*hydroxysuccinimide (**NHS**, 2.76 g, 24.2 mmol) were dissolved in dichloromethane (300 mL), and the reaction was stirred at room temperature overnight. After completion of the reaction, the reaction solution was backwashed twice with 0.1 mol/L HCl solution (150 mL*2), and backwashed once with saturated NaCl solution (150 mL). The dichloromethane phase was collected, dried with anhydrous MgSO₄, filtered, and concentrated to obtain the target compound **S1-2** (7.08 g, 99%).
Step b: The above-said compound **S1-2** (6.50 g, 20.0 mmol) was dissolved in dichloromethane (300 mL), compound **S1-3** (4.69 g, 22.0 mmol) and triethylamine (**TEA**, 3.03 g, 30.0 mmol) were added and the reaction was stirred at room temperature overnight to precipitate a large amount of solid. After completion of the reaction, the solid was filtered and pulped with methanol (100 mL), filtered, and rinsed twice with methanol. The solid was collected and dried to obtain the target compound **S1-4** (7.00 g, 82.5%).
Step c: Into an oven-dried reaction vial, the above-said compound S1-4 (6.36 g, 15.0 mmol) was added at room temperature and stirred, and the 1 mol/L borane-tetrahydrofuran solution (**BH₃·THF**, 32 mL) was added slowly. After that, the reaction solution was heated to 70°C and the reaction was conducted overnight. After completion of the reaction, the reaction solution was placed in an ice bath, the 0.5 mol/L HCl solution was added slowly to quench the reaction, and a large number of solid was precipitated. The reaction was heated to 70°C, stirred and continued overnight. After completion of the reaction, the reaction solution was cooled to room temperature and filtered, and the filter cake was rinsed with water and suction dried. The solid was collected, dissolved in methanol at 60°C, recrystallized in an ice bath, and filtered to obtain the target compound **S1-5** (4.51 g).
Step d: Into the round-bottom flask containing compound **S1-6** (4.20 g, 2.0 mmol; wherein, **S1-6** is a product of the reaction between 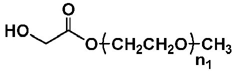 and methyl sulfonyl chloride, Mw≈2100, n₁≈45, PDI=1.02), 200 mL water was added and the reaction was stirred at room temperature to dissolve the compound. Compound **S1-5** (4.10 g, 10.0 mmol), **potassium carbonate** (2.76 g, 20.0 mmol) and **tetra-*n*-butylammonium bromide** (0.06 g, 0.2 mmol) were added successively, and the reaction was stirred at room temperature for 72 h. After completion of the reaction, the reaction solution was extracted twice with dichloromethane (200 mL*2), the organic phases were combined, backwashed once with saturated NaCl solution (100 mL), and then the organic phase was retained, dried with anhydrous MgSO₄, filtered, and concentrated to obtain the crude compound **E1-1**. The crude product was purified by column chromatography, concentrated, and dried with an oil pump to obtain the **PEGylated lipid E1-1** (3.36 g). ¹H NMR (500 MHz, CDCl₃) δ: 4.21 (t, 2H), 3.83-3.45 (m, 178H), 3.38 (s, 3H), 3.30 (t, 2H), 2.49 (t, 4H), 1.56-1.46 (m, 4H), 1.26-1.19 (m, 44H), 0.87 (t, 6H). The molecular weight of **E1-1** was determined to be 2461 Da by MALDI-TOF, PDI=1.02.

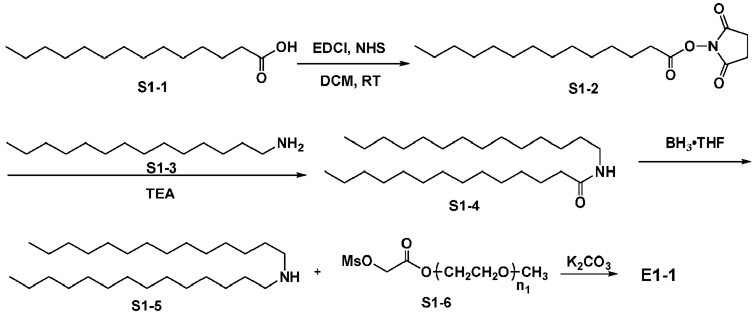

### Example-2: synthesis of methoxy-PEGylated lipid E2-1

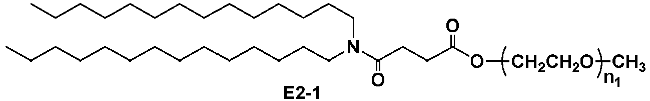

**E2-1** corresponds to the general formula (2); wherein, R₁ and R₂ are both tetradecyl groups, B₃ and B₄ are both linking bonds, L₇ and L₈ are both linking bonds, L₃ is - C(=O)CH₂CH₂C(=O)- or -C(=O)CH₂CH₂C(=O)O-, A is -OCH₂CH₂- or -CH₂CH₂O-, Rₐ and R_{b} are both hydrogen atoms, s is 2, n₁≈40, R is a methyl group or a methoxy group, and the total molecular weight is approximately 2280 Da.

The preparation process is represented as follows:
Compound **S2-1** (5.70 g, 3.0 mmol, 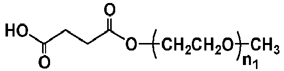 Mw≈1900, n₁≈40, PDI=1.02) was dissolved in dichloromethane (200 mL), compound **S1-5** (1.85 g, 4.5 mmol), 1-ethyl-3-(3-dimethylaminopropyl)carbodiimide hydrochloride (**EDCI**, 1.15 g, 6.0 mmol), 1-hydroxybenzotriazole (**HOBt**, 0.61 g, 4.5 mmol), and triethylamine (**TEA**, 0.61 g, 6.0 mmol) were added in batches and the reaction was stirred at room temperature overnight. After completion of the reaction, the reaction solution was backwashed twice with 0.1 mol/L HCl aqueous solution (10% NaCl) (200 mL*2), and then backwashed once with saturated sodium chloride solution (100 mL). The organic phase was dried with anhydrous MgSO₄, filtered, and concentrated to obtain the crude product. Furthermore, the crude product was purified by column chromatography, concentrated, and dried with an oil pump to obtain the **PEGylated lipid E2-1** (5.39 g). ¹H NMR (500 MHz, CDCl₃) δ: 4.17 (t, 2H), 3.83-3.45 (m, 158H), 3.38 (s, 3H), 3.20 (t, 2H), 3.16 (t, 2H), 2.62 (t, 2H), 2.37 (t, 2H), 1.55-1.46 (m, 4H), 1.27-1.18 (m, 44H), 0.86 (t, 6H). The molecular weight of **E2-1** was determined to be 2283 Da by MALDI-TOF, PDI=1.02.

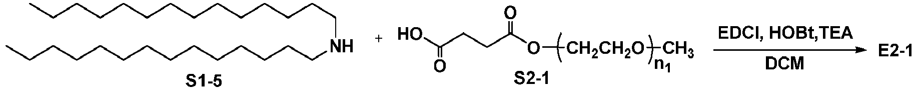

### Example-3: synthesis of methoxy-PEGylated lipid E3-1

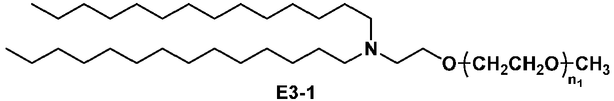

**E3-1** corresponds to the general formula (2); wherein, R₁ and R₂ are both tetradecyl groups, B₃ and B₄ are both linking bonds, L₇ and L₈ are both linking bonds, L₃ is -CH₂CH₂O- or -CH₂CH₂-, A is -OCH₂CH₂- or -CH₂CH₂O-, Rₐ and R_{b} are both hydrogen atoms, s is 2, n₁≈45, R is a methyl group or a methoxy group, and the total molecular weight is approximately 2450 Da.

The preparation process is represented as follows:
Step a: Compound **S3-1** (10.00 g, 5.0 mmol, mPEG-OH, Mw≈2000, n₁≈45, PDI=1.03), and **toluene** (300 mL) performed azeotropic water removal at 140°C. After 100 mL of solvent was steamed out, the temperature of reaction solution was cooled to room temperature. Triethylamine (**TEA**, 1.01 g, 10.0 mmol) and methyl sulfonyl chloride (**MsCl**, 1.03 g, 9.0 mmol) were added and the reaction was stirred at room temperature overnight. After completion of the reaction, the reaction solution was poured into water (120 mL), extracted twice with EtOAc (200 mL*2), and the water phase was retained and extracted twice with dichloromethane (200 mL*2). The organic phases were combined, dried, filtered, concentrated, dissolved in isopropanol at 50°C, recrystallized in an ice bath, and filtered to obtain the compound **S3-2** (9.45 g, 90%).
Step b: Into the round-bottom flask containing compound **S3-2** (6.30 g, 3.0 mmol), 300 mL water was added and the reaction was stirred at room temperature to dissolve said compound. **Potassium carbonate** (12.42 g, 90.0 mmol), compound **S3-3** (3.20 g, 15.0 mmol), and **tetra-n-butylammonium bromide** (0.10 g, 0.3 mmol) were added successively, and the reaction was stirred at room temperature for 72 h. After completion of the reaction, the reaction solution was extracted twice with dichloromethane (300 mL*2), the organic phases were combined, backwashed with saturated NaCl solution (200 mL), then the organic phase was retained, dried with anhydrous MgSO₄, filtered, and concentrated to obtain the crude product of **S3-4.** Furthermore, the crude product was purified by column chromatography, concentrated, and dried with an oil pump to obtain the target compound **S3-4** (4.60 g).
Step c: The above-said compound **S3-4** (2.30 g, 1.0 mmol) was dissolved in anhydrous THF (100 mL), **NaH** (60%, 0.40 g, 10.0 mmol) was added slowly in an ice bath and the reaction was conducted for 1 h in the ice bath. Compound **S3-5** (1.39 g, 5.0 mmol) was added, and the reaction was continued for 1 h in an ice bath; after that, the reaction solution was slowly returned to room temperature. After completion of the reaction, the reaction solution was placed in an ice bath, and 50 mL methanol was added slowly to quench the reaction. After stirring for 30 minutes, water (100 mL) was added and mixed by stirring. The mixture was extracted twice with EtOAc (200 mL*2), and the water phase was retained and extracted twice with dichloromethane (200 mL*2). The organic phases were collected and combined, backwashed with saturated NaCl solution (100 mL), dried with anhydrous sodium sulfate, filtered, and concentrated to obtain the crude product of **E3-1.** The crude product was purified by column chromatography, concentrated, and dried with an oil pump to obtain the **PEGylated lipid E3-1** (1.80 g). ¹H NMR (500 MHz, CDCl₃) δ: 3.85-3.45(m, 182H), 3.37(s, 3H), 2.50 (t, 4H), 2.62 (t, 2H), 1.58-1.48 (m, 4H), 1.36-1.19 (m, 44H), 0.86 (t, 6H). The molecular weight of **E3-1** was determined to be 2447 Da by MALDI-TOF, PDI=1.03.

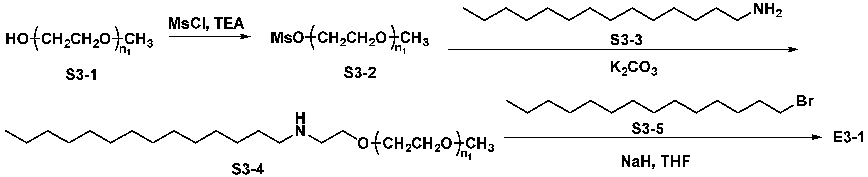

### Example-4: synthesis of methoxy-PEGylated lipid E4-1

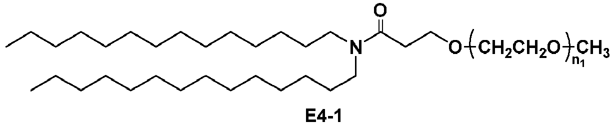

**E4-1** corresponds to the general formula (2); wherein, R₁ and R₂ are both tetradecyl groups, B₃ and B₄ are both linking bonds, L₇ and L₈ are both linking bonds, L₃ is - C(=O)CH₂CH₂O- or -C(=O)CH₂CH₂-, A is -OCH₂CH₂- or -CH₂CH₂O-, Rₐ and R_{b} are both hydrogen atoms, s is 2, n₁≈45, R is a methyl group or a methoxy group, and the total molecular weight is approximately 2480 Da.

The preparation process is represented as follows:
Compound **S4-1** (6.30 g, 3.0 mmol, mPEG-PA, Mw≈2100, n₁≈45, PDI=1.02) was dissolved in dichloromethane (200 mL), and then compound **S1-5** (1.85 g, 4.5 mmol), 1-ethyl-3-(3-dimethylaminopropyl)carbodiimide hydrochloride (**EDCI**, 1.15 g, 6.0 mmol), 1-hydroxybenzotriazole (**HOBt**, 0.61 g, 4.5 mmol), and triethylamine (**TEA**, 0.61 g, 6.0 mmol) were added successively, then stirred at room temperature overnight. After completion of the reaction, the reaction solution was backwashed twice with 0.1 mol/L HCl aqueous solution (10% NaCl) (100 mL*2), and backwashed once with saturated NaCl solution (100 mL). The organic phase was dried with anhydrous MgSO₄, filtered, and concentrated to obtain the crude product. Furthermore, the crude product was purified by column chromatography, concentrated, and dried with an oil pump to obtain the **PEGylated lipid E4-1** (6.30 g, 84%). ¹H NMR (500 MHz, CDCl₃) δ: 3.82-3.44 (m, 182H), 3.36 (s, 3H), 3.26 (t, 2H), 3.17 (t, 2H), 2.92 (t, 2H), 1.56-1.46 (m, 4H), 1.36-1.19 (m, 44H), 0.87 (t, 6H). The molecular weight of **E4-1** was determined to be 2475 Da by MALDI-TOF, PDI=1.02.

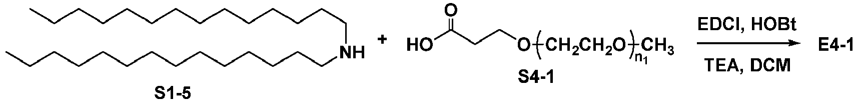

### Example-5: synthesis of methoxy-PEGylated lipid E5-1

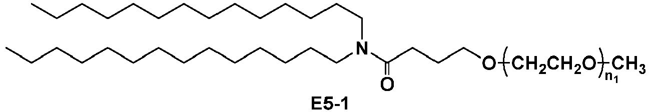

**E5-1** corresponds to the general formula (2); wherein, R₁ and R₂ are both tetradecyl groups, B₃ and B₄ are both linking bonds, L₇ and L₈ are both linking bonds, L₃ is - C(=O)CH₂CH₂CH₂O- or -C(=O)CH₂CH₂CH₂-, A is -OCH₂CH₂- or -CH₂CH₂O-, Rₐ and R_{b} are both hydrogen atoms, s is 2, n₁≈45, R is a methyl group or a methoxy group, and the total molecular weight is approximately 2470 Da.

The preparation process is represented as follows:
Compound **S5-1** (6.30 g, 3.0 mmol, mPEG-BA, Mw≈2100, n₁≈45, PDI=1.03) was dissolved in dichloromethane (200 mL), and then compound **S1-5** (1.85 g, 4.5 mmol), 1-ethyl-3-(3-dimethylaminopropyl)carbodiimide hydrochloride (ED**C**I, 1.15 g, 6.0 mmol), 1-hydroxybenzotriazole (**HOBt**, 0.61 g, 4.5 mmol) and triethylamine (**TEA**, 1.49 g, 10.0 mmol) were added successively, and the reaction was stirred at room temperature overnight. After completion of the reaction, the reaction solution was backwashed twice with 0.1 mol/L HCl aqueous solution (10% NaCl) (100 mL*2), and backwashed once with saturated NaCl solution (100 mL). The organic phase was dried with anhydrous MgSO₄, filtered, and concentrated to obtain the crude product. Furthermore, the crude product was purified by column chromatography, concentrated, and dried with an oil pump to obtain the **PEGylated lipid E5-1** (5.93 g). ¹H NMR (500 MHz, CDCl₃) δ: 3.83-3.45 (m, 182H), 3.38 (s, 3H), 3.27 (t, 2H), 3.18 (t, 2H), 2.73 (t, 2H), 1.83 (m, 2H), 1.56-1.46 (m, 4H), 1.36-1.19 (m, 44H), 0.87 (t, 6H). The molecular weight of **E5-1** was determined to be 2469 Da by MALDI-TOF, PDI=1.03.

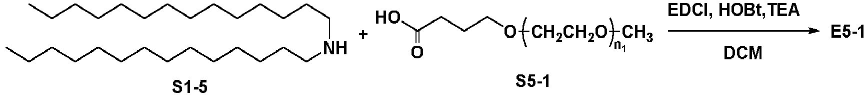

### Example-6: synthesis of methoxy-PEGylated lipid E6-1

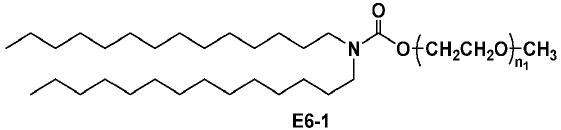

**E6-1** corresponds to the general formula (2); wherein, R₁ and R₂ are both tetradecyl groups, B₃ and B₄ are both linking bonds, L₇ and L₈ are both linking bonds, L₃ is -C(=O)O- or -C(=O)-, A is -OCH₂CH₂- or -CH₂CH₂O-, Rₐ and R_{b} are both hydrogen atoms, s is 2, n₁≈20, R is a methyl group or a methoxy group, and the total molecular weight is approximately 1350 Da.

The preparation process is represented as follows:
Compound **S6-1** (5.00 g, 5.0 mmol, mPEG-SC, Mw≈1000, n₁≈20, PDI=1.03) was dissolved in dichloromethane (200 mL), and then the compound **S1-5** from Example-1 (2.46 g, 6.0 mmol) and triethylamine (**TEA**, 0.75 g, 7.5 mmol) were added, and the reaction was stirred at room temperature overnight. After completion of the reaction, the reaction solution was concentrated to obtain the crude product. Furthermore, the crude product was purified by column chromatography, concentrated, and dried with an oil pump to obtain the **PEGylated lipid E6-1** (5.14 g). ¹H NMR (500 MHz, CDCl₃) δ: 4.21 (t, 2H), 3.83-3.45 (m, 78H), 3.39 (s, 3H), 3.19 (t, 2H), 3.04 (t, 2H), 1.58-1.48 (m, 4H), 1.36-1.19 (m, 44H), 0.86 (t, 6H). The molecular weight of **E6-1** was determined to be 1347 Da by MALDI-TOF, PDI=1.03.

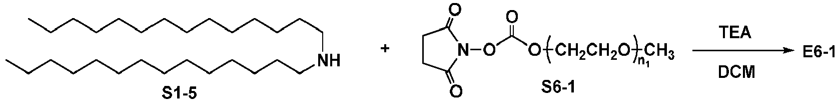

### Example-7: synthesis of hydroxyl-PEGylated lipid E7-1

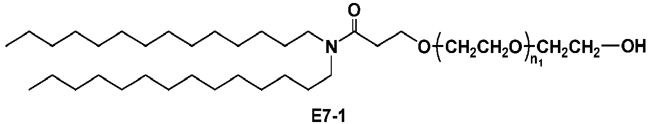

**E7-1** corresponds to the general formula (2); wherein, R₁ and R₂ are both tetradecyl groups, B₃ and B₄ are both linking bonds, L₇ and L₈ are both linking bonds, L₃ is - C(=O)CH₂CH₂O- or -C(=O)CH₂CH₂-, A is -OCH₂CH₂- or -CH₂CH₂O-, Rₐ and R_{b} are both hydrogen atoms, s is 2, n₁≈60, R is -OCH₂CH₂OH or -CH₂CH₂OH, and the total molecular weight is approximately 3170 Da.

The preparation process is represented as follows:
Compound **S7-1** (8.40 g, 3.0 mmol, HO-PEG-CM, Mw≈2800, m~60, PDI=1.02) was dissolved in dichloromethane (300 mL), and then compound **S1-5** (1.85 g, 4.5 mmol), 1-ethyl-3-(3-dimethylaminopropyl)carbodiimide hydrochloride (**EDCI**, 1.15 g, 6.0 mmol), 1-hydroxybenzotriazole (**HOBt**, 0.61 g, 4.5 mmol), and triethylamine (**TEA**, 0.61 g, 6.0 mmol) were added successively, and the reaction was stirred at room temperature overnight. After completion of the reaction, the reaction solution was backwashed twice with 0.1 mol/L HCl aqueous solution (10% NaCl) (150 mL*2), and backwashed once with saturated NaCl solution (150 mL). The organic phase was dried with anhydrous MgSO₄, filtered, and concentrated to obtain the crude product. Furthermore, the crude product was purified by column chromatography, concentrated, and dried with an oil pump to obtain the **hydroxyl-PEGylated lipid E7-1** (7.01 g). ¹H NMR (500 MHz, CDCl₃) δ: 3.83-3.45 (m, 246H), 3.26 (t, 2H), 3.16 (t, 2H), 2.92 (t, 2H), 1.57-1.46 (m, 4H), 1.36-1.20 (m, 44H), 0.87 (t, 6H). The molecular weight of **E7-1** was determined to be 3165 Da by MALDI-TOF, PDI=1.02.

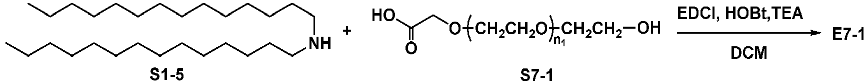

### Example-8: synthesis of ethylamine-PEGylated lipid E8-2

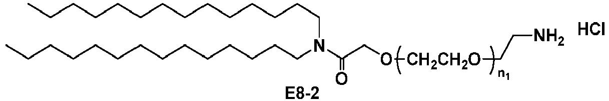

**E8-2** corresponds to the general formula (2); wherein, R₁ and R₂ are both tetradecyl groups, B₃ and B₄ are both linking bonds, L₇ and L₈ are both linking bonds, L₃ is - C(=O)CH₂O- or -C(=O)CH₂-, A is -OCH₂CH₂- or -CH₂CH₂O-, Rₐ and R_{b} are both hydrogen atoms, s is 2, n₁≈45, R is-OCH₂CH₂NH₂ or -CH₂CH₂NH₂, and the total molecular weight is approximately 2530 Da.

The preparation process is represented as follows:
Step a: Compound **S8-1** (12.00 g, 6.0 mmol, mPEG-OH, Mw≈2000, n₁≈45, PDI=1.02), and **toluene** (300 mL) performed azeotropic water removal at 140°C. After 100 mL of solvent was steamed out, the temperature of reaction solution was cooled to room temperature. Triethylamine (**TEA**, 1.21 g, 12.0 mmol) was added, methyl sulfonyl chloride **(MsCl,** 1.23 g, 10.8 mmol) was added dropwise, and the reaction was stirred at room temperature overnight. After completion of the reaction, the reaction solution was poured into water (200 mL) to be quenched, and then backwashed twice with EtOAc (200 mL*2). The water phase was retained, sodium chloride was added, and the reaction solution was extracted twice with dichloromethane (200 mL*2). The organic phases were combined, backwashed once with saturated NaCl solution and the dichloromethane phase was collected. The organic phase was dried with anhydrous MgSO₄, filtered, and concentrated and dried with an oil pump to obtain the compound **S8-2** (12.00 g, 95.2%).
Step b: The above-said compound **S8-2** (10.50 g, 5.0 mmol) was dissolved in ammonia (200 mL) and reacted at room temperature overnight. After completion of the reaction, sodium chloride was added and stirred to be dissolved, and the solution was extracted twice with dichloromethane (200 mL*2). The organic phases were combined, backwashed with saturated NaCl solution and the dichloromethane phase was collected. The organic phase was dried with anhydrous MgSO₄, filtered, and concentrated to obtain the crude product. The crude product was purified by column chromatography, concentrated, and dried with an oil pump to obtain the compound **S8-3** (5.40 g).
Step c: Compound **S8-3** (5.00 g, 2.5 mmol) was dissolved in dichloromethane (200 mL), triethylamine (**TEA**, 0.38 g, 3.8 mmol) and di-*t*-butyl decarbonate (**Boc₂O**, 0.75 g, 3.8 mmol) were added at room temperature, then the reaction was conducted overnight. After completion of the reaction, the reaction solution was concentrated and dissolved in isopropanol at 50°C, recrystallized in an ice bath, and filtered to obtain the compound **S8-4** (4.86 g, 92.6%).
Step d: The above-said compound **S8-4** (4.20 g, 2.0 mmol) was dissolved in 100 mL water, stirred in an ice bath. 2,2,6,6-tetramethylpiperidinooxy (**Tempo,** 1.60 mg, 0.01 mmol) and **KBr** (11.90 mg, 0.1 mmol) were added, 1 mol/L **NaClO** solution (10 mL, 9.6 mmol) was added slowly into the above solution in an ice bath, and the reaction was conducted in an ice bath for 3 h. After completion of the reaction, the pH was adjusted to 2~3 with 4 mol/L HCl solution, the reaction solution was extracted twice with dichloromethane (100 mL*2). The organic phases were combined and backwashed once with saturated NaCl solution. The organic phase was collected, dried with anhydrous MgSO₄, filtered, concentrated, dissolved in isopropanol at 50°C, recrystallized in an ice bath, and filtered to obtain the compound **S8-5** (3.87 g, 88%).
Step e: The above-said compound **S8-5** (3.30 g, 1.5 mmol) was dissolved in dichloromethane (100 mL), and then compound **S1-5** (0.92 g, 2.3 mmol) prepared in Example-1, 1-ethyl-3-(3-dimethylaminopropyl)carbodiimide hydrochloride (**EDCI**, 0.58 g, 3.0 mmol), 1-hydroxybenzotriazole (**HOBt**, 0.30 g, 2.3 mmol), and triethylamine (TEA, 0.30 g, 3.0 mmol) were added successively, and the reaction was stirred at room temperature overnight. After completion of the reaction, the reaction solution was backwashed twice with 0.1 mol/L HCl aqueous solution (10% NaCl) (50 mL*2), and backwashed once with saturated NaCl solution (50 mL). The organic phase was dried with anhydrous MgSO₄, filtered, and concentrated to obtain the crude product. Furthermore, the crude product was purified by column chromatography, concentrated and dried with an oil pump to obtain the target compound **E8-1** (3.01 g). ¹H NMR (500 MHz, CDCl₃) δ: 4.17 (s, 2H), 3.83-3.45 (m, 180-200H), 3.37 (s, 3H), 3.27 (t, 2H), 3.17 (t, 2H), 3.03 (t, 2H), 1.56-1.46 (m, 4H), 1.36-1.19 (m, 45-50H), 0.91 (s, 9H), 0.87 (t, 6H).
Step f: Compound **E8-1** (2.60 g, 1.0 mmol) was dissolved in 1,4-dioxane (5 mL), 4N HCl/dioxane (5 mL) was added in an ice bath by stirring, and the reaction was conducted in an ice bath for 2 h. After completion of the reaction, the reaction solution was poured into glacial n-hexane (50 mL). The solid was precipitated, filtered, collected, and dried with an oil pump to obtain the **ethylamine-PEGylated lipid E8-2** (2.43 g, 97.2%). ¹H NMR (500 MHz, CDCl₃) δ:4.16 (s, 2H), 3.83-3.45 (m, 182H), 3.26 (t, 2H), 3.17 (t, 2H), 2.83 (t, 2H), 1.56-1.46 (m, 4H), 1.35-1.19 (m, 44H), 0.87 (t, 6H). The molecular weight of **E8-2** was determined to be 2526 Da by MALDI-TOF, PDI=1.02.

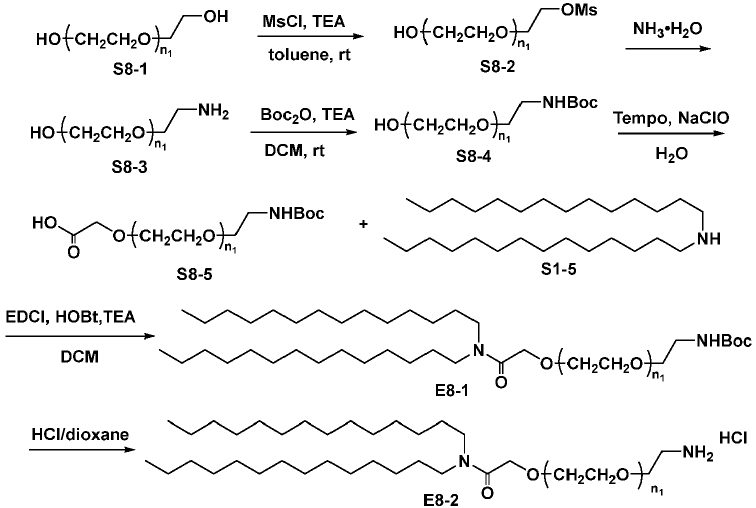

### Example-9: synthesis of methoxy-PEGylated lipid E9-1

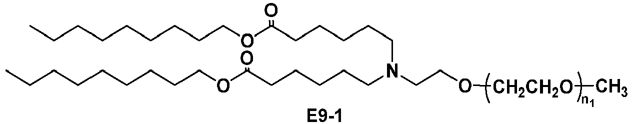

**E9-1** corresponds to the general formula (2); wherein, R₁ and R₂ are both nonyl groups, L₇ and L₈ are both -OC(=O)-, B₃ and B₄ are both pentylidene groups, L₃ is -CH₂CH₂O- or - CH₂CH₂-, A is -OCH₂CH₂- or -CH₂CH₂O-, Rₐ and R_{b} are both hydrogen atoms, s is 2, n₁≈60, R is a methyl group or a methoxy group, and the total molecular weight is approximately 3200 Da.

The preparation process is represented as follows:
Step a: Under argon atmosphere, into the round-bottom flask containing S9-2 (1.45 g, 10.0 mmol), S9-1 (3.90 g, 20.0 mmol) and 4-dimethylaminopyridine **(DMAP,** 0.30 g, 2.5 mmol) dissolved in dichloromethane (200 mL), *N,N'*-dicyclohexylcarbodiimide (DCC, 4.53 g, 22.0 mmol) was added and the reaction was conducted for 16 h at room temperature. After completion of the reaction, the precipitate was removed by filtering. The reaction solution was backwashed twice with saturated NaCl solution (100 mL). The organic phase was dried with anhydrous MgSO₄, filtered, and concentrated, and the residue obtained was purified by column chromatography to obtain the compound S9-3 (2.66 g).
Step b: Under nitrogen protection, the above-said compound S9-3 (1.61 g, 5.0 mmol) was dissolved in acetonitrile (200 mL), S9-4 (5.40 g, 2.0 mmol, 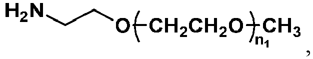 Mw≈2700, n₁≈60, PDI=1.02) and *N*,*N-*diisopropylethylamine (**DIPEA,** 0.36 g, 4.0 mmol) were added successively with stirring slowly, and the reaction was stirred at room temperature for about 20 h. After completion of the reaction, the reaction solution was concentrated, dissolved in dichloromethane, and extracted with 0.6 M HCl/10% NaCl solution and saturated sodium bicarbonate solution, successively. After standing for layering, the organic phases were combined, dried with anhydrous MgSO₄, filtered, concentrated, and purified by column chromatography to obtain the PEGylated lipid **E9-1** (5.12 g). ¹H NMR (500 MHz, CDCl₃) δ: 4.07 (t, 4H), 3.84-3.45 (m, 242H), 3.38 (s, 3H), 2.51 (t, 4H), 2.63 (t, 2H), 2.37 (t, 4H), 1.70-1.19 (m ,40H), 0.86 (t, 6H). The molecular weight of **E9-1** was determined to be 3195 Da by MALDI-TOF, PDI=1.02.

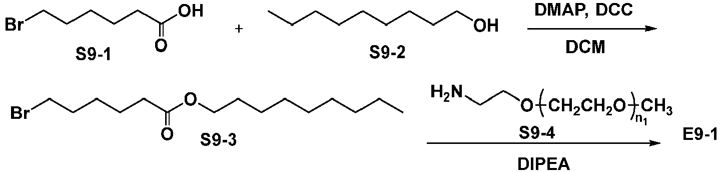

### Example-10: synthesis of propylamine-PEGylated lipid E10-2

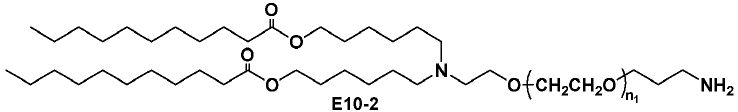

**E10-2** corresponds to the general formula (2); wherein, R₁ and R₂ are both decyl groups, L₇ and L₈ are both -C(=O)O-, B₃ and B₄ are both hexylene groups, L₃ is -CH₂CH₂O- or - CH₂CH₂-, A is -OCH₂CH₂- or -CH₂CH₂O-, Rₐ and R_{b} are both hydrogen atoms, s is 2, n₁≈50, R is -OCH₂CH₂CH₂NH₂ or -CH₂CH₂CH₂NH₂, and the total molecular weight is approximately 2850 Da.

The preparation process is represented as follows:
Step a: Under argon atmosphere, into the round-bottom flask containing **S10-2** (1.77 g, 15.0 mmol), **S10-1** (5.58 g, 30.0 mmol), and 4-dimethylaminopyridine (**DMAP,** 0.46 g, 3.8 mmol) dissolved in dichloromethane (300 mL), *N,N'*-dicyclohexylcarbodiimide (**DCC**, 6.80 g, 33.0 mmol) was added and the reaction was conducted for 16 h at room temperature. After completion of the reaction, the precipitate was removed by filtering. The reaction solution was backwashed twice with saturated NaCl solution (150 mL). The organic phase was dried with anhydrous MgSO₄, filtered, and concentrated to obtain the crude product. Furthermore, the residue obtained was purified by column chromatography to obtain the compound **S10-3** (3.50 g).
Step b: The above-said compound **S10-3** (2.86 g, 10.0 mmol) was dissolved in DCM (100 mL). After the temperature of the solution was cooled to 0°C, 2,2,6,6-tetramethylpiperidinooxy (**Tempo,** 0.73 g, 4.7 mmol) and **KBr** solution (1.32 g, 11.1 mmol) dissolved in 10 mL purified water were added into the above solution, and the **NaClO** solution (1 mL, 12.0 mmol) was added dropwise slowly, after which the TLC was used to monitor the reaction until the starting materials were consumed completely. Sodium sulfite solution was added to quench the reaction. The reaction solution was extracted twice with DCM (100 mL*2) after the temperature of the reaction liquid was returned to room temperature. The organic phase was dried with anhydrous MgSO₄, filtered, and concentrated to obtain the crude product. Furthermore, the crude product was separated and purified by column chromatography, and the target eluent was collected and concentrated to obtain the oxidation product **S10-4** (1.48 g).
Step c: The above oxidation product (**S10-4,** 1.16 g, 4.1 mmol) was dissolved in a mixed solution of THF (200 mL) and methanol (20 mL). After the temperature of the above solution was cooled to 0°C, compound **S10-5** (4.80 g, 2.0 mmol, 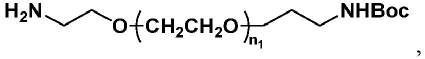 Mw≈2400, n₁≈50, PDI=1.03) and **glacial acetic acid** (0.12 g, 2.0 mmol) were added into the above solution, and then **NaBH(OAc)₃** (1.28 g, 6.1 mmol) was added slowly in batches. After the feeding, the reaction was continued for 2 h, and the TLC was used to monitor the reaction until the starting materials were consumed completely. Saturated sodium bicarbonate solution was added to quench the reaction, and then the reaction solution was returned to room temperature and concentrated to remove THF and methanol. The concentrated solution was extracted twice with 200 mL DCM. The organic phase was dried with anhydrous MgSO₄, filtered, and concentrated to obtain the crude product. Furthermore, the crude product was separated and purified by column chromatography, and the target eluent was collected and concentrated to obtain the product **E10-1** (3.36 g).
Step d: Removal of the Boc protecting group. Into a dry and clean round-bottom flask, a solution of trifluoroacetic acid/dichloromethane (1:2, v/v) was prepared, dichloromethane solution of **E10-1** (3.00 g, 1.0 mmol) was added dropwise slowly in an ice bath, and the reaction was continued for 2 h at room temperature. After completion of the reaction, the reaction solution was concentrated. Purified water was added into the above solution and the solution was then extracted with dichloromethane. The extract was dried with anhydrous MgSO₄, filtered, concentrated, and recrystallized to obtain the **propylamine-PEGylated lipid E10-2** (2.61 g, 90%). ¹H NMR (500 MHz, CDCl₃) δ: 4.07 (t, 4H), 3.83-3.45 (m, 204H), 3.15 (t, 2H), 2.50 (t, 4H), 2.65 (t, 2H), 2.37 (t, 4H), 1.92 (m, 2H), 1.70-1.19 (m, 48H), 0.86 (t, 6H). The molecular weight of **E10-2** was determined to be 2854 Da by MALDI-TOF, PDI=1.03.

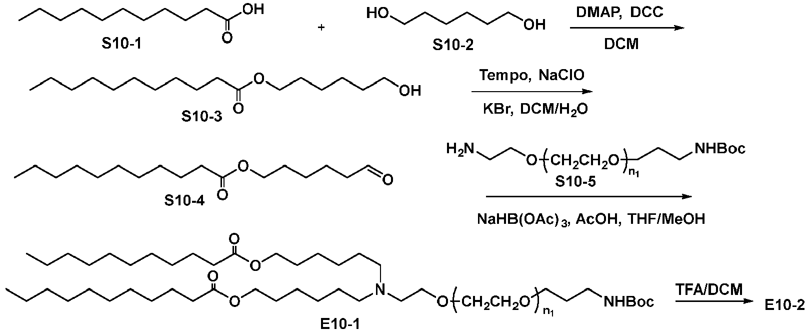

### Example-11: synthesis of methoxy-PEGylated lipid E11-1

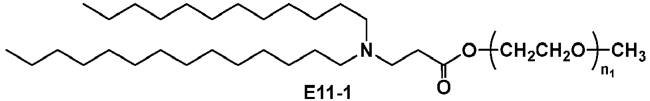

**E11-1** corresponds to the general formula (2); wherein, R₁ and R₂ are dodecyl group and tetradecyl group, respectively; B₃ and B₄ are both linking bonds, L₇ and L₈ are both linking bonds, L₃ is -CH₂CH₂C(=O)- or -CH₂C(=O)O-, A is -OCH₂CH₂- or -CH₂CH₂O-, Rₐ and R_{b} are both hydrogen atoms, s is 2, n₁≈35, R is a methyl group or a methoxy group, and the total molecular weight is approximately 2010 Da.

The preparation process is represented as follows:
Step a: The compound **S1-2** (6.50 g, 20.0 mmol) prepared in **Example-1** was dissolved in 300 mL dichloromethane, and then compound **S11-1** (4.07 g, 22.0 mmol) and triethylamine (**TEA**, 3.03 g, 30.0 mmol) were added and the reaction was conducted at room temperature overnight. After completion of the reaction, a large amount of solid was separated out. The solid was filtered, pulped with methanol (200 mL), filtered, rinsed twice with methanol, collected and dried to obtain the target compound **S11-2** (6.53 g).
Step b: Into an oven-dried reaction vial, the above-said compound **S11-2** (5.94 g, 15.0 mmol) was added at room temperature and stirred, and 1 mol/L borane tetrahydrofuran solution (**BH₃·THF,** 32 mL) was added slowly. After that, the reaction solution was heated to 70°C and the reaction was conducted overnight. After completion of the reaction, the reaction solution was placed in an ice bath, 0.5 mol/L HCl solution was added slowly to quench the reaction, and a large amount of solid was separated out. The reaction was heated to 70°C and the reaction was conducted overnight. After completion of the reaction, the reaction solution was returned to room temperature and filtered, and the filter cake was rinsed with water and suction dried. The solid was collected, dissolved in methanol at 60°C, recrystallized in an ice bath, and filtered to obtain the target compound **S11-3** (4.24 g).
Step c: Into the round-bottom flask containing compound **S11-4** (3.40 g, 2.0 mmol, 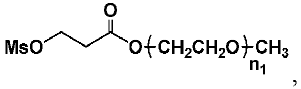 Mw≈1700, n₁≈35, PDI=1.03), 200 mL water was added and the reaction was stirred at room temperature to dissolve said compound. **Potassium carbonate** (2.76 g, 20.0 mmol), compound **S11-3** (3.82 g, 10.0 mmol), and **tetra-*n*-butylammonium bromide** (0.06 g, 0.2 mmol) were added successively, and the reaction was stirred at room temperature for 72 h. After completion of the reaction, the reaction solution was extracted twice with dichloromethane (200 mL*2). The organic phases were combined, backwashed once with saturated NaCl solution (200 mL). The organic phase was retained, dried with anhydrous MgSO₄, filtered, and concentrated to obtain the crude product of compound **E11-1.** Furthermore, the crude product was purified by column chromatography, concentrated and dried with an oil pump to obtain the **PEGylated lipid E11-1** (2.68 g). ¹H NMR (500 MHz, CDCl₃) δ: 4.07 (t, 2H), 3.84-3.45 (m, 140H), 3.38 (s, 3H), 2.49 (t, 4H), 2.37 (t, 2H), 1.56-1.47 (m, 4H), 1.36-1.19 (m, 40H), 0.87 (t, 6H). The molecular weight of **E11-1** was determined to be 2008 Da by MALDI-TOF, PDI=1.03.

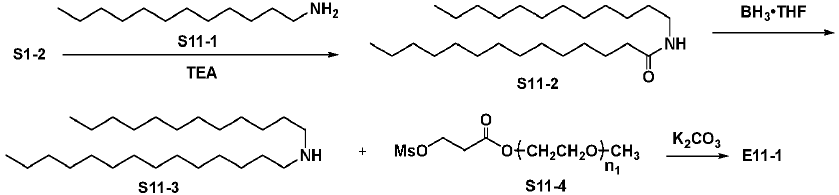

### Example-12: synthesis of ethyl ester-PEGylated lipid E12-1

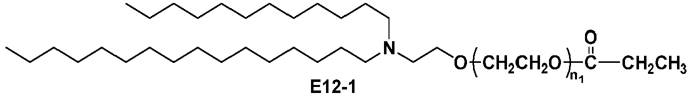

**E12-1** corresponds to the general formula (2); wherein, R₁ is a dodecyl group, R₂ is a hexadecyl group, B₃ and B₄ are both linking bonds, L₇ and L₈ are both linking bonds, L₃ is - CH₂CH₂O- or -CH₂CH₂-, A is -OCH₂CH₂- or -CH₂CH₂O-, Rₐ and R_{b} are both hydrogen atoms, s is 2, n₁≈80, R is -C(=O)-CH₂CH₃ or -OC(=O)-CH₂CH₃, and the total molecular weight is approximately 4030 Da.

The preparation process is represented as follows:
Step a: Compound **S12-1** (10.80 g, 3.0 mmol, 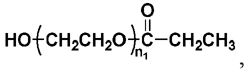 Mw≈3600, n₁≈80, PDI=1.03), and **toluene** (300 mL) performed azeotropic water removal at 140 °C. After 100 mL of solvent was steamed out, the temperature of reaction solution was cooled to room temperature. Triethylamine (**TEA**, 2.98 g, 20.0 mmol) and methyl sulfonyl chloride (**MsCl**, 0.62 g, 5.4 mmol) were added and the reaction was stirred at room temperature overnight. After completion of the reaction, the reaction solution was poured into water (200 mL), extracted twice with EtOAc (200 mL*2), and the water phase was retained and extracted twice with dichloromethane (200 mL*2). The organic phases were combined, dried, filtered, concentrated, dissolved in isopropanol at 50°C, recrystallized in an ice bath, and filtered to obtain the compound **S12-2** (9.99 g, 90%).
Step b: 300 mL water was added to dissolve the above-said compound **S12-2** (7.40 g, 2.0 mmol) while stirring at room temperature. **Potassium carbonate** (2.76 g, 20.0 mmol), compound **S12-3** (2.41 g, 10.0 mmol), and **tetra-*n*-butylammonium bromide** (0.06 g, 0.2 mmol) were added, and the reaction was stirred at room temperature for 72 h. After completion of the reaction, the reaction solution was extracted twice with dichloromethane (100 mL*2), the organic phases were combined and backwashed once with saturated NaCl solution (100 mL), and then the organic phase was retained, dried with anhydrous MgSO₄, filtered, and concentrated to obtain the crude product of **S12-4.** Furthermore, the crude product was purified by column chromatography, concentrated and dried with an oil pump to obtain the target compound **S12-4** (5.29 g).
Step c: The above-said compound **S12-4** (3.90 g, 1.0 mmol) was dissolved in anhydrous THF (100 mL), **NaH** (60%, 0.40 g, 10.0 mmol) was added slowly in an ice bath, and the reaction was conducted for 1 h. Compound **S12-5** (1.25 g, 5.0 mmol) was added and reacted for 1 h in an ice bath; after that, the reaction solution was slowly returned to room temperature, and the reaction was conducted overnight. After completion of the reaction, the reaction solution was placed in an ice bath, and 50 mL methanol was added slowly to quench the reaction. After stirring for 30 minutes, water (100 mL) was added and mixed by stirring. The mixture was extracted twice with EtOAc (100 mL*2), and the water phase was retained and extracted twice with dichloromethane (100 mL*2). The organic phases were collected, combined, backwashed once with saturated NaCl solution (100 mL), dried with anhydrous sodium sulfate, filtered, and concentrated to obtain the crude product of **E12-1.** The crude product was purified by column chromatography, concentrated, and dried with an oil pump to obtain the **ethyl ester-PEGylated lipid E12-1** (3.02 g). ¹H NMR (500 MHz, CDCl₃) δ: 4.07 (t, 2H), 3.83-3.45 (m, 320H), 2.50 (t, 4H), 2.37 (t, 2H), 2.25 (m, 2H), 1.56-1.46 (m, 4H), 1.35-1.20 (m, 44H), 1.16 (t, 3H), 0.86 (t, 6H). The molecular weight of **E12-1** was determined to be 4029 Da by MALDI-TOF, PDI=1.03.

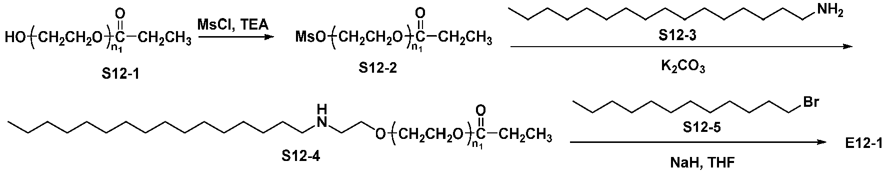

### Example-13: synthesis of butyl acid-PEGylated lipid E13-2

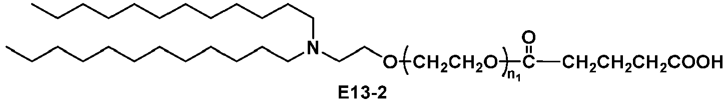

**E13-2** corresponds to the general formula (2); wherein, R₁ and R₂ are both dodecyl groups, B₃ and B₄ are both linking bonds, L₇ and L₈ are both linking bonds, L₃ is -CH₂CH₂O- or -CH₂CH₂-, A is -OCH₂CH₂- or -CH₂CH₂O-, Rₐ and R_{b} are both hydrogen atoms, s is 2, n₁≈40, R is -C(=O)CH₂CH₂CH₂COOH or -OC(=O)CH₂CH₂CH₂COOH, and the total molecular weight is approximately 2270 Da.

The preparation process is represented as follows:
Step a: Compound **S13-1** (11.40 g, 6.0 mmol, 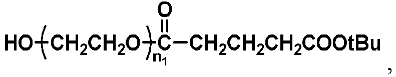 Mw≈2000, n₁≈40, PDI=1.02, a carboxyl group protected product after reaction between PEG and glutaric anhydride, and carboxyl protecting reaction), and **toluene** (300 mL) performed azeotropic water removal at 140°C. After 100 mL of solvent was steamed out, the temperature of reaction solution was cooled to room temperature. Triethylamine (**TEA**, 1.21 g, 12.0 mmol) and methyl sulfonyl chloride (**MsCl,** 1.23 g, 10.8 mmol) were added and the reaction was stirred at room temperature overnight. After completion of the reaction, the reaction solution was poured into water (200 mL) and extracted twice with EtOAc (200 mL*2), and the water phase was retained and extracted twice with dichloromethane (200 mL*2). The organic phases were combined, dried, filtered, concentrated, dissolved in isopropanol at 50°C, recrystallized in an ice bath, and filtered to obtain the compound **S13-2** (10.94 g, 90%).
Step b: 100 mL water was added to dissolve the above-said compound **S13-2** (6.00 g, 3.0 mmol) while stirring at room temperature. **Potassium carbonate** (4.14 g, 30.0 mmol), compound **S13-3** (2.78 g, 15.0 mmol), and **tetra-*n*-butylammonium bromide** (0.10 g, 0.3 mmol) were added, and the reaction was stirred at room temperature for 72 h. After completion of the reaction, the reaction solution was extracted twice with dichloromethane (100 mL*2). The organic phases were combined, backwashed with saturated NaCl solution (100 mL). The organic phase was retained, dried with anhydrous MgSO₄, filtered, and concentrated to obtain the crude product of **S13-4.** Furthermore, the crude product was purified by column chromatography, concentrated, and dried with an oil pump to obtain the target compound **S13-4** (44.47 g).
Step c: The above-said compound **S13-4** (4.40 g, 2.0 mmol) was dissolved in anhydrous THF (200 mL), **NaH** (60%, 0.80 g, 20.0 mmol) was added slowly in an ice bath and the reaction was conducted for 1 h. Compound **S12-5** (2.49 g, 10.0 mmol) was added and reacted for 1 h in an ice bath; after that, the reaction solution was slowly returned to room temperature and the reaction was conducted overnight. After completion of the reaction, the reaction solution was placed in an ice bath, and 50 mL methanol was added slowly to quench the reaction. After stirring for 30 minutes, water (200 mL) was added and mixed with stirring. The mixture was extracted twice with EtOAc (200 mL*2), and the water phase was retained and extracted twice with dichloromethane (200 mL*2). The organic phases were collected, combined, backwashed with saturated NaCl solution (100 mL), dried with anhydrous sodium sulfate, filtered, and concentrated to obtain the crude product of **E13-1.** The crude product was purified by column chromatography, concentrated, and dried with an oil pump to obtain the target compound **E13-1** (3.47 g).
Step d: The above-said compound **E13-1** (2.30 g, 1.0 mmol) was dissolved in dichloromethane (80 mL), **trifluoroacetic acid** (80 mL) was added and the reaction was conducted for 12 h at room temperature. After completion of the reaction, the reaction solution was concentrated, some purified water was added and the reaction solution was extracted with dichloromethane. The extract was dried with anhydrous MgSO₄, filtered, concentrated, and recrystallized to obtain the **butyl acid-PEGylated lipid E13-2** (1.68 g). ¹H NMR (500 MHz, CDCl₃) δ: 4.21 (t, 2H), 3.84-3.45 (m, 160H), 2.50 (t, 4H), 2.39-2.36 (t, 4H), 2.30 (t, 2H), 1.81-1.71 (m, 2H), 1.57-1.47 (m, 4H), 1.35-1.19 (m, 36H), 0.88 (t, 6H). The molecular weight of **E13-2** was determined to be 2271 Da by MALDI-TOF, PDI=1.02.

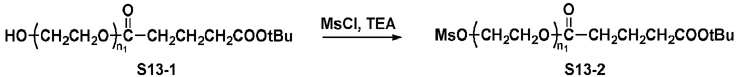

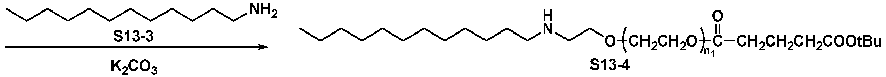

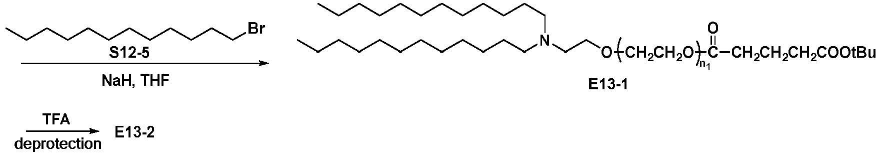

### Example-14: synthesis of glycol-PEGylated lipid E14-2

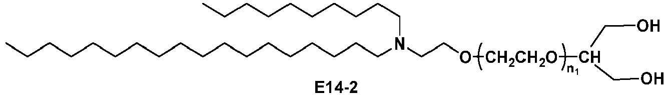

**E14-2** corresponds to the general formula (2); wherein, R₁ is a decyl group, R₂ is an octadecyl group, B₃ and B₄ are both linking bonds, L₇ and L₈ are both linking bonds, L₃ is - CH₂CH₂O- or -CH₂CH₂-, A is -OCH₂CH₂- or -CH₂CH₂O-, Rₐ and R_{b} are both hydrogen atoms, s is 2, n₁≈250, R is 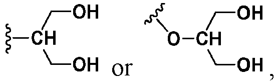 and the total molecular weight is approximately 11500 Da.

The preparation process is represented as follows:
Step a: Compound **S14-1** (14.69 g, 1.3 mmol, 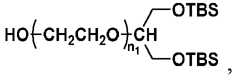 Mw≈11300, n₁≈250, PDI=1.02, a product of reaction between 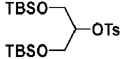 and PEG) and **toluene** (300 mL) performed azeotropic water removal at 140°C. After 100 mL of solvent was steamed out, the temperature of reaction solution was cooled to room temperature. Triethylamine (**TEA**, 0.26 g, 2.6 mmol) and methyl sulfonyl chloride (**MsCl**, 0.27 g, 2.3 mmol) were added and the reaction was stirred at room temperature overnight. After completion of the reaction, the reaction solution was poured into water (200 mL) and extracted twice with EtOAc (200 mL*2), and the water phase was retained and extracted twice with dichloromethane (200 mL*2). The organic phases were combined, dried, filtered, concentrated, dissolved in isopropanol at 50°C, recrystallized in an ice bath, and filtered to obtain the compound **S14-2** (13.51 g, 90%).
Step b: 300 mL water was added to dissolve the above-said compound **S14-2** (11.40 g, 1.0 mmol) while stirring at room temperature. **Potassium carbonate** (1.38 g, 10.0 mmol), compound **S14-3** (1.35 g, 5.0 mmol), and **tetra-*n*-butylammonium bromide** (0.03 g, 0.1 mmol) were added, and the reaction was stirred at room temperature for 72 h. After completion of the reaction, the reaction solution was extracted twice with dichloromethane (200 mL*2), and the organic phases were combined and backwashed once with saturated NaCl solution (100 mL). The organic phase was retained, dried with anhydrous MgSO₄, filtered, and concentrated to obtain the crude product of **S14-4.** Furthermore, the crude product was purified by column chromatography, concentrated, and dried with an oil pump to obtain the target compound **S14-4** (7.86 g).
Step c: The above-said compound **S14-4** (5.80 g, 0.5 mmol) was dissolved in anhydrous THF (100 mL), **NaH** (60%, 0.20 g, 5.0 mmol) was added slowly in an ice bath, and the reaction was conducted for 1 h. Compound **S14-5** (0.55 g, 2.5 mmol) was added and reacted for 1 h in an ice bath; after that, the reaction solution was slowly returned to room temperature, and the reaction was conducted overnight. After completion of the reaction, the reaction solution was placed in an ice bath, and 50 mL methanol was added slowly to quench the reaction. After stirring for 30 minutes, water (100 mL) was added and mixed with stirring. The mixture was extracted twice with EtOAc (100 mL*2), and the water phase was retained and extracted twice with dichloromethane (100 mL*2). The organic phases were combined, backwashed with saturated NaCl solution (50 mL), dried with anhydrous sodium sulfate, filtered, and concentrated to obtain the crude product of **E14-1.** The crude product was purified by column chromatography, concentrated, and dried with an oil pump to obtain the target compound **E14-1** (4.44 g).
Step d: Into a flask under nitrogen protection, the above substituted compound **E14-1** (3.54 g, 0.3 mmol) was dissolved in THF (100 mL), tetrabutylammonium fluoride solution (**TBAF,** 100 mL, 1M) was added, and the reaction was conducted overnight to remove the TBS protecting group. The reaction solution was dried with anhydrous sodium sulfate, filtered, and concentrated to obtain the crude product of **E14-2.** The crude product was purified by column chromatography, concentrated, and dried with an oil pump to obtain the **glycol-PEGylated lipid E14-2** (3.01 g, 87.3%). ¹H NMR (500 MHz, CDCl₃) δ: 3.83-3.45 (m, 1002H), 3.48 (d, 4H), 3.30 (m, 1H), 2.51 (t, 4H), 2.37 (t, 2H), 1.56-1.46 (m, 4H), 1.36-1.19 (m, 44H), 0.87 (t, 6H). The molecular weight of **E14-2** was determined to be 11527 Da by MALDI-TOF, PDI=1.02.

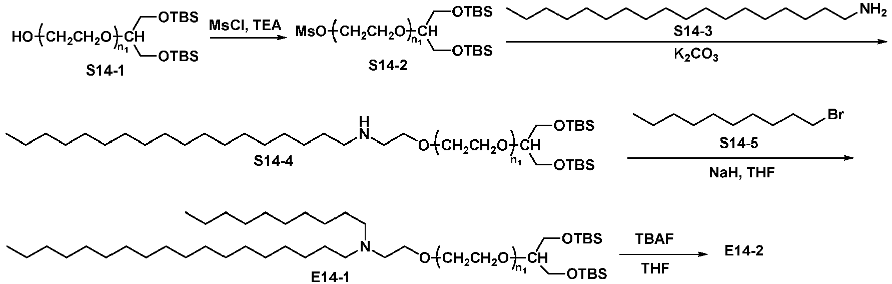

### Example-15: synthesis of lysine-PEGylated lipid E15-2

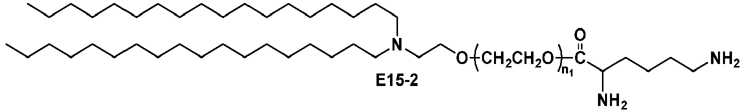

**E15-2** corresponds to the general formula (2); wherein, R₁ and R₂ are both octadecyl groups, B₃ and B₄ are both linking bonds, L₇ and L₈ are both linking bonds, L₃ is -CH₂CH₂O- or -CH₂CH₂-, A is -OCH₂CH₂- or -CH₂CH₂O-, Rₐ and R_{b} are both hydrogen atoms, s is 2, n₁≈45, R is 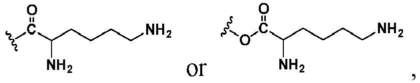 and the total molecular weight is approximately 2670 Da.

The preparation process is represented as follows:
Step a: Compound **S15-1** (10.40 g, 4.0 mmol, Mw≈2600, n₁≈45, PDI=1.02, a product of coupling reaction between lysine containing two Fmoc protecting groups and PEG) and **toluene** (300 mL) performed azeotropic water removal at 140°C. After 100 mL of solvent was steamed out, the temperature of reaction solution was cooled to room temperature. Triethylamine (**TEA**, 0.81 g, 8.0 mmol) and methyl sulfonyl chloride (**MsCl,** 0.82 g, 7.2 mmol) were added, and the reaction was stirred at room temperature overnight. After completion of the reaction, the reaction solution was poured into water (200 mL), extracted twice with EtOAc (200 mL*2), and the water phase was retained and extracted twice with dichloromethane (200 mL*2). The organic phases were combined, dried, filtered, concentrated, dissolved in isopropanol at 50°C, recrystallized in an ice bath, and filtered to obtain the compound **S15-2** (9.57 g, 92%).
Step b: 400 mL water was added to dissolve the above-said compound **S15-2** (7.80 g, 3.0 mmol) while stirring at room temperature. **Potassium carbonate** (4.14 g, 30.0 mmol), compound **S14-3** (4.05 g, 15.0 mmol), and **tetra-*n*-butylammonium bromide** (0.10 g, 0.3 mmol) were added, and the reaction was stirred at room temperature for 72 h. After completion of the reaction, the reaction solution was extracted twice with dichloromethane (400 mL*2). The organic phases were combined, backwashed once with saturated NaCl solution (200 mL). The organic phase was retained, dried with anhydrous MgSO₄, filtered, and concentrated to obtain the crude product of **S15-3.** Furthermore, the crude product was purified by column chromatography, concentrated, and dried with an oil pump to obtain the target compound **S15-3** (6.09 g).
Step c: The above-said compound **S15-3** (4.35 g, 1.5 mmol) was dissolved in anhydrous THF (200 mL), **NaH** (60%, 0.60 g, 15.0 mmol) was added slowly in an ice bath, and the reaction was conducted for 1 h. Compound **S15-4** (1.66 g, 7.5 mmol) was added and reacted for 1 h in an ice bath; after that, the reaction solution was slowly returned to room temperature, and the reaction was conducted overnight. After completion of the reaction, the reaction solution was placed in an ice bath, 100 mL methanol was added slowly to quench the reaction. After stirring for 30 minutes, water (300 mL) was added and mixed with stirring. The mixture was extracted twice with EtOAc (300 mL*2), and the water phase was retained and extracted twice with dichloromethane (300 mL*2). The organic phases were combined, backwashed once with saturated NaCl solution (150 mL), dried with anhydrous sodium sulfate, filtered, and concentrated to obtain the crude product of **E15-1.** The crude product was purified by column chromatography, concentrated, and dried with an oil pump to obtain the target compound **E15-1** (3.49 g). ¹H NMR (500 MHz, CDCl₃) δ: 7.80-7.20 (m, 16H), 4.45-4.36 (m, 5H), 4.22-4.20 (t, 2H), 3.83-3.45 (m, 180H), 2.50 (t, 4H), 2.37 (t, 2H), 2.26 (t, 2H), 2.00-1.76(m, 2H), 1.55-1.46 (m, 6H), 1.36-1.19 (m, 62H), 0.86 (t, 6H).
Step d: Removal of Fmoc protecting group. The above concentrated product **E15-1** (3.10 g, 1.0 mmol) was treated with 20% piperidine/DMF solution, the solvent was removed by rotary evaporation, and then the residue was dissolved in dichloromethane, precipitated by anhydrous ether, filtered, and recrystallized by isopropanol to obtain the lysine product containing two naked amino groups **E15-2** (1.75 g). The main ¹H-NMR spectrum data of **E15-2** were as follows: 4.36 (t, 1H). The characteristic peak of Fmoc disappeared in the ¹H-NMR spectrum. The molecular weight of **E15-2** was determined to be 2673 Da by MALDI-TOF, PDI=1.02.

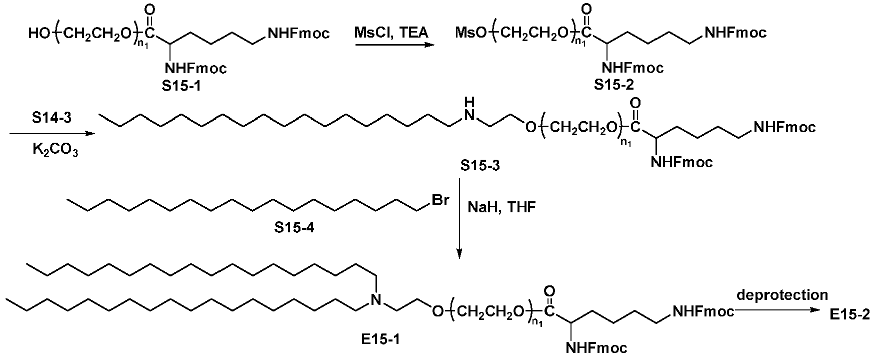

### Example-16: synthesis of ethyl ester-PEGylated lipid E16-1

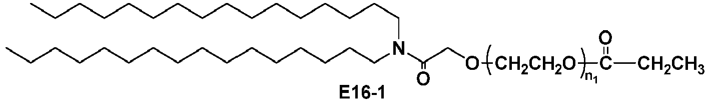

**E16-1** corresponds to the general formula (2); wherein, R₁ and R₂ are both hexadecyl groups, B₃ and B₄ are both linking bonds, L₇ and L₈ are both linking bonds, L₃ is - C(=O)CHzO- or -C(=O)CH₂-, A is -OCH₂CH₂- or -CH₂CH₂O-, Rₐ and R_{b} are both hydrogen atoms, s is 2, n₁≈55, R is -OC(=O)CH₂CH₃ or -C(=O)CH₂CH₃, and the total molecular weight is approximately 3000 Da.

The preparation process is represented as follows:
Compound **S16-2** (7.80 g, 3.0 mmol, 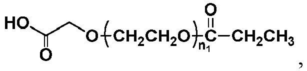 Mw≈2600, n₁≈55, PDI=1.03) was dissolved in dichloromethane (300 mL), and then compound **S16-1** (2.10 g, 4.5 mmol), 1-ethyl-3-(3-dimethylaminopropyl)carbodiimide hydrochloride (**EDCI**, 1.15 g, 6.0 mmol), 1-hydroxybenzotriazole (**HOBt,** 0.61 g, 4.5 mmol), and triethylamine (**TEA,** 0.61 g, 6.0 mmol) were added successively, and the reaction was stirred at room temperature overnight. After completion of the reaction, the reaction solution was backwashed twice with 0.1 mol/L HCl aqueous solution (10% NaCl) (200 mL*2), and backwashed once with saturated NaCl solution (200 mL). The organic phase was dried with anhydrous MgSO₄, filtered, and concentrated to obtain the crude product. Furthermore, the crude product was purified by column chromatography, concentrated, and dried with an oil pump to obtain the **ethyl ester-PEGylated lipid E16-1** (7.52 g). ¹H NMR (500 MHz, CDCl₃) δ: 4.21 (t, 2H), 4.17 (s, 2H), 3.83-3.45 (m, 218H), 3.28 (t, 2H), 3.17 (t, 2H), 2.25 (m, 2H), 1.56-1.46 (m, 4H), 1.36-1.19 (m, 52H), 1.15 (t, 3H), 0.87 (t, 6H). The molecular weight of **E16-1** was determined to be 3000 Da by MALDI-TOF, PDI=1.03.

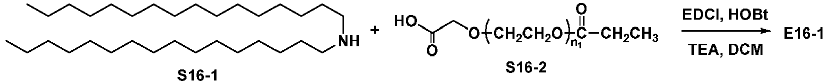

### Example-17: synthesis of butyl acid-PEGylated lipid E17-2

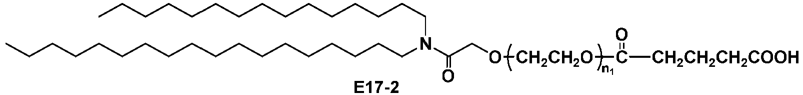

**E17-2** corresponds to the general formula (2); wherein, R₁ and R₂ are pentadecyl group and octadecyl group, B₃ and B₄ are both linking bonds, L₇ and L₈ are both linking bonds, L₃ is -C(=O)CH₂O- or -C(=O)CH₂-, A is -OCH₂CH₂- or -CH₂CH₂O-, Rₐ and R_{b} are both hydrogen atoms, s is 2, n₁≈30, R is -OC(=O)CH₂CH₂CH₂COOH or - C(=O)CH₂CH₂CH₂COOH, and the total molecular weight is approximately 1970 Da.

The preparation process is represented as follows:
Step a: The above-said compound **S17-2** (3.20 g, 2.0 mmol, 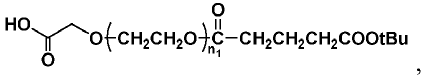 Mw≈1600, n₁≈30, PDI=1.02) was dissolved in dichloromethane (200 mL), and then compound **S17-1** (1.43 g, 3.0 mmol), 1-ethyl-3-(3-dimethylaminopropyl)carbodiimide hydrochloride (**EDCI**, 0.77 g, 4.0 mmol), 1-hydroxybenzotriazole (**HOBt**, 0.41 g, 3.0 mmol), and triethylamine (**TEA**, 0.41 g, 4.0 mmol) were added successively, and the reaction was stirred at room temperature overnight. After completion of the reaction, the reaction solution was backwashed twice with 0.1 mol/L HCl aqueous solution (10% NaCl) (100 mL*2) and backwashed once with saturated NaCl solution (100 mL). The organic phase was dried with anhydrous MgSO₄, filtered, and concentrated to obtain the crude product. Furthermore, the crude product was purified by column chromatography, concentrated, and dried with an oil pump to obtain the target compound **E17-1** (3.36 g, 84.1%).
Step b: The above-said compound **E17-1** (2.00 g, 1.0 mmol) was dissolved in dichloromethane (50 mL), trifluoroacetic acid (50 mL) was added, and the reaction was conducted for 12 h at room temperature. After completion of the reaction, the reaction solution was concentrated, some purified water was added and the reaction solution was extracted with dichloromethane, dried with anhydrous MgSO₄, filtered, concentrated, and recrystallized to obtain the **butyl acid-PEGylated lipid E17-2** (1.47 g). ¹H NMR (500 MHz, CDCl₃) δ: 4.21 (t, 2H), 4.17 (s, 2H), 3.83-3.45 (m, 118H), 3.26 (t, 2H), 3.17 (t, 2H), 2.37 (t, 2H), 2.30 (t, 2H), 1.81-1.70 (m, 2H), 1.56-1.46 (m, 4H), 1.36-1.19 (m, 54H), 0.88 (t, 6H). The molecular weight of **E17-2** was determined to be 1971 Da by MALDI-TOF, PDI=1.02.

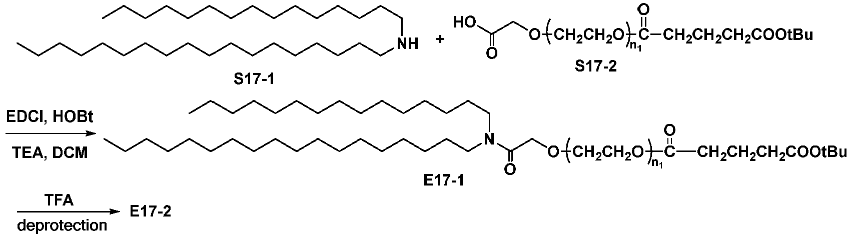

### Example-18: synthesis of glycol-PEGylated lipid E18-2

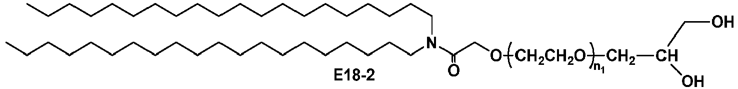

**E18-2** corresponds to the general formula (2); wherein, R₁ and R₂ are eicosyl groups, B₃ and B₄ are both linking bonds, L₇ and L₈ are both linking bonds, L₃ is -C(=O)CH₂O- or - C(=O)CH₂-, A is -OCH₂CH₂- or -CH₂CH₂O-, Rₐ and R_{b} are both hydrogen atoms, s is 2, n₁≈40, R is 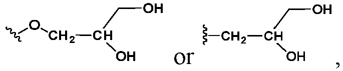 and the total molecular weight is approximately 2470 Da.

The preparation process is represented as follows:
Step a: The above-said compound **S18-2** (6.30 g, 3.0 mmol, 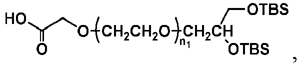 Mw≈2100, n₁≈40, PDI=1.02, a product of reaction between 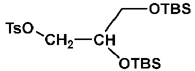 and polyethylene glycol) was dissolved in dichloromethane (300 mL), and then compound S18-1 (2.60 g, 4.5 mmol), 1-ethyl-3-(3-dimethylaminopropyl)carbodiimide hydrochloride (**EDCI**, 1.15 g, 6.0 mmol), 1-hydroxybenzotriazole (**HOBt**, 0.61 g, 4.5 mmol), and triethylamine (**TEA**, 0.61 g, 6.0 mmol) were added successively, and the reaction was stirred at room temperature overnight. After completion of the reaction, the reaction solution was backwashed twice with 0.1 mol/L HCl aqueous solution (10% NaCl) (200 mL*2) and backwashed with saturated NaCl solution (200 mL). The organic phase was dried with anhydrous MgSO₄, filtered, and concentrated to obtain the crude product. Furthermore, the crude product was purified by column chromatography, concentrated, and dried with an oil pump to obtain the target compound **E18-1** (6.81 g, 84.1%).
Step b: Into a flask under nitrogen protection, the above substituted product **E18-1** (5.40 g, 2.0 mmol) was dissolved in THF (100 mL), tetrabutylammonium fluoride solution (**TBAF**, 100 mL, 1M) was added and the reaction was conducted overnight to remove the TBS protecting group. The reaction solution was dried with anhydrous sodium sulfate, filtered, and concentrated to obtain the crude product of **E18-2.** The crude product was purified by column chromatography, concentrated, and dried with an oil pump to obtain the **glycol-PEGylated lipid E18-2** (4.36 g, 87.3%). ¹H NMR (500 MHz, CDCl₃) ¹H NMR (500 MHz, CDCl₃) δ: 4.23-4.21 (m, 1H), 4.17 (s, 2H), 3.83-3.45 (m, 164H), 3.28 (t, 2H), 3.18 (t, 2H), 1.55-1.45 (m, 4H), 1.36-1.19 (m, 68H), 0.87 (t, 6H). The molecular weight of **E18-2** was determined to be 2469 Da by MALDI-TOF, PDI=1.02.

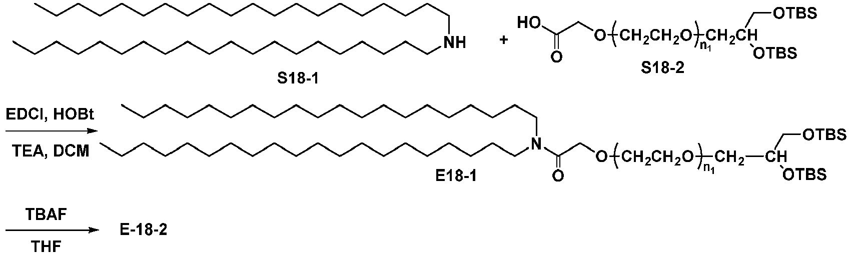

### Example-19: synthesis of lysine-PEGylated lipid E19-2

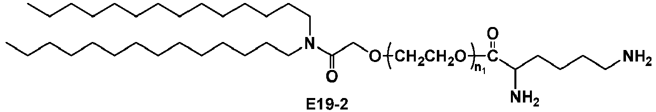

**E19-2** corresponds to the general formula (2); wherein, R₁ and R₂ are both tetradecyl groups, B₃ and B₄ are both linking bonds, L₇ and L₈ are both linking bonds, L₃ is - C(=O)CHzO- or -C(=O)CH₂-, A is -OCH₂CH₂- or -CH₂CH₂O-, Rₐ and R_{b} are both hydrogen atoms, s is 2, n₁≈100, R is 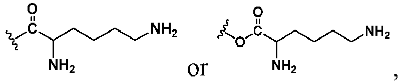 and the total molecular weight is approximately 5000 Da.

The preparation process is represented as follows:
Step a: The above-said compound **S19-1** (7.50 g, 1.5 mmol, n₁≈100, PDI=1.02 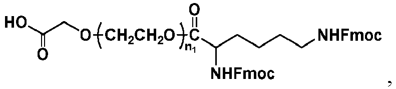 Mw≈5000, a product of coupling reaction between lysine containing two Fmoc protecting groups and polyethylene glycol) was dissolved in dichloromethane (200 mL), and then compound **S1-5** (0.92 g, 2.3 mmol), 1-ethyl-3-(3-dimethylaminopropyl)carbodiimide hydrochloride (**EDCI**, 0.58 g, 3.0 mmol), 1-hydroxybenzotriazole (**HOBt**, 0.30 g, 2.3 mmol), and triethylamine (**TEA**, 0.30 g, 3.0 mmol) were added successively, and the reaction was stirred at room temperature overnight. After completion of the reaction, the reaction solution was backwashed twice with 0.1 mol/L HCl aqueous solution (10% NaCl) (100 mL*2) and backwashed once with saturated NaCl solution (100 mL). The organic phase was dried with anhydrous MgSO₄, filtered, and concentrated to obtain the crude product. Furthermore, the crude product was purified by column chromatography, concentrated, and dried with an oil pump to obtain the target compound **E19-1** (6.84 g, 84.5%). ¹H NMR (500 MHz, CDCl₃) δ: 7.80-7.20 (m, 16H), 4.45-4.36 (m, 5H), 4.22-4.20 (t, 4H), 4.17 (s, 2H), 3.83-3.45 (m, 400H), 3.03 (t, 2H), 2.96 (t, 2H), 2.26 (t, 2H), 2.00-1.76 (m, 2H), 1.56-1.46 (m, 6H), 1.36-1.19 (m, 46H), 0.86 (t, 6H).
Step b: Removal of the Fmoc protecting group. The above concentrated product **E19-1** (5.40 g, 1.0 mmol) was treated with 20% piperidine/DMF solution, the solvent was removed by rotary evaporation, and then the residue was dissolved in dichloromethane, precipitated by anhydrous ether, filtered, and recrystallized by isopropanol to obtain the product **E19-2** (3.40 g) containing a lysine moiety with two free amino groups. The main ¹H-NMR spectrum data of **E19-2** were as follows: 4.36 (t, 1H). The characteristic peak of Fmoc disappeared in the ¹H-NMR spectrum. The molecular weight of **E19-2** was determined to be 4995 Da by MALDI-TOF, PDI=1.02.

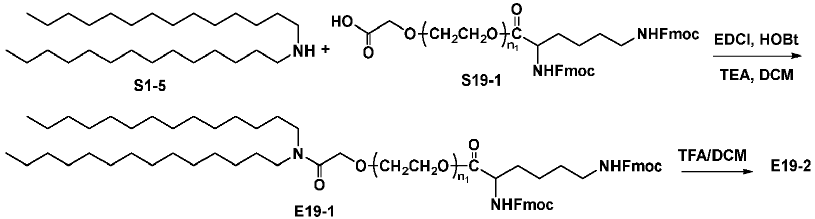

### Example-20: synthesis of dimethylamine-PEGylated lipid E20-1

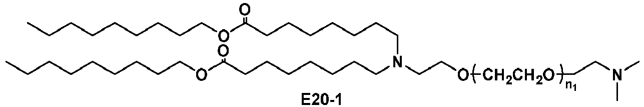

**E20-1** corresponds to the general formula (2); wherein, R₁ and R₂ are both nonyl groups, L₇ and L₈ are both -OC(=O)-, B₃ and B₄ are both heptylene groups, L₃ is -CH₂CH₂O- or - CH₂CH₂-, A is -OCH₂CH₂- or -CH₂CH₂O-, Rₐ and R_{b} are both hydrogen atoms, s is 2, n₁≈50, R is 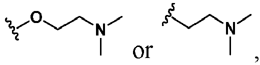 and the total molecular weight is approximately 2870 Da.

The preparation process is represented as follows:
Step a: Under argon atmosphere, into the round-bottom flask containing **S9-2** (1.44 g, 10.0 mmol), **S20-1** (4.46 g, 20.0 mmol), and 4-dimethylaminopyridine (**DMAP**, 0.31 g, 2.5 mmol) dissolved in dichloromethane (300 mL), *N,N'*-dicyclohexylcarbodiimide (**DCC**, 4.53 g, 22.0 mmol) was added, and the reaction was conducted for 16 h at room temperature. After completion of the reaction, the precipitate was removed by filtering. The reaction solution was backwashed twice with saturated NaCl solution (200 mL). The organic phase was dried with anhydrous MgSO₄, filtered, and concentrated to obtain the crude product. Furthermore, the crude product was purified by column chromatography to obtain the colorless oily product **S20-2** (2.90 g, 83%).
Step b: Under nitrogen protection, the above-said compound **S20-2** (1.75 g, 5.0 mmol) was dissolved in acetonitrile (200 mL), **S20-3** (4.60 g, 2.0 mmol, 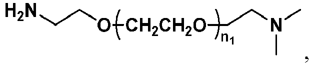 Mw≈2300, n₁≈50, PDI=1.03) and *N,N-*diisopropylethylamine (**DIPEA,** 0.36 g, 4.0 mmol) were added successively while stirring slowly, followed by reacting at room temperature while stirring for about 20 h. After completion of the reaction, the reaction solution was concentrated, dissolved in dichloromethane, and extracted with 0.6 M HCl/10% NaCl solution and saturated sodium bicarbonate solution successively. After standing for layering, the organic phases were combined, dried with anhydrous MgSO₄, filtered, concentrated, and purified by column chromatography to obtain the **dimethylamine-PEGylated lipid E20-1** (4.60 g). ¹H NMR (500 MHz, CDCl₃) δ: 4.07 (t, 4H), 3.83-3.45 (m, 204H), 2.49 (t, 4H), 2.62 (t, 2H), 2.58 (t, 2H), 2.36 (t, 4H), 2.30 (s, 6H), 1.70-1.19 (m, 48H), 0.86 (t, 6H). The molecular weight of **E20-1** was determined to be 2868 Da by MALDI-TOF, PDI=1.03.

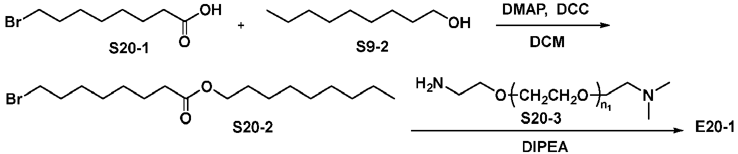

### Example-21: synthesis of thioether-PEGylated lipid E21-1

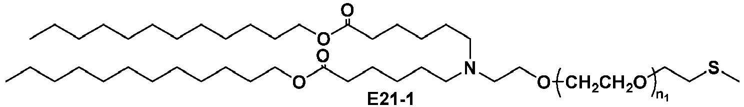

**E21-1** corresponds to the general formula (2); wherein, R₁ and R₂ are both dodecyl groups, L₇ and L₈ are both -OC(=O)-, B₃ and B₄ are both pentylene groups, L₃ is -CH₂CH₂O- or -CH₂CH₂-, A is -OCH₂CH₂- or -CH₂CH₂O-, Rₐ and R_{b} are both hydrogen atoms, s is 2, n₁≈60, R is 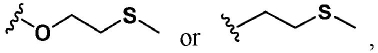 and the total molecular weight is approximately 3340 Da.

The preparation process is represented as follows:
Step a: Under argon atmosphere, into the round-bottom flask containing **S21-1** (1.86 g, 10.0 mmol), **S9-1** (3.90 g, 20.0 mmol), and 4-dimethylaminopyridine (**DMAP,** 0.31 g, 2.5 mmol) dissolved in dichloromethane (300 mL), *N,N'*-dicyclohexylcarbodiimide (**DCC**, 4.53 g, 22.0 mmol) was added and the reaction was conducted for 16 h at room temperature. After completion of the reaction, the precipitate was removed by filtering. The reaction solution was backwashed twice with saturated NaCl solution (200 mL). The organic phase was dried with anhydrous MgSO₄, filtered, and concentrated, and the residue was purified by column chromatography to obtain the colorless oily product **S21-2** (3.13 g, 86.3%).
Step b: Under nitrogen protection, the above-said compound **S21-2** (1.82 g, 5.0 mmol) was dissolved in acetonitrile (200 mL), **S20-3** (5.60 g, 2.0 mmol, 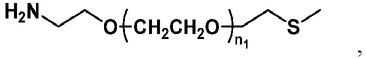 Mw≈2800, n₁≈60, PDI=1.03) and *N,N-*diisopropylethylamine (**DIPEA**, 0.36 g, 4.0 mmol) were added successively with stirring slowly, followed by reacting at room temperature while stirring for about 20 h. After completion of the reaction, the reaction solution was concentrated, dissolved in dichloromethane, and extracted with 0.6 M HCl/10% NaCl solution and saturated sodium bicarbonate solution successively. After standing for layering, the organic phases were combined, dried with anhydrous MgSO₄, filtered, concentrated, and purified by column chromatography to obtain the **thioether-PEGylated lipid E21-1** (5.32 g). ¹H NMR (500 MHz, CDCl₃) δ: 4.07 (t, 4H), 3.83-3.45 (m, 244H), 2.50 (t, 4H), 2.70 (t, 2H), 2.62 (t, 2H), 2.36 (t, 4H), 2.11 (s, 3H), 1.70-1.19 (m, 52H), 0.86 (t, 6H). The molecular weight of **E21-1** was determined to be 3339 Da by MALDI-TOF, PDI=1.03.

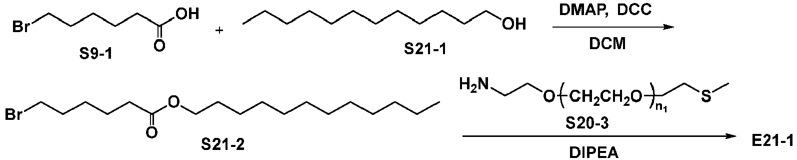

### Example-22: synthesis of maleimide-PEGylated lipid E22-2

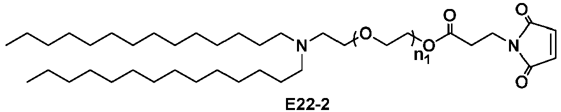

**E22-2** corresponds to the general formula (2); wherein, R₁ and R₂ are both tetradecyl groups, B₃ and B₄ are both linking bonds, L₇ and L₈ are both linking bonds, L₃ is -CH₂CH₂-, A is -OCH₂CH₂-, Rₐ and R_{b} are both hydrogen atoms, s is 2, n₁≈45, R is 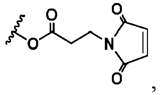 and the total molecular weight is approximately 2580 Da.

The preparation process is represented as follows:
Step a: In a 500 mL round-bottom flask, a condensation reaction between a polyethylene glycol derivative containing a Boc-protected amino group at one end and a free hydroxyl group at the other end (**S22-1**, 10.50 g, 5.0 mmol, Mw≈2100, n₁≈45, PDI=1.02) and a small molecule which was the furan-protected maleimide propionate (**S22-2**, 2.37 g, 10.0 mmol) in the presence of *N,N'*-dicyclohexylcarbodiimid (**DCC**, 2.27 g, 11.0 mmol) and 4-dimethylaminopyridine (**DMAP**, 0.15 g, 1.3 mmol) was carried out to obtain the crude reaction solution of the polyethylene glycol derivative **S22-3** containing a furan-protected maleimide group and a Boc-protected amino group. The reaction solution was concentrated, 300 mL dichloromethane was added, 100 mL trifluoroacetic acid was slowly added dropwise in an ice bath, and the reaction was conducted for 2 h at room temperature to remove the Boc protecting group. The reaction solution was concentrated and purified by column chromatography to obtain the polyethylene glycol derivative **S22-4** (8.46 g, 92%) containing a furan-protected maleimide group and a free amino group.
Step b: Under nitrogen protection, the above-said compound **S3-5** (2.08 g, 7.5 mmol) was dissolved in acetonitrile (200 mL), **S22-4** (7.20 g, 3.0 mmol) and *N,N-*diisopropylethylamine (**DIPEA**, 0.54 g, 6.0 mmol) were added successively while stirring slowly, followed by reacting at room temperature while stirring for about 20 h. After completion of the reaction, the reaction solution was concentrated, dissolved in dichloromethane, and extracted with 0.6 M HCl/10% NaCl solution and saturated sodium bicarbonate solution successively. After standing for layering, the organic phases were combined, dried with anhydrous MgSO₄, filtered, concentrated, and purified by column chromatography to obtain **E22-1** (6.40 g, 82.1%). **E22-1** was purified by column chromatography, and then the furan protecting group was removed to obtain the end-product **maleimide-PEGylated lipid E22-2** (4.78 g, 92%). ¹H NMR (500 MHz, CDCl₃) δ: 6.49 (s, 2H), 4.25-4.10 (t, 4H), 3.85-3.45 (m, 180H), 2.51 (t, 4H), 2.73 (t, 2H), 2.62 (t, 2H), 1.58-1.48 (m, 4H), 1.36-1.19 (m, 44H), 0.86 (t, 6H). The molecular weight of **E22-2** was determined to be 2584 Da by MALDI-TOF, PDI=1.02.

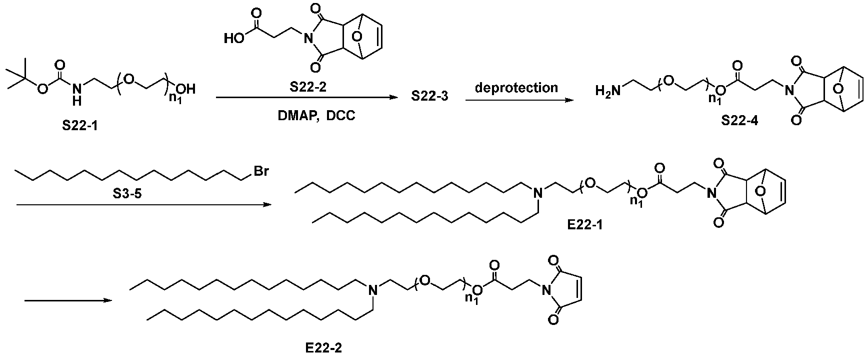

### Example-23: synthesis of methoxy-PEGylated lipid E23-1

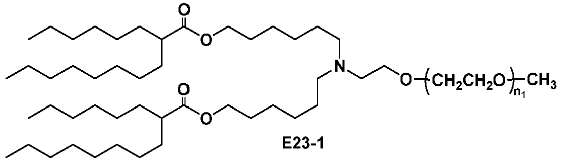

**E23-1** corresponds to the general formula (2); wherein, R₁ and R₂ are 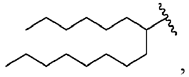 L₇ and L₈ are both -C(=O)O-, B₃ and B₄ are both hexylene groups, L₃ is -CH₂CH₂O-, A is - CH₂CH₂O-, Rₐ and R_{b} are both hydrogen atoms, s is 2, n₁≈45, R is a methyl group, and the total molecular weight is approximately 2730 Da.

The preparation process is represented as follows:
Step a: Under argon atmosphere, into the round-bottom flask containing **S10-2** (1.77 g, 15.0 mmol), **S23-1** (7.68 g, 30.0 mmol), and 4-dimethylaminopyridine (**DMAP,** 0.46 g, 3.8 mmol) dissolved in dichloromethane (300 mL), *N,N'*-dicyclohexylcarbodiimide (**DCC**, 6.80 g, 33.0 mmol) was added and the reaction was conducted for 16 h at room temperature. After completion of the reaction, the precipitate was removed by filtering. The reaction solution was backwashed twice with saturated NaCl solution (150 mL). The organic phase was dried with anhydrous MgSO₄, filtered, and concentrated, and the residue was purified by column chromatography to obtain the compound **S23-2** (4.42 g, 82.6%).
Step b: The above condensation product **S23-2** (3.57 g, 10.0 mmol) was dissolved in DCM (100 mL). After the temperature of the above solution was cooled to 0°C, 2,2,6,6-tetramethylpiperidinooxy (**Tempo,** 0.73 g, 4.7 mmol) and **KBr** solution (1.32 g, 11.1 mmol) dissolved in 10 mL purified water were added, and the **NaClO** solution (1 mL, 12.0 mmol) was added slowly. After the addition, the TLC was used to monitor the reaction until the starting materials were consumed completely. The sodium sulfite solution was added to quench the reaction. The reaction solution was extracted twice with DCM (100 mL*2) when restored to room temperature. The organic phase was dried with anhydrous MgSO₄, filtered, and concentrated to obtain the crude product. Furthermore, the crude product was separated and purified by column chromatography. The target eluent was collected and concentrated to obtain the oxidation product **S23-3** (1.81 g).
Step c: The above oxidation product **S23-3** (11.45 g, 4.1 mmol) was dissolved in a mixed solution of THF (100 mL) and methanol (100 mL). After the temperature of the above solution was cooled to 0°C, methoxy polyethylene glycol ethylamine (**S23-4,** 4.20 g, 2.0 mmol, Mw≈42100, n₁≈45, PDI=1.02) and **glacial acetic acid** (0.12 g, 2.0 mmol) were added, and then **NaBH(OAc)₃** (1.28 g, 6.1 mmol) was added slowly in batches. After the feeding, the reaction was continued for 2 h, and the TLC was used to monitor the reaction until the starting materials were consumed completely. Saturated sodium bicarbonate solution was added to quench the reaction, and then the reaction solution was returned to room temperature and concentrated to remove THF and methanol. The concentrated solution was extracted twice with 200 mL DCM. The organic phase was dried with anhydrous MgSO₄, filtered, and concentrated to obtain the crude product. Furthermore, the crude product was separated and purified by column chromatography. The target eluent was collected and concentrated to obtain the end-product **PEGylated lipid E23-1** (3.08 g). The main ¹H-NMR spectrum data of **E23-1** were as follows: ¹H NMR (400 MHz, CDCl₃) δ: 4.06 (t, 4H), 3.75-3.48 (m, 182H), 3.36 (s, 3H), 2.50 (t, 4H),2.64 (t, 2H), 2.32 (m, 2H), 1.66-1.58 (m, 12H), 1.53-1.22 (m, 52H), 0.88 (t, 12H). The molecular weight of **E23-1** was determined to be 2731 Da by MALDI-TOF, PDI=1.02.

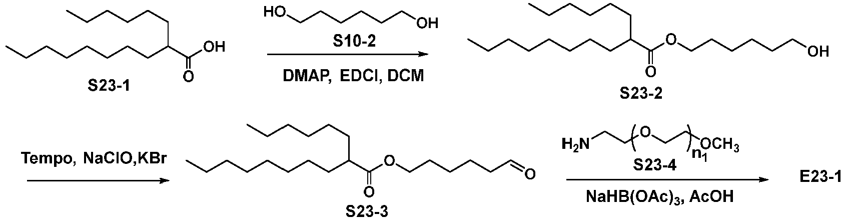

### Example-24: synthesis of methoxy-PEGylated lipid E24-1

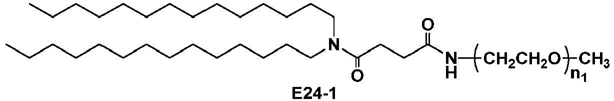

**E24-1** corresponds to the general formula (2); wherein, R₁ and R₂ are both tetradecyl groups, B₃ and B₄ are both linking bonds, L₇ and L₈ are both linking bonds, L₃ is - C(=O)CH₂CH₂C(=O)NH-, A is -CH₂CH₂O-, Rₐ and R_{b} are both hydrogen atoms, s is 2, n₁≈45, R is a methyl group, and the total molecular weight is approximately 2500 Da.

The preparation process is represented as follows:
Compound **S24-1** (5.67 g, 2.7 mmol, Mw≈2100, n₁≈45, PDI=1.02) and N-hydroxy succinimide (0.60 g, 6.0 mmol) were dissolved in anhydrous chloroform (30 mL). A 20 mL anhydrous chloroform solution of *N,N'*-dicyclohexylcarbodiimide (**DCC**, 1.85 g, 9.0 mmol) was added, and the reaction solution was stirred for 1 h. The above solution was added into the mixed solution of 20 mL anhydrous chloroform solution of S1-5 (2.46 g, 6.0 mmol) and triethylamine (1.7 mL, 12.0 mmol), the reaction was continued for 1 h while stirring. The TLC test showed that the most starting materials were reacted and the product was formed. After completion of the reaction, the reaction solution was filtered through the celite containing dichloromethane, acidified with hydrochloric acid, and washed with distilled water (100 mL*2) and saline (100 mL). The water layer was extracted with dichloromethane (200 mL), and then the organic phases were combined, dried with anhydrous MgSO₄, and purified by column chromatography to obtain the **PEGylated lipid E24-1** (5.18 g). ¹H NMR (500 MHz, CDCl₃) δ: 3.83-3.41 (m, 180H), 3.38 (s, 3H), 3.26 (t, 2H), 3.16 (t, 2H), 2.43-2.36 (m, 4H), 1.56-1.46 (m, 4H), 1.26-1.19 (m, 44H), 0.87 (t, 6H). The molecular weight of **E24-1** was determined to be 2503 Da by MALDI-TOF, PDI=1.02.

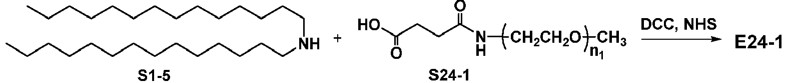

### Example-25.1: synthesis of methoxy-PEGylated lipid E25-1

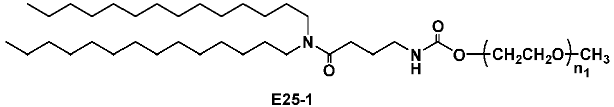

**E25-1** corresponds to the general formula (2); wherein, R₁ and R₂ are both tetradecyl groups, B₃ and B₄ are both linking bonds, L₇ and L₈ are both linking bonds, L₃ is - C(=O)CH₂CH₂CH₂NHC(=O)O- or -C(=O)CH₂CH₂CH₂NHC(=O)-, A is -CH₂CH₂O- or - OCH₂CH₂-, Rₐ and R_{b} are both hydrogen atoms, s is 2, n₁≈45, R is a methyl group or a methoxy group, and the total molecular weight is approximately 2500 Da.

The preparation process is represented as follows:
Step a: The compound **S25-2** (10.50 g, 5.0 mmol, Mw≈2100, n₁≈45, PDI=1.03) and **S25-1** (1.19 g, 7.5 mmol) were dissolved in DCM and cooled in an ice bath. Pyridine (15 mL) was added into the mixture, followed by stirring overnight. After completion of the reaction, the solvent was removed and the residue was dried under high vacuum. The residue was extracted with dichloromethane, and then washed with bicarbonate solution and water. The crude product was purified by column chromatography to obtain compound **S25-3** (10.01 g, 91%).
Step b: The above-said compound **S25-3** (6.60 g, 3.0 mmol) was dissolved in dichloromethane (50 mL), and then trifluoroacetic acid (50 mL) was added and the reaction was conducted for 12 h at room temperature. After completion of the reaction, the solvent was evaporated off under reduced pressure to obtain compound **S25-4** (4.56 g), and the compound was put into the next reaction directly without purification.
Step c: The above-said compound **S25-4** (3.78 g, 1.8 mmol) and *N*-hydroxy succinamide (0.40 g, 4.0 mmol) were dissolved in anhydrous chloroform (30 mL). 20 mL anhydrous chloroform solution of *N,N'*-dicyclohexylcarbodiimide (**DCC**, 0.62 g, 3.0 mmol) was added and followed by stirring for 1 h. The above solution was added to the mixed solution of 20 mL anhydrous chloroform solution of **S1-5** (0.82 g, 2.0 mmol) and triethylamine (1.1 mL, 8.0 mmol), and the reaction was continued for 1 h while stirring. The TLC test showed that the most starting materials were reacted and the product was formed. After completion of the reaction, the reaction solution was filtered through the celite containing dichloromethane, acidified with hydrochloric acid, and washed with distilled water (100 mL*2) and saline (100 mL). The water layer was extracted with dichloromethane (200 mL), and the organic phases were combined and dried with anhydrous MgSO₄. The crude product was purified by column chromatography to obtain the **PEGylated lipid E25-1** (3.42 g). ¹H NMR (500 MHz, CDCl₃) δ: 4.04-3.98 (t, 2H), 3.83-3.45 (m, 178H), 3.38 (s, 3H), 3.26 (t, 2H), 3.23 (t, 2H), 3.17 (t, 2H), 2.34 (t, 2H), 1.84 (m, 2H), 1.56-1.46 (m, 4H), 1.26-1.19 (m, 44H), 0.87 (t, 6H). The molecular weight of **E25-1** was determined to be 2533 Da by MALDI-TOF, PDI=1.03.

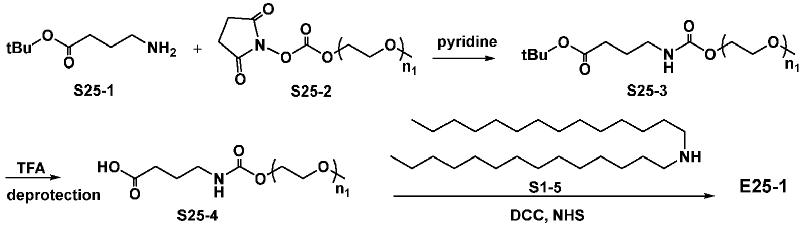

### Example-25.2: synthesis of methoxy-PEGylated lipid E25-2

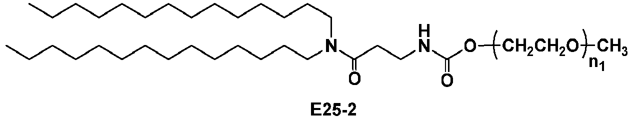

**E25-2** corresponds to the general formula (2); wherein, R₁ and R₂ are both tetradecyl groups, B₃ and B₄ are both linking bonds, L₇ and L₈ are both linking bonds, L₃ is - C(=O)CH₂CH₂NHC(=O)O- or -C(=O)CH₂CH₂CH₂NHC(=O)-, A is -CH₂CH₂O- or - OCH₂CH₂-, Rₐ and R_{b} are both hydrogen atoms, s is 2, n₁≈45, R is a methyl group or a methoxy group, and the total molecular weight is approximately 2520 Da.

This embodiment can refer to the foregoing embodiment **25.1,** wherein the starting material **S25-1** was replaced with **S25-4** 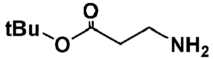 and the same steps were carried out to obtain **E25-2.** ¹H NMR (500 MHz, CDCl₃) δ: 4.04-3.98 (t, 2H), 3.83-3.45 (m, 178H), 3.40 (t, 2H), 3.37 (s, 3H), 3.26 (t, 2H), 3.17 (t, 2H), 2.61 (t, 2H), 1.56-1.46 (m, 4H), 1.26-1.19 (m, 44H), 0.88 (t, 6H). The molecular weight of **E25-2** was determined to be 2519 Da by MALDI-TOF, PDI=1.03.

### Example-26: synthesis of methoxy-PEGylated lipid E26-1

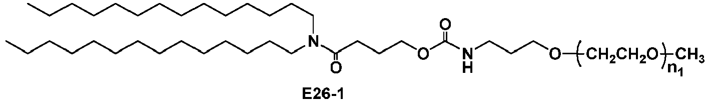

**E26-1** corresponds to the general formula (2); wherein, R₁ and R₂ are both tetradecyl groups, B₃ and B₄ are both linking bonds, L₇ and L₈ are both linking bonds, L₃ is - C(=O)CH₂CH₂CH₂OC(=O)NHCH₂CH₂CH₂O- or - C(=O)CH₂CH₂CH₂OC(=O)NHCH₂CH₂CH₂-, A is -CH₂CH₂O- or -OCH₂CH₂-, Rₐ and R_{b} are both hydrogen atoms, s is 2, n₁≈45, R is a methyl group or a methoxy group, and the total molecular weight is approximately 2590 Da.

The preparation process is represented as follows:
Step a: The compound **S26-1** (1.51 g, 5.0 mmol) and **S26-2** (15.75 g, 7.5 mmol, Mw≈2100, n₁≈45, PDI=1.03) were dissolved in DCM and cooled in an ice bath. Pyridine (15 mL) was added into the mixture, followed by stirring overnight. After completion of the reaction, the solvent was removed and the residue was dried under high vacuum. The residue was extracted with dichloromethane and washed with bicarbonate solution and water. The crude product was purified by column chromatography to obtain compound **S26-3** (10.07 g, 91.5%).
Step b: The above-said compound **S26-3** (6.60 g, 3.0 mmol) was dissolved in dichloromethane (50 mL), and then trifluoroacetic acid (50 mL) was added and the reaction was conducted for 12 h at room temperature. After completion of the reaction, the solvent was evaporated off under reduced pressure to obtain the compound **S26-4** (4.72 g), and the compound was put into the next reaction directly without purification.
Step c: The above-said compound **S26-4** (3.96 g, 1.8 mmol) and *N*-hydroxy succinamide (0.40 g, 4.0 mmol) were dissolved in anhydrous chloroform (30 mL). 20 mL anhydrous chloroform solution of *N,N'*-dicyclohexylcarbodiimide (**DCC**, 0.62 g, 3.0 mmol) was added, followed by reacting for 1 h while stirring. The above solution was added into the mixed solution of 20 mL anhydrous chloroform solution of **S1-5** (0.82 g, 2.0 mmol) and triethylamine (1.1 mL, 8.0 mmol), the reaction was continued for 1 h while stirring. The TLC test showed that the most starting materials were reacted and the product was formed. After completion of the reaction, the reaction solution was filtered through the celite containing dichloromethane, acidified with hydrochloric acid, and washed with distilled water (100 mL*2) and saline (100 mL). The water layer was extracted with dichloromethane (200 mL), and the organic phases were combined and dried with anhydrous MgSO₄. The crude product was purified by column chromatography to obtain the **PEGylated lipid E26-1** (3.58 mg). ¹H NMR (500 MHz, CDCl₃) δ: 4.10 (t, 2H), 3.83-3.45 (m, 180H), 3.37 (s, 3H), 3.28-3.26 (t, 4H), 3.20 (t, 2H), 3.17 (t, 2H), 2.35 (t, 2H), 1.90-1.78 (m, 4H), 1.56-1.46 (m, 4H), 1.26-1.19 (m, 44H), 0.88 (t, 6H). The molecular weight of **E26-1** was determined to be 2591 Da by MALDI-TOF, PDI=1.03.

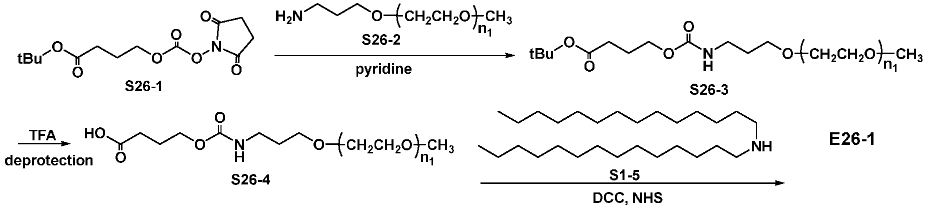

### Example-27: synthesis of methoxy-PEGylated lipid E27-1

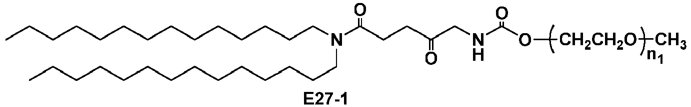

**E27-1** corresponds to the general formula (2); wherein, R₁ and R₂ are both tetradecyl groups, B₃ and B₄ are both linking bonds, L₇ and L₈ are both linking bonds, L₃ is-C(=O)CH₂CH₂C(=O)CH₂NHC(=O)O- or -C(=O)CH₂CH₂C(=O)CH₂NHC(=O)-, A is - CH₂CH₂O- or -OCH₂CH₂-, Rₐ and R_{b} are both hydrogen atoms, s is 2, n₁≈45, R is a methyl group or a methoxy group, and the total molecular weight is approximately 2560 Da.

The preparation process is represented as follows:
Step a: The compound **S25-2** (10.50 g, 5.0 mmol) and **S27-1** (1.40 g, 7.5 mmol) were dissolved in DCM and cooled in an ice bath. Pyridine (15 mL) was added into the mixture, followed by stirring overnight. After completion of the reaction, the solvent was removed and the residue was dried under high vacuum. The residue was extracted with dichloromethane solution, and washed with bicarbonate solution and water. The crude product was purified by column chromatography to obtain compound S27-2 (10.16 g, 91.3%).
Step b: The above-said compound S27-2 (6.67 g, 3.0 mmol) was dissolved in dichloromethane (50 mL), and then trifluoroacetic acid (50 mL) was added and the reaction was conducted for 12 h at room temperature. After completion of the reaction, the solvent was evaporated off under reduced pressure to obtain **S27-3** (4.63 g), and the compound was put into the next reaction directly without purification.
Step c: The above-said compound **S27-3** (3.96 g, 1.8 mmol) and N-hydroxy succinamide (0.40 g, 4.0 mmol) were dissolved in anhydrous chloroform (30 mL). 20 mL anhydrous chloroform solution of *N,N'*-dicyclohexylcarbodiimide (**DCC**, 0.62 g, 3.0 mmol) was added, followed by reacting for 1 h while stirring. The above solution was added into the mixed solution of 20 mL anhydrous chloroform solution of **S1-5** (0.82 g, 2.0 mmol) and triethylamine (1.1 mL, 8.0 mmol), and the reaction was continued for 1 h while stirring. The TLC test showed that the most starting materials were reacted and the product was formed. After completion of the reaction, the reaction solution was filtered through the celite containing dichloromethane, acidified with hydrochloric acid, and washed with distilled water (100 mL*2) and saline (100 mL). The aqueous layer was extracted with dichloromethane (200 mL), and the organic phases were combined and dried with anhydrous MgSO₄. The crude product was purified by column chromatography to obtain the **PEGylated lipid E27-1** (3.45 g). ¹H NMR (500 MHz, CDCl₃) δ: 4.07 (s, 2H), 4.04-3.98 (t, 2H), 3.83-3.45 (m, 178H), 3.37 (s, 3H), 3.28-3.23 (t, 4H), 3.17 (t, 2H), 2.87 (t, 2H), 1.56-1.46 (m, 4H), 1.26-1.19 (m, 44H), 0.88 (t, 6H). The molecular weight of **E27-1** was determined to be 2561 Da by MALDI-TOF, PDI=1.03.

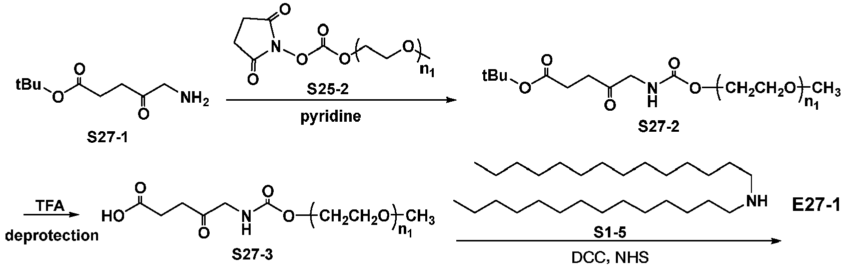

### Example-28: synthesis of methoxy-PEGylated lipid E28-1

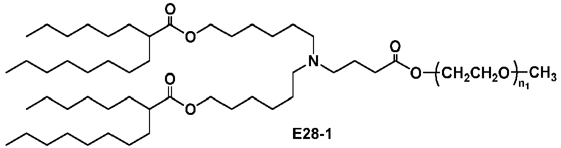

**E28-1** corresponds to the general formula (2); wherein, R₁ and R₂ are 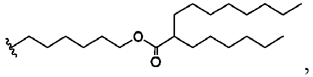 B₃ and B₄ are hexylene groups, L₇ and L₈ are ester groups, L₃ is -CH₂CH₂CH₂C(=O)O- or -CH₂CH₂CH₂C(=O)-, A is -CH₂CH₂O- or -OCH₂CH₂-, Rₐ and R_{b} are both hydrogen atoms, s is 2, n₁≈45, R is a methyl group, and the total molecular weight is approximately 2800 Da.

The preparation process is represented as follows:
Step a: The above oxidation product **S23-4** (7.47 g, 21.1 mmol) was dissolved in a mixed solution of THF (50 mL) and methanol (50 mL). After the temperature of the above solution was cooled to 0°C, compound **S25-1** (1.59 g, 10.0 mmol, Mw≈2000, n₁≈45, PDI=1.02) and **glacial acetic acid** (0.60 g, 10.0 mmol) were added, and then **NaBH(OAc)₃** (6.57 g, 31.0 mmol) was added slowly in batches. After the feeding, the reaction was continued for 2 h, and the TLC was used to monitor the reaction until the starting materials were consumed completely. Saturated sodium bicarbonate solution was added to quench the reaction, and the reaction solution was returned to room temperature and concentrated to remove THF and methanol. The concentrated solution was extracted twice with 200 mL DCM. The organic phase was dried with anhydrous MgSO₄, filtered, and concentrated to obtain the crude product. Furthermore, the crude product was separated and purified by column chromatography. The target eluent was collected and concentrated to obtain the intermediate **S28-1** (4.77 g).
Step b: The above-said compound **S28-1** (4.18 g, 5.0 mmol) was dissolved in dichloromethane (50 mL), and then trifluoroacetic acid (50 mL) was added and the reaction was conducted for 12 h at room temperature. After completion of the reaction, the solvent was evaporated off under reduced pressure to obtain **S28-2** (2.77 g), and the compound was put into the next reaction directly without purification.
Step c: Under argon atmosphere, into the round-bottom flask containing **S28-2** (1.56 g, 2.0 mmol), **S23-1** (2.00 g, 1.0 mmol), and 4-dimethylaminopyridine **(DMAP,** 30.50 mg, 0.3 mmol) dissolved in dichloromethane (100 mL), *N,N'*-dicyclohexylcarbodiimide (**DCC**, 0.45 g, 2.2 mmol) was added and the reaction was conducted for 16 h at room temperature. After completion of the reaction, the precipitate was removed by filtering. The reaction solution was backwashed twice with saturated NaCl solution (100 mL). The organic phase was dried with anhydrous MgSO₄, filtered, and concentrated, and the obtained residue was purified by column chromatography to obtain the **PEGylated lipid E28-1** (6.00 g, 86.3%). ¹H NMR (500 MHz, CDCl₃) δ: 4.07 (t, 6H), 3.83-3.45 (m, 178H), 3.37 (s, 3H), 2.49 (m, 4H), 2.32-2.25 (m, 6H), 1.78-1.22 (m, 66H), 0.88 (t, 12H). The molecular weight of **E28-1** was determined to be 2773 Da by MALDI-TOF, PDI=1.02.

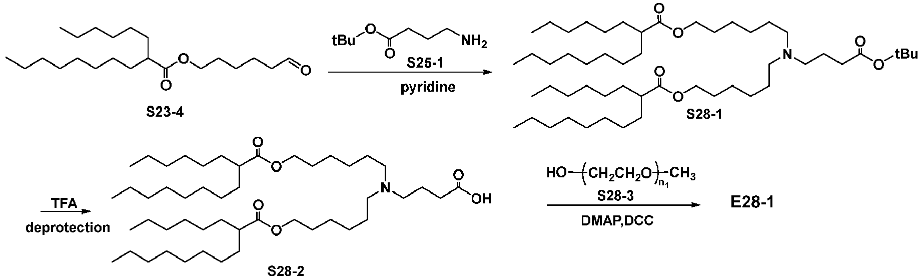

### Example-29: synthesis of PEGylated lipid folic acid derivative E29-1

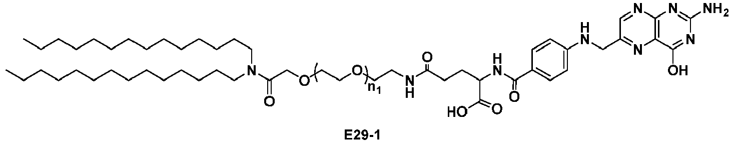

**E29-1** corresponds to the general formula (2); wherein, R₁ and R₂ are both tetradecyl groups, L₇ and L₈ are both linking bonds, B₃ and B₄ are both linking bonds, L₃ is - C(=O)CHzO- A is -CH₂CH₂O-, Rₐ and R_{b} are both hydrogen atoms, s is 2, n₁≈45, R is 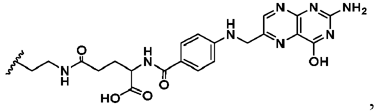 and the total molecular weight is approximately 2900 Da.

The preparation process is represented as follows:
Into a 100 mL dry and clean round-bottom flask, folic acid (**S29-1,** 1.00 g, 2.4 mmol) dissolved in DMSO, ethyl amine-PEGylated lipid (**E8-2**, 3.50 g, 1.4 mmol, Mw≈2500, n₁≈45, PDI=1.02), pyridine (20 mL), and *N,N'*-dicyclohexylcarbodiimide (**DCC**, 1.30 g, 6.3 mmol) were added, followed by reacting for 4 h at room temperature while stirring. The reaction was monitored by spotting the TLC plate, and continued until the production of the end-product and the disappearance of the starting material ethyl amine-PEGylated lipid from the reaction mixture. Then, the pyridine was removed at reduced pressure by a rotary evaporator, 300 mL water was added to the solution, and the solution was centrifuged to remove the trace insoluble. The supernatant after the centrifugation was dialyzed in saline and water, monitored by TLC until the dialysate contained only the end-product (i.e., only one spot was monitored by spotting the TLC plate). Then, the aqueous solution of dialysate was freeze-dried to obtain the end-product PEGylated lipid folic acid derivative (**E29-1,** 3.67 g, 90%). ¹H NMR(500 MHz, CDCl₃) δ: 8.68 (s, 1H), 7.68 (d, 2H), 6.67 (d, 2H), 4.51 (d, 2H), 4.36 (m, 1H), 4.17 (s, 2H), 3.83-3.45 (m, 182H), 3.27 (t, 4H), 3.17 (t, 2H), 2.34 (t, 2H), 2.08-1.94 (m, 2H), 1.56-1.46 (m, 4H), 1.36-1.19 (m, 44H), 0.87 (t, 6H). The molecular weight of **E29-1** was determined to be 2913 Da by MALDI-TOF, PDI=1.02.

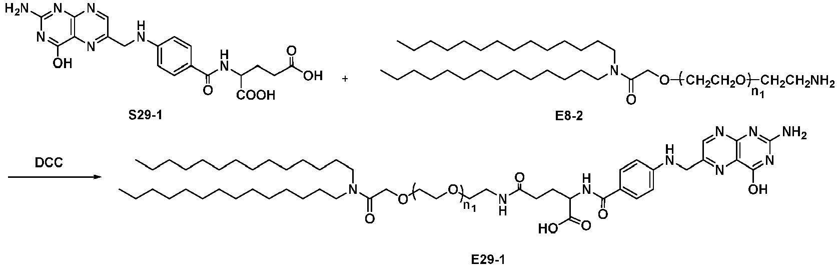

### Example-30: synthesis of PEGylated lipid biotin derivative E30-1

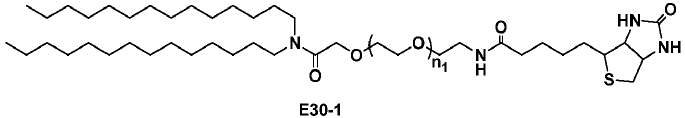

**E30-1** corresponds to the general formula (2); wherein, R₁ and R₂ are both tetradecyl groups, L₇ and L₈ are both linking bonds, B₃ and B₄ are both linking bonds, L₃ is - C(=O)CH₂O-, A is -CH₂CH₂O-, Rₐ and R_{b} are both hydrogen atoms, s is 2, n₁≈45, R is 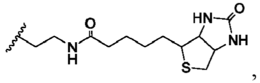 and the total molecular weight is approximately 2700 Da.

The preparation process is represented as follows:
Into a dry and clean round-bottom flask, the biotin succinimidyl ester (**S30-1**, 1.88 g, 5.5 mmol) dissolved in DMF and the ethyl amine-PEGylated lipid (**E8-2**, 12.50 g, 5.0 mmol, Mw≈2500, n₁≈45, PDI=1.02) dissolved in DCM were added, mixed and stirred well, and then **triethylamine** (**TEA,** 2.1 mL, 15.0 mmol) was added and the reaction was continued at room temperature until the reaction was completed according to TLC. After completion of the reaction, the reaction solution was filtered, concentrated, and purified by column chromatography. The target eluent was collected, combined, concentrated, and freeze-dried to obtain the **PEGylated lipid biotin derivative** (**E30-1,** 9.45 g). ¹H NMR (500 MHz, CDCl₃) δ: 4.36-4.25 (m, 2H), 4.18 (s, 2H), 3.84-3.44 (m, 182H), 3.35 (t, 2H), 3.27-3.20 (m,3H), 3.14 (t, 2H), 2.83 (d, 2H), 2.22-2.18 (m, 2H), 1.70-1.40 (m, 8H), 1.36-1.19 (m, 46H), 0.87 (t, 6H). The molecular weight of **E30-1** was determined to be 2716 Da by MALDI-TOF, PDI=1.02.

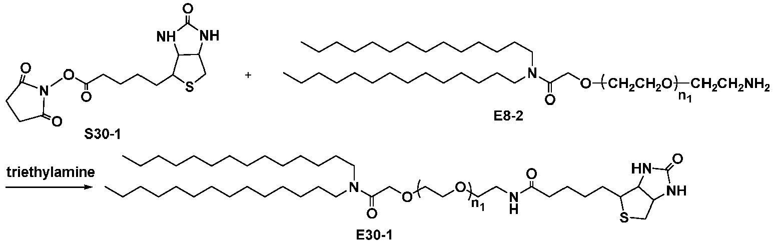

### Example-31: synthesis of cationic lipid P-9

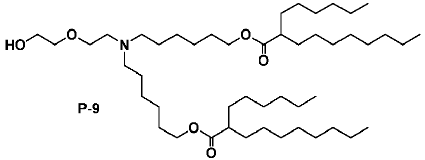

**P-9** corresponds to the general formula (1); wherein, L₁ and L₂ are both -C(=O)O-, L₃ is a linking bond, B₁ and B₂ are both 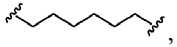 R₁ and R₂ are both 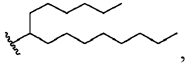 R₃ is a hydrogen atom, A is -(CRₐR_{b})ₛO-, Rₐ and R_{b} are both hydrogen atoms, s is 2, and n is 2.

The preparation process is represented as follows:
Step a: Into a flask under nitrogen protection, compound **2-hexyldecanoic acid** (**S31-1,** 100.00 g, 390.6 mmol) was dissolved in anhydrous dichloromethane (DCM, 1 L). After the temperature of the above mixture solution was cooled to 0~10°C, **1,6-hexanediol** (**S31-2,** 92.30 g, 781.3 mmol) and DMAP (57.20 g, 468.7 mmol) were added carefully, then EDCI (81.60 g, 430.0 mmol) was added in batches, and the reaction solution was returned to room temperature and the reaction was continued. After reacting for 16 h, **S31-1** was completely consumed according to the TLC. The reaction solution was washed twice with 500 mL mixed solution of 0.4N HCl/10% NaCl, and then washed once with saturated saline. The organic phase was dried with anhydrous MgSO₄, filtered, and concentrated to obtain the crude product. Furthermore, the crude product was separated and purified by silica gel column. The target eluent was collected and concentrated to obtain the product **S31-3** (79.00 g).
Step b: The above condensation product (**S31-3,** 50.0 g, 140.5 mmol) was dissolved in 500 mL DCM. After the temperature of the above solution was cooled to 0°C, 2,2,6,6-tetramethylpiperidinooxy (Tempo, 11.00 mg) and the **KBr** solution (20.10 g, 168.6 mmol) dissolved in 50 mL purified water were added, and the NaClO solution (182.6 mmol) was added dropwise slowly. After the addition, the reaction was monitored by TLC until the starting materials were consumed completely. Then, sodium sulfite solution was added to quench the reaction. The reaction was returned to room temperature and extracted twice with 500 mL DCM. The organic phase was dried with anhydrous MgSO₄, filtered, and concentrated to obtain the crude product. Furthermore, the crude product was separated and purified by silica gel column. The target eluent was collected and concentrated to obtain the oxidation product **S31-4** (23.00 g).
Step c: The above oxidation product (**S31-4,** 20.00 g, 56.5 mmol) was dissolved in a mixed solution of 200 mL THF and 20 mL methanol. After the temperature of the above solution was cooled to 0°C, 2-(2-aminoethoxy)ethanol (**S31-5,** 2.80 g, 26.9 mmol) and glacial acetic acid (1.61 g, 26.9 mmol) were added, and then NaBH(OAc)₃ ( 17.50 g, 82.5 mmol) was added slowly in batches. After the feeding, the reaction was continued for 2 h and monitored by TLC until the starting materials were consumed completely. Saturated sodium bicarbonate solution was added to quench the reaction. The reaction solution was returned to room temperature and concentrated to remove THF and methanol. The concentrated solution was extracted twice with 200 mL DCM, and the organic phase was dried with anhydrous MgSO₄, filtered, and concentrated to obtain the crude product. Furthermore, the crude product was separated and purified by silica gel column. The target eluent was collected and concentrated to obtain the product **P-9** (12.00 g). The main ¹H-NMR spectrum data of **P-9** were as follows: ¹H NMR (400 MHz, CDCl₃) δ: 4.07 (t, *J* = 5.9 Hz, 4H), 3.70 (s, 2H), 3.63 (m, 4H), 2.65 (m, 2H), 2.49 (m, 4H), 2.32 (m, 2H), 1.70-1.22 (m, 64H), 0.88 (d, *J* = 6.3 Hz, 12H).

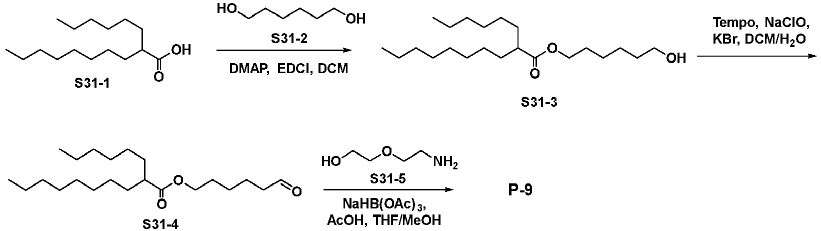

### Example-32: Preparation of cationic liposomes

In this example, multiple groups of cationic liposomes were prepared for comparison. In the compositions of every group of cationic liposomes, the neutral lipids contained were all DSPC, the sterol lipids contained were all cholesterol, and the differences lied in the cationic lipid and the PEGylated lipid components. Wherein, for the control group (L-0), the cationic lipid was DLin-MC3-DMA (MC3 for short) and the PEGylated lipid was PEG₂ₖ-DMG (DMG for short); for the experimental groups (L-1 to L-25 and L-29), the cationic lipid was MC3, and the PEGylated lipids were the PEGylated lipids prepared in the present application; for the experimental group (L-26 to L-28), the cationic lipid was the cationic lipid P-9 prepared in the present application, and the PEGylated lipids were the PEGylated lipids prepared in the present application, specifically as shown in Table 1.

**The preparation method of cationic liposomes is as follows:**
Step a: The cationic lipid CL (15.0 µmol), distearoylphosphatidylcholine (DSPC, 3.0 µmol), cholesterol (12.0 µmol), and the PEGylated lipid **PL** (0.45 µmol) of each group from L-0 to L-29 as listed in Table 1 were weighed at a molar ratio of 50:10:40:1.5, and added into a 100 mL round-bottom flask, 30 mL chloroform was added to dissolve the solid and shaken well;
Step b: The solvent chloroform was removed by a rotary evaporator with 140 rpm at 55°C to form a thin oil film which was dried with a vacuum pump for 12 h to ensure the chloroform was removed completely;
Step c: Into a flask, 30 mL phosphate buffer (PBS, pH=7.4) containing 10% lactose was added. The reaction solution was ultrasonicated by ultrasonic cleaner at the frequency of 90% for 30 min, and a translucent emulsion was formed;
Step d: The emulsion was added into a high-pressure homogenizer, and overpressurized for 5 times under the pressure of 100 MPa; then, the emulsion was added into the liposome extruder and overpressurized for 10 times under the pressure of 150 MPa to prepare the cationic liposome powders of groups L-1 to L-21 and L-23.

Following the above step a-d, the cationic lipid CL (16.0 µmol), distearoylphosphatidylcholine (DSPC, 3.0 µmol), cholesterol (14.0 µmol), and the PEGylated lipid **PL** (0.5 µmol) of group the L-29 listed in Table 1 were weighed at a molar ratio of 48:9:42:1.5 to prepare the cationic liposome powder of L-29 with the rest conditions unchanged.

### Example-33: Preparation of the formulations of cationic liposome-nucleic acid pharmaceutical compositions

Step a: 0.03 mL physiological saline was used as the working solution for the formulations of nucleic acid pharmaceutical compositions;
Step b: 0.1 mg cationic liposome (from L-0 to L-29) prepared in **Example-32** was weighed, dissolved in physiological saline, and equilibrated for 30 min. According to the N/P ratio of 10/1 for the complexation, 1.00 µg siRNA was weighed, dissolved in 10 µL physiological saline, and complexed with the cationic liposome dissolved in physiological saline for 30 min to prepare 1 control group (**L-0**/siRNA) and the formulations of said cationic liposome-nucleic acid pharmaceutical compositions of the present invention ("LNP/siRNA formulation" for short, L-1/siRNA~ L-29/siRNA).

**Table 1 Summary of the liposome formulations and the particle size and encapsulation efficiency of the prepared LNP/siRNA**

| **Liposome** | **L-0** \| | **L-1** | **L-2** | **L-3** | **L-4** | **L-5** |
|---|---|---|---|---|---|---|
| **Cationic lipid CL** | **MC3** | | | | | |
| **PEGylated lipid** PL | DMG | **E1-1** | **E2-1** | **E3-1** | **E6-1** | **E7-1** |
| **Particle size /nm** | 91.29 | 102.35 | 96.83 | 103.81 | 102.17 | 123.18 |
| **Encapsulation efficiency** /% | 93.2 | 92.8 | 93.7 | 90.5 | 88.1 | 79.4 |

| **Liposome** | **L-6** | **L-7** | **L-8** | **L-9** | **L-10** | **L-11** |
|---|---|---|---|---|---|---|
| **Cationic lipid CL** | **MC3** | | | | | |
| **PEGylated lipid PL** | **E9-1** | **E10-2** | **E11-1** | **E12-1** | **E13-2** | **E14-2** |
| **Particle size /nm** | 100.19 | 102.79 | 92.09 | 116.11 | 95.46 | 101.11 |
| **Encapsulation efficiency /%** | 93.6 | 89.5 | 88.5 | 83.1 | 86.7 | 85.4 |

| **Liposome** | L-12 | **L-13** | **L-14** | **L-15** | **L-16** | **L-17** |
|---|---|---|---|---|---|---|
| **Cationic lipid CL** | **MC3** | | | | | |
| **PEGylated lipid PL** | **E15-2** | **E16-1** | **E18-2** | **E20-1** | **E21-1** | **E22-2** |
| **Particle size /nm** | 118.09 | 103.54. | 125.16 | 97.21 | 99.92 | 96.55 |
| **Encapsulation efficiency /%** | 85.7 | 92.3 | 88.4 | 90.2 | 83.6 | 85.1 |

| **Liposome** | **L-18** | **L-19** | **L-20** | **L-21** | **L-22** | **L-23** |
|---|---|---|---|---|---|---|
| **Cationic lipid CL** | **MC3** | | | | | |
| **PEGylated lipid PL** | **E23-1** | **E24-1** | **E25-1** | **E25-2** | **E26-1** | **E27-1** |
| **Particle size /nm** | 106.36 | 97.55 | 98.21 | 98.33 | 105.18 | 102.79 |
| **Encapsulation efficiency /%** | 93.2 | 93.8 | 93.2 | 93.9 | 93.4 | 91.6 |

| **Liposome** | **L-24** | **L-25** | **L-26** | **L-27** | **L-28** | **L-29** |
|---|---|---|---|---|---|---|
| **Cationic lipid CL** | MC3 | MC3 | **P-9** | **P-9** | **P-9** | MC3 |
| **PEGylated lipid PL** | **E28-1** | **E29-1** | **E1-1** | **E2-1** | **E25-1** | **E2-1** |
| **Particle size /nm** | 108.10 | 93.95 | 95.3 | 97.9 | 106.4 | 98.3 |
| **Encapsulation efficiency /%** | 93.4 | 90.7 | 90.2 | 93.7 | 94.1 | 91.6 |

### Example-34: Biological activity assays for the formulations of cationic liposome-nucleic acid pharmaceutical compositions

### (1) Determination of nanoparticle size and gene complexation ability

The gel electrophoresis experiment was used to evaluate the gene complexation ability of LNP/siRNA in each group. 0.8 g agarose was weighed and dissolved in 40 mL TAE solution. The solution was heated in a microwave oven until the granules of the agarose were dissolved completely, and then cooled. 5 µL nucleic acid dye GelGreen was added into the cooled agarose gel which was then added into the gel slot and dried natural air. A mixed solution of LNP/siRNA and 2 µL loading buffer was added into the agarose gel well, and the electrophoresis voltage was set to 90 V for the electrophoresis experiment. The electrophoresis was carried out at room temperature for 10 min. The results showed that free siRNA was essentially absent in the experimental groups (**L-1**/siRNA~**L-23**/siRNA) and the control group (**L-0**/siRNA), indicating that the formulations of cationic liposome-nucleic acid pharmaceutical compositions prepared by the cationic lipids of the present invention had a stronger gene complexation ability, and the ionizable cationic lipids could be well combined with the negatively charged nucleic acids because of bearing partial positive charges.

Determination of encapsulation efficiency: The LNP/siRNA was ultracentrifuged (4 °C, 60000 rpm, 1 h) with an ultracentrifuge, the concentration of unencapsulated siRNA in the supernatant was determined by a nucleic acid quantifier, and the encapsulation efficiency of the liposome for siRNA was calculated. The results were summarized in Table 1, showing that the cationic liposomes of the present invention (L-1 ~ L-29) had higher encapsulation efficiency for nucleic acid drugs. The DMG is a commercially available PEGylated lipid. The groups L-1 to L-25 used the same cationic lipid MC3; wherein, L-1 (containing the PEGylated lipid E2-1), L-6 (containing the PEGylated lipid E9-1), L-19 (containing the PEGylated lipid E24-1), L-21 (containing the PEGylated lipid E25-2), L-22 (containing the PEGylated lipid E26-1), and L-24 (containing the PEGylated lipid E28-1) were all higher in the encapsulation efficiency for siRNA than that of DMG; L-26~L-28 containing the PEGylated lipids and cationic lipids prepared in the present invention, and the resulting cationic liposomes also showed higher encapsulation efficiency for nucleic acid; L-29 adopted a different ratio of lipids, and the prepared cationic liposome showed higher encapsulation efficiency for nucleic acid as well.

Determination of particle size: In this example, the particle size of LNP/siRNA was determined by dynamic light scattering (DLS). The measured sizes of LNP/siRNA had a relatively high uniformity with PDI values all smaller than 0.3. When n₁ was in the range of approximately 40-100, the particle sizes of the prepared LNP/siRNA with the PEGylated lipids of the present invention were in the range of 90-130 nm.

### (2) Study on the stability in serum

In this experiment, the stability of the PEGylated lipid-modified formulation of cationic liposome-nucleic acid pharmaceutical composition in serum was determined by measuring the variation of the size of liposome particles. In this example, 0.5 mL PEGylated lipid-modified formulation of cationic liposome-nucleic acid pharmaceutical composition of the present invention (**as described in Example-33**), whose N/P ratio was 10/1, was added to the 0.5 mL culture medium containing 10% fetal bovine serum (FBS), followed by stirring at 37°C. Samples were taken at a regular interval to measure the variation of the particle size of the formulation of cationic liposome-nucleic acid pharmaceutical composition, based on which the stability of the formulation of cationic liposome-nucleic acid pharmaceutical composition in serum was analyzed. In the other group, 0.5 mL PBS was added to replace the culture medium to determine the stability of the formulation of cationic liposome-nucleic acid pharmaceutical composition in serum, using the same method as aforementioned. Results of the experiments showed that, in 48 h, compared with **L-0,** the variations in the particle size of the formulation of cationic liposome-nucleic acid pharmaceutical composition of **L-1~L-29** in PBS solution and serum were relatively small, indicating that the PEGylated lipid-modified cationic liposomes of the present invention were relatively stable, which had good stability in serum and met the requirements of long circulating cationic liposomes.

### (3) Study on the cytotoxicity (biocompatibility)

The cytotoxicity of the formulation of cationic liposome-nucleic acid pharmaceutical composition of the present invention was examined by the MTT assay. The formulations of cationic liposome-nucleic acid pharmaceutical compositions were dissolved in culture medium to the required concentration, and an appropriate amount of solubilizer could be added when necessary. With HeLa cells as a cell model, the cell suspension was inoculated onto a 96 well plate at 100 µL/well and a density of 1×10⁴ cells/well. After the inoculation, the cells were incubated in a cell incubator at 37°C, 4% CO₂ for 24 h. Then, the culture medium was aspirated and 100 µL culture medium containing 3.3 µg/mL LNP/siRNA (formulation of cationic liposome-nucleic acid pharmaceutical composition prepared in Example-33) was added to each well, 100 µL fresh culture medium was added to the blank control group, and each concentration of each group corresponded to 6 replicate wells. After the formulations of cationic liposome-nucleic acid pharmaceutical compositions were incubated with the HeLa cells for 24 h, 20 µL PBS buffer solution containing 5 mg/mL MTT was added to each well. After the MTT was incubated with the cancer cells for 4 h, the mix solution of the culture medium and the buffer solution containing MTT was aspirated, and DMSO was added at 150 µL/well to dissolve the purple formazan crystals in living cells. After shaking evenly, the absorbance was measured by a microplate reader. The result was calculated according to the measured absorbance value, and showed that compared with the blank control group, the cell viability in groups containing the formulations of cationic liposome-nucleic acid pharmaceutical compositions prepared in the present invention were all over 95%, indicating that the PEGylated lipid-modified formulations of cationic liposome-nucleic acid pharmaceutical composition of the present invention had good biocompatibility.

### (4) Study on the nucleic acid transport efficiency

In order to examine the nucleic acid transport efficiency of each group of formulation of cationic liposome-nucleic acid pharmaceutical composition prepared with the PEGylated lipid of the present invention, the Luc-HeLa cells which could stably express the luciferase were used as the cell model to evaluate the nucleic acid transport rate of each group of the formulations of cationic liposome-nucleic acid pharmaceutical compositions (**as described in Example-33**) whose N/P ratio was 10/1. In a 96-well plate, when the cells were cultured to a cell density of 80%, each group of formulation of cationic liposome-nucleic acid pharmaceutical composition was added to the culture medium, and the cells were incubated in a cell incubator at 37 °C, 4% CO₂ for 24 h. The culture medium was then aspirated, culture medium containing 3.3 µg/mL LNP/siRNA was added and the cells were cultured for 24 h. The cells were treated with lyse buffer, and the fluorescence intensity was measured using a luciferase assay kit and a chemiluminescence detector. On the other hand, the siRNA group, i.e., the group with the cultured cells added with naked siRNA at the same dosage as aforementioned, was set. Considering the siRNA which was transported into cells could inhibit the expression of the luciferase gene Flue mRNA, the untreated cells were used as the negative control group, and the expression (i.e., fluorescence intensity) of the target gene in this blank cell group was designated as 100%. The fluorescence intensity of the siRNA group was approximately 78%, the fluorescence intensity of the L-0/siRNA group was approximately 45%, the fluorescence intensity of the L-1/siRNA group was approximately 39%, the fluorescence intensity of the L-2/siRNA group was approximately 32%, the fluorescence intensity of the L-7/siRNA group was approximately 47%, the fluorescence intensity of the L-19/siRNA group was approximately 32%, the fluorescence intensity of the L-22/siRNA group was approximately 31%, the fluorescence intensity of the L-23/siRNA group was approximately 35%, and the fluorescence intensity of the L-28/siRNA group was approximately 33%. Compared with the blank cell group and the siRNA group, the positive control group and the experimental groups showed better inhibitory effects on the luciferase gene, indicating that cationic liposomes could improve the transport efficiency for nucleic acid drugs; compared with the control group L-0/siRNA, the experimental groups had better inhibitory effects on the luciferase gene except the L-7/siRNA group, indicating that the cationic liposomes prepared with the PEGylated lipids of the present invention could improve the nucleic acid transport efficiency of liposomes.

### (5) Study on the cellular uptake efficiency

In order to investigate whether the PEGylated lipid-modified cationic liposomes containing the targeting group folic acid can increase the uptake of cationic liposomes by tumor cells or not, in the present invention, the nasopharymneal carcinoma cells (KB) with high expression of cell-surface folate receptor and the hepatoma cells (HepG2) with low expression of cell-surface folate receptor were respectively used as cell models to evaluate the cellular uptake of liposomes. The cells were inoculated onto 6-well plates. The supernatant was discarded when the cells grown to a density of approximately 80%, and then 0.2 mL cationic liposome formulation of **L-23/**siRNA without the targeting group folic acid or **L-25**/siRNA with the targeting group folic acid, together with 1.8 mL culture medium, was respectively added to each well, and each sampling point corresponded to 3 replicate wells. After 1, 3 and 6 hours of incubation, respectively, the supernatant was retrieved, and the cells were digested with pancreozyme, washed with PBS, and transferred into a flow cytometer tube. 1×10⁴ cells using cell counting were acquired on a flow cytometer to calculate the percentage of positive cells (i.e., cells whose fluorescence intensity exceeded 10 units). The uptake efficiency was represented by the percentage of positive cells. Results of the experiments showed that, for the hepatoma cells with low expression of folate receptors, the difference of the cellular uptake efficiency for the PEGylated lipid-modified cationic liposome was relatively small between those without and with the targeting group folic acid. Compared with the PEGylated lipid-modified cationic liposome without the targeting group folic acid, the PEGylated lipid-modified cationic liposome with the targeting group folic acid permitted a higher cellular uptake efficiency of HepG2 cells which was increased by approximately 5%. However, for the nasopharymneal carcinoma cells with high expression of folate receptors, compared with the PEGylated lipid-modified cationic liposome without the targeting group folic acid, the PEGylated lipid-modified cationic liposome with the targeting group folic acid permitted a higher cellular uptake efficiency of KB cells which was increased by approximately 13%. It meant that the targeting effect of folic acid and the enhanced transport efficiency of cationic liposomes for nucleic acid drugs can act synergistically to further improve the effects of gene therapy on tumor.

Those described above are only embodiments of the present invention, and are not for setting a limitation on the patent scope of the present invention. Any modification of equivalent structures or equivalent routes according to the present invention, which may be applied in other related art in a direct or an indirect way, should be included into the scope of the present invention.

For those skilled in the art, without departing from the spirit and scope of the present invention, and without unnecessary experimentation, the present invention can be implemented in a wide range under equivalent parameters, concentrations and conditions. While the present invention has given particular examples, it should be understood that the present invention can be further modified. In conclusion, in accordance with the principles of the present invention, the present application is intended to cover any alterations, uses, or improvements of the present invention, including changes departing from the scope disclosed in this application but made using conventional techniques known in the art.

## Claims

1. A PEGylated lipid, wherein, the structure is represented by the general formula (2): 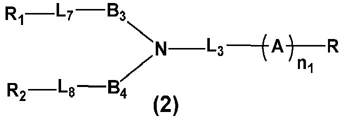
wherein, L₇ and L₈ are each independently a linking bond, -OC(=O)- or -C(=O)O-;
wherein, L₃ is a linking bond, -L₄-, -L₄-Z-, -Z-L₄-, -Z-L₄-Z-, -L₄-Z-L₅-, -Z-L₄-Z-L₅-, -L₄-Z-L₅-Z-, -Z-L₄-Z-L₅-Z- or -L₄-Z-L₄-Z-L₅-Z-; said L₄ and L₅ are carbon chain linking groups, each independently represented as -(CRₐR_{b})ₜ-(CRₐR_{b})ₒ-(CRₐR_{b})ₚ-, wherein, t, o and p are each independently an integer from 0 to 12, and t, o and p are not 0 simultaneously; Rₐ and R_{b} are, at each occurrence, each independently a hydrogen atom or a C₁₋₁₂ alkyl group; said Z is, at each occurrence, each independently selected from the group consisting of -C(=O)-, -OC(=O)-, -C(=O)O-, -OC(=O)O-, -O-, -S-, -C(=O)S-, -SC(=O)-, -NR_{c}C(=O)-, -C(=O)NR_{c}-, -NR_{c}C(=O)NR_{c}-, -OC(=O)NR_{c}-, -NR_{c}C(=O)O-, -SC(=O)NR_{c}-, -NRcC(=O)S-, and 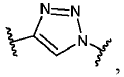 wherein, R_{c} is, at each occurrence, independently a hydrogen atom or a C₁₋₁₂ alkyl group;
wherein, B₃ and B₄ are each independently a linking bond or a C₁₋₃₀ alkylene group;
wherein, R₁ and R₂ are each independently a C₁₋₃₀ aliphatic hydrocarbon group;
wherein, R is a hydrogen atom, -R_{d}, -OR_{d}, -NR_{d}R_{d}, -SR_{d}, -C(=O)R_{d}, -C(=O)OR_{d}, -OC(=O)R_{d}, -OC(=O)OR_{d}, or 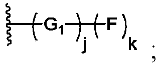 wherein, R_{d} is, at each occurrence, independently a C₁₋₁₂ alkyl group, G₁ is a (k+1)-valent terminal branching group, j is 0 or 1, and F contains the functional group R₀₁; when j is 0, G₁ is absent; when j is 1, G₁ protrudes F with the number of k, and k is an integer from 2 to 8; when L₃ is -C(=O)CH₂-, R is not a hydrogen atom, an alkyl group, an alkoxy group, or a hydroxyl group;
wherein, A is -(CRₐR_{b})ₛO- or -O(CRₐR_{b})ₛ-, wherein, s is 2, 3, or 4;
wherein, n₁ is an integer from 20 to 250;
said alkyl group, alkylene group, alkoxy group, and aliphatic hydrocarbon group are each independently substituted or unsubstituted.

2. The PEGylated lipid according to claim **1,** wherein, the number-average molecular weight of said polyethylene glycol chain is selected from the group consisting of 900, 1000, 1500, 2000, 2500, 3000, 3350, 3500, 4000, 5000, 5500, 6000, 6500, 7000, 7500, 8000, 8500, 9000, 9500, 10000, and 11,000.

3. The PEGylated lipid according to claim **1,** wherein, said polyethylene glycol chain is polydisperse, and the number-average degree of polymerization n₁ is preferably an integer from 20 to 100, more preferably an integer from 20 to 60, more preferably an integer from 40 to 60, and more preferably selected from the group consisting of 44, 45, 46, 48, 50, 52, 54, 56, 58, and 60.

4. The PEGylated lipid according to claim **1,** wherein, said polyethylene glycol chain is monodisperse, and the number of EO units is preferably from 20 to 70, more preferably from 20 to 60, more preferably selected from the group consisting of 20, 22, 24, 26, 27, 28, 29, 30, 32, 34, 36, 38, 40, 42, 44, 45, 46, 48, 50, 52, 54, 56, 58, and 60.

5. The PEGylated lipid according to claim **1,** wherein, said R₁ and R₂ are preferably each independently a C₅₋₃₀ aliphatic hydrocarbon group, more preferably a C₁₀₋₃₀ aliphatic hydrocarbon group, and most preferably a C₁₀₋₂₀ aliphatic hydrocarbon group.

6. The PEGylated lipid according to claim **1,** wherein, said L₇ and L₈ are selected from any one of the following Groups:
Group (1): L₇ and L₈ are both linking bonds;
Group (2): one of L₇ and L₈ is a linking bond, and the other is -OC(=O)- or -C(=O)O-;
Group (3): L₇ and L₈ are each independently -OC(=O)- or -C(=O)O-;preferably L₇ and L₈ are both -OC(=O)- or -C(=O)O-.

7. The PEGylated lipid according to claim **1,** wherein, said B₃ and B₄ are each independently a linking bond or a C₁₋₂₀ alkylene group; preferably selected from any one of the following Groups:
Group (1): B₃ and B₄ are both linking bonds;
Group (2): one of B₃ and B₄ is a linking bond, and the other is a C₁₋₂₀ alkylene group;
Group (3): B₃ and B₄ are each independently selected from the group consisting of a methylene group, an ethylene group, a propylene group, a butylene group, a pentylene group, a hexylene group, a heptylene group, an octylene group, a nonylene group, a decylene group, an undecylene group, a dodecylene group, a tridecylene group, a tetradecylene group, a pentadecylene group, a hexadecylene group, a heptadecylene group, an octadecylene group, a nonadecylene group, and an eicosylene group.

8. The PEGylated lipid according to claim **1,** wherein, said L₃ contains degradable groups, said degradable group refers to the group which can degrade under any condition selected from the group consisting of light, heat, low temperature, enzymatic condition, oxidation-reduction condition, acidic condition, basic condition, physiological condition, and simulated physiological environment in vitro; L₄ and L₅ in L₃ are preferably each independently -(CH₂)ₜ-; L₃ is selected from the group consisting of -(CH₂)ₜ-, -(CH₂)ₜZ-, -Z(CH₂)ₜ-, -(CH₂)ₜZ(CH₂)ₜ-, -Z(CH₂)ₜZ-, -(CH₂)ₜZ(CH₂)ₜZ-, -Z(CH₂)ₜZ(CH₂)ₜ-, and -Z(CH₂)ₜZ(CH₂)ₜZ-, wherein, t is an integer from 0 to 12, and Z is, at each occurrence, independently selected from the group consisting of -C(=O)-, -OC(=O)-, -C(=O)O-, -OC(=O)O-, -O-, -S-, -C(=O)S-, -SC(=O)-, -NHC(=O)-, -C(=O)NH-, -NHC(=O)NH-, -OC(=O)NH-, -NHC(=O)O-, -SC(=O)NH-, and -NHC(=O)S-; L₃ is preferably selected from the group consisting of -(CH₂)ₜ-, -(CH₂)ₜO-, -(CH₂)ₜC(=O)-, -(CH₂)ₜC(=O)O-, -(CH₂)ₜOC(=O)-, -(CH₂)ₜC(=O)NH-, -(CH₂)ₜNHC(=O)-, -(CH₂)ₜOC(=O)O-, -(CH₂)ₜNHC(=O)O-, -(CH₂)ₜOC(=O)NH-, -(CH₂)ₜNHC(=O)NH-, -O(CH₂)ₜ-, -C(=O)(CH₂)ₜ-, -C(=O)O(CH₂)ₜ-, -OC(=O)(CH₂)ₜ-, -C(=O)NH(CH₂)ₜ-, -NHC(=O)(CH₂)ₜ-, -OC(=O)O(CH₂)ₜ-, -NHC(=O)O(CH₂)ₜ-, -OC(=O)NH(CH₂)ₜ-, -NHC(=O)NH(CH₂)ₜ-, -(CH₂)ₜO(CH₂)ₜ-, -(CH₂)ₜC(=O)(CH₂)ₜ-, -(CH₂)ₜC(=O)O(CH₂)ₜ-, -(CH₂)ₜOC(=O)(CH₂)ₜ-, -(CH₂)ₜC(=O)NH(CH₂)ₜ-, -(CH₂)ₜNHC(=O)(CH₂)ₜ-, -(CH₂)ₜOC(=O)O(CH₂)ₜ-, -(CH₂)ₜNHC(=O)O(CH₂)ₜ-, -(CH₂)ₜOC(=O)NH(CH₂)ₜ-, -(CH₂)ₜNHC(=O)NH(CH₂)ₜ-, -O(CH₂)ₜO-, -C(=O)(CH₂)ₜC(=O)-, -C(=O)O(CH₂)ₜC(=O)O-, -OC(=O)(CH₂)ₜOC(=O)-, -C(=O)O(CH₂)ₜOC(=O)-, -OC(=O)(CH₂)ₜC(=O)O-, -OC(=O)O(CH₂)ₜOC(=O)O-, -C(=O)NH(CH₂)ₜC(=O)NH-, -NHC(=O)(CH₂)ₜNHC(=O)-, -NHC(=O)(CH₂)ₜC(=O)NH-, -C(=O)NH(CH₂)ₜNHC(=O)-, -NHC(=O)O(CH₂)ₜNHC(=O)O-, -OC(=O)NH(CH₂)ₜOC(=O)NH-, -NHC(=O)O(CH₂)ₜOC(=O)NH-, -OC(=O)NH(CH₂)ₜNHC(=O)O-, -NHC(=O)NH(CH₂)ₜNHC(=O)NH-, -C(=O)(CH₂)ₜO-, -C(=O)(CH₂)ₜC(=O)O-, -C(=O)(CH₂)ₜOC(=O)-, -C(=O)(CH₂)ₜOC(=O)O-, -C(=O)(CH₂)ₜNHC(=O)O-, -C(=O)(CH₂)ₜOC(=O)NH-, -C(=O)(CH₂)ₜNHC(=O)NH-, -C(=O)(CH₂)ₜC(=O)O(CH₂)ₜ-, -C(=O)(CH₂)ₜOC(=O)(CH₂)ₜ-, -C(=O)(CH₂)ₜOC(=O)O(CH₂)ₜ-, -C(=O)(CH₂)ₜNHC(=O)O(CH₂)ₜ-, -C(=O)(CH₂)ₜOC(=O)NH(CH₂)ₜ-, -C(=O)(CH₂)ₜNHC(=O)NH(CH₂)ₜ-, and -C(=O)(CH₂)ₜC(=O)(CH₂)ₜNHC(=O)O-, wherein, t is an integer from 2 to 12, and most preferably selected from the group consisting of -C(=O)O-, -(CH₂)ₜO-, -(CH₂)ₜC(=O)O-, -(CH₂)ₜOC(=O)-, -(CH₂)ₜO-, -C(=O)(CH₂)ₜO-, -C(=O)(CH₂)ₜOC(=O)-, -C(=O)(CH₂)ₜC(=O)O-, -C(=O)(CH₂)ₜC(=O)NH-, -C(=O)(CH₂)ₜOC(=O)NH-, -C(=O)(CH₂)ₜNHC(=O)O-, -C(=O)(CH₂)ₜOC(=O)NH(CH₂)ₜ-, and -C(=O)(CH₂)ₜC(=O)(CH₂)ₜNHC(=O)O-.

9. The PEGylated lipid according to claim 1, wherein, said R is a hydrogen atom, -R_{d}, -OR_{d}, -C(=O)R_{d}, -C(=O)OR_{d}, -OC(=O)R_{d}, -OC(=O)OR_{d}, or , the structure of said F is represented by -(Z₂)_{q}-(Z₁)_{q1}-R₀₁, wherein, q and q1 are each independently 0 or 1; Z₁ and Z₂ are each independently a divalent linking group, preferably each independently selected from the group consisting of -L₄-, -L₄-Z-, -Z-L₄-, -Z-L₄-Z-, -L₄-Z-L₅-, -Z-L₄-Z-L₅-, and -L₄-Z-L₅-Z-, wherein, t is an integer from 1 to 12; R₀₁ is a functional group capable of interreacting with bio-related substances.

10. The PEGylated lipid according to claim 1, wherein, the stability of said divalent linking groups L₃, L₄, L₅, L₇, L₈, Z, Z₁, and Z₂ are not particularly limited, wherein, any divalent linking group or a divalent linking group consisting of any aforementioned divalent linking group and adjacent heterosubstituted groups thereof is independently a stable linking group STAG or a degradable linking group DEGG; the condition of being stable for a said stable linking group STAG is selected from light, heat, low temperature, enzymatic condition, oxidation-reduction, acidic condition, basic condition, physiological condition, and simulated physiological environment in vitro, preferably selected from light, heat, enzymatic condition, oxidation-reduction, acidic condition, and basic condition; the condition of said degradable linking groups DEGG to be degraded is selected from light, heat, low temperature, enzymatic condition, oxidation-reduction, acidic condition, basic condition, physiological condition, and simulated physiological environment in vitro, preferably selected from light, heat, enzymatic condition, oxidation-reduction, acidic condition, and basic condition;
said stable linking group STAG is further preferably selected from the group consisting of an alkylene group, a divalent heteroalkyl group, a carbon-carbon double bond, a carbon-carbon triple bond, a divalent dienyl group, a divalent cycloalkyl group, a divalent cycloalkenyl group, a divalent cycloalkenyl hydrocarbon group, a divalent cycloalkynyl hydrocarbon group, an aromatic ring, an aliphatic heterocyclic group, a heterophenylene group, an aromatic-fused heterocyclic group, a hetero-fused heterocyclic group, a substituted alkylene group, a substituted heteroalkyl group, a substituted divalent heteroalkyl group, a substituted double bond, a substituted triple bond, a substituted dienyl group, a substituted divalent cycloalkyl group, a substituted divalent cycloalkenyl group, a substituted divalent cycloalkenyl hydrocarbon group, a substituted divalent cycloalkynyl hydrocarbon group, a substituted aromatic ring, a substituted aliphatic heterocyclic group, a substituted heterophenylene group, a substituted aromatic-fused heterocyclic group, a substituted hetero-fused heterocyclic group, an ether bond, a thioether bond, a urea bond, a thiourea bond, a carbamate bond, a thiocarbamate bond, -P(=O)-, a divalent silyl group without active hydrogen atoms, a divalent boron-containing linking group, a secondary amino group, a tertiary amino group, a carbonyl group, a thiocarbonyl group, an amide group, a thioamide group, a sulfonamide group, an enamino group, a triazole group, a 4,5-dihydroisoxazole group, a skeleton of an amino acid or its derivative, and a stable divalent linking group composed of any two or more groups thereof;
said degradable linking group DEGG is further preferably degradable divalent linking group containing any divalent linking group or combination of any two or more thereof selected from the group consisting of a disulfide bond, a vinylether bond, an ester group, a thioate group, a thioester group, a dithioester group, a carbonate group, a thiocarbonate group, a dithiocarbonate group, a trithiocarbonate group, a carbamate group, a thiocarbamate group, a dithiocarbamate group, an acetal group, a cycloacetal group, a thioacetal group, an azaacetal group, an azacycloacetal group, an azathiaacetal group, a dithioacetal group, a hemiacetal group, a thiohemiacetal group, an azahemiacetal group, a ketal group, a thioketal group, an azaketal group, an azacycloketal group, an azathiaketal group, an imine bond, a hydrazone bond, an acylhydrazone bond, an oxime bond, a thiooxime ether bond, a semicarbazone bond, a thiosemicarbazone bond, a hydrazino group, a hydrazide group, a thiocarbohydrazide group, an azocarbohydrazide group, an azothiocarbohydrazide group, a hydrazino formate group, a hydrazino thioformate group, a carbohydrazide group, a thiocarbohydrazide group, an azo group, an isourea group, an isothiourea group, an allophanate group, a thioallophanate group, a guanidino group, an amidino group, an aminoguanidinyl group, a carbamimidamido group, an imino acid group, a thioimidate group, a sulfonate group, a sulfinate group, a sulfonyl hydrazide group, a sulfonyl ureido group, a maleimide group, an orthoester group, a phosphate group, a phosphirate group, a phosphinate group, a phosphonate group, a phosphosilicate group, a silicate group, an amide group, a thioamide group, a sulfonamide bond, a polyamide group, a phosphamide group, a phosphoramidite group, a pyrophosphamide group, a cyclophosphamide group, an ifosfamide group, a thiophosphamide group, an aconityl group, a peptide fragment, a skeleton of a nucleotide or its derivative, and a skeleton of a deoxynucleotide or its derivative.

11. The PEGylated lipid according to claim **1,** wherein, the structure of said PEGylated lipid is selected from the group consisting of the following formulas:

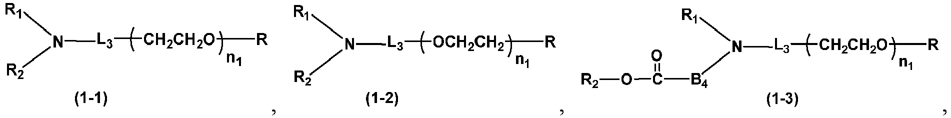

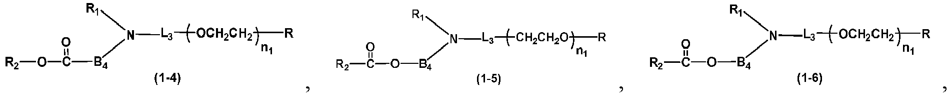

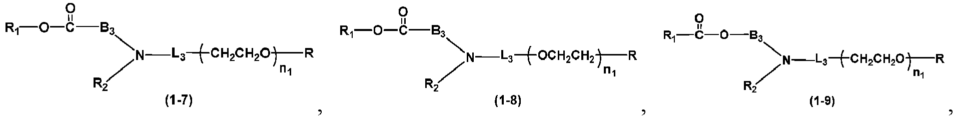

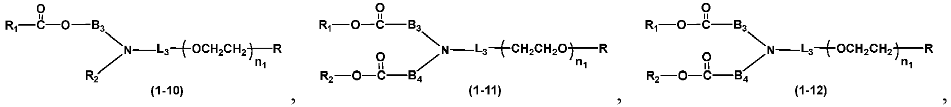

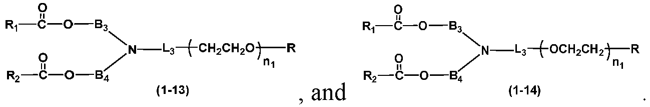

12. The PEGylated lipid according to claim 1, wherein, said R₁ and R₂ are each independently a linear alkyl group, a branched alkyl group, a linear alkenyl group, a branched alkenyl group, a linear alkynyl group, or a branched alkynyl group, preferably a linear alkyl group, more preferably a C₁₋₂₅ linear alkyl group, more preferably a C₁₋₁₇ linear alkyl group, and most preferably selected from the group consisting of a dodecyl group, a tridecyl group, a tetradecyl group, a pentadecyl group, a hexadecyl group, a heptadecyl group, and an octadecyl group.

13. The PEGylated lipid according to claim **12,** wherein, said R₁ and R₂ are each independently a branched alkyl group, a branched alkenyl group, or a branched alkynyl group, and each independently represented as 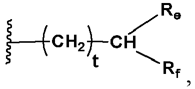 wherein, Rₑ and R_{f} are each independently selected from the group consisting of a C₁₋₁₅ alkyl group, a C₂₋₁₅ alkenyl group, and a C₂₋₁₅ alkynyl group, preferably selected from the group consisting of a methyl group, an ethyl group, a propyl group, a butyl group, a pentyl group, a hexyl group, a heptyl group, an octyl group, a nonyl group, a decyl group, an undecyl group, a dodecyl group, a tridecyl group, a tetradecyl group, a vinyl group, a propenyl group, an allyl group, a butenyl group, an enyl butyl group, a pentenyl group, an enyl pentyl group, a hexenyl group, an enyl hexyl group, a heptenyl group, an enyl heptyl group, an octenyl group, an enyl octyl group, a nonenyl group, an enyl nonyl group, a decenyl group, an enyl decyl group, an ethynyl group, a propynyl group, a propargyl group, a butynyl group, an ynyl butyl group, a pentynyl group, an ynyl pentyl group, a hexynyl group, an ynyl hexyl group, a heptynyl group, an ynyl heptyl group, an octynyl group, an ynyl octyl group, a nonynyl group, an ynyl nonyl group, a decynyl group, and an ynyl decyl group; and Rₑ and R_{f} are each independently more preferably selected from the group consisting of a methyl group, an ethyl group, a propyl group, a butyl group, a pentyl group, a hexyl group, a heptyl group, an octyl group, a nonyl group, and a decyl group.

14. The PEGylated lipid according to claim **13,** wherein, said R₁ and R₂ are each independently selected from the group consisting of the following structures: 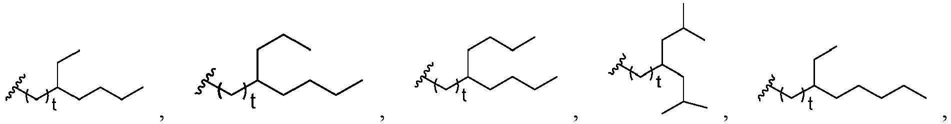 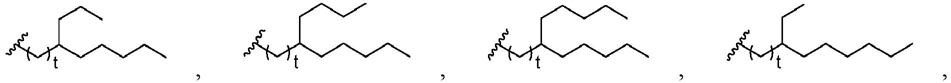 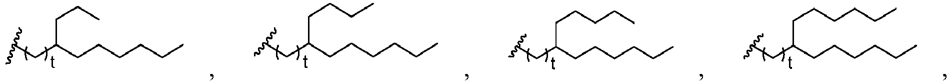 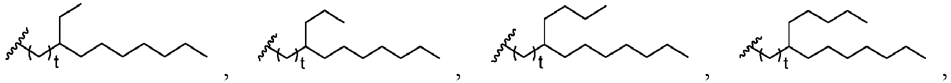 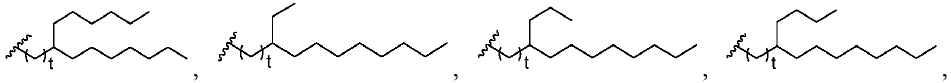 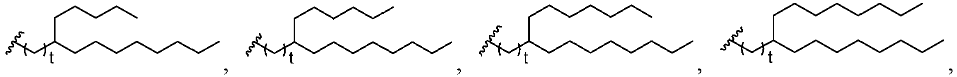 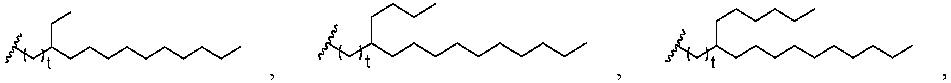 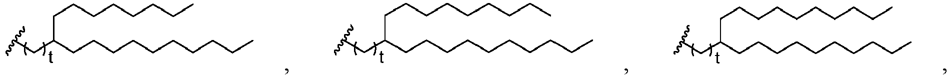 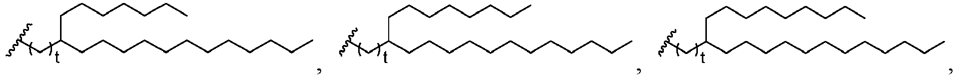 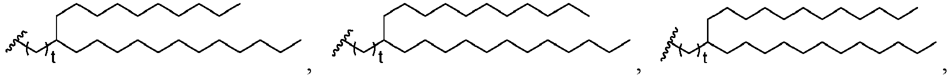 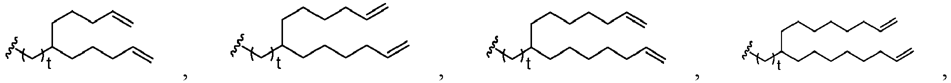 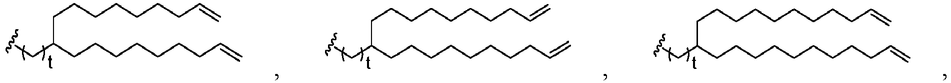 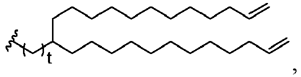 wherein, t is an integer from 0 to 12.

15. The PEGylated lipid according to claim **1,** wherein, said R contains any atom or group selected from the group consisting of a hydrogen atom, an alkyl group, an alkoxy group, an alcoholic hydroxyl group, a protected alcoholic hydroxyl group, a thiol group, a protected thiol group, a carboxyl group, a protected carboxyl group, an amino group, a protected amino group, an aldehyde group, a protected aldehyde group, an ester group, a carbonate group, a urethane group, a succinimidyl group, a maleimide group, a protected maleimide group, a dimethylamino group, an alkenyl group, an alkenoate group, an azido group, an alkynyl group, a folate group, a rhodamine group, a biotinyl group, a monosaccharide group, and a polysaccharide group; and preferably selected from the group consisting of H, -CH₃, -CH₂CH₃, -(CH₂)ₜOH, -(CH₂)ₜSH, -OCH₃, -OCH₂CH₃, -(CH₂)ₜNH₂, -(CH₂)ₜC(=O)OH, -C(=O)(CH₂)ₜC(=O)OH, -C(=O)CH₃, -(CH₂)ₜN₃, -C(=O)CH₂CH₃, -C(=O)OCH₃, -OC(=O)OCH₃, -C(=O)OCH₂CH₃, -OC(=O)OCH₂CH₃, -(CH₂)ₜN(CH₃)₂, -(CH₂)ₜN(CH₂CH₃)₂, -(CH₂)ₜCHO,

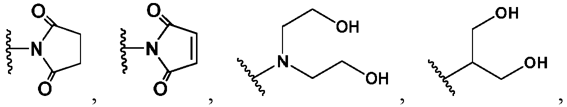

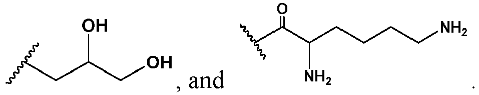

16. The PEGylated lipid according to claim **9,** wherein, said G₁ is a branching group with a valence of three or more, and G₁ is preferably a trivalent or tetravalent branching group; G₁ is preferably a trivalent branching group, more preferably a trivalent branching group of glycerol or amino acid residues.

17. The PEGylated lipid according to claim **1,** wherein, the structure of said PEGylated lipid is selected from the group consisting of the following structures: 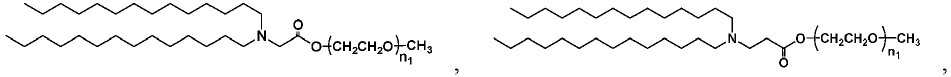 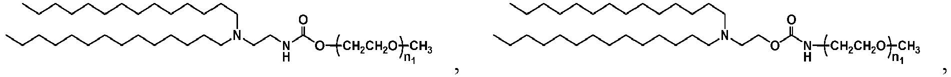 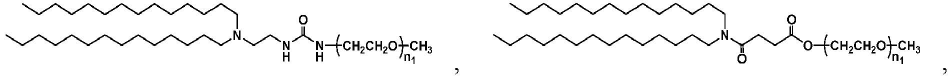 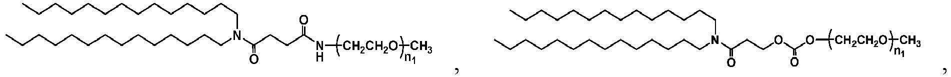 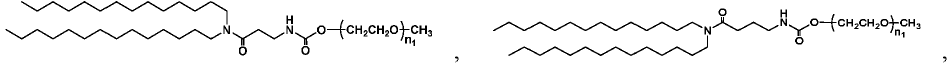 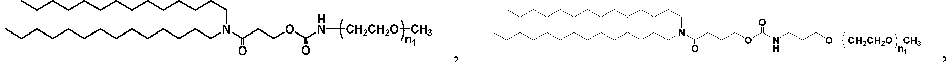 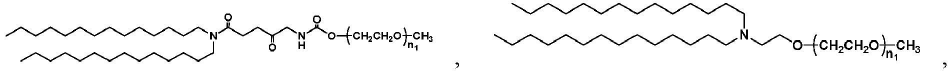 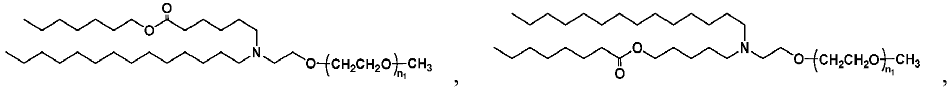 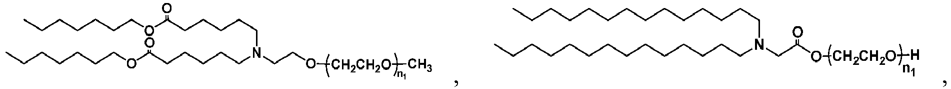 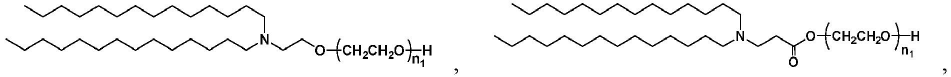 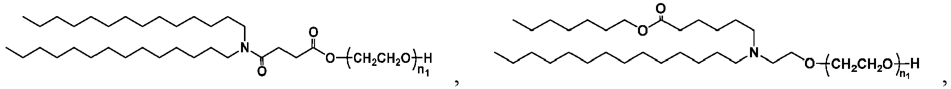 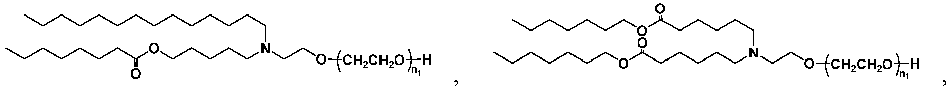 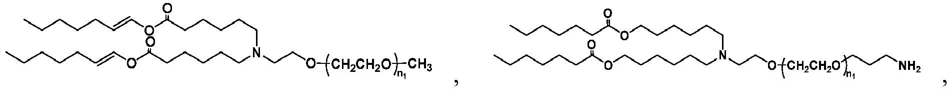 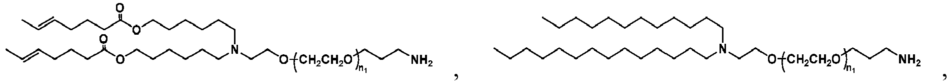 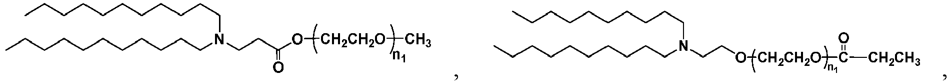 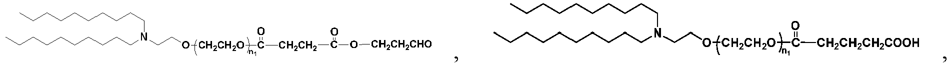 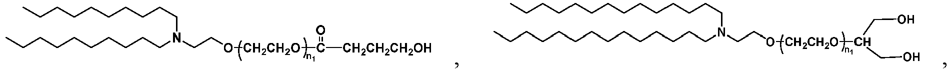 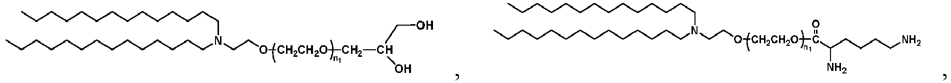 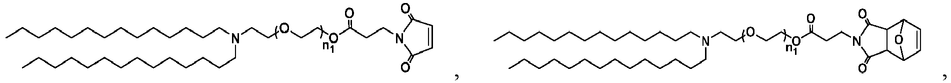 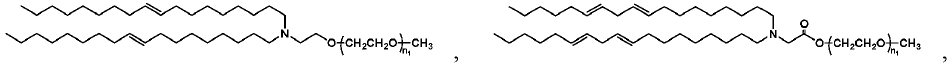 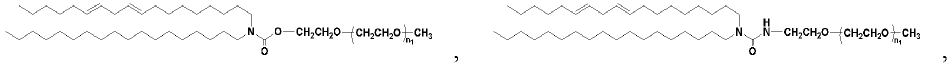 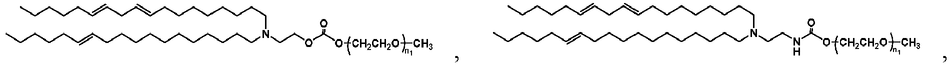 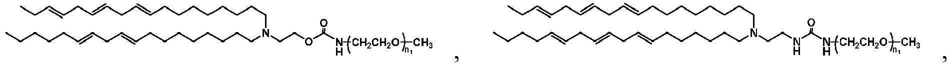 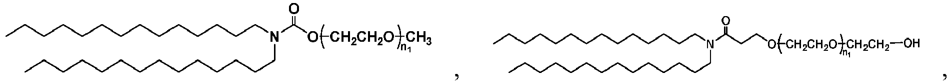 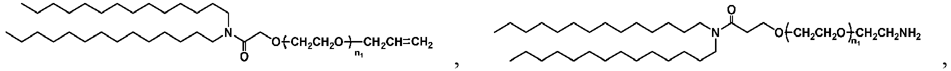 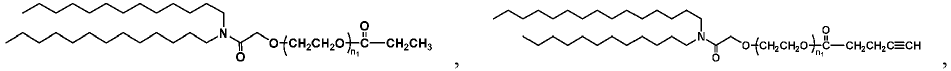 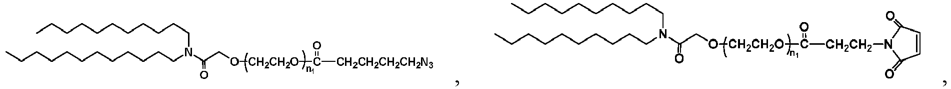 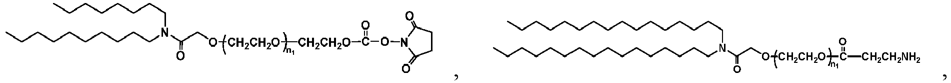 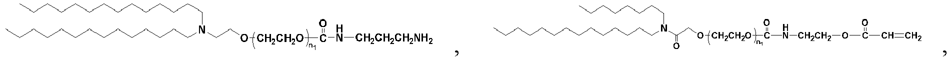 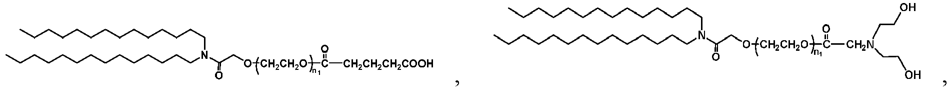 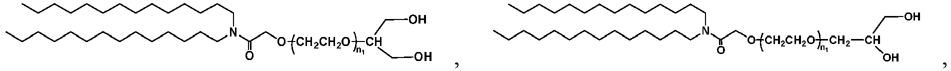 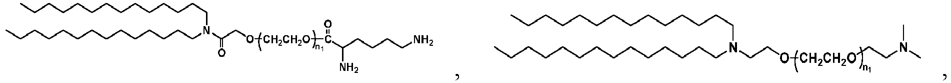 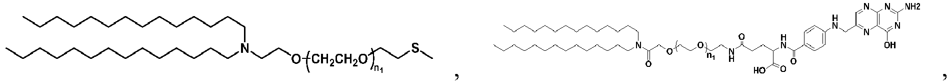 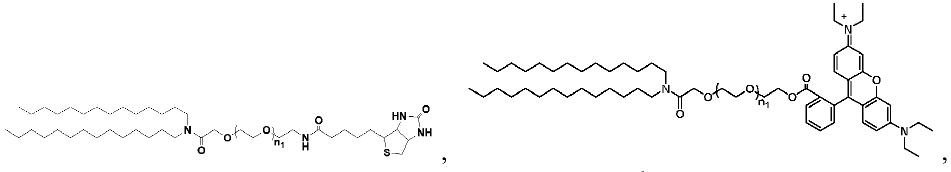 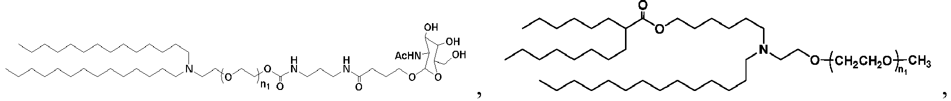 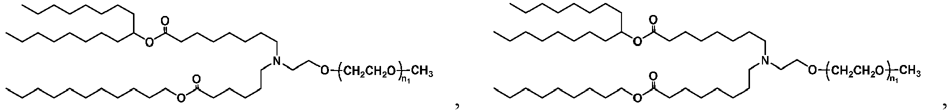 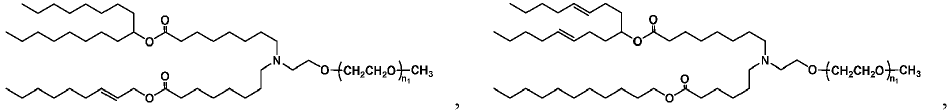 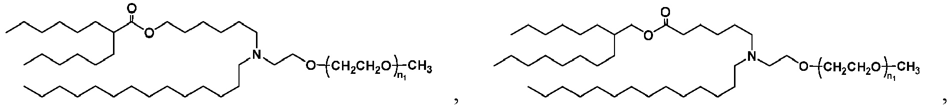 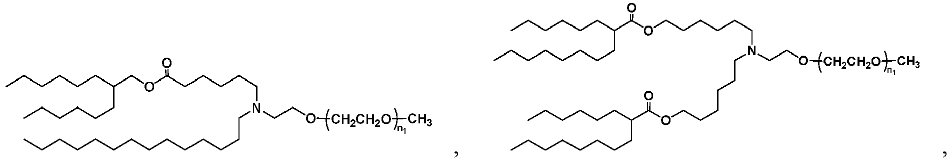 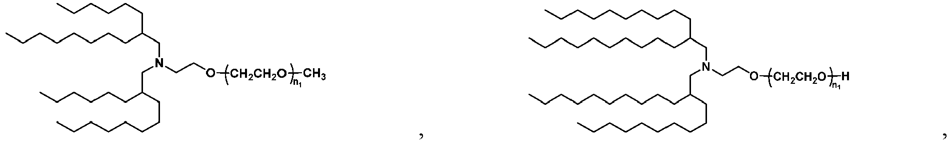 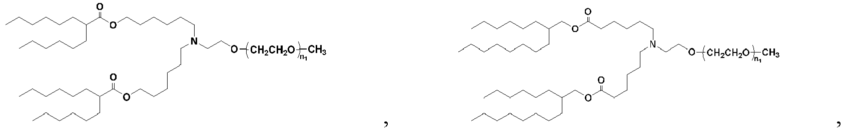 and

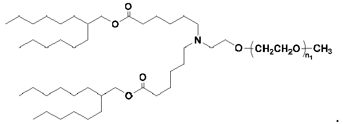

18. A method for preparing a PEGylated lipid according to one of claims **1-17**, wherein, said PEGylated lipid can be prepared by the following steps:
Step 1: Activating the carboxyl terminus of the acid A-1 or A-1' containing an unprotected carboxyl group with carboxyl activating agents to obtain the ester A-2 or A-2' containing an activated carboxyl terminus, wherein B₃' and B₄' are each independently a linking bond or an alkylene group having one less methylene group than B₃ and B₄; R₁' and R₂' are each independently R₁, R₂, or an aliphatic hydrocarbon group having one less methylene group than R₁ and R₂; R₇ is a carboxyl-activating group; when either B₃' or L₇ is not a linking bond, R₁' is R₁; when both B₃' and L₇ are linking bonds, R₁' is an aliphatic hydrocarbon group having one less methylene group than R₁; when any of B₄' and L₈ is not a linking bond, R₂' is R₂; when both B₄' and L₈ are linking bonds, R₂' is an aliphatic hydrocarbon group having one less methylene group than R₂;
Step 2: Carrying out the condensation reaction between the ester A-2 or A-2' containing an activated carboxyl terminus and the primary amine derivative A-3 or A-3' containing a nitrogen-source terminal group to obtain the amide intermediate A-4 or A-4';
Step 3: Using reducing agents to reduce the amide intermediate A-4 or A-4' to the secondary amine intermediate A-5 or A-5';
Step 4: Carrying out the coupling reaction between the secondary amine intermediate and the dual end-functionalized PEG derivative which is the biLPEG molecule A-6, to obtain the PEGylated lipid derivative A-7 or A-7'; wherein, the biLPEG is monodisperse or polydisperse; wherein, the two terminal functional groups of biLPEG can be the same or different; wherein, the R' end of biLPEG contains the functional group R₀₁ or a micro variant form of R₀₁; said micro variant form refers to the group which can be transformed into R₀₁ through any chemical process selected from deprotection, salt complexation and decomplexation, ionization, protonation, deprotonation, and leaving group transformation; wherein, the F₁ contains active functional groups, which can react with the amino group of the secondary amine intermediate A-5 or A-5' to obtain the nitrogen atom as a branching center and the divalent linking group L₃;
when R' is R, the structure of A-7 or A-7' is represented by the general formula (2);
when R' is not R, A-8 or A-8' represented by the general formula (2) is obtained via terminal micro-modification of A-7 or A-7'; said terminal micro-modification is selected from chemical reactions including deprotection, salt complexation and decomplexation, ionization, protonation, deprotonation, and leaving group transformation;
Step 1: 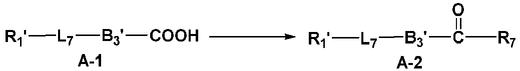 or 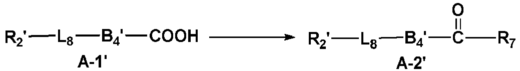
Step 2: 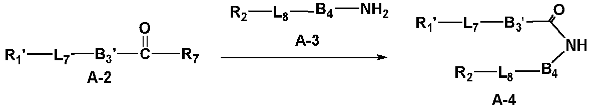 or 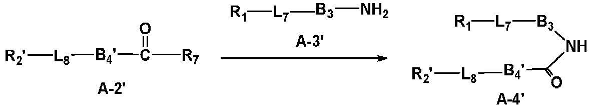
Step 3: 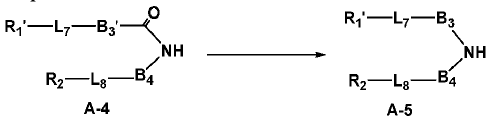 or 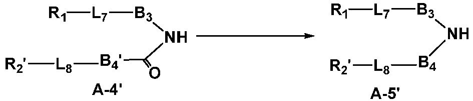
Step 4: 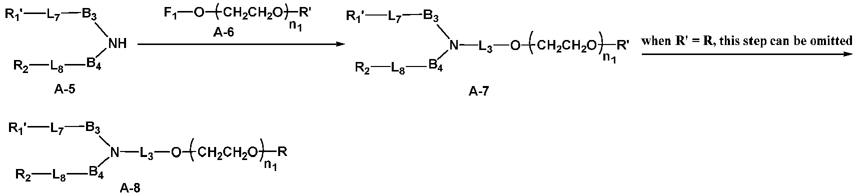 or

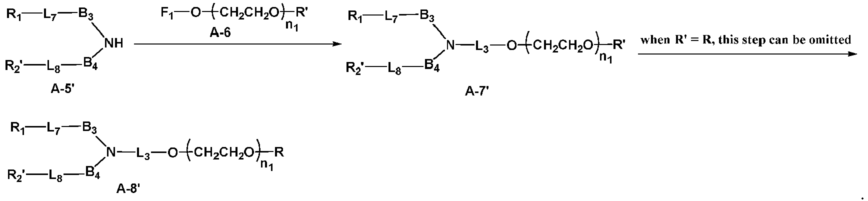

19. The preparation method of PEGylated lipid according to claim **18,** wherein, said A-1 is R₁'-COOH or R₂'-COOH, said A-3 is preferably R₁-NH₂ or R₂-NH₂, said carboxyl activating agent is preferably selected from the group consisting of N-hydroxysuccinimide (NHS), 1-ethyl-3-(3-dimethylaminopropyl)carbodiimide hydrochloride (EDCI), N-hydroxy-5-norbomene-2,3-dicarboximide (HONb), and *N*,*N*'-dicyclohexylcarbodiimide (DCC); said PEGylated lipid is preferably prepared by the following routes:

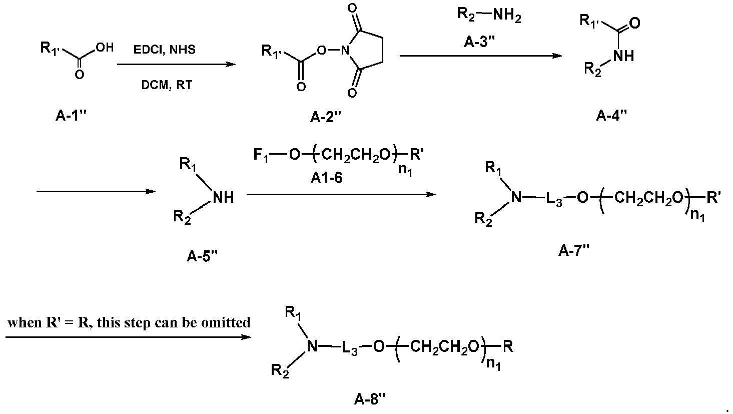

20. A method for preparing a PEGylated lipid according to one of claims **1-17**, wherein, said PEGylated lipid can be prepared by the following steps:
Step 1: Carrying out the coupling reaction between the PEG derivative biLPEG molecule B-1 and the primary amine derivative B-2 or B-2' containing a nitrogen-source terminal group to obtain the secondary amine derivative B-3 or B-3'; wherein, the biLPEG can be monodisperse or polydisperse, the two terminal functional groups of biLPEG can be the same or different, and the R' end of biLPEG contains the functional group R₀₁ or a micro variant form of R₀₁; said micro variant form refers to the group which can be transformed into R₀₁ through any chemical process selected from deprotection, salt complexation and decomplexation, ionization, protonation, deprotonation, and leaving group transformation; wherein, the F₁ contains active functional groups, which can react with the amino group of the primary amine B-2 or B-2' to obtain the secondary amine derivative B-3 or B-3' containing the divalent linking group L₃;
Step 2: Carrying out the alkylation reaction between the secondary amine derivative B-3 or B-3' and the compound B-4 or B-4' with the functional group F_{N} to obtain the PEGylated lipid derivative B-5 or B-5'; said F_{N} is a functional group which can react with amino group or secondary amino group, preferably -OMs, -OTs, -CHO, -F, -Cl, or -Br;
when R' is R, the structure of B-5 or B-5' is represented by the general formula (2);
when R' is not R, B-6 or B-6' represented by the general formula (2) is obtained via terminal micro-modification of B-5 or B-5'; said terminal micro-modification is selected from chemical reactions including deprotection, salt complexation and decomplexation, ionization, protonation, deprotonation, and leaving group transformation;
Step 1: 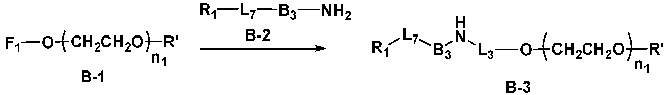 or 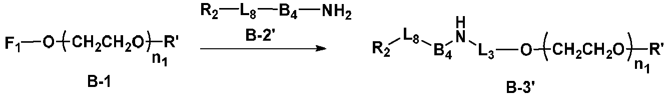
Step 2: 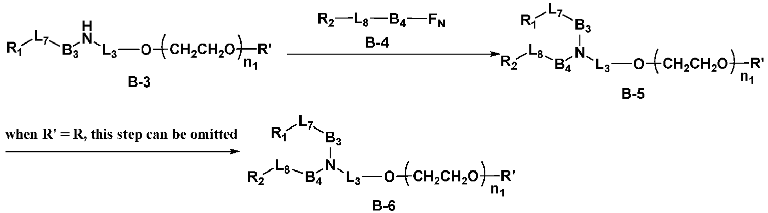 or

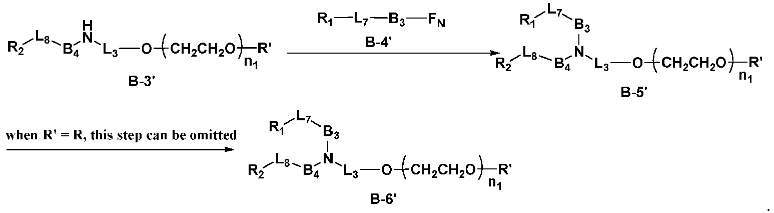

21. The preparation method of PEGylated lipid according to claim **20,** wherein, said B-3 or B-3' is R₁-NH₂ or R₂-NH₂, said B-4 or B-4' is R₂-L₈-B₄-Br or R₁-L₇-B₃-Br, and said F₁ contains the -OMs group.

22. A method for preparing a PEGylated lipid according to one of claims **1-21**, wherein, said PEGylated lipid can be prepared by the following steps:
Step 1: Carrying out the reaction between the small molecule C-1 and the small molecule C-2 to obtain the small molecule intermediate C-3 which contains the divalent linking group L₇, the functional group F_{N} at one end, and an aliphatic hydrocarbon group R₁ at the other end; wherein, F₃ and F₄ are each independently a functional group, which can be transformed into the divalent linking group L₇ via reaction; wherein, C-2 contains a pair of heterofunctional groups of F₃ and F_{N}, said F_{N} is the functional group which can react with amino group or secondary amino group, preferably -OMs, -OTs, -CHO, -F, -Cl, or -Br;
Step 2: Carrying out the alkylation reaction between two molecules of the small molecule intermediate C-3 and the PEGylated primary amine derivative C-4 containing a nitrogen-source terminal group to obtain the PEGylated lipid C-5, wherein, the R' end contains the functional group R₀₁ or a micro variant form of R₀₁; said micro variant form refers to the group which can be transformed into R₀₁ through any chemical process selected from deprotection, salt complexation and decomplexation, ionization, protonation, deprotonation, and leaving group transformation;
when R' is R, the structure of C-5 is represented by the general formula (2);
when R' is not R, the structure of C-6 represented by the general formula (2) is obtained via terminal micro-modification of C-5; said terminal micro-modification is selected from chemical reactions including deprotection, salt complexation and decomplexation, ionization, protonation, deprotonation, and leaving group transformation; wherein, R₁ is the same as R₂, B₃ is the same as B₄, and L₇ is the same as L₈;
Step 1: 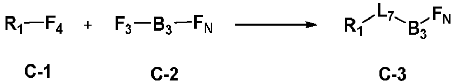
Step 2:

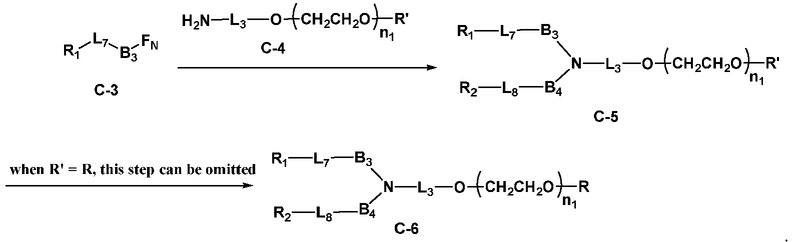

23. A method for preparing a PEGylated lipid according to one of claims **1-17**, wherein, said PEGylated lipid can be prepared by the following steps:
Step 1: Carrying out the reaction between the small molecule D-1 and the small molecule D-2 to obtain the small molecule intermediate D-3 which contains the divalent linking group L₇, a hydroxyl group at one end, and an aliphatic hydrocarbon group R₁ at the other end; wherein, F₃ and F₄ are each independently a functional group, which can be transformed into the divalent linking group L₇ via reaction; wherein, D-2 contains a pair of heterofunctional groups of F₃ and a hydroxyl group;
Step 2: Carrying out the reaction in which the hydroxyl group of the small molecule intermediate D-3 is oxidized to an aldehyde group to obtain the small molecule intermediate D-4 containing an aldehyde group, wherein B₃' is an alkylene group having one less methylene group than B₃;
Step 3: Carrying out the addition reaction between two molecules of the small molecule intermediate D-4 containing an aldehyde group and the PEGylated primary amine derivative D-5 containing a nitrogen-source terminal group to obtain the PEGylated lipid D-6, wherein, the R' end contains the functional group R₀₁ or a micro variant form of R₀₁; said micro variant form refers to the group which can be transformed into R₀₁ through any chemical process selected from deprotection, salt complexation and decomplexation, ionization, protonation, deprotonation, and leaving group transformation;
when R' is R, the structure of D-6 is represented by the general formula (2);
when R' is not R, D-6 represented by the general formula (2) is obtained via terminal micro-modification of D-7; said terminal micro-modification is selected from chemical reactions including deprotection, salt complexation and decomplexation, ionization, protonation, deprotonation, and leaving group transformation; wherein, R₁ is the same as R₂, B₃ is the same as B₄, and L₇ is the same as L₈;
Step 1: 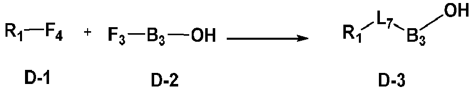
Step 2: 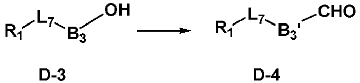
Step 3:

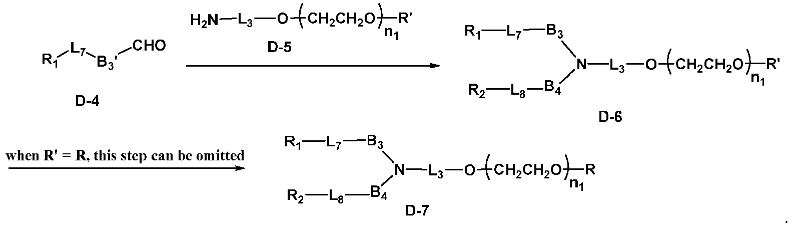

24. A cationic liposome, wherein, the cationic liposome contains any foregoing PEGylated lipid according to one of claims **1-17.**

25. The cationic liposome according to claim **24,** wherein, said cationic liposome also contains one or more types of lipids selected from the group consisting of neutral lipid, steroid lipid, and cationic lipid; said neutral lipid is preferably phospholipid.

26. The cationic liposome according to claim **25,** wherein, said neutral lipid is selected from the group consisting of 1,2-dilinoleoyl-sn-glycero-3-phosphocholines (DLPC), 1,2-dimyristoleoyl-sn-glycero-3-phosphocholines (DMPC), 1,2-dioleoyl-sn-glycero-3-phosphocholines (DOPC), 1,2-dipalmitoyl-sn-glycero-3-phosphocholines (DPPC), 1,2-distearoyl-sn-glycero-3-phosphatidylcholines (DSPC), 1,2-diundecanoyl-sn-glycero-3 -phosphatidylcholines (DUPC), 1-plamitoyl-2-oleoyl-sn-glycero-3-phosphocholines (POPC), 1,2-di-*O*-octadecenyl-sn-glycero-3-phosphatidylcholines (18:0 Diether PC), 1-oleoyl-2-cholesterylhemisuccinyl-sn-glycero-3-phosphocholines (OChemsPC), 1-*O*-hexadecyl-sn-glycero-3-phosphatidylcholines (C16 Lyso PC), 1,2-dilinolenoyl-sn-glycero-3-phosphatidylcholines, 1,2-diarachidonoyl-sn-glycero-3-phosphatidylcholines, 1,2-didecosahexaenoyl-sn-glycero-3-phosphocholines, 1,2-dioleoyl-sn-glycero-3-phosphoethanolamines (DOPE), 1,2-diphytanyl-sn-glycero-3-phosphoethanolamines (ME 16.0 PE), 1,2-distearoyl-sn-glycero-3-phosphoethanolamines, 1,2-dilinoleoyl-sn-glycero-3-phosphoethanolamines, 1,2-dilinolenoyl-sn-glycero-3-phosphoethanolamines, 1,2-diarachidonoyl-sn-glycero-3-phosphoethanolamines, 1,2-didecosahexaenoyl-sn-glycero-3 -phosphoethanolamines, 1,2-dioleoyl-sn-glycero-3-phospho-rac-(1-glycerol) sodium salts (DOPG), dioleoyl phosphatidylserines (DOPS), dipalmitoylphosphatidylglycerols (DPPG), palmitoyloleoyl phosphatidylethanolamines (POPE), distearoyl phosphatidylethanolamines (DSPE), dipalmitoyl phosphatidylethanolamines (DPPE), dimyristoleoyl phosphoethanolamines (DMPE), 1-stearoyl-2-oleoyl-stearoylethanolamines (SOPE), 1-stearoyl-2-oleoyl-phosphatidylcholines (SOPC), sphingomyelins, phosphatidylcholines, phosphatidylethnolamines, phosphatidylserines, phosphatidylinositols, phosphatidic acids, palmitoyloleoyl phosphatidylcholines, lysophosphatidylcholines, lysophosphatidylethanolamines (LPE), and combinations thereof.

27. The cationic liposome according to claim **25,** wherein, said steroid lipid is selected from the group consisting of cholesterols, coprostanols, sitosterols, ergosterols, campesterols, stigmasterols, brassicasterol tomatidines, ursolic acids, α-tocopherols, and any mixture thereof.

28. The cationic liposome according to claim **25,** wherein, the structure of said cationic lipid is represented by the general formula (1): 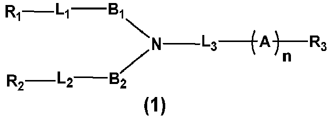
wherein, N is the nitrogen branching center;
wherein, L₁ and L₂ are divalent linking groups, each independently selected from the group consisting of -OC(=O)-, -C(=O)O-, -OC(=O)O-, -C(=O)-, -O-, -S-, -C(=O)S-, -SC(=O)-, -NR_{c}C(=O)-, -C(=O)NR_{c}-, -NR_{c}C(=O)NR_{c}-, -OC(=O)NR_{c}-, -NR_{c}C(=O)O-, -SC(=O)NR_{c}-, and -NR_{c}C(=O)S-; wherein, said R_{c} is a hydrogen atom or a C₁₋₁₂ alkyl group;
wherein, L₃ is a linking bond or -L₄-Z-L₅-; said L₄ and L₅ are carbon chain linking groups, each independently represented as -(CRₐR_{b})ₜ- or -(CRₐR_{b})ₜ-(CRₐR_{b})ₚ-(CRₐR_{b})_{q}-, wherein, t, p and q are each independently an integer from 0 to 12, Rₐ and R_{b} are each independently a hydrogen atom or a C₁₋₁₂ alkyl group; said Z is a linking bond or a divalent linking group selected from the group consisting of -OC(=O)-, -C(=O)O-, -OC(=O)O-, -C(=O)-, -O-, -S-, -C(=O)S-, -SC(=O)-, -NR_{c}C(=O)-, -C(=O)NR_{c}-, -NR_{c}C(=O)NR_{c}-, -OC(=O)NR_{c}-, -NR_{c}C(=O)O-, -SC(=O)NR_{c}-, and -NR_{c}C(=O)S-;
wherein, B₁ and B₂ are each independently a C₁₋₁₂ alkylene group;
wherein, R₁ and R₂ are each independently a C₂₋₃₀ aliphatic hydrocarbon group;
wherein, R₃ is hydrogen atom, alkyl group, alkoxy group, -C(=O)R_{d}, -C(=O)OR_{d}, -OC(=O)R_{d}, -OC(=O)OR_{d}, or 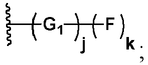 wherein, R_{d} is a C₁₋₁₂ alkyl group, G₁ is a terminal branching group with the valence of k+1, j is 0 or 1, and F contains functional groups, when j is 0, G₁ is absent, when j is 1, G₁ protrudes F with the number of k, and k is an integer from 2 to 8;
wherein, A is selected from the group consisting of -(CRₐR_{b})ₛO-, -O(CRₐR_{b})ₛ-, -(CRₐR_{b})ₛS-, -S(CRₐR_{b})ₛ-, -(CRₐR_{b})ₛO(CRₐR_{b})ₛS-, -(CRₐR_{b})ₛS(CRₐR_{b})ₛO-, -(CRₐR_{b})ₛNR_{c}(CRₐR_{b})ₛS-, -(CRₐR_{b})ₛS(CRₐR_{b})ₛNR_{c}-, -(CRₐR_{b})ₛNR_{c}(CRₐR_{b})ₛO-, and -(CRₐR_{b})ₛO(CRₐR_{b})ₛNR_{c}-, wherein, s is 2, 3, or 4, Rₐ and R_{b} are each independently a hydrogen atom or a C₁₋₁₂ alkyl group;
wherein, when A is -(CRₐR_{b})ₛO- or -O(CRₐR_{b})ₛ-, n is an integer from 2 to 6; when A is not -(CRₐR_{b})ₛO- or -O(CRₐR_{b})ₛ-, n is an integer from 1 to 6;
wherein, said alkyl group, alkylene group, alkoxy group, and aliphatic hydrocarbon group are each independently substituted or unsubstituted.

29. The cationic liposome according to claim **28,** wherein, the structure of said cationic lipid is selected from the following structures:

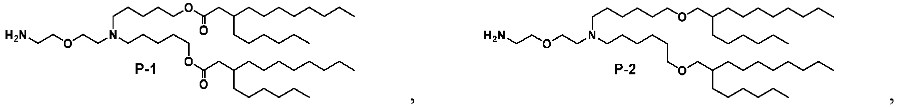

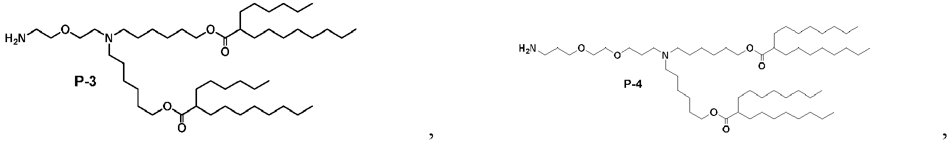

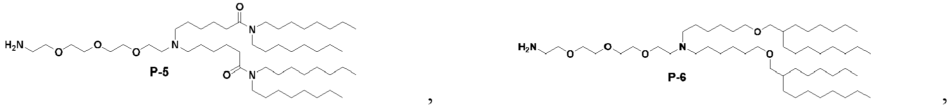

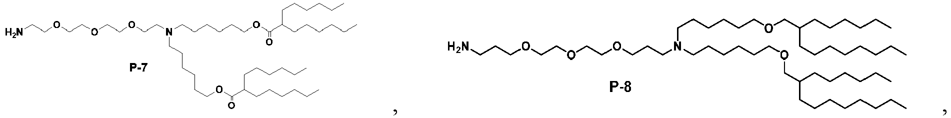

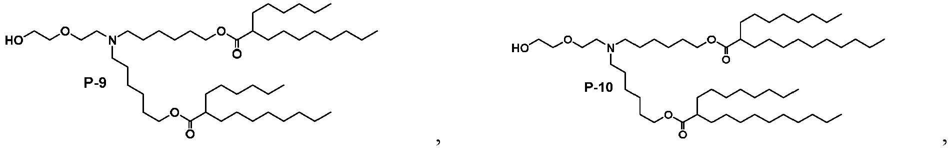

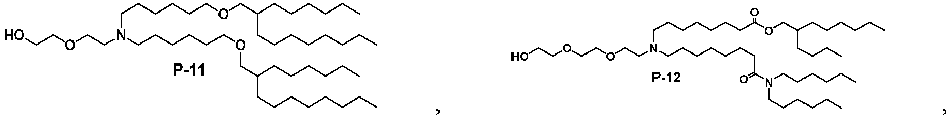

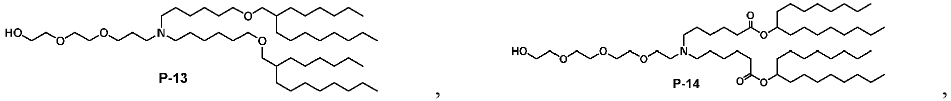

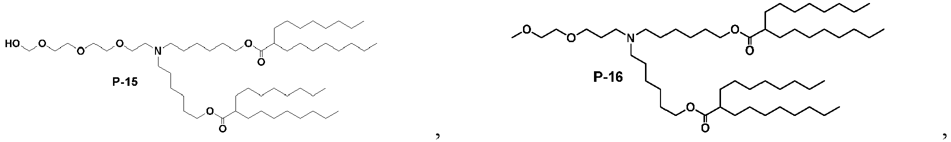

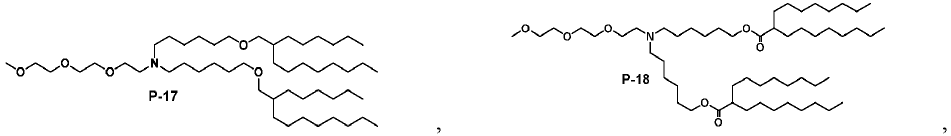

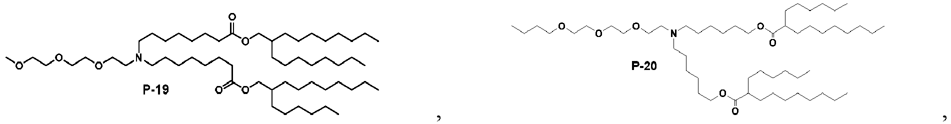

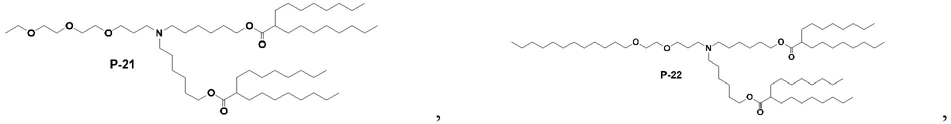

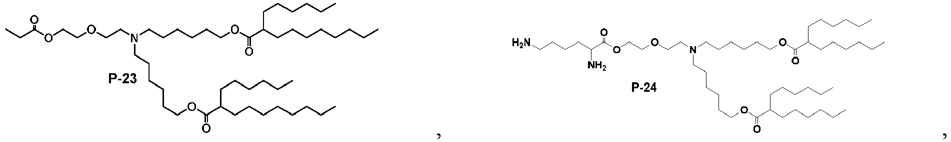

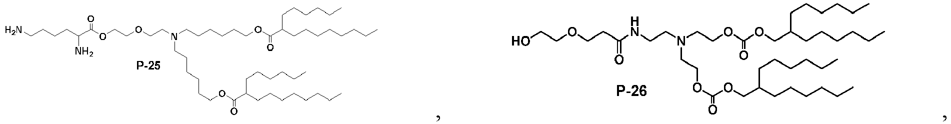

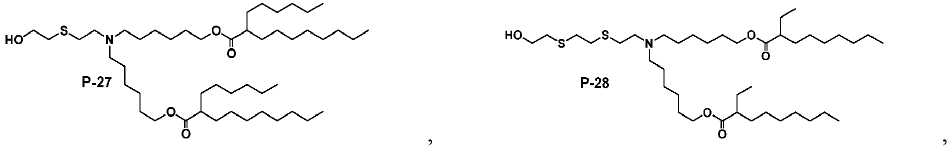

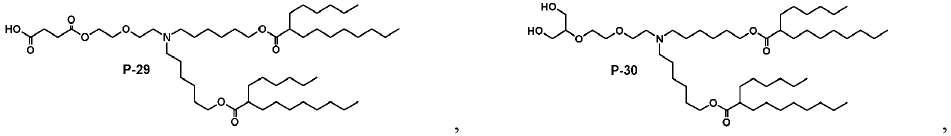

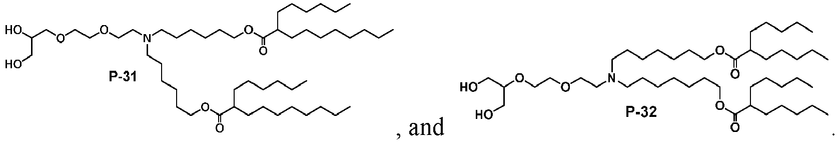

30. The cationic liposome according to claim **25,** wherein, said cationic lipids in the cationic liposomes can be selected from the group consisting of *N*,*N*-dioleyl-*N*,*N*-dimethylammonium chloride (DODAC), *N*,*N-*distearyl-*N*,*N*-dimethylammonium bromide (DDAB), 1,2-dioleoyl-3-trimethylammonium-propane (DOTAP), *N*-[1-(2,3-dioleoyloxy)propyl]-*N*,*N*,*N*-trimethylammonium chloride (DOTMA) 1,2-dioleyloxy-*N*,*N*-dimethylaminopropane (DODMA), 3-(didodecylamino)-*N1*,*N1*,4-tridodecyl-1-piperazineethanamine (KL10), *N1*-[2-(didodecylamino)ethyl]-*N1*,*N4*,*N4*-tridodecyl-1,4-piperazinediethanamine (KL22), 14,25-ditridecyl-15, 18,21,24-tetraaza-octatriacontane (KL25), 1,2-dilinoleyloxy-*N*,*N*-dimethylaminopropane (DLin-DMA), 2,2-dilinoleyl-4-dimethylaminomethyl-[1,3]-dioxolane (DLin-K-DMA), heptatriaconta-6,9,28,31-tetraen-19-yl 4-(dimethylamino)butanoate (DLin-MC3-DMA), 2,2-dilinoleyl-4-(2-dimethylaminoethyl)-[1,3]-dioxolane (DLin-KC2-DMA), and mixtures thereof.

31. The cationic liposome according to one of claims **25-30,** wherein, said cationic liposome contains 20% to 80% cationic lipids, 5% to 15% neutral lipids, 25% to 55% steroid lipids, and 0.5% to 10% PEGylated lipids represented by the general formula (2), said percentage is the molar percentage of each lipid relative to the total lipids in a solution containing the solvent.

32. The cationic liposome according to one of claims **25-30,** wherein, the molar percentage of said cationic lipids relative to the total lipids in a solution containing the solvent is 30% to 65%; more preferably 35%, 40%, 45%, 46%, 47%, 48%, 49%, 50%, or 55%.

33. The cationic liposome according to one of claims **25-30,** wherein, the molar percentage of said neutral lipids relative to the total lipids in a solution containing the solvent is 7.5% to 13%, more preferably 8%, 9%, 10%, 11%, or 12%.

34. The cationic liposome according to one of claims **25-30,** wherein, the molar percentage of said steroid lipids relative to the total lipids in a solution containing the solvent is 35% to 50%, more preferably 40%, 41%, 42%, 43%, 44%, 45%, 46%, 47%, 48%, 49%, or 50%.

35. The cationic liposome according to one of claims **25-30,** wherein, the molar percentage of said PEGylated lipids relative to the total lipids in a solution containing the solvent is 0.5% to 5%, preferably 1% to 3%, more preferably 1.5%, 1.6%, 1.7%, 1.8%, or 1.9%.

36. A cationic liposome pharmaceutical composition, wherein, the cationic liposome pharmaceutical composition contains the cationic liposome according to one of claims **25-30** and drugs.

37. The cationic liposome pharmaceutical composition according to claim **36,** wherein, said drug is a nucleic acid drug or an anti-tumor agent, said nucleic acid drug is selected from the group consisting of DNA, antisense nucleic acid, plasmid, mRNA, interfering nucleic acid, aptamer, antagomir, miRNA, ribozyme, and siRNA, and preferably selected from the group consisting of DNA, mRNA, miRNA, and siRNA.

38. The cationic liposome pharmaceutical composition according to claim **36,** wherein, said cationic liposome pharmaceutical composition is used as a drug, selected from the group consisting of drugs for treating cancer, drugs for treating malignant tumor, anti-infectious agents, antiviral agents, antifungal agents, and vaccines.

39. A formulation of cationic liposome pharmaceutical composition, wherein, the said formulation of cationic liposome pharmaceutical composition contains the cationic liposome pharmaceutical composition according to one of claims **37-38** and pharmaceutically acceptable diluents or excipients, said diluent or excipient is preferably deionized water, ultrapure water, phosphate buffer, or physiological saline, more preferably phosphate buffer or physiological saline, and most preferably physiological saline.
